# EUROPEAN PATENT APPLICATION

(11) **EP 1 854 880 A1**
(43) Date of publication of application: **14.11.2007**
(21) Application number: 07011519.1
(22) Date of filing: 15.03.2000
(51) Int. Cl.: C12N 15/00, C07K 14/705, C12N 5/10, A01K 67/027, A61K 38/17, A61K 48/00, A61K 38/45, A61K 31/00, A61K 31/70, G01N 33/68, C12Q 1/68, C12N 15/11, C12N 15/12

(54) **Methods and reagents for modulating cholesterol levels**

(30) Priority: 15.03.1999 US 124702 P; 08.06.1999 US 138048 P; 17.06.1999 US 139600 P; 01.09.1999 US 151977 P
(62) Divisional of application: 00917240.4
(71) Applicant: UNIVERSITY OF BRITISH COLUMBIA, Vancouver, British Columbia V6T 1Z4 (CA); XENON PHARMACEUTICALS INC., Burnaby, British Columbia V5G 4W8 (CA)
(72) Inventor: Hayden, Michael R., Vancouver British Columbia V6R 1W9 (CA); Brooks-Wilson, Angela R., Richmond British Columbia V7C 4B2 (CA); Pimstone, Simon N., Vancouver British Columbia V6T 1C5 (CA)
(74) Representative: Zwicker, Jörk

(57) **Abstract**

The invention features ABC1 nucleic acids and polypeptides for the diagnosis and treatment of abnormal cholesterol regulation. The invention also features methods for identifying compounds for modulating cholesterol levels in an animal (e.g., a human).

SEQUENCE LISTING

<110> University of British Columbia
      Xenon Pharmaceuticals Inc.

<120> METHODS AND REAGENTS FOR MODULATING CHOLESTEROL LEVELS

<130> 171-73 T1

<140> EP
<141> 2000-03-15

<150> 60/124,702
<151> 1999-03-15

<150> 60/138,048
<151> 1999-06-08

<150> 60/139,600
<151> 1999-06-17

<150> 60/151,977
<151> 1999-09-01

<160> 287

<170> FastSEQ for Windows Version 4.0

<210> 1
<211> 2261
<212> PRT
<213> Homo sapiens

<400> 1
Met Ala Cys Trp Pro Gln Leu Arg Leu Leu Leu Trp Lys Asn Leu Thr
 1               5                  10                  15
Phe Arg Arg Arg Gln Thr Cys Gln Leu Leu Leu Glu Val Ala Trp Pro
            20                  25                  30
Leu Phe Ile Phe Leu Ile Leu Ile Ser Val Arg Leu Ser Tyr Pro Pro
        35                  40                  45
Tyr Glu Gln His Glu Cys His Phe Pro Asn Lys Ala Met Pro Ser Ala
    50                  55                  60
Gly Thr Leu Pro Trp Val Gln Gly Ile Ile Cys Asn Ala Asn Asn Pro
65                  70                  75                  80
Cys Phe Arg Tyr Pro Thr Pro Gly Glu Ala Pro Gly Val Val Gly Asn
                85                  90                  95
Phe Asn Lys Ser Ile Val Ala Arg Leu Phe Ser Asp Ala Arg Arg Leu
            100                 105                 110
Leu Leu Tyr Ser Gln Lys Asp Thr Ser Met Lys Asp Met Arg Lys Val
        115                 120                 125
Leu Arg Thr Leu Gln Gln Ile Lys Lys Ser Ser Ser Asn Leu Lys Leu
    130                 135                 140
Gln Asp Phe Leu Val Asp Asn Glu Thr Phe Ser Gly Phe Leu Tyr His
145                 150                 155                 160
Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190
Ser Leu Cys Asn Gly Ser Lys Ser Glu Glu Met Ile Gln Leu Gly Asp
        195                 200                 205
Gln Glu Val Ser Glu Leu Cys Gly Leu Pro Arg Glu Lys Leu Ala Ala
    210                 215                 220
Ala Glu Arg Val Leu Arg Ser Asn Met Asp Ile Leu Lys Pro Ile Leu
225                 230                 235                 240
Arg Thr Leu Asn Ser Thr Ser Pro Phe Pro Ser Lys Glu Leu Ala Glu
                245                 250                 255
Ala Thr Lys Thr Leu Leu His Ser Leu Gly Thr Leu Ala Gln Glu Leu
            260                 265                 270
Phe Ser Met Arg Ser Trp Ser Asp Met Arg Gln Glu Val Met Phe Leu
        275                 280                 285
Thr Asn Val Asn Ser Ser Ser Ser Ser Thr Gln Ile Tyr Gln Ala Val
    290                 295                 300
Ser Arg Ile Val Cys Gly His Pro Glu Gly Gly Gly Leu Lys Ile Lys
305                 310                 315                 320
Ser Leu Asn Trp Tyr Glu Asp Asn Asn Tyr Lys Ala Leu Phe Gly Gly
                325                 330                 335
Asn Gly Thr Glu Glu Asp Ala Glu Thr Phe Tyr Asp Asn Ser Thr Thr
            340                 345                 350
Pro Tyr Cys Asn Asp Leu Met Lys Asn Leu Glu Ser Ser Pro Leu Ser
        355                 360                 365
Arg Ile Ile Trp Lys Ala Leu Lys Pro Leu Leu Val Gly Lys Ile Leu
    370                 375                 380
Tyr Thr Pro Asp Thr Pro Ala Thr Arg Gln Val Met Ala Glu Val Asn
385                 390                 395                 400
Lys Thr Phe Gln Glu Leu Ala Val Phe His Asp Leu Glu Gly Met Trp
                405                 410                 415
Glu Glu Leu Ser Pro Lys Ile Trp Thr Phe Met Glu Asn Ser Gln Glu
            420                 425                 430
Met Asp Leu Val Arg Met Leu Leu Asp Ser Arg Asp Asn Asp His Phe
        435                 440                 445
Trp Glu Gln Gln Leu Asp Gly Leu Asp Trp Thr Ala Gln Asp Ile Val
    450                 455                 460
Ala Phe Leu Ala Lys His Pro Glu Asp Val Gln Ser Ser Asn Gly Ser
465                 470                 475                 480
Val Tyr Thr Trp Arg Glu Ala Phe Asn Glu Thr Asn Gln Ala Ile Arg
                485                 490                 495
Thr Ile Ser Arg Phe Met Glu Cys Val Asn Leu Asn Lys Leu Glu Pro
            500                 505                 510
Ile Ala Thr Glu Val Trp Leu Ile Asn Lys Ser Met Glu Leu Leu Asp
        515                 520                 525
Glu Arg Lys Phe Trp Ala Gly Ile Val Phe Thr Gly Ile Thr Pro Gly
    530                 535                 540
Ser Ile Glu Leu Pro His His Val Lys Tyr Lys Ile Arg Met Asp Ile
545                 550                 555                 560
Asp Asn Val Glu Arg Thr Asn Lys Ile Lys Asp Gly Tyr Trp Asp Pro
                565                 570                 575
Gly Pro Arg Ala Asp Pro Phe Glu Asp Met Arg Tyr Val Trp Gly Gly
            580                 585                 590
Phe Ala Tyr Leu Gln Asp Val Val Glu Gln Ala Ile Ile Arg Val Leu
        595                 600                 605
Thr Gly Thr Glu Lys Lys Thr Gly Val Tyr Met Gln Gln Met Pro Tyr

610                 615                 620
Pro Cys Tyr Val Asp Asp Ile Phe Leu Arg Val Met Ser Arg Ser Met
625                 630                 635                 640
Pro Leu Phe Met Thr Leu Ala Trp Ile Tyr Ser Val Ala Val Ile Ile
                645                 650                 655
Lys Gly Ile Val Tyr Glu Lys Glu Ala Arg Leu Lys Glu Thr Met Arg
            660                 665                 670
Ile Met Gly Leu Asp Asn Ser Ile Leu Trp Phe Ser Trp Phe Ile Ser
        675                 680                 685
Ser Leu Ile Pro Leu Leu Val Ser Ala Gly Leu Leu Val Val Ile Leu
    690                 695                 700
Lys Leu Gly Asn Leu Leu Pro Tyr Ser Asp Pro Ser Val Val Phe Val
705                 710                 715                 720
Phe Leu Ser Val Phe Ala Val Val Thr Ile Leu Gln Cys Phe Leu Ile
                725                 730                 735
Ser Thr Leu Phe Ser Arg Ala Asn Leu Ala Ala Ala Cys Gly Gly Ile
            740                 745                 750
Ile Tyr Phe Thr Leu Tyr Leu Pro Tyr Val Leu Cys Val Ala [?]p Gln
        755                 760                 765
Asp Tyr Val Gly Phe Thr Leu Lys Ile Phe Ala Ser Leu Leu Ser Pro
    770                 775                 780
Val Ala Phe Gly Phe Gly Cys Glu Tyr Phe Ala Leu Phe Glu Glu Gln
785                 790                 795                 800
Gly Ile Gly Val Gln Trp Asp Asn Leu Phe Glu Ser Pro Val Glu Glu
                805                 810                 815
Asp Gly Phe Asn Leu Thr Thr Ser Val Ser Met Met Leu Phe Asp Thr
            820                 825                 830
Phe Leu Tyr Gly Val Met Thr Trp Tyr Ile Glu Ala Val Phe Pro Gly
        835                 840                 845
Gln Tyr Gly Ile Pro Arg Pro Trp Tyr Phe Pro Cys Thr Lys Ser Tyr
    850                 855                 860
Trp Phe Gly Glu Glu Ser Asp Glu Lys Ser His Pro Gly Ser Asn Gln
865                 870                 875                 880
Lys Arg Ile Ser Glu Ile Cys Met Glu Glu Glu Pro Thr His Leu Lys
                885                 890                 895
Leu Gly Val Ser Ile Gln Asn Leu Val Lys Val Tyr Arg Asp Gly Met
            900                 905                 910
Lys Val Ala Val Asp Gly Leu Ala Leu Asn Phe Tyr Glu Gly Gln Ile
        915                 920                 925
Thr Ser Phe Leu Gly His Asn Gly Ala Gly Lys Thr Thr Thr Met Ser
    930                 935                 940
Ile Leu Thr Gly Leu Phe Pro Pro Thr Ser Gly Thr Ala Tyr Ile Leu
945                 950                 955                 960
Gly Lys Asp Ile Arg Ser Glu Met Ser Thr Ile Arg Gln Asn Leu Gly
                965                 970                 975
Val Cys Pro Gln His Asn Val Leu Phe Asp Met Leu Thr Val Glu Glu
            980                 985                 990
His Ile Trp Phe Tyr Ala Arg Leu Lys Gly Leu Ser Glu Lys His Val
        995                 1000                1005
Lys Ala Glu Met Glu Gln Met Ala Leu Asp Val Gly Leu Pro Ser Ser
    1010                1015                1020
Lys Leu Lys Ser Lys Thr Ser Gln Leu Ser Gly Gly Met Gln Arg Lys
1025                1030                1035                1040
Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070
Glu Leu Leu Leu Lys Tyr Arg Gln Gly Arg Thr Ile Ile Leu Ser Thr
        1075                1080                1085
His His Met Asp Glu Ala Asp Val Leu Gly Asp Arg Ile Ala Ile Ile
    1090                1095                1100
Ser His Gly Lys Leu Cys Cys Val Gly Ser Ser Leu Phe Leu Lys Asn
1105                1110                1115                1120
Gln Leu Gly Thr Gly Tyr Tyr Leu Thr Leu Val Lys Lys Asp Val Glu
                1125                1130                1135
Ser Ser Leu Ser Ser Cys Arg Asn Ser Ser Ser Thr Val Ser Tyr Leu
            1140                1145                1150
Lys Lys Glu Asp Ser Val Ser Gln Ser Ser Ser Asp Ala Gly Leu Gly
        1155                1160                1165
Ser Asp His Glu Ser Asp Thr Leu Thr Ile Asp Val Ser Ala Ile Ser
    1170                1175                1180
Asn Leu Ile Arg Lys His Val Ser Glu Ala Arg Leu Val Glu Asp Ile
1185                1190                1195                1200
Gly His Glu Leu Thr Tyr Val Leu Pro Tyr Glu Ala Ala Lys Glu Gly
                1205                1210                1215
Ala Phe Val Glu Leu Phe His Glu Ile Asp Asp Arg Leu Ser Asp Leu
            1220                1225                1230
Gly Ile Ser Ser Tyr Gly Ile Ser Glu Thr Thr Leu Glu Glu Ile Phe
        1235                1240                1245
Leu Lys Val Ala Glu Glu Ser Gly Val Asp Ala Glu Thr Ser Asp Gly
    1250                1255                1260
Thr Leu Pro Ala Arg Arg Asn Arg Arg Ala Phe Gly Asp Lys Gln Ser
1265                1270                1275                1280
Cys Leu Arg Pro Phe Thr Glu Asp Asp Ala Ala Asp Pro Asn Asp Ser
                1285                1290                1295
Asp Ile Asp Pro Glu Ser Arg Glu Thr Asp Leu Leu Ser Gly Met Asp
            1300                1305                1310
Gly Lys Gly Ser Tyr Gln Val Lys Gly Trp Lys Leu Thr Gln Gln Gln
        1315                1320                1325
Phe Val Ala Leu Leu Trp Lys Arg Leu Leu Ile Ala Arg Arg Ser Arg
    1330                1335                1340
Lys Gly Phe Phe Ala Gln Ile Val Leu Pro Ala Val Phe Val Cys Ile
1345                1350                1355                1360
Ala Leu Val Phe Ser Leu Ile Val Pro Pro Phe Gly Lys Tyr Pro Ser
                1365                1370                1375
Leu Glu Leu Gln Pro Trp Met Tyr Asn Glu Gln Tyr Thr Phe Val Ser
            1380                1385                1390
Asn Asp Ala Pro Glu Asp Thr Gly Thr Leu Glu Leu Leu Asn Ala Leu
        1395                1400                1405
Thr Lys Asp Pro Gly Phe Gly Thr Arg Cys Met Glu Gly Asn Pro Ile
    1410                1415                1420
Pro Asp Thr Pro Cys Gln Ala Gly Glu Glu Glu Trp Thr Thr Ala Pro
1425                1430                1435                1440
Val Pro Gln Thr Ile Met Asp Leu Phe Gln Asn Gly Asn Trp Thr Met
                1445                1450                1455
Gln Asn Pro Ser Pro Ala Cys Gln Cys Ser Ser Asp Lys Ile Lys Lys
            1460                1465                1470
Met Leu Pro Val Cys Pro Pro Gly Ala Gly Gly Leu Pro Pro Pro Gln
        1475                1480                1485
Arg Lys Gln Asn Thr Ala Asp Ile Leu Gln Asp Leu Thr Gly Arg Asn

1490                1495                1500
Ile Ser Asp Tyr Leu Val Lys Thr Tyr Val Gln Ile Ile Ala Lys Ser
1505                1510                1515                1520
Leu Lys Asn Lys Ile Trp Val Asn Glu Phe Arg Tyr Gly Gly Phe Ser
                1525                1530                1535
Leu Gly Val Ser Asn Thr Gln Ala Leu Pro Pro Ser Gln Glu Val Asn
            1540                1545                1550
Asp Ala Ile Lys Gln Met Lys Lys His Leu Lys Leu Ala Lys Asp Ser
        1555                1560                1565
Ser Ala Asp Arg Phe Leu Asn Ser Leu Gly Arg Phe Met Thr Gly Leu
    1570                1575                1580
Asp Thr Arg Asn Asn Val Lys Val Trp Phe Asn Asn Lys Gly Trp His
1585                1590                1595                1600
Ala Ile Ser Ser Phe Leu Asn Val Ile Asn Asn Ala Ile Leu Arg Ala
                1605                1610                1615
Asn Leu Gln Lys Gly Glu Asn Pro Ser His Tyr Gly Ile Thr Ala Phe
            1620                1625                1630
Asn His Pro Leu Asn Leu Thr Lys Gln Gln Leu Ser Glu Val Ala Leu
        1635                1640                1645
Met Thr Thr Ser Val Asp Val Leu Val Ser Ile Cys Val Ile Phe Ala
    1650                1655                1660
Met Ser Phe Val Pro Ala Ser Phe Val Val Phe Leu Ile Gln Glu Arg
1665                1670                1675                1680
Val Ser Lys Ala Lys His Leu Gln Phe Ile Ser Gly Val Lys Pro Val
                1685                1690                1695
Ile Tyr Trp Leu Ser Asn Phe Val Trp Asp Met Cys Asn Tyr Val Val
            1700                1705                1710
Pro Ala Thr Leu Val Ile Ile Ile Phe Ile Cys Phe Gln Gln Lys Ser
        1715                1720                1725
Tyr Val Ser Ser Thr Asn Leu Pro Val Leu Ala Leu Leu Leu Leu Leu
    1730                1735                1740
Tyr Gly Trp Ser Ile Thr Pro Leu Met Tyr Pro Ala Ser Phe Val Phe
1745                1750                1755                1760
Lys Ile Pro Ser Thr Ala Tyr Val Val Leu Thr Ser Val Asn Leu Phe
                1765                1770                1775
Ile Gly Ile Asn Gly Ser Val Ala Thr Phe Val Leu Glu Leu Phe Thr
            1780                1785                1790
Asp Asn Lys Leu Asn Asn Ile Asn Asp Ile Leu Lys Ser Val Phe Leu
        1795                1800                1805
Ile Phe Pro His Phe Cys Leu Gly Arg Gly Leu Ile Asp Met Val Lys
    1810                1815                1820
Asn Gln Ala Met Ala Asp Ala Leu Glu Arg Phe Gly Glu Asn Arg Phe
1825                1830                1835                1840
Val Ser Pro Leu Ser Trp Asp Leu Val Gly Arg Asn Leu Phe Ala Met
                1845                1850                1855
Ala Val Glu Gly Val Val Phe Phe Leu Ile Thr Val Leu Ile Gln Tyr
            1860                1865                1870
Arg Phe Phe Ile Arg Pro Arg Pro Val Asn Ala Lys Leu Ser Pro Leu
        1875                1880                1885
Asn Asp Glu Asp Glu Asp Val Arg Arg Glu Arg Gln Arg Ile Leu Asp
    1890                1895                1900
Gly Gly Gly Gln Asn Asp Ile Leu Glu Ile Lys Glu Leu Thr Lys Ile
1905                1910                1915                1920
Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950
Ser Ser Thr Phe Lys Met Leu Thr Gly Asp Thr Thr Val Thr Arg Gly
        1955                1960                1965
Asp Ala Phe Leu Asn Lys Asn Ser Ile Leu Ser Asn Ile His Glu Val
    1970                1975                1980
His Gln Asn Met Gly Tyr Cys Pro Gln Phe Asp Ala Ile Thr Glu Leu
1985                1990                1995                2000
Leu Thr Gly Arg Glu His Val Glu Phe Phe Ala Leu Leu Arg Gly Val
                2005                2010                2015
Pro Glu Lys Glu Val Gly Lys Val Gly Glu Trp Ala Ile Arg Lys Leu
            2020                2025                2030
Gly Leu Val Lys Tyr Gly Glu Lys Tyr Ala Gly Asn Tyr Ser Gly Gly
        2035                2040                2045
Asn Lys Arg Lys Leu Ser Thr Ala Met Ala Leu Ile Gly Gly Pro Pro
    2050                2055                2060
Val Val Phe Leu Asp Glu Pro Thr Thr Gly Met Asp Pro Lys Ala Arg
2065                2070                2075                2080
Arg Phe Leu Trp Asn Cys Ala Leu Ser Val Val Lys Glu Gly Arg Ser
                2085                2090                2095
Val Val Leu Thr Ser His Ser Met Glu Glu Cys Glu Ala Leu Cys Thr
            2100                2105                2110
Arg Met Ala Ile Met Val Asn Gly Arg Phe Arg Cys Leu Gly Ser Val
        2115                2120                2125
Gln His Leu Lys Asn Arg Phe Gly Asp Gly Tyr Thr Ile Val Val Arg
    2130                2135                2140
Ile Ala Gly Ser Asn Pro Asp Leu Lys Pro Val Gln Asp Phe Phe Gly
2145                2150                2155                2160
Leu Ala Phe Pro Gly Ser Val Leu Lys Glu Lys His Arg Asn Met Leu
                2165                2170                2175
Gln Tyr Gln Leu Pro Ser Ser Leu Ser Ser Leu Ala Arg Ile Phe Ser
            2180                2185                2190
Ile Leu Ser Gln Ser Lys Lys Arg Leu His Ile Glu Asp Tyr Ser Val
        2195                2200                2205
Ser Gln Thr Thr Leu Asp Gln Val Phe Val Asn Phe Ala Lys Asp Gln
    2210                2215                2220
Ser Asp Asp Asp His Leu Lys Asp Leu Ser Leu His Lys Asn Gln Thr
2225                2230                2235                2240
Val Val Asp Val Ala Val Leu Thr Ser Phe Leu Gln Asp Glu Lys Val
                2245                2250                2255
Lys Glu Ser Tyr Val
            2260

<210> 2
<211> 7864
<212> DNA
<213> Homo sapiens

<400> 2
gtccctgctg tgagctctgg ccgctgcctt ccagggctcc cgagccacac gctgggggtg      60
ctggctgagg gaacatggct tgttggcctc agctgaggtt gctgctgtgg aagaacctca     120
ctttcagaag aagacaaaca tgtcagctgt tactggaagt ggcctggcct ctatttatct     180
tcctgatcct gatctctgtt cggctgagct acccacccta tgaacaacat gaatgccatt     240
ttccaaataa agccatgccc tctgcaggaa cacttccttg ggttcagggg attatctgta     300
atgccaacaa cccctgtttc cgttacccga ctcctgggga ggctcccgga gttgttggaa     360

actttaacaa atccattgtg gctcgcctgt tctcagatgc tcggaggctt cttttataca 420
gccagaaaga caccagcatg aaggacatgc gcaaagttct gagaacatta cagcagatca 480
agaaatccag ctcaaacttg aagcttcaag atttcctggt ggacaatgaa accttctctg 540
ggttcctgta tcacaacctc tctctcccaa agtctactgt ggacaagatg ctgagggctg 600
atgtcattct ccacaaggta tttttgcaag gctaccagtt acatttgaca agtctgtgca 660
atggatcaaa atcagaagag atgattcaac ttggtgacca agaagtttct gagctttgtg 720
gcctaccaag ggagaaactg gctgcagcag agcgagtact tcgttccaac atggacatcc 780
tgaagccaat cctgagaaca ctaaactcta catctccctt cccgagcaag gagctggctg 840
aagccacaaa aacattgctg catagtcttg ggactctggc ccaggagctg ttcagcatga 900
gaagctggag tgacatgcga caggaggtga tgtttctgac caatgtgaac agctccagct 960
cctccaccca aatctaccag gctgtgtctc gtattgtctg cgggcatccc gagggagggg 1020
ggctgaagat caagtctctc aactggtatg aggacaacaa ctacaaagcc ctctttggag 1080
gcaatggcac tgaggaagat gctgaaacct tctatgacaa ctctacaact ccttactgca 1140
atgatttgat gaagaatttg gagtctagtc ctctttcccg cattatctgg aaagctctga 1200
agccgctgct cgttgggaag atcctgtata cacctgacac tccagccaca aggcaggtca 1260
tggctgaggt gaacaagacc ttccaggaac tggctgtgtt ccatgatctg gaaggcatgt 1320
gggaggaact cagccccaag atctggacct tcatggagaa cagccaagaa atggaccttg 1380
tccggatgct gttggacagc agggacaatg accacttttg ggaacagcag ttggatggct 1440
tagattggac agcccaagac atcgtggcgt ttttggccaa gcacccagag gatgtccagt 1500
ccagtaatgg ttctgtgtac acctggagag aagctttcaa cgagactaac caggcaatcc 1560
ggaccatatc tcgcttcatg gagtgtgtca acctgaacaa gctagaaccc atagcaacag 1620
aagtctggct catcaacaag tccatggagc tgctggatga gaggaagttc tgggctggta 1680
ttgtgttcac tggaattact ccaggcagca ttgagctgcc ccatcatgtc aagtacaaga 1740
tccgaatgga cattgacaat gtggagagga caaataaaat caaggatggg tactgggacc 1800
ctggtcctcg agctgacccc tttgaggaca tgcggtacgt ctgggggggc ttcgcctact 1860
tgcaggatgt ggtggagcag gcaatcatca gggtgctgac gggcaccgag aagaaaactg 1920
gdgtgtctat atgcaacaga tgccctatcc ctgttacgtt gatgacatct ttctgcgggt 1980
gatgagccgg tcaatgcccc tcttcatgac gctggcctgg atttactcag tggctgtgat 2040
catcaagggc atcgtgtatg agaaggaggc acggctgaaa gagaccatgc ggatcatggg 2100
cctggacaac agcatcctct ggtttagctg gttcattagt agcctcattc ctcttcttgt 2160
gagcgctggc ctgctagtgg tcatcctgaa gttaggaaac ctgctgccct acagtgatcc 2220
cagcgtggtg tttgtcttcc tgtccgtgtt tgctgtggtg acaatcctgc agtgcttcct 2280
gattagcaca ctcttctcca gagccaacct ggcagcagcc tgtgggggca tcatctactt 2340
cacgctgtac ctgccctacg tcctgtgtgt ggcatggcag gactacgtgg gcttcacact 2400
caagatcttc gctagcctgc tgtctcctgt ggcttttggg tttggctgtg agtactttgc 2460
cctttttgag gagcagggca ttggagtgca gtgggacaac ctgtttgaga gtcctgtgga 2520
ggaagatggc ttcaatctca ccacttcggt ctccatgatg ctgtttgaca ccttcctcta 2580
tggggtgatg acctggtaca ttgaggctgt ctttccaggc cagtacggaa ttcccaggcc 2640
ctggtatttt ccttgcacca agtcctactg gtttggcgag gaaagtgatg agaagagcca 2700
ccctggttcc aaccagaaga gaatatcaga aatctgcatg gaggaggaac ccacccactt 2760
gaagctgggc gtgtccattc agaacctggt aaaagtctac cgagatggga tgaaggtggc 2820
tgtcgatggc ctggcactga atttttatga gggccagatc acctccttcc tgggccacaa 2880
tggagcgggg aagacgacca ccatgtcaat cctgaccggg ttgttccccc cgacctcggg 2940
caccgcctac atcctgggaa aagacattcg ctctgagatg agcaccatcc ggcagaacct 3000
gggggtctgt ccccagcata acgtgctgtt tgacatgctg actgtcgaag aacacatctg 3060
gttctatgcc cgcttgaaag ggctctctga gaagcacgtg aaggcggaga tggagcagat 3120
ggccctggat gttggtttgc catcaagcaa gctgaaaagc aaaacaagcc agctgtcagg 3180
tggaatgcag agaaagctat ctgtggcctt ggcctttgtc gggggatcta aggttgtcat 3240
tctggatgaa cccacagctg gtgtggaccc ttactcccgc aggggaatat gggagctgct 3300
gctgaaatac cgacaaggcc gcaccattat tctctctaca caccacatgg atgaagcgga 3360
cgtcctgggg gacaggattg ccatcatctc ccatgggaag ctgtgctgtg tgggctcctc 3420
cctgtttctg aagaaccagc tgggaacagg ctactacctg accttggtca agaaagatgt 3480
ggaatcctcc ctcagttcct gcagaaacag tagtagcact gtgtcatacc tgaaaaagga 3540
ggacagtgtt tctcagagca gttctgatgc tggcctgggc agcgaccatg agagtgacac 3600
gctgaccatc gatgtctctg ctatctccaa cctcatcagg aagcatgtgt ctgaagcccg 3660

gctggtggaa gacatagggc atgagctgac ctatgtgctg ccatatgaag ctgctaagga 3720
gggagccttt gtggaactct ttcatgagat tgatgaccgg ctctcagacc tgggcatttc 3780
tagttatggc atctcagaga cgaccctgga agaaatattc ctcaaggtgg ccgaagagag 3840
tggggtggat gctgagacct cagatggtac cttgccagca agacgaaaca ggcgggcctt 3900
cggggacaag cagagctgtc ttcgcccgtt cactgaagat gatgctgctg atccaaatga 3960
ttgctgacat agacccagaa tccagagaga cagacttgct cagtgggatg gatggcaaag 4020
ggtcctacca ggtgaaaggc tggaaactta cacagcaaca gtttgtggcc cttttgtgga 4080
agagactgct aattgccaga cggagtcgga aaggattttt tgctcagatt gtcttgccag 4140
ctgtgtttgt ctgcattgcc cttgtgttca gcctgatcgt gccacccttt ggcaagtacc 4200
ccagcctgga acttcagccc tggatgtaca acgaacagta cacatttgtc agcaatgatg 4260
ctcctgagga cacgggaacc ctggaactct taaacgccct caccaaagac cctggcttcg 4320
ggacccgctg tatggaagga aacccaatcc cagacacgcc ctgccaggca ggggaggaag 4380
agtggaccac tgccccagtt ccccagacca tcatggacct cttccagaat gggaactgga 4440
caatgcagaa cccttcacct gcatgccagt gtagcagcga caaaatcaag aagatgctgc 4500
ctgtgtgtcc cccaggggca ggggggctgc ctcctccaca aagaaaacaa aacactgcag 4560
atatccttca ggacctgaca ggaagaaaca tttcggatta tctggtgaag acgtatgtgc 4620
agatcatagc caaaagctta aagaacaaga tctgggtgaa tgagtttagg tatggcggct 4680
tttccctggg tgtcagtaat actcaagcac ttcctccgag tcaagaagtt aatgatgcca 4740
tcaaacaaat gaagaaacac ctaaagctgg ccaaggacag ttctgcagat cgatttctca 4800
acagcttggg aagatttatg acaggactgg acaccagaaa taatgtcaag gtgtggttca 4860
ataacaaggg ctggcatgca atcagctctt tcctgaatgt catcaacaat gccattctcc 4920
gggccaacct gcaaaaggga gagaacccta gccattatgg aattactgct ttcaatcatc 4980
ccctgaatct caccaagcag cagctctcag aggtggctct gatgaccaca tcagtggatg 5040
tccttgtgtc catctgtgtc atctttgcaa tgtccttcgt cccagccagc tttgtcgtat 5100
tcctgatcca ggagcgggtc agcaaagcaa aacacctgca gttcatcagt ggagtgaagc 5160
ctgtcatcta ctggctctct aattttgtct gggatatgtg caattacgtt gtccctgcca 5220
cactggtcat tatcatcttc atctgcttcc agcagaagtc ctatgtgtcc tccaccaatc 5280
tgcctgtgct agcccttcta cttttgctgt atgggtggtc aatcacacct ctcatgtacc 5340
cagcctcctt tgtgttcaag atccccagca cagcctatgt ggtgctcacc agcgtgaacc 5400
tcttcattgg cattaatggc agcgtggcca cctttgtgct ggagctgttc accgacaata 5460
agctgaataa tatcaatgat atcctgaagt ccgtgttctt gatcttccca catttttgcc 5520
tgggacgagg gctcatcgac atggtgaaaa accaggcaat ggctgatgcc ctggaaaggt 5580
ttggggagaa tcgctttgtg tcaccattat cttgggactt ggtgggacga aacctcttcg 5640
ccatggccgt ggaaggggtg gtgttcttcc tcattactgt tctgatccag tacagattct 5700
tcatcaggcc cagacctgta aatgcaaagc tatctcctct gaatgatgaa gatgaagatg 5760
tgaggcggga aagacagaga attcttgatg gtggaggcca gaatgacatc ttagaaatca 5820
aggagttgac gaagatatat agaaggaagc ggaagcctgc tgttgacagg atttgcgtgg 5880
gcattcctcc tggtgagtgc tttgggctcc tgggagttaa tggggctgga aaatcatcaa 5940
ctttcaagat gttaacagga gataccactg ttaccagagg agatgctttc cttaacaaaa 6000
ataggtatct tatcaaacat ccatgaagta catcagaaca tgggctactg ccctcagttt 6060
gatgccatca cagagctgtt gactgggaga gaacacgtgg agttctttgc ccttttgaga 6120
ggagtcccag agaaagaagt tggcaaggtt ggtgagtggg cgattcggaa actgggcctc 6180
gtgaagtatg gagaaaaata tgctggtaac tatagtggag gcaacaaacg caagctctct 6240
acagccatgg ctttgatcgg cgggcctcct gtggtgtttc tggatgaacc caccacaggc 6300
atggatccca aagcccggcg gttcttgtgg aattgtgccc taagtgttgt caaggagggg 6360
agatcagtag tgcttacatc tcatagtatg gaagaatgtg aagctctttg cactaggatg 6420
gcaatcatgg tcaatggaag gttcaggtgc cttggcagtg tccagcatct aaaaaatagg 6480
tttggagatg gttatacaat agttgtacga atagcagggt ccaacccgga cctgaagcct 6540
gtccaggatt tctttggact tgcatttcct ggaagtgttc taaaagagaa acaccggaac 6600
atgctacaat accagcttcc atcttcatta tcttctctgg ccaggatatt cagcatcctc 6660
tcccagagca aaaagcgact ccacatagaa gactactctg tttctcagac aacacttgac 6720
caagtatttg tgaactttgc caaggaccaa agtgatgatg accacttaaa agacctctca 6780
ttacacaaaa accagacagt agtggacgtt gcagttctca catcttttct acaggatgag 6840
aaagtgaaag aaagctatgt atgaagaatc ctgttcatac ggggtggctg aaagtaaaga 6900
ggaactagac tttcctttgc accatgtgaa gtgttgtgga gaaaagagcc agaagttgat 6960

gtgggaagaa gtaaactgga tactgtactg atactattca atgcaatgca attcaatgca 7020
atgaaaacaa aattccatta caggggcagt gcctttgtag cctatgtctt gtatggctct 7080
caagtgaaag acttgaattt agttttttac ctatacctat gtgaaactct attatggaac 7140
ccaatggaca tatgggtttg aactcacact tttttttttt tttttgttcc tgtgtattct 7200
cattggggtt gcaacaataa ttcatcaagt aatcatggcc agcgattatt gatcaaaatc 7260
aaaaggtaat gcacatcctc attcactaag ccatgccatg cccaggagac tggtttcccg 7320
gtgacacatc cattgctggc aatgagtgtg ccagagttat tagtgccaag tttttcagaa 7380
agtttgaagc accatggtgt gtcatgctca cttttgtgaa agctgctctg ctcagagtct 7440
atcaacattg aatatcagtt gacagaatgg tgccatgcgt ggctaacatc ctgctttgat 7500
tccctctgat aagctgttct ggtggcagta acatgcaaca aaaatgtggg tgtctccagg 7560
cacgggaaac ttggttccat tgttatattg tcctatgctt cgagccatgg gtctacaggg 7620
tcatccttat gagactctta aatatactta gatcctggta agaggcaaag aatcaacagc 7680
caaactgctg gggctgcaac tgctgaagcc agggcatggg attaaagaga ttgtgcgttc 7740
aaacctaggg aagcctgtgc ccatttgtcc tgactgtctg ctaacatggt acactgcatc 7800
tcaagatgtt tatctgacac aagtgtatta tttctggctt tttgaattaa tctagaaaat 7860
gaaa 7864

<210> 3
<211> 22
<212> DNA
<213> Homo sapiens

<400> 3
gcagagggca tggctttatt tg 22

<210> 4
<211> 24
<212> DNA
<213> Homo sapiens

<400> 4
ctgccaggca ggggaggaag agtg 24

<210> 5
<211> 23
<212> DNA
<213> Homo sapiens

<400> 5
gaaagtgact cacttgtgga gga 23

<210> 6
<211> 20
<212> DNA
<213> Homo sapiens

<400> 6
aaaggggctt ggtaagggta 20

<210> 7
<211> 20
<212> DNA
<213> Homo sapiens

<400> 7
catgcacatg cacacacata                                                    20

<210> 8
<211> 27
<212> DNA
<213> Homo sapiens

<400> 8
ctttctgcgg gtgatgagcc ggtcaat                                            27

<210> 9
<211> 20
<212> DNA
<213> Homo sapiens

<400> 9
ccttagcccg tgttgagcta                                                    20

<210> 10
<211> 26
<212> DNA
<213> Homo sapiens

<400> 10
cctgtaaatg caaagctatc tcctct                                             26

<210> 11
<211> 26
<212> DNA
<213> Homo sapiens

<400> 11
cgtcaactcc ttgatttcta agatgt                                             26

<210> 12
<211> 20
<212> DNA
<213> Homo sapiens

<400> 12
gggttcccag ggttcagtat                                                    20

<210> 13
<211> 21
<212> DNA
<213> Homo sapiens

<400> 13
gatcaggaat tcaagcacca a                                                  21

<210> 14
<211> 10545
<212> DNA
<213> Homo sapiens

<220>
<221> misc_feature
<222> (1)...(10545)
<223> n = a, t, c, or g

<400> 14
acctcttata gaatgataga attcctctgg aatgattgga taacttcatt tcatccttga 60
cttttacctt ggaggatttc ttaccccttt tggcttctca aatttgacta ttaaaatgtt 120
gcctttaaaa ataggaacac agtttcaggg gggagtacca gcccatgacc cttctgcaag 180
gccccctaac tcaaggtagt ttccctggaa ctgtggttta tggaatgttt caggagtgtg 240
aggaggtata atttaaggct gtcctagcaa ggataccctt aaggatagag ggcccagtag 300
catctggagg ccagaaaagt taaactgagg cagtcagatt agcttcaggc tcaattaagc 360
tgatgggtca gcctgggaga aattgcagga tgactctcaa tatcccctcc cacccccaca 420
gcagccacga tctgtctgtc tttaatcatg ggtgcagtga acctgttctt tccaggtgtc 480
ttggccttca gtaaccttgt taggcttgtc cctgaacgtg gctaccgatc caaagacaca 540
tgatcagaga ggcaattaga gaacagacct tttccaaagc aagcatgttc tgttgggctt 600
agaagtttca tgtcctaata ttataggacc ctgtgcatct ctctggagat gaggcacatg 660
agtcatatct gtgattcttg cttttgtgtc aacatctcat gaataggcaa tcagagcttt 720
ggcaccaatg tattttcagt tcatatctga tgtagttaaa tccacctcct gctttgtagt 780
ttactggcaa gctgtttttg atataagaca tctagaacac tgtaaatata taacattttt 840
atttgtctat tatacctcaa ttacgaaaaa gacatctaga agcaacctca tcaagagaga 900
tactgaggcc gggcatggta gctcacactt gcaatcccat tactttggga ggctgaggca 960
ggtagatcac ttgaggtcaa gagtttgaaa ccagcctggc caacatgttg aaaccctgtc 1020
tctattaaaa atacaaaaaa gttagctggg cttggtggtg ggcacctgta atcccagcta 1080
ctccggaggc tgaggcagga gaatcacttg aacctgggag gcagaggttg cagtgagctg 1140
agatcacacc actgcactcc aacctgggca ccagagtgag attacatcta aaaaataaaa 1200
taaagtaata aaaaagagag atattgatag ctgttgttgg aaatttcaac ttccatctca 1260
cttctggtaa ctttttggaa gtttgttgaa caaagtggaa tacacgcaca tacacacaca 1320
cacatactct cttgtttgtt taaggtttaa tgaaatagct gtcatataat cactgttttt 1380
gaaagaggag aattagttgc tatctgtaca ttttgggtat gtgaactatt tggatagaac 1440
tctgagaaat gcattcagaa caacaaacaa aatcatagga gaaatagcta agtgggaagg 1500
ggcatataag agttgttgaa aaagttattt cttgagaaac cagctctaat gctaggcaag 1560
tcacttgctt tgggggaggc ctcagcttct ctgtctataa gattgcagca ggggtgtagt 1620
gggaatgagt cttcaacatt ccaagagatt ttatctacta atacgacagt caaatggagc 1680
atgactttgt ggaagcctct cctcttccac ccagaggggc caatttctct gtcccagtga 1740
gatgttgaca cttgtatgat ccctgcttgg agacttccct cttctggaac ctgccctggc 1800
tcaggcatga gggctgactg tcacccttcg ataggagccc agcactaaag ctcatgtgtt 1860
ggcagtgttc ttgcgggaag gaaaaagacc agccagccca tttgttactg cacaagcaaa 1920
cagcttctgg tagctgtaca gatacatgca ctttctttcc tcactgtgtt tccatagaca 1980
gatttagtgc tgtagaagag tagagggcag tcacgggaag gagttcctgt ttttcttttg 2040
gctatgccaa atggggaaaa atcctcctat cttgtctttt tagtgtcatc ctctctcccc 2100
ttttcttctt ctttataatt ctcatctctc atctctcctg gaaatgtgca tgtcaagttc 2160
aaaagggcac aatgttttgg tgaggaagag gtgggagaac acgtgccagg tgctaactag 2220
ggtcatcatt tcccccttca cagccagctt cctgtgaatg tgtgtgtgtg tgtgtgtgtg 2280
tgtgtgtgtg tgtgtgtgtg tgtgtatttc ttttgccagc atcactgaat ctgtctgctg 2340
tctggtattc caggttttgg tttagggaaa agtaaaagta attttataat cccagctgtc 2400
atttaagcca cccctttgtg ggtagcatat ggtccactct ctcagttcat tgtcctaaag 2460
atgcttcatc agaaaggaat aacttccacc ccgttactct ctgtcccctt actctgcttt 2520
atttttcttc gtcaatccta ccaccaccac ccactgtttg aacaacccac tattatttgt 2580
ctgtttccca tccctggtag aataggagcc ccatgaatga aggaactttg cttctgttgt 2640
tcaccactga atctctaagg tatggaacac acctggcatg tgataggcac tcgataaata 2700
tttgttgtgg ctcatgggca ccttgcagag ttaaggctgc agttgtttgt ggaatttata 2760
agtggtaatg aatatttatc tactattcct cttccaaggc gatcacacaa taatcaggct 2820
ttacactatc cagttcttag gtcttccaag ttatgacttg tgaggtatgt taattatgat 2880
aatagaaggc agtttatttg gttcagattt attgatgtgt aatttaccac agtaagactt 2940

cccctttaca aaagtatgat gagttttgac aaatggatac acatgtgtat ctaccactgc 3000
catgctcctt ttcagtctgt cgtcccctcc acccatgacc actggtcacc actgcagtga 3060
tttctgtccc cttcatttca ccttttccag aatgtcatat aaatggaatc atgcagtatg 3120
tagttttttg tgtctggctt atttttctta gcattaggct tttgggattc atccaggttg 3180
tcgcatgtaa cagtagctta ttccttttta tggctgagta agtgtcccag ttttatttat 3240
atatttattt atgaggaggt gtctcactct gtcacccagg ctggagtgcg gtagcgcgat 3300
ctcagctcac tgcaacctcc gcctcccagg ttcaagcaat tctcctgcct cctgagtagc 3360
tgggattaca ggcacccacc gccacgccca actaattttt atatttttag tagagatggg 3420
gtttcaccat gttggccagg ctgatctcaa actcttgacc tcaggtgatc cgcccacctc 3480
tggctcccaa agtgctagga ttacaggcat gagccactgt gcccagcccc agttttattt 3540
attcaccagt tgatggtctt ttcgacaact aattgtttcc agtttttggc tattctgtat 3600
aaggcttcta taaatattca caaataccta ggatgggatg actgggtcat ataatagtac 3660
tgtataacct tagcagaaac tgtcaaacta ttttccaaag tggctcttcc attttacaat 3720
tccacagtgt attgagtccc agtgtctcca tacacatgct agcactttta atatttaatt 3780
tagtgggtat gtaatgatat ctcattgtgg ttttaatttg catttctctg cagctaatga 3840
tgagtgtttc tgcttatttg ggaaggtttt aatttagcag tctgttgtat tctgtagata 3900
ttaataactt caaaatatca gtggcatttg cagttaaaat ttccttaaaa aattggccaa 3960
aggtttccag cagtcacttc tgccatgccc aaactgtatg aaacaaggct gaggtgtgga 4020
gattgtcaca ttttggcaag gagtgatcca cttgggtgac tgatgagacc cagagagcgt 4080
acgcctcggg cttgagggtg aggacgggcg ggaagtcgac tgcatggccc tgctggcctt 4140
gggaggctgc ccagtcctta gctaaagctg gcagttatgg gaaacagact tagattctat 4200
tacgtttttc aggatgtccc aggagtcacc tgggaagctc agcagtcctt tgtgactttc 4260
aagcatatgg tagaagctgc tgaacacaga gctccctctt tggggataat ttgcccaaat 4320
catttaatca ggcttgagaa atgagttacc acaggtccag gagtgctgcc acccttgaat 4380
tctgacaccc tatttctcct atccgtctct taattaatta agcagacatc cccaagtgct 4440
tacgacaagc caggaccctt ttgcatacta aggaaaacag ggatgaagga aacagaaatg 4500
gtctctgctc tgactcagaa ggtagaaatc ctctttccca gccaagtctt cctagggagc 4560
acgtaggaag ggctctgaac ccacgtgtca gttgcagggg aggatatcag gaaaggacat 4620
tgaagaagtg gagacctaag tttgagacct aggcattagc caggctagca gtgcttgaaa 4680
aagtgtctta ggacaagaga actcaccagt gaagtcccag tggtaggaga gcgtgcagca 4740
tattctgagc ctgtatacac atctccaggg cattgcttag caggtgggga gtggcaagag 4800
agtaggctgg agtcacagaa gggaggccag gtagaccttg gtgagcactg gactctatgt 4860
tcaggtgctg aggagctggc aaaaggtttt aagtcgggga gaggcatgtt cagatatttg 4920
gtctagctga gtaactttgg gtgctctgtg acaaatggtt gggagaccag tgaggtggca 4980
gttgcggtca tctaggagca ggatcagagt ggcctattga ctgggatgac tgtgaagtgg 5040
gatcctttcc agccagtaac tggaaatgtg tatgagggca gaagtgagtg tactgcattt 5100
gaaacattga gaaatctagt acatagtact gtctctttta tatctttttt tttttttttt 5160
ttgattttgg tttgtttgtt cactaacttg gaaaactgat gtggaaatgt ccctttggct 5220
tcagttacct gagcagaagg ggccgggcat tgccaaactc tcctcttagg acagaattgc 5280
tcccagtatt gatcattgtg ttctgagttg ggggagcaaa ttgtgcagga ggccaggtca 5340
gtgccaaggt gggtgggagg aattggagca ggaagcttgc ctaagtgtgc ccagcaaagc 5400
cacggtagaa ctttctactg tggctctatg ctacttctta gcaaccttct ccatgtgctt 5460
cctggagagt ccttggagtc agaacctttt tcttgaaacc cagacacttt acttccaaga 5520
aaatgctgtc caagaaaact catccttccc ttcttctcat gaacgttgtg tagaggtgtg 5580
tcttctcttc ctttgagctt ttccactcag ggtttagggg aggtgatatt ctatatttgg 5640
gtttggctct gggtactgca acactaggct attaagattt catccttact gctttgcccc 5700
tcctatcttt ccagaaaccc acaatggatt tgctagaaat aatggaacgt cctgtttgga 5760
caggatataa ccatttctca gctagaggat attgttggaa tgaagaaaga taaatgggga 5820
gaagggaact cacattgctt tggcacttaa attaagccat gtactgtgtt gggaaattat 5880
ttatattatc tcgttgaatc cacagtagaa cacagttgaa caccatacaa ggtaagtatt 5940
gtcatcctta ttttaccatg aggaaattga tgcttagaga gcataaagcc ttggccaggg 6000
gcacatagtt gggaagccgg ggctaattca tgcctgggct ctttctgata gttttccttt 6060
tttaattgtc ccctcctcat tgttaccttg gggatttcaa gagattcatg tagcttctaa 6120
atcaacgaac tgattcctgg agagcagctt ctgtatgaga aaaatctagc taattattta 6180
tttcagtgtc tctggaatgc aagctctgtc ctgagccact tagaaaacaa tttgggatga 6240

caagcatgtg tctcacaatg ctgctctggt tgccagtgct gtgctgccag ttgtcatctt 6300
tgaacaaact gatgcagtgc tggtttaact cttcctcttt ttggagtaag aaactttgga 6360
ggcctgtgtc cttctagaag tttgctgagc aaatggtaag gaaaagaaat aggtcctaag 6420
gcttgactat ttcagagaat ttcttgattt attggactgt caatgaatga attggaatac 6480
atagtggtag gctgtctttt cttctcagac actgcaattt cctccaatct cttgactttt 6540
ctagaagttt taatccaagt ccttgttggg tggtagataa aagggtattg ttctactaga 6600
gactgacctt ggcatggaga tctcatttgg actcacagat ttctagtcta gcgcttggtt 6660
ttgtatccat acctcgctac tgcattctta gttccttctg ctccttgttc ctcatgccca 6720
gtgtcccacc ctacccttgc ccctactcct ctagaggcca cagtgattca ctgagccatt 6780
tcataagcac agctaggaga gttcatggct accaagtgcc agcagggccg aattttcacc 6840
tgtgtgtcct cccttccatt tttcatcttc tgccccctcc ccagctttaa ctttaatata 6900
actacttggg actattccag cattaaataa gggtaactgc tggatgggtg gctgggatac 6960
acagaatgta gtatcccttg ttcacgagaa gaccttcttg ccctagcatg gcaaacagtc 7020
ctccaaggag gcacctgtga cacccaacgg agtagggggg cggtgtgttc aggtgcaggt 7080
ggaacaaggc cagaagtgtg catatgtgct gaccatggga gcttgtttgt cggtttcaca 7140
gttgatgccc tgagcctgcc atagcagact tgtttctcca tgggatgctg ttttctttcc 7200
agagacacag cgctagggtt gtcctcatta cctgagagcc aggtgtcggt agcattttct 7260
tggtgtttac tcacactcat ctaaggcacg ttgtggtttt ccagattagg aaactgcttt 7320
attgatggtg cttttttttt ttttttttga gacagagtct cgctctgtcg ccatgctgga 7380
gtgtagtggc acaatcttgg ctcactgcac ctccgcctgc caggttcagc gattctcctg 7440
cctcagcctc ccaagtagct gggactacag gtgcctgcca ccatgcccag ctaatttttg 7500
tatttttagt agagacgggg tttcaccgta ttggctagga tggtctcgat ttcttgacct 7560
cgtgatccgc ctgcctcggc ctcccaaagt gctgggatta taggcttgag ccaccacgcc 7620
tggccgatgg tgctttttat catttgaagg actcagttgt ataacccact gaaaattagt 7680
atgtaaggaa gttcagggaa tagtataagt cactccaggc ttgaggcaaa atttacaaat 7740
gctgctgact ttgtatgtaa ggggaggcat tttcttagaa aagagaggta ggtctctggg 7800
attccagtat gccatttcca tcctcagtgt ttttggccac ctgagagagg tctattttca 7860
gaaatgcatt cttcattccc agatgataac atctatagaa ctaaaatgat taggaccata 7920
acacgtagct cctagcctgc tgtcggaaca cctcccgagt ccctctttgt gggtgaaccc 7980
agaggctggg agctggtgac tcatgatcca ttgagaagca gtcatgatgc agagctgtgt 8040
gttggaggtc tcagctgaga gggctggatt agcagtcctc attggtgtat ggctttgcag 8100
caataactga tggctgtttc ccctcctgct ttatctttca gttaatgacc agccacggcg 8160
tccctgctgt gagctctggc cgctgccttc cagggctccc gagccacacg ctgggggtgc 8220
tggctgaggg aacatggctt gttggcctca gctgaggttg ctgctgtgga agaacctcac 8280
tttcagaaga agacaaacag taagcttggg tttttcagca gcggggggtt ctctcatttt 8340
ttctttgtgg ttttgagttg gggattggag gagggaggga gggaaggaag ctgtgttggt 8400
tttcacacag ggattgatgg aatctggctc ttatggacac agaactgtgt ggtccggata 8460
tggcatgtgg cttatcatag agggcagatt tgcagccagg tagaaatagt agctttggtt 8520
tgtgctactg cccaggcatg agttctgatc cctaggacct ggctccgaat cgcccctgag 8580
caccccactt tttccttttg ctgcagccct gggaccacct ggctctccaa aagcccctaa 8640
tgggcccctg tatttctgga agctgtgggt gaagtgagtt agtggcccca ctcttagaga 8700
tcaatactgg gtatcttggt gtcaatctgg attctttcct tcaggcctgg aggaatataa 8760
taactgagac ttgttttatt tctgcagagg gttctaagcc attcacttcc cagatgggcc 8820
aataatgctt tgagtaatct ggagatcatc tttaatgcgc aggtgaatgg aactcttcca 8880
cagagggatg tgagggctgt agagcagagt gaactccctg aaactcagac gtcagctctt 8940
tgtctctcta tctctgaaca cccttcctta gagatcccat ctctaggatg catttctctg 9000
tagttagttt ctaagtctct tgttcctgtt ctgcctttat ttttttttcc tggattctaa 9060
gccagtatcc ccacttggct gtcttaatgt agcttaacat gtctgtaatc aaaatgatca 9120
tctttctgag attcaaaggg ctataaggga ctttggagag aatttcattc agttttcctc 9180
aaactagaat aatgcttgca ctgtctgtaa aagaacaaaa gtgtcaaagc atccttttgt 9240
tcactaaatt tcctttttta ttatagtgtt acttaaatat taggaagtta aaagtaggta 9300
taaacttctt ataggctgtt attatacaac tatatgaccc atacatattt acaaattaag 9360
tgcagccaaa attgcaaaat caataccatt caaattaata ccttaaatgt ggtgaggcag 9420
ctgttgttca actgaaacca aattataagt tgcatggcag taaatgctat catgctgatc 9480
attttgagtt tggccagtct atattatcat gtgctaatga ttgaattctc cacccatttt 9540

tctacttgta tgaccttaat ttgatggcac ctgttccatc ctcatgagtt tgctacaatt 9600
atactggtgc caacacaatc ataaacacaa atataaactt gggctttgaa atcttgtgcc 9660
agaacttggc tttaaagtaa gcatttaaaa aatccatatg tgtttattag actttgttta 9720
gatgactgtt gaaatgaaaa caaagtgttt aaaatcctct tagagaactt aaatataatc 9780
cctcagcaat atgtatacag atcttccttt gagaaaaact gattgtgttc agcctctcat 9840
gttacaaatg gggaacctga attctgaggt ctctagtgag agaacaggga ctggaatctg 9900
tggatcctat ctgttttaat aataattgta aagtataata gataatatta tattaaaaag 9960
agagnnnnnn acacttagaa tgagcttcca tgtgtgaggc actaactgat taggcattat 10020
taactagatt tattcctttt aaggccccgc gatgtactgt tatttccaca tgttgtagct 10080
ggggaacgtg ctactcagag aggttaagta acttgtctga ggtccacacc actaacaagg 10140
agcacaggta gggttcaaat ccagataatc tgactttgga gctggcactc taactcaatg 10200
tgcctaatcg cttttcagtg gtgtcattat tttgcctatt ctccatctga gaatattgaa 10260
gtttctgact ccttccttgc ctttctccct gcctcccgtg gttatcccca ggtcttggtg 10320
ttccagtcct ctatgtccgt ccttactctt attcctttgc tacagtgtga tccagggctc 10380
ctgcccttct tatcctggta gagggggccc acttgctggg aaattgtctc cgccatggtt 10440
tatccatgtt gtgtgtccat tagtgagtag tgggaagaat catatcatgt tggcaatgaa 10500
aggggggcta tggctctggg gtagtctagt ctgaactctt atttt 10545

<210> 15
<211> 4736
<212> DNA
<213> Homo sapiens

<400> 15
cttttttttt tttttttttt tttttttttt tgaggtgaag tctcactctg ttgcccaggc 60
tggagtgcaa tggagcgatc ttggctcacc ccaacctctg tctcctgggt tcaaacagtt 120
ctcctgcctc agcctcccga gtagctggga ttacaggctc ccgccaccat gcccagctat 180
ttttttgtat tttcagtaga gatggggttt cacccttttg accaggctgg tcttgaactc 240
ctgacctcat gatcaaccca cctcagcctc ccaaagtgct gggattacag gtgtgagcca 300
ccacgcccgg cctcataagt attttctaaa tttatttaca gtcatgccat ttaaaaggaa 360
agttgtattc ctgtctttgt taatatttat aagtgatttt attcagctac aagcttggaa 420
tggcatataa ttttgtattc tgcttttttc acttaatatt acatggctaa tgatttctgt 480
gtttcataaa cattattctg atgatggcat gatatattgt tgagtacatg taccataatt 540
gaatcatttc cctattgcta tgcaattaag ttgtttccaa tattttgcaa ttataatgtt 600
tcaatgaatg aataacttta tgcatatagc tttttgatat cttaagttca gtttcctagg 660
atgaatttcc aggaatagta attgggcaaa tgggataaac atgactcttg aatacgtatt 720
gttaacattg ctttcccaaa gggctcaact gatttatatt tccgtgttca ttatctttta 780
aaccagctca tttactcacc aaacattttt aaagccatta tcatgtggta ggcttagtaa 840
gaagaaagtg accctaaggg agaagcttat atataaatag ggtccctggt gtaccaagtg 900
ctgatacaga cacaaagtac ctggggaaat tgagatgagg gagtcctggc tcagctggga 960
gaaaagttca ttttcataga gtcatggttt tgttctttgg cagaaagaaa attgctttct 1020
tccccacccc cacccccagc tttattgagg tataattgac aaataaaaat tgtatatctt 1080
taagatatgc aatgtgatat atatgtatat ctcaacttaa aaaataagct acagaataaa 1140
aaggtgtttg ctattaaaaa aaaagaaaag gctgaatgtc attcccaagc ttggaaattt 1200
gagtatgttg cctctttggg attatttaca gaaatattag caagaccagc cccatctttg 1260
gtcttgagta ctccactgtc agcatgcttt cttccagaga gggatccatt tgcctttatt 1320
tttcattctg ttgtgccgtc tatgcaaact attcttgata gttttatggt aacagtgttt 1380
ttttgttcca tgagataaat ttatacatgc tcattgtgga aaatttagaa aagacaggaa 1440
agtattaaaa acatcmcytt tttttttttt tttttttttt tttttttamg cagacagagt 1500
cttgctctgt cgcccaggcc ggagtgcagt ggcgtgatct cagctcacag caacctccgc 1560
ttcccaggtt taagtgattc tcctgcctca gcctcccaag tagctgggag tacaggcatg 1620
caccaccacg cccggctaat tttgtatttt tagtagagat ggggtttcac catgttggcc 1680
aggctggtct caaactcctg acctcaggtg atccgcctgc cttggcctcg caaagttctg 1740

ggattatagg caggagccac tgcgccagcc acacctacgt tcttatcatc ctagtacatc 1800
cactgtcatt atcttgctgt atttccttct gcccagtctc actctgatca tgcagtggcg 1860
tgatcatgca gtgatctcgg ctcactgcaa cctaggcctt ctgggttcga gtgattctcc 1920
tgccttagcc tcctgggttc aagtgattct cttgccttgg cctcccaagt agctgggatt 1980
acaggcatac acccccatgc ccatctaatt tttgtatttt tagtagacac agcgtttcac 2040
taaaattttg tatttttagt agagatgggg tttcaccatg ttggccaggc tggtctccaa 2100
ctcctgacct caggtgatcc gcctgccttg gcctcacaaa gtgattacag gcatgagcca 2160
ctgcatccat cgccaaaaag attttttaaa agagtttaat gtagaaccat atcaaaggtc 2220
tttggaaata aaaaacagtt ttttaaaaat atcagaaata aaacaacaaa taaataaata 2280
aataaaaaca cccaaaacaa tctgaagcac gagcacctag cagaaaggtt caattatgat 2340
ctattcatag agtggaatat caagtagaca ttacaggaca tgttttaaga ttatatttta 2400
tgtcatggga aatgctctcc cagtatgatg ttaaatgaaa aaacagaata caaaagtata 2460
tatgctgcat agtctcaata ttgtagagaa aaaatattat ttatgtatgc atgaaaaaag 2520
acaaaagatg ttaacagaga tccattgtta cttcagttta ctagggattg tctctgggag 2580
gtaggattaa ggtgatttat atttaccttt ttaaactttt ctgtattttt ttattttcaa 2640
attttccata aaaatataag gacttgaaga tcaagaaaaa atttctgctt tggctcagtg 2700
cagtcgtcac gcctgtaatc ccagcagttt gggagcccta ggggagagga tcacttgaac 2760
ccaagagttt gacgttccag tgagctatga tctccggatc gtaccgcctg gacgatggag 2820
caagaccctg tctcaaaaaa aaaaatcttt gctttttttt tttgtttgtt tttgagacgg 2880
agtctctctc tgttgcccca gctggagtac agtggcacaa tctcagctca ccgcaacctc 2940
tgcctcctgg gttcaagcga ttctcttgcc tcagcctccc aagtacctgg gattccatgc 3000
acccaccact atgcccagct acttttttgt attttcagta gagacagggt ttcaccatgt 3060
tggccaggct ggtctcgaat tcctgacctc agctgatcca ccggccttgg cctcccaaag 3120
tgctgggatt acaggcatga gccactgtgc ccagcccaat cttttgcttt ttttaaaaaa 3180
agaagacaaa aagggatttt ataccagtat tatcttggct gtgtgactct gaagccacag 3240
ttgtaagcta taattactct gaaacacaag gccctgtgac tcttttgggc tctttggtgt 3300
ttatcttgat tacaacgttg gaatatagaa atgaaaggaa tgggagaggt gatagacttc 3360
aggcagtgta actagttgtc tgaacactac tggctcaatt atattgtgtc tagtgatttc 3420
catcttgtcc gtctgctaat ttatcgcctg gtaactcact gaggcagggt tttcctttgg 3480
agaaacctca ttgttttaac cagtgtatca tgcttgttta gaagttcaat gatcttttta 3540
actcatcgga gaagatgatg accagacctg gacagatggg gaaggacttt gcactctctc 3600
tttacagtcc tgagtgcaca caggtcaata tggaactatg tgtgaatttt cattgtcttt 3660
gagagccctc ttctctgccc catagggagc agctttgtgt gcaattagag gagcaagggt 3720
tgtgtgtatt tagcacagca ggttggcctg gtcctctcct ctcaacatag tcaccacata 3780
cctggcacta tgctaaggct gggaatgcag acagatgggt gcctgctttc agagtgctca 3840
atgtgctgag gaagccagca acagaaacag atgatttcag gagctccagg aaaatgctac 3900
aggaggagtg tgcctgggtt actggagtag cacaggagga gggcttctag ctcaggctga 3960
gattttagta aaggaaatta tgccacgatg aatcctgaag aatgaataga agtgaaccag 4020
ataaagcacg ataggaagca tcttccctta cctaagggaa gacacagagg tatatggaat 4080
ggtatgttaa aaggttggga ctccaaacag ttctgttaaa gcttagagag tggtgggaga 4140
gactggagaa gttgattaat tagtaaatga agttgtctgt ggatttccca gatcccagtg 4200
gcattggata tccatattat ttttaaattt acagtgttct atcttatttc ccactcagtg 4260
tcagctgctg ctggaagtgg cctggcctct atttatcttc ctgatcctga tctctgttcg 4320
gctgagctac ccaccctatg aacaacatga atgtaagtaa ctgtggatgt tgcctgagac 4380
tcaccaatgg cagggaaaat ccaggcaatt aacgtgggct aaattggact tttccaaaga 4440
tgctgtcttt gggaaacatc acacatgctt tggatcagaa aacctaggct tctaatttgt 4500
tgataaggca tgaactcagg agactgtttt cagtcctagt gaatggtgat aattgtaatt 4560
ataacagtag acaacatctc ttttacacat tttaaatcat gaaaatagaa taaccttact 4620
gataatttta gaaagtggtg attaaaagca catttaagat aatgccttaa cacctagtct 4680
tttccatatg catgatgtct taatcacaca ttgcaaatca tggaacacag aatttt 4736

<210> 16
<211> 4768
<212> DNA
<213> Homo sapiens

<400> 16
atcttacaat cacagtcttt ctcttagggc tgggctcagt gggtggattg acactgcaga 60
aatggccaga tctaaaggat caacatttac gtagctggga aatgtagctg ggacttcagt 120
ttcactgccc tagtgatttt tcctaccact aagcagctca gtccataccc ctacgagacc 180
cacaagctta tgagatactg ttcttccagg aaagcagtgg ggccagggcc accttttaat 240
tgtgtttctt ggcctggtcc catctttctc acaatatata gcaacagtta tttacttgct 300
gattttctaa tgcacatcac acatagtcat attaaacaca cacacacaca cacacacaca 360
cacacacccc tcaagaaaca ttttctgaga cgtgatttcc tgatttcatc aaaaaagaaa 420
agagcgggcc aggcacagtg ggaagtcaag gtgggtggat cacttgaggt caggagtttg 480
aaaccagcct ggccaacacg gtggaacctc gtctctacta aaaatacaaa aattagccag 540
gcgtggtggc gcacacctgt aatcccagct actggggagg ctgaggcagg agaattgctt 600
caacctgcga ggctgaggtt gcagtgagcc gagattgcgc cattgcactc cagcctgggc 660
aacagagtga gactctgtct caaaaaaaaa aaaaaaaaaa aaagcataaa ctgaaattta 720
tatgcaattt atatgcctgt gagataattc tgttttctct tttggaaccc caaagagatt 780
tttttgattg atgagcaaat acattttaga ttttatttaa gcattatgcc aagcaccact 840
gaagtataag tttcaagggc aaactcagtt ttttcatcta ctagacgaat gattttctgg 900
aatgattaca agcaggcaag atggtgtagt ggaaatagca aatgtcttcg gcatcagaca 960
agttggggtt tgtttgtatc ctgcctctgc ccttcaccga ggttgtgatc ttgggcagat 1020
tgttgagttt taacctagat tcctctgact ccagatcata aattttcaga aaagttctga 1080
aattcttgta tatactgatg gtaaatgaga cttttcctta catctatgca cttctttgtt 1140
tgtttgtttt gagatggtct tgctctgttg cccagactgg agtgcagtag tgcaatctcc 1200
gctcactaca atgtctgcct cccaggttcc agtgagcctc ctgcctcagc ctcccaaata 1260
gctgagacta caggcatgtg ccaccacgtc cggctaattt ttgtattttt agtagagaca 1320
gggttttgcc atgttgacca cactggtctc gaactcctgg cctcaggtga ttcgcccgcc 1380
tcagcctccc aaagtgctgg gattacaggc atgagccacc atgcccggcc atatccatgc 1440
acttcttgca accttacctt cttttctcat caccctccag ggacctagtt ggaagagcag 1500
agttaaaagt taaggtgaaa cttggagagg tgtcttgtcc ctaggaacaa aggactggtt 1560
tgaaattctc tgtaaatctt ccccagttca aaccagagtt atcaaggtct taaaaacttc 1620
cctgggtcct gagagcccat tatattattt acttgtcttc ctgtacaccc actgcctagt 1680
cctgatccta cttttgtttg caaataggat ggggcacaac gtacaaggaa gggcctttgc 1740
cacccctgct aagggataac ctgaaatacc ttcaccatca ctgccctgtg ctgcttttca 1800
cctatgccag tctgtctaca gtgccagtgt ctcctggcat tgaaagggga gaatcttttg 1860
gtcctttgag tatttggttg ggttacataa atctccctga atgaagagca gctgacttag 1920
gcaaggggcc ttgtttggtt ttccttgaac tattaacagg aagataggga gattaactgt 1980
gtaaatgttc aataggccag agtccctgca gagggtggcc acagtgatca gatcttatca 2040
catccttgct ttgggtgttg cctctctggt tggagtatgg atagaaaaga aagaaagacc 2100
ctatattgaa atgcaaagtg cagcaagtcc tgactttgga ttaacttctc agcccatttg 2160
catgaaaata aaaagatgaa taaaacaagg ttcccacttt ggagggaggt ggtagctgtg 2220
agatggaagg agtgttcctg ctgggcaaca gcagagtaag tgctggggta gattcactcc 2280
cacagtgcct ggaaaatcct cataggctca tttgttgagt ctttgtccta caccaggcac 2340
tctgcaaaaa cgctttgcct gcaaggtctc atgcgatgct caccacagct ctgtgaagtt 2400
aattgtactt ttatcaccat tttacagatg agaaaactga gggtatgggg tcaatgactt 2460
ggctaaagtc actgcttagc aagctgcagg gactggatgt gaattccaat tggtttgact 2520
ccaaagcctg tgaagctact tgttcttcac cacctagagc tgtggttctt gataactgtg 2580
aactcttttg gggtcacaaa tagccctgag aatatgatag aagcaggagc tctggccttt 2640
ctgtccatac ctgaacaggt ccttgggtta agagcccctc gtccagggcc tattaatctt 2700
gatcctcata agcagcatcc atgtattacg gccgcaaacc aaactgtgcc agaccgaatc 2760
ctaggaccaa gcccaaatat gtcccatcat ccttttggta agaagctcat tgtaagaaag 2820
aaagaggaga gcaagaggat gacctagtgc atggggcctc attgttttaa ttagtgacaa 2880
aacaacaata ataacaacaa aacccccgaa gcttcacaga tgacatcaga ccccaagcct 2940
gtgtgttttt caggtgccct tgaggagctt tgtagctggc agaggaggtg aaactgacaa 3000
atgtttggca gatggaggag agtaccagag gggtttgaga tgagctaaat tccaatctaa 3060
ccgcagtgtt gaggaagagg cttggattgg gaccatggag atgggggttc tactcccagt 3120
cacgccagct gactttgcga gtgttctttg tcagtcactt tatcttattt tatttatttt 3180
tatttttttg aaatggagtt tcgctcttgt cgcccaggct ggagtgaaat ggcgcgatct 3240

tggctcactg caacctcccc ctcctgagtt caagcgattc tcctgcctca gcctccagag 3300
tacctgggat tacaggcgcc tgccaccaag cccatcgaat ttttgtatgc ttagtagaga 3360
cagggtttcg ccatgttggc cagggtggtc ttgaactcct gacctcaggt gatccgccca 3420
ccttggcctc ccaaagtgct gggattacag gcgcgagcca ctgtgcccag cccacttcat 3480
cttaccgtag ttacctcctt agagtatgaa aaaataggct tagggcatcc ccaagtcccc 3540
tctatgtctg agagctgagg ctggctgtca aagaggaact aaggatgcca gggactttct 3600
gcttaggacc cctctcatca cttctccaac gctggtatca tgaaccccat tctacagatg 3660
atgtccacta gattaagaat ggcatgtgag gccaagtttc cacctgagag tcagttttat 3720
tcagaagaga caggtctctg ggatgtgggg aatgggacgg acagacttgg catgaagcat 3780
tgtataaatg gagcctcaaa atcgcttcag ggaattaatg tttctccctg tgtttttcta 3840
ctcctcgatt tcaacaggcc attttccaaa taaagccatg ccctctgcag gaacacttcc 3900
ttgggttcag gggattatct gtaatgccaa caacccctgt ttccgttacc cgactcctgg 3960
ggaggctccc ggagttgttg gaaactttaa caaatccatg taagtatcag atcaggtttt 4020
ctttccaaac ttgtcagtta atccttttcc ttcctttctt gtcctctgga gaattttgaa 4080
tggctggatt taagtgaagt tgtttttgta aatgcttgtg tgatagagtc tgcagaatga 4140
gggaagggag aattttggag aatttggggt atttggggta tccatcacct cgagtattta 4200
tcatttctgt atgttgtgaa catttcaagt cctgtctgct agctattttg gaatatacta 4260
tatgttgtta atgatatcat gcagcagacg tgcatctgaa tgggctggct ctaggagcta 4320
gagggtaggg gctggcacaa agatgcatgc tggaagggtc cttgcccata agaagcttac 4380
agccaaggct aggggagttc tgtcttctct gcatcaggtc acctctctca cctctgtcac 4440
tgccccatca gactacaatg tctgcaggtc tttctcccct gagtgtgagc tccctgagca 4500
aagcaggatg ctgccccttc cctttgtatt ccttgctcct tgcttcagtg cctgtacata 4560
agtatgggca taataagtgt cccccaaatg agacattgag gattcttcaa atgcacagga 4620
ccgtgatgtg agttaggacg gagtaaggac gatgggatgt ggctcatgac aatcctgagg 4680
aagctgcagc tgcggcacgc agggccacac tgtcatgttc atggacccta gactggcttt 4740
gtagcctcca tgggcccctt ccatacac 4768

<210> 17
<211> 1295
<212> DNA
<213> Homo sapiens

<400> 17
tcatgactgc cattggtata aagatgaata taatccagac cagattcatg attattcata 60
catttttagt gtattaactt ttaattctgc ttttaaaata aattaaaaca ttctaatatg 120
cccttaagag tatcccagcc caggccactg agcctactgt ggttcatgga taagtttgcc 180
cctgggggca tgtgtgtgca tgcatgtgtg tgcacatgca tgatgagccg ggccttgaag 240
ggtggtaaga tttgggtgtg tagaccaatg gagaaaggca tttggggcag tgatgatggg 300
tgggggaggg aacatggtga tgaatggagc tgggtgtggg gagccatggg agtgggttag 360
ggccagcctg tggaggacct gggagccagg ctgagttcta tgcacttggc agtcacttct 420
gtaaagcagc agaggcagtt ggcctagcta aagcctttcg ccttttcttg caccctttac 480
agtgtggctc gcctgttctc agatgctcgg aggcttcttt tatacagcca gaaagacacc 540
agcatgaagg acatgcgcaa agttctgaga acattacagc agatcaagaa atccagctca 600
agtaagtaaa aaccttctct gcatccgttt ataattggaa attgacctgc accagggaaa 660
agagtagccc aggtgtctgg ggcttgttcc cattagatct tccccaaggg gtttttctcc 720
ttggtggctg gcctgtgggg cccctctcca ggaggcattg gtgaagaaac taggggagct 780
ggttgccaca gacagtgatg tactaatctt ctctgggaag acagaagaaa agtccccagg 840
gaagaatact acagacttgg ccttagggac agctaggggt gcagattgct gccaactgca 900
ttttttctga agttggccat atggttgcag tgaatggatt tatagacaga gtatttctgt 960
gcatataaga gcaattacag ttgtaagttg atatggataa gtgaaagtta agcacttctt 1020
tctaaaaaga gaatgcaatt cattttcccc taatcatttc aattagtctg atgggcattt 1080
gaacttgttg tctttaaaaa gtgaaatctt tacctctgat ctggtaagta tccaggcaat 1140
ttcttgtgtg ccacccagga ggtatctggg gagtgggcat tttctgactg aggcattggc 1200
tgccatagca tcagagcagc cttccaggca gtggcctggc aaggggacag aggctggtgg 1260
gagcagctgg ctgagtgcag ccagtaatgg catgt 1295

<210> 18
<211> 2188
<212> DNA
<213> Homo sapiens

<400> 18
agctctccag gtgattctga tgcatactta agtttgagaa ccattgcttg ttttgcatta 60
aacaggagat tagtctctgc agcttgtggg aataaagctt taaatctctc caattttagc 120
tctgtgaaaa ggcagtgggg agacaggaat gaacggacta gtgccacaaa gctcaggtgg 180
ggtgggtgag atcatttaga agagaaagac cgggcatggt ggctcacgcc tgtactgtca 240
gcactttggg aggccaaggc aggttggatc acaaggtcag gagtttgaga ccagcctgcc 300
tatcatggtg aaaccctgtc tgtactaaag ataaaaaaaa aaaaatttgc cagtcatggt 360
gatgcatacc tgtaatccca gctactcggg aggctgaggc aggagaatct cttgaacccg 420
ggaggcgggg gttgcagtga gctgagattc caccattgca ctccaaccta ggtgacaggg 480
tgagactccg tctcaaaata aaaaaaaaaa aagaaaagga aaggctgtgt gtgtgtgtat 540
gtgtgtgtgt gtgtgtgtgt gtgtgtgtaa cagcaccatc acactgtttg agttgaggag 600
cacatgctga gtgtggctca acatgttacc agaaagcaat attttcatgc ctctcctgat 660
atggcgatgc tcccctatct cattcctgtg tgtgtttagc caggcaactg ttgatcatca 720
atattatgat aacgtttctc cactgtccca ttgtgcccac tttttttttt tttttgagtt 780
acttactaaa taaaaataaa acactatttc tcaatagact tgaagcttca agatttcctg 840
gtggacaatg aaaccttctc tgggttcctg tatcacaacc tctctctccc aaagtctact 900
gtggacaaga tgctgagggc tgatgtcatt ctccacaagg taagctgatg cctccagctt 960
cctcagtagg gctgatggca attacgttgt gcagctactg gaaagaaatg aataaaccct 1020
tgtccttgta atggtggtga aggggaggga ggtagtttga atacaacttc acttaatttt 1080
acttccctat tcaggcagga attgccaaac catccaggag tggaatatgc aacctggcgt 1140
catgggccag ctggttaaaa taaaattgat ttctggctta tcacttggca tttgtgatga 1200
tttcctccta caagggatac attttaagtt gagttaaact taaaaaatat tcacagttct 1260
gaggcaataa ccgtggttaa gggttattga tctggaggag ctctgtctaa aaaattgagg 1320
acaggagact ttagacaagg gtgtatttgg agacttttaa gaattttata aaataagggc 1380
tggacgcagt ggcactgagt tgagaactgt tgcttgcttt gcattaaata ggagatcagt 1440
ccctgcagct tgtgggaata aggctttaaa tctctccaat tttagctctg tgagatggca 1500
ctggggaaac agaaatgaac ggactagtgt cacaaagctc aggtgggatg gacgagatca 1560
cttcaaaggt ctgtaatccc acgtctataa tcccagcact ttgggaggcc aaggcgggaa 1620
aatcacttga ggtcaggagt tcgagaccat cctggccaac aatgcaaagc ctgtctctac 1680
taaaaatatg aaaattagct cagcgtggtg gcatgctcct gtagtcccag ctactcgtga 1740
ggctgagaca ggagaatcgt ttgaacctgg gaggcggagg ttgcagtgag ccaatatcac 1800
gccattgcac tccagcctgg ctgacagagt gagactccat ctcaaaaaaa aaaaaaaaaa 1860
aagaatttta taaaatcagg aaataatatt agtgtttatg ttgaatttta actttagaat 1920
catagaaaac ttcctctggc atcattatta gacagctctt gtgcagtggg tagcaccaga 1980
cccagcttgc atggttattg atttttcaga gacacttttt gagcttattc tctggcagaa 2040
aggggaactg cttcctcccc tatctcgtgt ctgcatacta gcttgtcttt acaagaagca 2100
gaagtagtgg aaatgtttat tcttgaaaat aagctttttg cttcacatga tctagaattt 2160
ttaaaattag aaaaatgtgc ttactgcg 2188

<210> 19
<211> 1183
<212> DNA
<213> Homo sapiens

<220>
<221> misc_feature
<222> (1)...(1183)
<223> n = a, t, c, or g

<400> 19

agtaaaatgg agaattccaa attctgaaat tgttagaaca tagttctgtg tcttagttaa 60
atatcgacac ttacagataa atagcataaa tgctttctcc ccatatttca gcccagtcct 120
acttaaagac aacataaatt gcaaaatagt gaggatgttg ttcatctaat aaaagtggtt 180
ccaggaattc agactctgga ttcctgtttg ccaaatcatg tgtcccactc ttaagaaaac 240
gagttggact ntggattttt ctttgcaaga gggacaagag tgtgggagat actgagttaa 300
tgcaacttgc aggttttaag tgtcctgtca ttgtgccttg tgctttgata cattctgagt 360
ttcagtaaag agacctgatg cattggactg ttgcaatgga acctgtttta agatcttcaa 420
agctgtattg atatgaagtt ctccaaaaga cttcaaggac ccagcttcca atcttcataa 480
tcctcttgtg cttgtctctc tttgcatgaa atgcttccag gtatttttgc aaggctacca 540
gttacatttg acaagtctgt gcaatggatc aaaatcagaa gagatgattc aacttggtga 600
ccaagaagtt tctgagcttt gtggcctacc aagggagaaa ctggctgcag cagagcgagt 660
acttcgttcc aacatggaca tcctgaagcc aatcctggtg agtagacttg ctcactggag 720
aaacttcaag cactaatgct ttcggaatgt gaggcttttc cttggacagc atgactttgt 780
tttgtagaaa agtacggctg gctgggagtt tgtgatataa tttagttcag tggtattcta 840
agtgttctta gtgttctttc agactttggg gccatctccc aaagggtgaa tgggaagaat 900
aagctgggtg tggctgagtt taagccaaaa gttttttgtg cttgtttcaa tcagagaaga 960
cctgcttttt catgttttta ctattataat actaagcaag agctcatttg aaaacagagt 1020
tcttcatatt taaaaaaaaa aagtcttgaa accattgatg ggaagatgga tatctattta 1080
tgtttaaaaa cccatcataa agatgacatt gtgggctgtc acagttggaa ggccctggaa 1140
ttagatgaga ccacactatt tagcttactt agtaataaca ttg 1183

<210> 20
<211> 8981
<212> DNA
<213> Homo sapiens

<400> 20
ccgtttggca aatgctcagt aaaagaaaag ggttagaagg ggagaaaggc attttatccc 60
aagccttcag gaatcaggat gaggatgtct tcaccttgtg gtggggagta attatacaat 120
tagagacagc acattggagt gtggctgata tgctgtgtga tgatagctct agctctctgc 180
ctagcagagg aaggacattt caatagaaga aaaagtttaa gaccttgccg agaaacagag 240
aaaggatgtt tgtcttttta agaagttgaa aaccctgttt gcagacaaaa gccctccagt 300
tttggcagta aactttcatg caagggaaga aaaaggcagg ggatgacatt gttgacaatt 360
gtgaggaatt accatgtgcc aggcactgtg cgaggggctt tgtacatatc ctctagtttt 420
agtgcttata aaaactctgt gatatgtgca cagcatttta aactttgctg catagtcgag 480
aaaatggaag gatggggaat ttgagtcatt tgcccagggt tctatagcta ccccaggttc 540
ccatgactgg agaattgggg cacagggtgg cgggggagag tgagtgacaa gaatcctaac 600
aatcttattt ccattgagtc cttataaaag aagtggatta actaccacgt ttttaagttt 660
ttcttaaatt taggttatgt ggatctggcg tttcttgttt tgtcctgggt ttgttttgtt 720
tttgctatgc tgtcttgaac atctgtcatc ttgtaggcct aacggtaaac acaaaaacac 780
tttacctcct atagctttca attaagatct ctcagtttgt gtttgtaata gttttccagg 840
caagttctcc ctaggttcgg cttctagtgt gttaaccttt agttataaag tgaacccaaa 900
gagagaaagt agaaacaaaa cacctcacct gtttttgctc atgaattact ctctatggaa 960
ggaacaatca tgaacacctc tgcgtatcac agaggcctat ctgagtctga cgtttaaggg 1020
agaccgcgta ggtccctttg aggactgtga atgtgggagt cctgggactc tggtgaagaa 1080
cccgttccag aagagatgaa tgagctggac aagttctttc atagaacctt taggcaggtt 1140
ttcttagaaa tgcacattga ggattatgct tggatattgt gatgatcaga atgatactca 1200
atcccttctg catttggaat tctctttgaa agaaaacatc ccaggcagct atttctcaga 1260
gatagtgagt cccagccact tctagacatt ttcttgtgta gtctacatta taatttcaca 1320
gcagtctctg atatgacaaa tgtcaaaata gcccaacctt ctctaaactt cagagatgtc 1380
tgatatgata ttgaataaaa caatgctcat agaaacatca agaaaggtgg attttccctg 1440
gatacttttt tcctgcttga caaataacag tgaagaaact gatctcacgt ctttttctct 1500
ttggaagcct gaacactcag aacccaactt gaggctcctc agctatagca attctgactt 1560
cacagtctgt aaattattgt tctttttttt ctttagctta tgctttctgc cctaatttat 1620
cttttccctg ttctaatgaa ttattgtcct atatctgctg tgcagttagg tgacatataa 1680

cagcaattaa atatatgaat tggtacatat aaagatttga ctaaaactcg atgtaaaaat 1740
aagtgttcta cattcaattt ccagtgttag aaacagtgct gacttgaaca gagtgacaga 1800
attccatctt tccctatttt tgacagcttt aaactttata ttttcttcct ttcttgtgag 1860
ccgtcattaa cttgtttctc aaagccattc ccgtattacc catcttgcag acgcagacag 1920
atttgggaat ttgcggtcag agttgtattg gacacatccc cccagcccac atgagatcct 1980
tttaatctat tgcatattaa ctagttttaa gtacaatatt cctacttcat ttaaaaccat 2040
taatcaaaga atgagtttga aaatgaacaa aatgcaaact tacagttaga aataattgta 2100
gtgtctttag ttttggttag gagtcggttt cttgtttgtt aaactcaaga ttgtgaacag 2160
ttttaattca cttgtttatt tccaatagag atttcaggtt tacatttgaa ttcagaaaca 2220
aagttttctt tctcattaca gagaacacta aactctacat ctcccttccc gagcaaggag 2280
ctggccgaag ccacaaaaac attgctgcat agtcttggga ctctggccca ggaggtaagt 2340
tgtgtctttc cagtaccagg aagcggatca tccactgtat cagtattttc attcctgagt 2400
ctggcaagag gtccttttga gttgaatatc acatggatgg taatatcaat tttcaaagta 2460
taagtgatgt aaacaataat gttttgattt ccttatttta gaaatgaaga aacctaaaac 2520
tcatagatgt ctcagagcta attggttagt ggctaacagc tggatatcta gtttagaacc 2580
ttctccattt tttctttttg cccctaggta atcatacatt tgtaaagagg agaattatct 2640
ctgccactgc ccatgcactg cttttgtctg accagcaatt tctccatatt gcttcttcag 2700
tagcaaggcc aatcatttta ccaacacaca tgcttgctaa ctaacaggaa taacgtggta 2760
cccctaattc agccctttcc cttgaaagca tctggcttct gaggttcaac tatgggaata 2820
tggtctctta atgaacatta agttgagttt gccttttagg tccacatgtt gacaaatgta 2880
tcagagtaat ctctgtccta ggatcagagg gcctgtaggc acttgcaaaa gcagttagct 2940
ctgactccca gccagtgcac actccacctt tctgactccc agccttgtct caaattaggc 3000
ttggaagcga ggaactgtct ggtgtccccc agcataggaa gctgagccag ggggcagtgc 3060
tcacaaacaa tacagacttt aacgtgtagg atattggaaa ataataattt gtggggaaat 3120
tgtctcagac ttggtccacc cttattttta gctgcttctc taatccgttt ttcttttttt 3180
ggtgcttgta tctaacctac ccattttttg gtgcttgcat cattttttca aatatcaaaa 3240
acgaacttta tgttttctaa caatgaaagt attgcatgtt cattgtggaa aatgctgaag 3300
acttggaaaa tacaaaaatg ctgagatcaa acactattga tacgttagtg tatttcttcc 3360
tgtcctgttc tactttcttt ctttgaattc tgctcacgtg tttctgactg atgaggtctg 3420
acttttgggt tccttttcca gaggagaagc cttctttcag cttgccattt gttaccctgg 3480
ttatgaaggc tggtaacctt ttttactagg tagagaagct ggaccaactg gggttcttcc 3540
agggggagaa tgagaaagag aaactgtttt gcaagtccgt agctatttct ctagggccct 3600
gttagctgac attgacatgc cttgcattgc tctgcagatc ccctcgcagc cctctgtccc 3660
ttgttcattt ctggccttag agaaagcaaa gcagggtctg taacagggga ggctgcctct 3720
aaactcaggg tttggttaca gctgttttca cttacatcac tggccctggt tttttttttt 3780
tttctggcat taaaaaaaaa aattggaagc aggtgatgtt cccattgctg atgtggtgga 3840
aactctccaa gtgaacaata tacgtttttc ttggcagctg tttcttgtgc cctgcttgct 3900
cctggtccag gacaagcaag gaccatctgc ctctttcaat agaacacctc cagatccctt 3960
tgatcaaaag ttactcattg tctgacttgc tatttctgtg agataaatgg gagaagatca 4020
ataaatgcac ttgtttgtcc agtcagcgtg tggaaagttg ataattttga ccaaagcaca 4080
accctgaaag gaaaagaaaa agggagtgaa tgtcttctga gaagctgcct aggttcagac 4140
agtgtcaccc atttccctgt atgctccaca tgacaaacct gagtgggtct catcatgtcc 4200
attttgcaga tggcaccaag gctcagaaag gttaggcaac ttttccagtc acccaatgag 4260
ttaattgaca aaactgggat tcaaacccag aactgttgga ttccaaagcc tgtgttgttg 4320
cctgcttcgt gaaaaactcc agtagcgact ggaatagaaa ggagaacctt ccaagaaaga 4380
aaatacgcac tagcagaacc tggaaattgg gaggaaatga ggacttgagg aataagatga 4440
atgaaagctg acctgagttt cacatctggg tgatgggaag ggaggacagg gaggcagcat 4500
ctcagatgtc cacccagcac cgaccagctg cctggcattg ctaggtgttg aggactcagc 4560
agtgaacacg ctaacttctc tgctttcttg gggcacgtat agggtgagag acagaaacaa 4620
acaggtcagt gtacaatgcc acaggaggga tatatgcagt gaagaaaaag cagggtaagg 4680
ggcatagagc atgagaaggt gcttttttta aaggggktga ttaggaaagc tctctctaag 4740
gtgacagttg gacctgaagg agatgatagc atgtctgtgg tgagggaagg aaactccgaa 4800
caggaagaat ggcagataca aagacattga tgctagagca tgcctaagga atgtgtttaa 4860
ggaccaggga aagtgagcaa gtggtggggg gaggagagga gctcagagca ggaggaggtg 4920
agtgccatac aggcctggca agactttgga ttcctgctgg gtgagatgag aatccagcgg 4980

agggcttgag ggaggggaca tgatgtgatc tagagtttag actgtttaca ctctggttgt 5040
tgggttgaga agagactggg atgggggaaa gggaggacaa aggacattgt gctggattga 5100
gaaagcagta agtcagtttc attcattcac tcaaccgatg atgttcaaat accaccatca 5160
tccgtgggct aaaggatgaa gagccatccc tccctgagag tcaggaagca cttcccagat 5220
aaagtttgga gtgtgagctg aggtgtagga gaaagagtaa gagtttaccc ctgaaacggg 5280
tgctgggaag agtcaatagt ttggaataac tcaataattt atggtgcttc tttagaaaga 5340
tttgctggct ttatgtggga agaaatttkt ttttttgatt ggggagtggt gggttggtgg 5400
tgaggctgcc tgtggaaaga gaagtgagtg ttttgactca ctgttattta aaaatctcta 5460
gggctgttcc aataagcaac aaaaggcaaa atggcctggt tctctgtccc ctttctgtct 5520
gtatgcctcg tacaggttat gaaaagaaaa agttgggaaa agctgtccac ctcacctaat 5580
tgtgttcttg tggagtgtgc tagatgcccc ctctctggag aaaaaaaatc cttgtggcct 5640
ctgacccacc tctggagagc ctagttccct tctggaggca gaaggcaaag cttaggacct 5700
agagagtgct ggaccacgcc actcacagga accagcaggc tgtgaggttg aaagctaggc 5760
atatggagct ttccaggctg ggtgcagggc ctcgtggccc ttcccctccc ctctgtgctc 5820
tatagctcag tcttcccagg cggtgtgaac acgcagtgac atttccagga atacagggat 5880
ttattaatga tttcttgtga aatgtttgga aatacaaagt actctataaa tatttcataa 5940
tagcattggg gctgagaact ccacaaagtg ccggaataca tttgcatgta agacagaacg 6000
ctgcctgggt cattgatgcc tgttgagtgg cagtcacaga cactgcctag ggtttctgac 6060
tcacgctgtt gggactgttc tatgcagggc accctcttgt gtggcatagg atttgtgcct 6120
caccacacac tgttgtagct ttgctgtctt gatgatgagt agagggcagt gtccaggcca 6180
tggtataagc atctactgcc ccccagggtt accaaaacca agccaagttg tgtctcagcg 6240
agctccgtga agcatggaga agttgagtac tcagagacat gacgtgactt ttcaaaggct 6300
gtaagctgac gagggacata gctagggttc agacttgagt ttttcttttt cttttctttt 6360
ttcttttttt tttaagactg agtcttgctt ttgtcgccca ggctggattg cagtggtgct 6420
tggctcactg caacctctgc ctcccgggtt caagcaattc tcctgcctca gcctccccag 6480
tagctgggat tacaggcacc tgccaccatg cctggccaac atttttgtat ttttttagta 6540
gagatggggt ttcaccatgt tggccaggct ggtcttgaac tcctgacctc aggtgatcca 6600
cccgcctcga cctcccaaag tactgggatt acaggtgtga gccactgcac ccggcccaga 6660
ctcgagtttt tcatcttaat gctttttcat tgcctgacac tttactgaga ccaagatagg 6720
gaacttcaca tacagtacct tttctcccaa ggcggaagag ggctgttcaa tttctacact 6780
agagttcggg gagttttaga aatgagtcag ttatcgagga tgagagcagt tcctgatagg 6840
ctcaaccaca atgagatgta gctgttcaga gaaagcattc ttttatctat aaactggaag 6900
ataatcccgg tgaaacgaag cccagcccca ggggcttcac taactccagg ctgtgcttct 6960
caaactttag tgagcatagg aatcacctgg gcatcttgtg aagctgtaga tttgaattct 7020
gcaggtcggc agaggggtct cagaatccgc atttccaaca atgtctccag taatgctgat 7080
gctgctcgtc cctggaccac agattgggta gccaggttct ggcaagctca tcccaaggct 7140
ttgagatgac atcagacaaa atatgttctg ggacatggct tttgagaggt caagaaaata 7200
agatgtttct ttctcttctc atccccaacc cttgcactgc ccttttctcc cttcccctac 7260
cctcctttct gtccccatcc ctgacgccag ctgttcagca tgagaagctg gagtgacatg 7320
cgacaggagg tgatgtttct gaccaatgtg aacagctcca gctcctccac ccaaatctac 7380
caggctgtgt ctcgtattgt ctgcgggcat cccgagggag gggggctgaa gatcaagtct 7440
ctcaactggt atgaggacaa caactacaaa gccctctttg gaggcaatgg cactgaggaa 7500
gatgctgaaa ccttctatga caactctaca agtgagtgtc catgcagacc ccagccctgt 7560
ccccaacccc atccctccct tagttctggc cttggcctgt gtcatctcct ccctctgtag 7620
cagcgttaga tgtctacatg cccatttgcc caccagactg agctcttcct agaggagaga 7680
ggcttctctt gaatagctac ctgtccccag ttctctgaat gcagcctggc acatctcagg 7740
tgcacagtag tgtttatcaa tggaatgaat gattgacagc caaccttctg gttttctggg 7800
ggatgtggaa gggtggcttc cagggtgatc aagaatgaga taatggcaga aggacaaatc 7860
ctgcaagatc tcacttatat atggaatata tgtaaggtag aaagtgtcag tttcacatga 7920
tgaataagtt cctgggatct tgatgtacat cgtgatgact atagttagta acactgtata 7980
gtatacttga aatttgctaa gagagtagat ccgaagtgtt cacactacac aaaaaaggca 8040
actatgaggt gatggattta ttaacagctt gattgtggtg atccttttac aaagtataca 8100
tatattaaaa catcacattg tataccttaa atatatacaa tttttatttg tcagttgtaa 8160
ctcaaaaaag ctagaaaagc atttttaaaa aggatgatgt actggtctta atattaccat 8220
tgagataagc tttataataa cataaaaaga aataacagta atgataatag caacaacaac 8280

aacaacaaag aactaacatt taagtagaat ttcttgtgca ctgtgcattc tgtttaagtt 8340
atctcatttt accctcatga taacctgcag ggaagattct ttaaccccac atttcatagg 8400
ctcagagagg ttaagtgcct tggttagagc cacatcagag ttaatccaca agagccagga 8460
ttcaagccca aatctgcctg gatctgtgct ctctaagata actgttagtg gtggcgtgtg 8520
tgttctcaca ctcagacatt tgatctgccc tttgtttccc attcttagct gcaaggcagt 8580
gttaaagaac cctgtgtctc catatccact ccccacactt aagcactttt gtgggcccgt 8640
gtgccgtatg cctcgtggca gcagggatcc aatgtcacag ttttaggcag tggcatcctt 8700
ttccttgaaa acttgatgca ggggaacctt tctccatttc caaccacagg tgtgtctttc 8760
agacactgag tgaggcaggt tttgtacttt attgtaacac aagaaccttt tcttctctgg 8820
agtaaagcac tccagacatt cgcaagttgc tttacaagcc ttaaaaggat ggtattgtag 8880
gcaactttaa ttaaatccca tctcctcctc tcccccagct tgcaagttga cccaaggaag 8940
ccttcatttc catgacagac ttaattgtga gggcatcctc a 8981

<210> 21
<211> 20284
<212> DNA
<213> Homo sapiens

<220>
<221> misc_feature
<222> (1)...(20284)
<223> n = a, t, c, or g

<400> 21
actgtgttag caaggatggt ctcgatctcc tgacctcgtg atccgcctgt atcggcctcc 60
caaagtgctg ggattacagg cgtgaaccac tgcgccctgt tgagaatttt tttttttttt 120
tttgggagaa agagtttcgc tcttgttgcc cgggctagag tgcagtgaca caatctcggc 180
tcactgcaac ctctgcctcc tgggttcaag caattctcct gcctcagcct catgcgtcac 240
cacgcccagc taattttgta tttttagtag agacagggtt tctccatgtt ggtcaggctg 300
gtctcgaact cccaacctca ggtggttcgc ccgccttggc ctcccaaagt gctgggattg 360
caggcatgag ccactgcgcc cagccccaaa ttttggtttt tgcttgaaaa ctgaggtctg 420
aattcagcct tctggttgcc cctcaagagt cagtttaaat gttggtcatg ttagttgtca 480
gtgaaaacaa tggtgaggct ggcatgagag tgtgaatctg gatgggaggg cttgtgcttc 540
atgaaaacat ttttccagat cagctcagtc gtgagttatc cgtcattgac gttataataa 600
gctctgatta tttatcaagc atcattcttt atagatatct cagtttaatc tgagataatc 660
ttctccacat ctctccacat agatgttatg aattttactt ttacagagga gccaactgag 720
gctcagataa gttacttatt atatgactag tagtggtaga gctggggttt caactaagaa 780
ctctctggct ccaaagccct tgtaagtttc tatcagtata tgaccatgca tatgagcatt 840
tgtctctcct cttcttcata gctccttact gcaatgattt gatgaagaat ttggagtcta 900
gtcctctttc ccgcattatc tggaaagctc tgaagccgct gctcgttggg aagatcctgt 960
atacacctga cactccagcc acaaggcagg tcatggctga ggtaagctgc ccccagccca 1020
agactccctc cccagaatct ccccagaact gggggcaaaa aactcaaggt agcttcagag 1080
gtgtgcgcta agtatactca cggctcttct ggaattccca gagtgaaaac ctcaagtctg 1140
atgcagacca gagctgggcc agctccccag tcgtgggtat agaatcatag ttacaagcag 1200
gcatttcttg ggatggggga ggactggcac agggctgctg tgatggggta tcttttcagg 1260
gaggagccaa acgctcattg tctgtgcttc tcctcctttt tctgcggtcc ctggctcccc 1320
acctgactcc aggtgaacaa gaccttccag gaactggctg tgttccatga tctggaaggc 1380
atgtgggagg aactcagccc caagatctgg accttcatgg agaacagcca agaaatggac 1440
cttgtccggg tgagtgtccc tcccattatt accatgtgcc tgcttgatac tggagaggtg 1500
agtttctggt cactttccca ggtgtgagtg aggtgagaat tctttcagtt tatctagctg 1560
ggggaatgta gtgagcatag ctaaagtcac agggcaccac ctctccagaa gtacaggcca 1620
tggtgcagag ataacgctgt gcatatcagc atccatgcca ctcacggtca aatagcagtt 1680
ttctgcaaaa cttagtgagg gctggtgttt ggaagtggag ttgagtaatt gcagtaccct 1740

attttccttt ttgctgcagc ctctcagcca gccacagcat ctccctgtgt cttggtaggt 1800
tttggaaaga agtgtgggag caaaagcatg atgttacatg tagactggcc tgagatactc 1860
attctcaggg cactgtgtga atgatgagct gctgttactg tgtggagggg aaatgcactt 1920
agtgcttcag agccacttga aagggataag tgctctagag acaattgggt tcaaatgtgg 1980
agcaggctga gcaagaacag aatgtctcct ttgcctgagc ctgagtgctg ttaatcacat 2040
cttcctgcct tgggctgagt tagagaatca ttagactatt tcctgtttcc atggtgaggg 2100
aggcctcttc cttttgtctc tgctcccctt aagaagcagg tgaggatttt gccaggtttc 2160
ttgttttgaa ccttattgac tttaagggcg gctgggtttt agagactgta cctacctagg 2220
gggaacactt ccgaagttta ggactattcc ctgatccgct gggaggcagg ttactgagga 2280
agtcccttta aaaacaaagg agtttatact gagaaaagca taaacagtga tttgtatgga 2340
ttcacactga ctaatatagc tcatgccatt aaagtggggt ctcttctcta aaggagggtt 2400
atatgatcta gccccgtaga cctaagtgtg gtttcagacc tgttcttcct ggtcctctcc 2460
ttggaatcca tatttctact agttggactt tttctgtttg tctggctctc agaggattat 2520
aggaggccct gtgaagtgac tcagtgaatt ttgatttgtg ggcaagtaga tggttcccta 2580
gtctgaaatt gactttgcct taggtgcttc aattcttcat aagctcccag ttcttaaagg 2640
acaagatcct tgtaaacatg gcaatggcat tcattaggaa tctagctggg aaaatccagt 2700
gtgtatgctt ggaaatgagg gatctggggc tggagagaaa ggcatgggca tgccttggag 2760
ggacttgtgt gtcaagctga ggacctttac tttaagctct aggggaccag gcaaggggag 2820
atgtagatac gttactctga tggggtggat gaattgaaga aggatgaggc aagaatgaag 2880
gcagagacca gggaggaggc tctccaagtg gccaaggcat aaagcaagaa atgaggcctg 2940
gtgactgctt agtggcagag cagtgaaaga gagggaggca tcaaagtgag tctcgatttc 3000
tagctgggtg ggtggtagcg atgtccagta ggccagtggc tactgaggtc tgcagtggag 3060
gagggtggtt gggctggaga cagatgatga gggagtcatc agcctgtggg tggaagaaaa 3120
gggaacctct tccaactgtt ttctttgctt cttccctctc tttctctttt tttttttttt 3180
tggacagagt cttgctctgt cacccaggct gaaatgcagt ggcatgatct tggctcacca 3240
cagcctccgc ctcctgggtt caagcaattc tcctgtctca gcctccagag tagctgggat 3300
tacaggcaca tatcactgtg cccggctaat tttgtatttt tcagtggaga tgggatttca 3360
ccatgttggt cgggctggaa tgaactcctg acctcaagtg atccacctgc ctcagcctcc 3420
caaagtgttg ggattacagg catgagccac cgcgcccggc ctttcttccc tctcttaaag 3480
agtgtttatt taattccaca aacatgagct tgtcaccccc tgtagcctgg catctcctac 3540
acgaggtgat ggctgaggct tctgcttctg ctggggtagc tctgatcttt ctgctttctc 3600
tggcactgtc tacccatgtt gcctcacccc acaggtccca gggcacctct ctcgggcaag 3660
tcttggaacc ctctgacact gatttgctct cttttctgag ctgcttttag ccacccatcc 3720
tcgggacctg ttttctctct gcctccaccc ctgcgggcag tcttaggtct cctgcccctc 3780
acgagcaccc cagagaggcc acgtgctcag tgatctcagt gggcgcatct ttctagtctt 3840
gctattcttt ttggccatgt tgttcagaaa ccatactggg cagggccgac ttcaccctaa 3900
aggctgcgtc tcttcactct gcttttgttt gttccaaata aagtggcttc agaattgcta 3960
accctagcct ctgtgaactt gtgaggtaca attttgtgtc tgttatgtta acaaaaatac 4020
atacatacct tcctggtgat ggtataaatt gctattctct attggaaagc aatttggaat 4080
gaaaatttaa agaaccattt taaaatatgc tatcctgcgt acctccattc cacccacccc 4140
cagggatgta gcctactgaa ataattttaa agaagtcacc atatgagaga aaatgttatt 4200
gctatattgt tattgtgaga aattggaaat agactaaatg ttcagcacta taggaataat 4260
taatgaaatt acatatactc tatacaatca ttatgctgcc attgaaataa taaatacaaa 4320
ggcgcaaggg gggaaaagct tataatgtta gtgaaactaa gactgatttt tttataaagc 4380
agcagttttc agacccttgg agactccaat tcggtagaac cagagcttca tcttctctgt 4440
cgaagctgtg acaggagttg caaatgcctc tcctttttgc tgagtttgca gctgctgttt 4500
ttccggcagc acatctgtgc aggcctctgc ctcggcccct ctggatctgc tgattgagca 4560
gcggattgat ctgtccttct ctttcgtgtt gacccatgtg aggaaccaac tggcaaggga 4620
acaagaaatg gaaataggcc tcctttgcat catgacctgt acatcctgca attggaaaag 4680
attgtacttt agttggttta accagcagca ttatttttct aaactaagca gtaagaagga 4740
attaggtttt atgtgggatc aacagactgg gtctcaaaag aggaaggtga tagaacacag 4800
tggggagggg gaggtgcact agaaacagag ggcctatgct ttcattctgg ctttgctact 4860
taatagctgt gtgacccaat cttagagact taacctctct gaacttccat tttctcatgt 4920
ataaaatggg aaatattaaa ggatactcac tgggctggtg gcttgtgcct gtaatcccag 4980
cacttgggga ggttgaggtg ggaggatcac ttgagcccag gtgttcaaga ccagcccagg 5040

caacatggca agactctgtc tctatgaaaa aattaaaaat tagccaggtg tggtggtgtg 5100
cacctgtagt cttagctact tggtaggctg agatgggagg atcacttggg cttgggaggt 5160
caaggctgcg gtgagctgtg attccatcac tgcactccag cccgggcggc agagcgagac 5220
actgaatcca aacgacaaca acaacaaaag gcaaaaaaat aaaagtgccc tctttatgga 5280
gttgtgtaag gtgaagcata tacactattc aacatagtaa ctatataaag gaagtattgt 5340
tgttgttact gtagttaata ccattaagtg agatgtttcg tatagtggaa agcacatgga 5400
ctctgaattc agactggtct gactttgagt ctcagctcca catctagtaa tactatgacc 5460
aagccctggt taaaatcatg tttttttttc ttcagcctca gtcttctcac atataaaata 5520
gggacactgt catttacctc agttttctgt gaggataaaa caacgacagt gtatatgcaa 5580
gtattttgta aattttgtag tgctcctcaa gatttagttg gtgtttacta cttgtacttt 5640
ctcactggaa tggcagatgc tgttggacag cagggacaat gaccactttt gggaacagca 5700
gttggatggc ttagattgga cagcccaaga catcgtggcg tttttggcca agcacccaga 5760
ggatgtccag tccagtaatg gttctgtgta cacctggaga gaagctttca acgagactaa 5820
ccaggcaatc cggaccatat ctcgcttcat ggaggtgaat ctgttgctgg gatcatttag 5880
aaaagactta acggcttctt tctctgagac gttacaataa ggttcaggca ggaggcaagt 5940
ttagaaataa tgtatagtct catttacaaa actatccctc aagcctaaca caggatttga 6000
taacaaaagg cacttaataa atgttagttg agtggttgaa tgagtaaata aactctagct 6060
ttagtaaatt aactctagct tattctatat aggctcaaga gaatatttct acccattttc 6120
ttctaggttt tcctatctca gtgactaatg gtagcaaagc attcccttaa aaaggcatta 6180
tttgtgaaac ttayctaaaa tcgaattcgg gtccaattaa atttttgaaa ttttatatta 6240
aaaattatat tagtagggat gggtaagagg tgttttggtc tggttggttg gttagttgct 6300
atgactcaga attgctaaga aaacagaaaa gtaagataag atcattgttt taacctcttt 6360
tcctccacaa aatcaataaa taacatatcc ctaaattact cttagaattt ctcttaaatt 6420
gcagtgaaaa accaaaatcc ttcattcttg gttgaaggtt ggaaaactac gttagagagg 6480
attagagaga gaggatgagc aatcgtgtag tcagcccttg cctcctagtg taggatttgt 6540
ctcagccact gcttgttgtc ctggctgcca acgttctcat gaaggctgtt cttctatcag 6600
tgtgtcaacc tgaacaagct agaacccata gcaacagaag tctggctcat caacaagtcc 6660
atggagctgc tggatgagag gaagttctgg gctggtattg tgttcactgg aattactccm 6720
rgcagcattg agctgcccca tcatgtcaag tacaagatcc gaatggacat tgacaatgtg 6780
gagaggacaa ataaaatcaa ggatgggtaa gtggaatccc atcacaccag cctggtcttg 6840
gggaggtcca gagcacctat tatattagga caagaggtac tttattttaa ctaaaaattt 6900
ggtagaaatt tcaacaacaa caaaaaaact caacttggtg tcatgatttt ggtgaaattg 6960
gtacatgact tgctggaagg tttttcatag gtcataaaat aacagtatct tttgatttag 7020
catttctact caagggaatt aattccagga attttggtgg caggcacctg taatcccagc 7080
tactcgggag gctgaggcag gagaattgct tgaacccagg aggcagaggt tgcagtgagc 7140
taagatcgca tcattgcact cccgcctggg caataagagt gaaactccat ctcaaaaaaa 7200
aaaaagatac aaaaatagaa aaaggggctt ggtaagggta gtagggtttt gggcaatttt 7260
tttttttttt ttttttttta ttgtatggtt ctaaaggaat ggttgattac ctgtggtttg 7320
gttttaggta ctgggaccct ggtcctcgag ctgacccctt tgaggacatg cggtacgtct 7380
gggggggctt cgcctacttg caggatgtgg tggagcaggc aatcatcagg gtgctgacgg 7440
gcaccgagaa gaaaactggt gtctatatgc aacagatgcc ctatccctgt tacgttgatg 7500
acatgtaagt tacctgcaag ccactgtttt taaccagttt atactgtgcc agatgggggt 7560
gtatatatgt gtgtgcatgt gcatgcatgt gtgaatgatc tggaaataag atgccagatg 7620
taagttgtca acagttgcag ccacatgaca gacatagata tatgtgcaca cactagtaaa 7680
cctctttcct tctcatccat ggttgccact tttatctttt tatttttatt tttttttttg 7740
agatggagtc tcgctctgac gcccaggctg gagtgcagtg gctcgatctc ggctcactgc 7800
aacctttgcc tcccgggttc aagctattct cctgcctcag cctccacagt agctgggact 7860
acaggctcat gctgccacgc ccggctgact ttttgtattt tagtagagac gaggtttcac 7920
catgttaccc aggctagact tcaactcctg agctcaggca atccaccctc cttggcctcc 7980
caaagtgctg ggattacagg tgtgagccac tgcacccagc ccaccacttt aattttttac 8040
actctaccct tttggtcaaa atttgctcaa tctgcaagct taaaatgtgt catgacaaac 8100
acatgcaagc acatactcac acatagatgc agaaacagcg tctaaactta taaaagcaca 8160
gtttatgtaa atgtgtgcac ttcttctccc taggtggtaa accacatttc aaaacaaccc 8220
aaataaaact gaacaaagct tcttcctctt agacttttta gaaaatcttt cagtgctgag 8280
tcactaagct gccaagttct cattgtggga actatgcctt tggatgtaat gatttcttct 8340

aagacaatgg gcggaggtgt agttattgca gacatctgaa atatgtaatg tttcttccag 8400
attctggaaa ttctcttatt ctctgtggtt ggtggtggtg gtgggatgtg tgtgtgtgtg 8460
tgtgtgtgtg tgtgtgtgtg tgtgtaggga tcaggatgcg ggaggagctg ggttctgctt 8520
gtattggttc tctgttttgc attgaatagt gtgtttcctt gtatggctat ctatagcttt 8580
tcaaggtcac cagaaattat cctgtttttc accttctaaa caattagctg gaatttttca 8640
aaggaagact tttacaaaga cccctaagct aaggtttact ctagaaagga tgtcttaaga 8700
cagggcacag gagttcagag gcattaagag ctggtgcctg ttgtcatgta gtgagtatgt 8760
gcctacatgg taaagctttg acgtgaacct caagttcagg gtccaaaatc tgtgtgcctt 8820
tttactttgc acatctgcat tttctattct agcttggaat ctgaaacatt gacaagagct 8880
gcctgaaatg tatgtctgtg gtgtgattag agttacgata agcaagtcaa tagtgagatg 8940
accttggaga tgttgaactt ttgtgagaga atgagttgtt tttttgtttt ggtttttagt 9000
actttaacat aatctacctt tagtttaagt atcgctcaca gttacctagt tactgaagca 9060
agcccccaaa gaaatttggt ttggcaacac tttgttagcc tcgtttttct ctctacattg 9120
cattgctcgt gaagcattgg atcatacgta catttcagag tctagagggc ctgtccttct 9180
gtggcccaga tgtggtgctc cctctagcat gcaggctcag aggccttggc ccatcaccct 9240
ggctcacgtg tgtctttctt tctccccttg tccttccttg gggcctccag ctttctgcgg 9300
gtgatgagcc ggtcaatgcc cctcttcatg acgctggcct ggatttactc agtggctgtg 9360
atcatcaagg gcatcgtgta tgagaaggag gcacggctga aagagaccat gcggatcatg 9420
ggcctggaca acagcatcct ctggtttagc tggttcatta gtagcctcat tcctcttctt 9480
gtgagcgctg gcctgctagt ggtcatcctg aaggtaaggc agcctcactc gctcttccct 9540
gccaggaaac tccgaaatag ctcaacacgg gctaagggag gagaagaaga aaaaaaatcc 9600
aagcctctgg tagagaaggg gtcatacctg tcatttcctg caatttcatc catttatagt 9660
tggggaaagt gaggcccaga gaggggcagt gacttgccca aggtcaaccc agccgggtag 9720
cagctaagta ggatgagagt gcagggttca tgctttccag ataaccacat gctcaactgt 9780
gccatgctgt ctcattggta gtggttcatg gcagcatctg aaagctattt attttcttag 9840
atatattggg tggcgattct tcctaagttt ctaagaacaa taatcagaag gatatatatt 9900
gttgcaggtt agactgtctg gaagcagagg ctgaaataga gtttgatgta tgggtattta 9960
tgagggctca atacctatgg aagagatatg gaagatgcag gattgggcag agggaggagt 10020
tgaactgtga tatagggcca accccgtggg gcactctaga gaatatgcag cttgttggag 10080
ttgttcttca tcgagctgaa acatccagcc ctttgtgctc ccccaaggcc tccctcctga 10140
caccacctac ctcagccctc tcaatcaatc actggatgtg ggctgccctg ggaaggtcgt 10200
gccccagggc ctacatggct ctctgctgct gtgacaaacc cagagttgct gatgcctgag 10260
gccgtctact gacagctggg caacaaggct tccctgaatg gggactctgg gcagtgcagt 10320
tttgtgtctg aaccatacat taatatattt atatccgaat ttctttctct tgcaagcatt 10380
tcatataaag acacatcagg taaaaataaa tgtttttgaa gcaaaaggag tacaaagaga 10440
taagaactaa ctaatttaat actagttacc atctgttaca aatagttcct actgattgcc 10500
aaggactgtt taaacacatc acatgggctt cttcttctat cctcactaac ccttttaaca 10560
gacaaggaaa tgaggctcag gaaggtcaag gactttattg aggttccaca gtaggataca 10620
gttcttgcta aaagcaaccc ctccctcatg ctctgttatc taactgcaag gggaaggtca 10680
gtggcagagg tagtggtccc atggttggtg cataagagct gctctgagac aactgcatgc 10740
tggtgggtcc tgcagacatg tacccatcag ccggagatag gctcaaaata tccacaagag 10800
tttggatgat tgtgggaatg cagaatccat ggtgatcaag agggaaagtc aagttgcctg 10860
gccatttccc ttggctttta gacagaaaag ttacgtggga tattatctcc cacagctctt 10920
ctgtggtgcc accagtcata gtccttatat aaggagaaac cagttgaaat tacctattga 10980
agaaacaaag agcaaactcg cccactgaaa tgcgtagaaa gccctggact ctgttgtatt 11040
cataactctg ccattatttt tctgcgtagt tttgggtaag tcacttatct tctttaggat 11100
ggtaatgatc agttgcctca tcagaaagat gaacagcatt acgcctctgc attgtctcta 11160
acatgagtag gaataaaccc tgtctttttt ctgtagatca tacaagtgag tgcttgggat 11220
tgttgaggca gcacatttga tgtgtctctt ccttcccagt taggaaacct gctgccctac 11280
agtgatccca gcgtggtgtt tgtcttcctg tccgtgtttg ctgtggtgac aatcctgcag 11340
tgcttcctga ttagcacact cttctccaga gccaacctgg cagcagcctg tgggggcatc 11400
atctacttca cgctgtacct gccctacgtc ctgtgtgtgg catggcagga ctacgtgggc 11460
ttcacactca agatcttcgc tgtgagtacc tctggccttt cttcagtggc tgtaggcatt 11520
tgaccttcct ttggagtccc tgaataaaag cagcaagttg agaacagaag atgattgtct 11580
tttccaatgg gacatgaacc ttagctctag attctaagct ctttaagggt aagggcaagc 11640

attgtgtttt attaaattgt ttacctttag tcttctcagt gaatcctggt tgaattgaat 11700
tgaatggaat ttttccgaga gccagactgc atcttgaact gggctgggga taaatggcat 11760
tgaggaatgg cttcaggcaa cagatgccat ctctgccctt tatctcccag ctctgttggc 11820
tatgttaagc tcatgacaaa ccaaggccac aaatagaact gaaaactctt gatgtcagag 11880
atgacctctc ttgtcttcct tgtgtccagt atggtgtttt gcttgagtaa tgttttctga 11940
actaagcaca actgaggagc aggtgcctca tcccacaaat tcctgacttg gacacttcct 12000
tccctcgtac agagcagggg gatatcttgg agagtgtgtg agcccctaca agtgcaagtt 12060
gtcagatgtc cccaggtcac ttatcaggaa agctaagagt gactcatagg atgctcctgt 12120
tgcctcagtc tgggcttcat aggcatcagc agccccaaac aggcacctct gatcctgagc 12180
catccttggc tgagcaggga gcctcagaag actgtgggta tgcgcatgtg tgtgggggaa 12240
caggattgct gagccttggg gcatctttgg aaacataaag ttttaaaagt tttatgcttc 12300
actgtatatg catttctgaa atgtttgtat ataatgagtg gttacaaatg gaatcatttt 12360
atatgttact tggtagccca ccactcccta aaggactctt ataggtaaat actacttctg 12420
caccttatga ttgatccatt ttgcaaattc aaatttctcc aggtataatt tacactagaa 12480
gagatagaaa aatgagactg accaggaaat ggataggtga ctttgcctgt ttctcacaga 12540
gcctgctgtc tcctgtggct tttgggtttg gctgtgagta ctttgccctt tttgaggagc 12600
agggcattgg agtgcagtgg gacaacctgt ttgagagtcc tgtggaggaa gatggcttca 12660
atctcaccac ttcggtctcc atgatgctgt ttgacacctt cctctatggg gtgatgacct 12720
ggtacattga ggctgtcttt ccaggtacac tgctttgggc atctgtttgg aaaatatgac 12780
ttctagctga tgtcctttct ttgtgctaga atctctgcag tgcatgggct tccctgggaa 12840
gtggtttggg ctatagatct atagtaaaca gatagtccaa ggacaggcag ctgatgctga 12900
aagtacaatt gtcactactt gtacagcact tgtttcttga aaactgtgtg ccaggcagca 12960
tgcaaaatgt tttatacaca ttgcttcatt taattctcac aaggctactc tgaagtagtt 13020
actataataa ccagcaattt tcaaatgaga gaactgtgac tcaaagacgt taagtaacca 13080
gctttggtca cacaactgtt aaatgttggt acgtggaggt gaatccactt cggttacact 13140
gggtcaataa gcccaggcga atcctcccaa tgctcaccca attctgtatt tctgtgtcct 13200
cagagggggt acaactagga gaggttctgt ttcctgagta caggttgtta ataattaaat 13260
atactagctc taaggcctgc ctgtgattta attagcattc aataaaaatt catgttgaat 13320
ttttctttag tacttctttc ttaatataat acatcttctt gaccaagtcc aagaggaacc 13380
tgcgttggac agttttcata tgagatcaaa ttctgagaga gcaagattta accctttttg 13440
gttcaccttc tgatcctccc ctaaggaggt atacatgaaa tatttattac tcctgcctga 13500
acttctttca ttgaatatgc aattttgcag catgcagatt ctggatttaa attctgagtc 13560
ttaacttact ggctgaggga ccttggatag gctccttatc cctcagtttc ctcatctcta 13620
aaatggggat ggcacctgcc ccgtgggttg ttggaaggac ttacagaggt gcagaatgta 13680
cgttgtacat agcaggtttc agcaaatgtt agctccctct ttccccacat ccattcaaat 13740
ctgttccttc tccaaaggat gtgtcaagga ggaaatggac ctggctggga aaccctcaga 13800
atactgggat gatgctgagc ttggctcata cctgtgcttt gctttcaggc cagtacggaa 13860
ttcccaggcc ctggtatttt ccttgcacca agtcctactg gtttggcgag gaaagtgatg 13920
agaagagcca ccctggttcc aaccagaaga gaatgtcaga aagtaagtgc tgttgacctc 13980
ctgctctttc tttaacctag tgctgctgcc tctgctaact gttgggggca agcgatgtct 14040
cctgcctttc taaaagactg tgaaaccact ccaggggcag agaaatcaca tgcagtgtcc 14100
ctttccaaat cctcccatgc catttatgtc caatgctgtt gacctattgg gagttcacgg 14160
tctcgatccc tgagggacat tttctttgtt gtcttggctt ctagaagagt atcttttact 14220
tgccccctcc caaacacaca tttcatggtc tcctaacaag ctagaagaaa gaggtaaaga 14280
caagcgtgat tgtggaacca tagcctcgct gcctgcctgt gacatggtga cctgtgtatc 14340
agcctgtgtg ggctgagacc aagtggctac cacagagctc agcctatgct tcataatgta 14400
atcattaccc agatccctaa tcctctcttg gctcttaact gcagacagag atgtccacag 14460
ctcatcaaag gctctgcttc tgggttcttt gtgcttagag tggcttccta aatatttaat 14520
aggtcccttt tctgccagtc tcttctgtgc ccatcccctg attgcccttg gtaaaagtat 14580
gatgcccctt agtgtagcac gcttgcctgc tgttcctaat catcttctcc tacctcctct 14640
ttacacctag ctcctgtttc agtcacctag aaatgctcac agtcgctgga atatgtcatg 14700
ttcttccaca cctccatgcc tttgtaggta ctgtttgctc tcacaggaga actttctctc 14760
taacttgcct atcttctcaa ctcctccttt ctctccaaga tctagttccg gatcccctcc 14820
cctgagcatc cctccttggt tctcaggtag tcagtcactc tctgccctga acttccatgg 14880
cacgtgaaag aaaatctttt tattttaaaa caattacaga ctcacaagaa gtaatacaaa 14940

ttacatgagg gggttccctt aaacctttca tccagtttcc ccaatggtag cagcatgtgt 15000
aactgtagaa tagtatcaaa accatgaaat tgacataggt acaattcaca aaccttcttc 15060
agatttcact agctttatgt gcgctcattt gtgtgtgtgt gtgcgtattt agttctatgc 15120
aattttatca tgtgtgaatt catgtaatta ctagctcagt caagctgcag aaatatctca 15180
ttgtcacaaa gctccttcat gctacccctt aatggccaca gccacctccc ttcttcctca 15240
gttcctgaca cctgtcaacc actaatgcgt tcctcgtttt tacagtttta ttatttctag 15300
aatgttacat aaatggaacc atacagtagg tatccttttg atactggctt tttttttttt 15360
ttcactcagc agtattccct tagatctatc caagttgtgt gtgtcaacag ttcattcctc 15420
ttcactgctg agtagtgttc cctgggaggg gtgtatcaca gttccatggc atttttagat 15480
gtatttttta aacagctttc agcatcctct attttaattg ttcatcaagt cctttttccc 15540
aatagactct gaatgctcct ttatcatcgt attcccatca ccaacatcag tacccaaata 15600
ggccctaaat aaacatttat agcctcctgc ctgcctgaga aaccagggtg gacatggaga 15660
gaaggcactt ctgaaagttc aagcgcagtg csctgtgtcc ttacactcca ctcctcagtg 15720
ctttctgtgg gttcatttct gtcttctctc ctgtcacagt ctgcatggag gaggaaccca 15780
cccacttgaa gctgggcgtg tccattcaga acctggtaaa agtctaccga gatgggatga 15840
aggtggctgt cgatggcctg gcactgaatt tttatgaggg ccagatcacc tccttcctgg 15900
gccacaatgg agcggggaag acgaccacca tgtaagaaga gggtgtggtt cccgcagaat 15960
cagccacagg agggttctgc agtagagtta gaaatttata ccttaggaaa ccatgctgat 16020
ccctgggcca agggaaggag cacatgagga gttgccgaat gtgaacatgt tatctaatca 16080
tgagtgtctt tccacgtgct agtttgctag atgttatttc ttcagcctaa aacaagctgg 16140
ggcctcagat gacctttccc atgtagttca cagaattctg cagtggtctt ggaacctgca 16200
gccacgaaaa gatagattac atatgttgga gggagttggt aattcccagg aactctgtct 16260
ctaagcagat gtgagaagca cctgtgagac gcaatcaagc tgggcagctg gcttgattgc 16320
cttccctgcg acctcaagga ccttacagtg ggtagtatca ggaggggtca ggggctgtaa 16380
agcaccagcg ttagcctcag tggcttccag cacgattcct caaccattct aaccattcca 16440
aagggtatat ctttgggggg tgacattctt ttcctgtttt ctttttaatc tttttttaaa 16500
acatagaatt aatatattat gagcttttca gaagattttt aaaaggcagt cagaaatcct 16560
actacctaac acaaaaattg tttttatctt tgaataatat gttcttgttt gtccattttc 16620
catgcatgcg atgttaggca tacaaaatac attttttaaa gaatactttc attgcaaatt 16680
ggaaacttcg tttaaaaaat gctcatacta aaattggcat ttctaaccca taggcccact 16740
tgtagttatt taccgaagca aaaggacagc tttgctttgt gtgggtctgg tagggttcat 16800
tagaaaggaa tgggggcggt gggagggttg gtgttctgtt ctctctgcag actgaatgga 16860
gcatctagag ttaagggtag gtcaaccctg acttctgtac ttctaaattt ttgtcctcag 16920
gtcaatcctg accgggttgt tccccccgac ctcgggcacc gcctacatcc tgggaaaaga 16980
cattcgctct gagatgagca ccatccggca gaacctgggg gtctgtcccc agcataacgt 17040
gctgtttgac atgtgagtac cagcagcacg ttaagaatag gccttttctg gatgtgtgtg 17100
tgtcatgcca tcatgggagg agtgggactt aagcatttta ctttgctgtg tttttgtttt 17160
ttcttttttt ctttttattt tttttgagat ggagtctcgc tctgtagcca ggctggactg 17220
tagtggcgcg atctcggctc actgcaacct tggcctccca ggttcaagcg attctcctgc 17280
ctcagcctcc cgagtagctg ggactctagg cacacaccac catgcccagc taatttttgt 17340
gtttttagta gagacggggt ttcaccatgt tggccaggat ggtctcaatg tcttgacctc 17400
gtgatccgcc cacctcggtc tcccaaagtg ctgggaacac aggcatgagc cactgtgtct 17460
ggccacattt tactttcttt gaatatggca ggctcacctc cgtgaacacc ttgagaccta 17520
gttgttcttt gattttagga gaagtgggag gtgaatggtt gagctgtaga ggtgacatca 17580
gcccagccag tggatggggg cttgggaaac attgcttccc attattgtca tgctggaggg 17640
ccctttagcc catcctctcc ccccgccacc ctccttattg aggcctggag cagacttccc 17700
agacctggta gtgcttcagg gccctggtat gatggaccta tatttgctgc ttaagacatt 17760
tgctcccact caggttgtcc catcagccat aaggccccca gggagcccgt gtgatggagc 17820
agagagagac ctgagctctg caatcttggg caaggctttt cccttatgtt tcttcttatc 17880
taaagtgaac agctggggct catgtgctcc ctcctcatct aaagtgaaca catggggctc 17940
atgtgcaggg tcctccccgc tttcagagcc tgaggtcccc tgaggctcag gaaggctgct 18000
ccaggtgagt gccgagctga cttcttggtg gacgtgctgt ggggacagcc cattaaagac 18060
cacatcttgg ggccctgaaa ttgaaagttg taactgcctg gtgcatggtg gccaggcctg 18120
ctggaaacag gttggaagcg atctgtcacc tttcactttg atttcctgag cagctcatgt 18180
ggttgctcac tgttgttcta ccttgaatct tgaagattat ttttcagaaa ttgataaagt 18240

tattttaaaa agcacgggga gagaaaaata tgcccattct catctgttct gggccagggg 18300
acactgtatt ctggggtatc cagtagggcc cagagctgac ctgcctccct gtccccaggc 18360
tgactgtcga agaacacatc tggttctatg cccgcttgaa agggctctct gagaagcacg 18420
tgaaggcgga gatggagcag atggccctgg atgttggttt gccatcaagc aagctgaaaa 18480
gcaaaacaag ccagctgtca ggtgcggccc agagctacct tccctatccc tctcccctcc 18540
tcctccggct acacacatgc ggaggaaaat cagcactgcc ccagggtccc aggctgggtg 18600
cggttggtaa cagaaacttg tccctggctg tgcccctagg tcctctgcct tcactcactg 18660
tctggggctg gtcctggagt ttgtcttgct ctgttttttt gtaggtggaa tgcagagaaa 18720
gctatctgtg gccttggcct ttgtcggggg atctaaggtt gtcattctgg atgaacccac 18780
agctggtgtg gacccttact cccgcagggg aatatgggag ctgctgctga aataccgaca 18840
aggtgcctga tgtgtattta ttctgagtaa atggactgag agagagcggg gggctttgga 18900
gaagtgtggc tgtatctcat ggctaggctt ctgtgaagcc atgggatact cttctgttak 18960
cacagaagag ataaagggca ttgagactga gattcctgag aggagatgct gtgtctttat 19020
tcatcttttt gtccccaaca tggtgcacta aatttatggt tagttgaaag ggtggatgct 19080
taaatgaatg gaagcggaga ggggcaggaa gacgattggg ctctctggtt agagatctga 19140
tgtggtacag tatgaggagc acaggcaggc ttggagccaa ctctggcttg gccctgagac 19200
attgggaaag tcacaacttg cctcaccttc tttgccgata ataatagtgg tgcgttacct 19260
catagaggat taaattaaat gagaatgcac acaaaccacc tagcacaatg cctggcatat 19320
agcaagttcc caaataaaat gcgtactgtt cttacctctg tgaggatgtg gtacctatat 19380
atacaaagct ttgccattct aggggtcata gccatacagg gtgaaaggtg gcttccaggt 19440
ctcttccagt gcttacccct gctaatatct ctctagtccc tgtcactgtg acaaatcaga 19500
actgagaggc ctcacctgtc ccacatcctt gtgtttgtgc ctggcaggcc gcaccattat 19560
tctctctaca caccacatgg atgaagcgga cgtcctgggg gacaggattg ccatcatctc 19620
ccatgggaag ctgtgctgtg tgggctcctc cctgtttctg aagaaccagc tgggaacagg 19680
ctactacctg accttggtca agaaagatgt ggaatcctcc ctcagttcct gcagaaacag 19740
tagtagcact gtgtcatacc tgaaaaaggt gagctgcagt cttggagctg ggctggtgtt 19800
gggtctgggc agccaggact tgctggctgt gaatgatttc tccatctcca ccccttttgc 19860
catgttgaaa ccaccatctc cctgctctgt tgcccctttg aaatcatatc atacttaagg 19920
catggaaagc taaggggccc tctgctccca ttgtgctagt tctgttgaat cccgttttcc 19980
ttttcctatg aggcacanag agtgatggag aaggtcctta gaggacatta ttatgtcaaa 20040
gaaaagagac ttgtcaagag gtaagagcct tggctacaaa tgacctggtc gttcctgctc 20100
attacttttc aatctcattg accttaactt ttaaactata aaacagccaa tatttattag 20160
gcactgattt catgccagag acactctggg cattgaaaga aagtaatgat aatagttaat 20220
tttatatagc gttgttacca tttcaacctt tttttttttt taacctctat catctcaatt 20280
aaag 20284

<210> 22
<211> 7052
<212> DNA
<213> Homo sapiens

<400> 22
   gtgaacacac attaaagcat gagaagcatg aactagacat gtagccaggt aaaggccttg 60
   ctgagatggt tggcaaaggc ctcattgcag cattcattgg caggccacag ttcttttggc 120
   agctctgctt cctgaccttt caccctcagg aagcgaggct gttcacacgg cacacacatg 180
   ccagacaggg tcctctgaag ccacggctgc cagtgcatgt gtcccaggga aagctttttc 240
   ctttagttct cacacaacag agcttcttgg aagccctccc cggcgaaggt gctggtggct 300
   ctgccttgct ccgtccctga cccgttctca cctccttctt tgccatcagg aggacagtgt 360
   ttctcagagc agttctgatg ctggcctggg cagcgaccat gagagtgaca cgctgaccat 420
   cggtaaggac tctggggttt cttattcagg tggtgcctga gcttccccca gctgggcaga 480
   gtggaggcag aggaggagag gtgcagaggc tggtggcgct gactcaaggt ttgctgctgg 540
   gctggggctg ggtggctgcg ggggtgggag cagcttggtg gcgggttggc ctaatgcttg 600
   ctggggtgcc tggggctcgg tttgggagct agcagggcag tgtcccagag agctgagatg 660
   attggggttt ggggaatccc ttaggggagt ggacactgaa taccagggat gaggagctga 720
   gggccaagcc aggagggtgg gatttgagct tagtacataa gaagagtgag agcccaggag 780
   atgaggaaca gccttccaga tttttcttgg gtagcgtgtg taggaggcca gtgtcaccag 840
   tagcatatgt ggaacagaag tcttgaccct tgctatctct gcctagtcct aatggctggc 900
   ttttcccagg aaggcttctg cttccatgga ctgttagatt aaccctttat ttaggtaaat 960
   gagggaacct actttataag cataggaaag ggtgaagaat cttttaagat tcctttactc 1020
   aagttttctt ttgaagaatc ccagagctta ggcaatagac accagacttt gagcctcagt 1080
   tatccattca cccatccacc cacccaccca cccatccttc catcctccca tcctcccatt 1140
   cacccatcca cccatccagc tgtccaccca ttctacactg agtacctata atgtgcctgg 1200
   ctttggtgat acaaaggtga ataagacata gtcctttcct ttgcccccaa ccctcagacc 1260
   agagatgaac atgtggaatg acctaaacac ctggaacagg tgtggtgtat gagcggcagg 1320
   cctctgatga gagggtgggg gatggccagc cctcactccg aagcccctct gagttgattg 1380
   agccatcttt gcattctggt cctgcagatg tctctgctat ctccaacctc atcaggaagc 1440
   atgtgtctga agcccggctg gtggaagaca tagggcatga gctgacctat gtgctgccat 1500
   atgaagctgc taaggaggga gcctttgtgg aactctttca tgagattgat gaccggctct 1560
   cagacctggg catttctagt tatggcatct cagagacgac cctggaagaa gtaagttaag 1620
   tggctgactg tcggaatata tagcaaggcc aaatgtccta aggccagacc agtagcctgc 1680
   attgggagca ggattatcat ggagttagtc attgagtttt taggtcatcg acatctgatt 1740
   aatgttggcc ccagtgagcc atttaagatg gtagtgggag atagcaggaa agaagtgttt 1800
   tcctctgtac cacagtacat gcctgagatt tgtgtgttga aaccagtggt acctaacaca 1860
   tttacatccc aaccttaaac tcctatgcac ttatttaccc tttaatgagc ctctttactt 1920
   aagtacagtg kgaggaacag cggcatcagg atcacttggg aacttgttag aaattcagca 1980
   acttgggccc agctcagacc tactgaatca gaatcaggag caattctctg gtgtgactgt 2040
   gtcacagcca ggtatcaact ggattctcat acataggaaa tgacaaacgt ttatggatgg 2100
   atagtctact tgtgccaggt gctgagattt gtttttgttt ttttgatttt tttttaatca 2160
   ctgtgacctc atttaattct caaaaaaaga tgaaaaaatg aacactcagg aatgctgaca 2220
   tgagattcag aatcaggggt ttggggcttc aaagtccatc ctctctttat ccatgtaatg 2280
   cctcccctta gagatacaac atcacagacc ttgaaggctg aaggggatat aaaagctgtc 2340
   tggccaagtg gtctccaagc ttgacagtgc agcagaatca cctggggata ttattaaaaa 2400
   taaacatact aaggtttggc ttcagggcct gtgaatcaga atttctggag gtgaggcctt 2460
   gaagtctgta tttctattgc atactttgga cacagtggtc tatagactag agtttggaaa 2520
   tgattgcgct cattcagatt ctcttctgat gtttgaattg ctgccatcat atttctagtg 2580
   ctctatttcc tcctgctcat tctgtcttgg ataacttatc atagtactag cctactcaaa 2640
   gatttagagc cacagtcctg aaagaagcca cttgactcat tccctgtagg ttcagaataa 2700
   atttcttctg cgcagtgtct gtcatagctt tttttaaatt tttttttatt tttgatgaga 2760
   ctggagtttt gctcttattg cccaagctgg agtgcagtgg tgcgattttg gctcactgca 2820
   acctccacct cccaggttca agcgattctc ctgcctcagc ctcccaagta gctgagatta 2880
   caagcatgtg ctaccacgcc cagctaattt tgtattttta gtagagatgg ggtttatcca 2940
   tgttggtcag gctggtctcg agctccagac ctcaggtgat ctgcccgcct cggcctccca 3000
   aagtgctggg attataggcc tgagccacag cgctcagcca taactttaat ttgaaaatga 3060
   ttgtctagct tgatagctct caccactgag gaaatgttct ctggcaaaaa cggcttctct 3120
   cccaggtaac tctgagaaag tgttattaag aaatgtggct tctactttct ctgtcttacg 3180
   gggctaacat gccactcagt aatataataa tcgtggcagt ggtgactact ctcgtaatgt 3240
   tggtgcttat aatgttctca tctctctcat tttccagata ttcctcaagg tggccgaaga 3300
   gagtggggtg gatgctgaga cctcaggtaa ctgccttgag ggagaatggc acacttaaga 3360
   tagtgccttc tgctggcttt ctcagtgcac gagtattgtt cctttccctt tgaattgttc 3420
   tattgcattc tcatttgtag agtgtaggtt tgttgcagat ggggaaggtt tgttttgttg 3480
   taaataaaat aaagtatggg attctttcct tgtgccttca gatggtacct tgccagcaag 3540
   acgaaacagg cgggccttcg ggacaagcag gagctgtctt cgcccgttca ctgaagatga 3600
   tgctgctgat ccaaatgatt ctgacataga cccaggtctg ttagggcaag atcaaacagt 3660
   gtcctactgt ttgaatgtga aattctctct catgctctca cctgttttct ttggatggcc 3720
   tttagccaag gtgatagatc cctacagagt ccaaagagaa gtgaggaaat ggtaaaagcc 3780
   acttgttctt tgcagcatcg tgcatgtgat caaacctgaa agagcctatc catatcactt 3840
   cctttaaaga cataaagatg gtgcctcaat cctctgaacc catgtattta ttatcttttc 3900
   tgcggggtcc tagtttcttg tatacattag gtgtttaatt gttgaacaaa tattcattcg 3960
   agtagatgag tgattttgaa agagtcagaa aggggaattt gctgttagag ttaattgtac 4020
   cctaagactt agatatttga ggctgggcat ggtggctcat gccagtaatc ccagcgcttt 4080
   gagaggctga ggtgggtaga tcacctgagg tcaggagttt gagaccagtc tgaccaacaa 4140
   ggtgaaaccc cgtctctact aaatacaaaa aattagccga gtgtggtggc acatgcctgt 4200
   catcccagct acttgggagg ctgaggcagg agaatcgctt gaacccagga ggcagaggtt 4260
   gcagtcagcc acggttgcgc cattgcactc cagactgggc aacaagagtg aaaactccat 4320
   ctcaaaaaag aaaaaaaaag aattagatat tttggatgag tgtgtctttg tgtgtttaac 4380
   tgagatggag aggagagcta agacatcaaa caaatattgt taagatgtaa aagcacatca 4440
   gttaggtatc attagtttag gacaaggatt tctagaaaat ttttaggaac agaaaacttt 4500
   ccagttctct cacccctgct caaagagtgt atggctctta cattatatat aactgcctga 4560
   cttcatacag tatcagtact tagatcattt gaaatgtgtc cacgttttac caaaatataa 4620
   tagggtgaga agctgagatg ctaattgcca ttgtgtattc tcaaatatgt caagctacgt 4680
   acatggcctg tttcatagag tagtctataa gaaattgatg acttgattca tccgaatggc 4740
   tggctgtaac acctggttac gcatgaacac ctcttttcag ttgtctcaag acacctttct 4800
   tttctgtact tatcagacaa ggactgaaag gcagagactg ctactgttag acattttgag 4860
   tcaagctttt ccttggacat agctttgtca tgaaagccct ttacttctga gaaacttcta 4920
   gcttcagaca catgccttca agatagttgt tgaagacacc agaagaagga gcatggcaat 4980
   gccgaaaaca cctaagataa taggtgacct tcagtgttgg cttcttgcag aatccagaga 5040
   gacagacttg ctcagtggga tggatggcaa agggtcctac caggtgaaag gctggaaact 5100
   tacacagcaa cagtttgtgg cccttttgtg gaagagactg ctaattgcca gacggagtcg 5160
   gaaaggattt tttgctcagg tgagacgtgc tgttttcgcc agagactctg gcttcatggg 5220
   tgggctgcag gctctgtgac cagtgaaggc aggatagcat cctggtcaag atatggatgc 5280
   cggagccaga tttatctgta tttcaatccc agttctattc cttgccagtt gtgtatccgc 5340
   tggcaagtta cttctctatg cctcaatctc ctcatctgta aaatggggat aataatatta 5400
   cctgcaatac agggttgtta cgaaaataaa aatgaatagg tgcttagaat ggggcctgac 5460
   attagtaagt gcttagtttt gtgtgtgtat atgttatttt tattttggag gagaacataa 5520
   aaaggacaaa gtgtagaaaa actggttggg tgtattcagc tgtcataaca tgagagttgt 5580
   tatgcccaga tgcacttgac atgtgaattt attagaaaca tgatttttct ctgagttgat 5640
   gtttaactca aactgataga aaagataggt cagaatatag ttggccaaca gagaagactt 5700
   gttagactat tgtctgcatg tcagtgtttg catgctaact tgcttagtta gaaaggttaa 5760
   attttttcac tctataaaat caagaaatat agagaaaagg tctgcagaga gtctttcatt 5820
   tgatgatgtg gatattgtta agagcgggag tttggagcat acagagctca agttgaatcc 5880
   tgactttgct acttattggc tatatgacct tgggcaagct gcttagtctc tctgatcctc 5940
   agttaccttt gtttgttgat gatgaccatt gataacacaa ccataaataa tgacaacata 6000
   gagatagttc tcattatagt agttgttata cagaattatt cactcaatgt taattttctg 6060
   cattgaaatc ccagaacatt agaattgggg gcattatttg aatctttaag gttataagga 6120
   atacatttct cagcaataaa tggaaggagt tttgggttaa cttataaagt atacccaagt 6180
   catttttttt cagagaagat atggtagaaa gtcttaggag gttgaagaag gaattggata 6240
   tttattcttt ctgagactat catgggagat aatgactatg gttgtccatg attggagccg 6300
   ttgctgtaga gttggtttta ttatagtgta ggatttgaat gggccatgtg ttctcagacc 6360
   tcagaataaa aagagaaaac tgaggccagt ggggagcgtg acttcacatg ggtacacttg 6420
   tgctagagac agaaccagga ttcaggactt ctggctcctg gtcctgggtt catggcccaa 6480
   tgtagtcttt ctcagtcttc aggaggagga agggcaggac ccagtgttct gagtcaccct 6540
   gaatgtgagc actatttact tcgtgaactt cttggcttag tgcctctgcc aggtggccat 6600
   aacctctggc cttgtgttgc cagagaaaag gtttagtttt caggctccat tgcttcccag 6660
   ctgccaagaa tgccttggtg cagcacagtc ataggccctg cattcctcat tgccgtgctg 6720
   gttggtcggg gaggtgggct ggactcgtag ggatttgccc cttggccttg tttctaacac 6780
   ttgccgtttc ctgctgtccc cctgccccct ccactgcctg ggtaaagatt gtcttgccag 6840
   ctgtgtttgt ctgcattgcc cttgtgttca gcctgatcgt gccacccttt ggcaagtacc 6900
   ccagcctgga acttcagccc tggatgtaca acgaacagta cacatttgtc aggtatgttt 6960
   gtcttctaca tcccaggagg gggtaagatt cgagcagacc aaagatgttt acgagggcca 7020
   agggaatgga cttcagaatt acacggtgga at 7052

<210> 23
<211> 2534
<212> DNA
<213> Homo sapiens

<400> 23
gggaagcatt taaaaaaaaa aaagtatata tatatatata tatatatata tgtaatgtga 60
attggcctct ttttctctaa gcccacattt tcttcttaca tagttcaggt ttactttatt 120
ttttcctttc cggctgctga ccctgtattg cccgtagttg tggaacatag catgtgtttg 180
tgacctgtgc ctgttatttt tgtgctttct agttgtgcat gcaaagagta caaagttttc 240
ttgccctttc ttggaaaatc ctgcttgtct gtgccaaagg gataattgtg aaagcacttt 300
tgaaatactt aatgagttga ttttcttcaa attaaaaaaa atatataaat gtatatgtgt 360
atgtacatgt gtgtacacat acacaccttt atacatacag cccatttaaa acaagctcca 420
ctttggagtg ctctacgtca ccctgatgcc gaatacaggg ccagagtctg agatccttct 480
gggtggtttc tgtgttttgt tcatttctgt tttaagagcc tgtcacagag aaatgcttcc 540
taaaatgttt aatttataaa aacattttta tctctcgatt actggtttta atgaattact 600
aagctggctg cctctcatgt acccacagca atgatgctcc tgaggacacg ggaaccctgg 660
aactcttaaa cgccctcacc aaagaccctg gcttcgggac ccgctgtatg gaaggaaacc 720
caatcccgtg agtgccactt tagccataag cagggcttct tgtgcttgtt gcctggtttg 780
atttctaata tgctgcattt atcaactgca tgccacattg tgaccgccag catttgccct 840
ttgaattatt attatgtttt atttacaaaa agcgaaggta gtaaccgaac taaattatct 900
aggaacaaac gtttggagag tcttctaaca ccgyscaaag cacgtcatta cagacatttg 960
tttactgatt tagaacctta atatttaatt taaatacgca ctttacactt actgatgaaa 1020
tgcttttcct ttctttctct cccagcccct gtacttaagt gcttcaatag gctctcatta 1080
tatatgattt ttaggttttg cttatcagct tcttcgcttt tataatctga aaagatggca 1140
tatgaatttt tataaaaagg gacactttct tcttctcaaa ttgtatattt ttattgtact 1200
ttccttcaaa accccctttt aaaaagtaag cagtggataa ataaattcag tgaagcatcc 1260
atatgaccct taagtgagtg taggggaagg gaggtcacca gatcactgtg agtgaagatg 1320
gtggagaggt gaggatctta tgaggccgtg ctcaaggctg gtagaggtgg gttagtgttt 1380
ccaggtttag gcagaatctc agctgaggtc atgaaacaac agtgatctct gaaaaattat 1440
ggcaaggtgg gaaggtgctg gagaattgga gagggggcaa acttgacttt caagtttcaa 1500
tgggaagata ggtgactctg cacaccacag aacagtgagc atgataacct gtttatacaa 1560
ggttctagag cagatttcta aatggatagc tactgtgtgc ttgtttgttc ttaattagta 1620
ttggatagtt actaaatact tgttagtact tagtacataa tgggtggtaa atcctagcag 1680
ctaatattgg ttcccaaata accagatgac aaggatagag aaggacacag acacggccta 1740
tctggatttc atggtgcctt tgattttcca catgaaggtt gtgtagggaa gatagaagca 1800
tgagatgaga tgataatata gttatctgga ttcatcactg gccagctgaa ccatatgaac 1860
tcatggattg atgctagctt aggaaggctc tgtaggagcc agaactgggc tgagagccag 1920
cccatagaga caaaagaggc ccggccctga catcagaggg ttcaaacatg atgtctgagc 1980
cccacctaca gtctgccgga ggtggttgga aggaagagcc tttatcctta caattcttac 2040
tgaaattcaa atttttaggt tttgcaaaaa aatggtggac ctgaaggaaa tttgacagga 2100
gcatgtctca gctgtattta aatttgtctc agccaatccc cttttgaatg ttcagagtgt 2160
aagcttcagg agggcagcgc gtcttagtgt gacttttctg gtcagttcag gtgctttaag 2220
gagacaatta gagatcaatc tggaaaactt catttgaatt tttaatacat aagaaaacaa 2280
taagaaatag ttaaaaatat atatttatat aatatatata tgtgtgtgtg tgtgtgtgtg 2340
tgtgtgtgtg tatatatata tatattttat ttatttattt ttttttgaga tggagtctcg 2400
ctctgttgcc caggctggag tgcagtggct caatcttggc tcactgccac ctctgcctcc 2460
caggttcaag tgattctcct acctcagcct cctgagtagc tgggattaca agcatgtgcc 2520
accacactgg ctaa 2534

<210> 24
<211> 2841
<212> DNA
<213> Homo sapiens

<400> 24
tcttgccagt ctctactcat ttttcagcac atcgagcata agatccagac tctttcccag 60
gcctctctca tctggctcct ctcctcctcc tttatcatta ctcttcttcg tagcttatcc 120
tactccagcc atgctgtctt cctattattc ctaaaaarta gaaatgcatt tcttcctagg 180
gcctttgtac ctgcacttgc catcgctttt gctcagaatg ttctttttgc caagcttttg 240
cccagcttgt tctccatcat tgttatgttt tggctgaaat gtcttctctt agtaggttca 300
ttctccccag tcactgtctt tttattttgc tttattttgg gccatctaag gttatcttat 360
tagtgtattt gttgttcgtc tcctccatgg gcatacacct ccatgaaggc aggtattttc 420
accttaggcc ctcgaatata ctggacagca tctggcacgt agtagatgct caacgaatgt 480
ttgttgtgtg agcaaatggt tggttgattg gattgaactg agttcagtat gtaaatattt 540
agggcctctt tgcattctat tttacttatg tataaaatga tacataatga tgatataaat 600
gatgtcacag tgtacaaggc tgttgtggga tcaagcaatc aaatgagatc atgcttgtct 660
tttccaaatg gtgagggaat agatgcatgt ttgtggttgt tacggaatga tcctgtgctc 720
ctgaggcaac agaaaggcca ggccatctct ggtaatccta ctcttgctgt cttccctttg 780
cagagacacg ccctgccagg caggggagga agagtggacc actgccccag ttccccagac 840
catcatggac ctcttccaga atgggaactg gacaatgcag aacccttcac ctgcatgcca 900
gtgtagcagc gacaaaatca agaagatgct gcctgtgtgt cccccagggg caggggggct 960
gcctcctcca caagtgagtc actttcaggg ggtgattggg cagaaggggt gcaggatggg 1020
ctggtagctt ccgcttggaa gcaggaatga gtgagatatc atgttgggag ggtctgtttc 1080
agtctttttt gtttttgttt tttttttctg aggcggagtc ttgctctgtc gcccaggctg 1140
gagtgctgtg gcatgatctt gcctcactgc aacctccacc tcccaggttc aagcgattct 1200
cctgcctcag cctcctgagt agctgggatt acaggcacgc accaccatgt ctggctaatt 1260
tttgtgtttt tagtagagat agggtttcgc cgtgttggct aggctggtct ggaattcctg 1320
acctcaggtg atccacccgc ctcggcctcc caaagtgctg ggattacagg cgtgagccac 1380
tacgcccagc cctgtttcag tctttaactc gcttcttgtc ataagaaaaa gcatgtgagt 1440
tttgagggga gaaggtttgg accacactgt gcccatgcct gtcccacagc agtaaagtca 1500
caggacagac tgtggcaggc ctggcttcca atcttggctc tgcaacaaat gagctggtag 1560
cctttgacag gcctgggcct gtttcttcac ctctgaatta gggaggctgg accagaaaac 1620
tcctgtggat cttgtcaact ctggtattct tagagactct gtttgggaag gagtcctgag 1680
ccattttttt tttcttgaga atttcaggaa gaggagtgct tatgatagct ctctgctgct 1740
tttatcagca accaaattgc aggatgagga caagcaattc taaatgagta caggaactaa 1800
aagaaggctt ggttaccact cttgaaaata atagctagtc caggtgcggg gtggctcaca 1860
cctgtaatct cagtattttg ggatgccgag gtggactgat cacctaaggt caggagttcg 1920
aaaccagctt ggccaatgtg gcgaaaccct gtctctacta aaaattcaaa aattagccag 1980
gcatggtggc acatgcctgt aatcccagtt acttgggagg ctgaagcagg agaattgctt 2040
gaacctggga ggtggaggtc gcagggagcc aaaattgcgc cactgtactc cagcctgagc 2100
aacacagcaa aactccatat caaaaaataa aatgaataaa ataacagcta atctagtcat 2160
cagtataact ccagtgaaca gaagatttat taggcatagt gaatgatggt gcttcctaaa 2220
aatctcttga ctacaaagaa tctcatttca atgtttattg tttagatgtt cagaataaat 2280
tcttgggaaa gaccttggct tggtgtaagt gaattaccag tgccgagggc agggtgaacc 2340
aagtctcagt gctggttgac tgagggcagt gtctgggacc tgtagtcagg tttccggtca 2400
cactgtggac atggtcactg ttgtccttga tttgttttct gtttcaattc ttgtctataa 2460
agacccgtat gcttggtttt catgtgatga cagagaaaac aaaacactgc agatatcctt 2520
caggacctga caggaagaaa catttcggat tatctggtga agacgtatgt gcagatcata 2580
gccaaaaggt gactttttac taaacttggc ccctgcctta ttattactaa ttagaggaat 2640
taaagaccta caaataacag actgaaacag tgggggaaat gccagattat ggcctgattc 2700
tgtctattgg aagtttagga tattatccca aactagaaaa gatgacgaga gggactgtga 2760
acattcagtt gtcagcttca aggctgaggc agcctggtct agaatgaaaa tagaaatgga 2820
ttcaacgtca aattttgcca c 2841

<210> 25
<211> 852
<212> DNA
<213> Homo sapiens

<400> 25
gcatgctgga gtgatagtga ccatgagttt ctaagaaaga agcataattt ctccatatgt 60
catccacaat tgaaatatta ttgttaattg aaaaagcttc taggccaggc acggtggctc 120
atgcctgtaa tcccagcact ttaggagcca aggcgggtgg atcacttgag gtcaggagtt 180
tgagaccagc ctggccaaca tggggaaacc ctgtctctac taaaaataca aaataagctg 240
ggcgtggtgg tgcgtgcctg taatcccagc tacttgggag gctgaggcag gagaactgct 300
tgaatctggg aggcggaggt tgcagtgagc tgagttcatg ccattgcatt ccagcctggg 360
caacaagagc gaaaccatct cccaaaagaa aaaaaaaaga aagaaaaagc ttctagtttg 420
gttacatctt ggtctataag gtggtttgta aattggttta acccaaggcc tggttctcat 480
ataagtaata gggtatttat gatggagaga aggctggaag aggcctgaac acaggcttct 540
tttctctagc acaaccctac aaggccagct gattctaggg ttatttctgt ccgttcctta 600
tatcctcagg tggatattta ctccttttgc atcattagga ataggctcag tgctttcttt 660
gaactgattt tttgtttctt tgtctctgca gcttaaagaa caagatctgg gtgaatgagt 720
ttaggtaagt tgctgtcttt ctggcacgtt tagctcaggg ggaggatggt gttgtaggtg 780
tgcttggatt gaagaaagcc ttggggattg tttgtcactc acacacttgt gggtgccatc 840
tcactgtgag ga 852

<210> 26
<211> 6289
<212> DNA
<213> Homo sapiens

<400> 26
gctttataga gtttctgcct agagcatcat ggctcagtgc ccagcagccc ctccagaggc 60
ctctgaatat ttgatatact gatttccttg aggagaatca gaaatctcct gcaggtgtct 120
agggatttca agtaagtagt gttgtgaggg gaatacctac ttgtactttc cccccaaacc 180
agattcccga ggcttcttaa ggactcaagg acaatttcta ggcatttagc acgggactaa 240
aaaggtctta gaggaaataa gaagcgccaa aaccatctct ttgcactgta tttcaaccca 300
tttgtccttc tgggttttga aggaacaggt gggactgggg acagaagagt tcttgaagcc 360
agtttgtcca tcatggaaaa tgagataggt gatgtggcta cgtcaggggg cccgaaggct 420
ccttgttact gatttccgtc ttttctctct gccttttccc caagggccag gacccctgga 480
tctctgggca gagcagacgc aggcccctat aatagccctc atgctagaaa ggagccggag 540
cctgtgtata aggccagcgc agcctactct ggacagtgca gggttcccac tctcccaact 600
ccccatctgc ttgcctccag acccacattc acacacgagc cactgggttg gaggagcatc 660
tgtgagatga aacaccattc tttcctcaat gtctcagcta tctaactgtg tgtgtaatca 720
ggccaggtcc tccctgctgg gcagaaacca tgggagttaa gagattgcca acatttatta 780
gaggaagctg acgtgtaact tctgaggcaa aatttagccc tcctttgaac aggaatttga 840
ctcagtgaac cttgtacaca ctcgcactga gtctgctgct gatgatactg tgcaccccac 900
tgtctgggtt ttaatgtcag gctgttcttt taggtatggc ggcttttccc tgggtgtcag 960
taatactcaa gcacttcctc cgagtcaaga agttaatgat gccatcaaac aaatgaagaa 1020
acacctaaag ctggccaagg taaaatatct atcgtaagat gtatcagaaa aatgggcatg 1080
tagctgctgg gatataggag tagttggcag gttaaacgga tcacctggca gctcattgtt 1140
ctgaatatgt tggcatacag agccgtcttt ggcatttagc gatttgagcc agacaaaact 1200
gaattactta gttgtacgtt taaaagtgta ggtcaaaaac aaatccagag gccaggagct 1260
gtggctcatg cctgtaatcc tagcactttg ggaggctgaa gcgggtggat cacttgaggt 1320
caggagttcg agaccagcct ggcctacatg acaaaacccc gtatctacta aaaatacaaa 1380
aaaattagct gggcttggtg gcacacacct gtaatcccag ctacttggga ggctgaggca 1440
ggagaattgc ttgaaccctg taggaagagg ttgtagtgag ccaagatcgc accgttgcac 1500
tccagcctgg gcaacaagag caaaactcca tctcaaaaaa caaattaaat ccagagattt 1560
aaaagctctc agaggctggg cgcggtggct tacacctgtt atcccagcat tttgggatgc 1620
cgaggcgggc aaagcacaag gtcaggagtt tgagaccagc ctggccaaca tagtgaaacc 1680
ctgtctctgc taaaaacata gaaaaattag ccgggcatgg tggcgtgcgc ctgtaatccc 1740
agctactcgg gaggctgagg tgagagaatt rcttgaaccc gggaggcgga ggttgcagtg 1800
agcccagatt gcaccactgc actccagcct gggcgacaga gcaagactcc atctcaaaaa 1860
aagctctcag aacaaccagg tttacaaatt tggtcagttg gtaaataaac tgggtttcaa 1920

acatactttg ctgaaayaat cactgactaa ataggaaatg aatctttttt tttttttttt 1980
taagctggca agctggtctg taggacctga taagtactca cttcatttct ctgtgtctca 2040
ggtttcccat ttttaggtga gaattaaggg gctctgataa aacagaccct aggattgtgg 2100
acagcagtga tagtcctaga gtccacaagt ctgcttttga gtgatgggcc catgtatctg 2160
gcacatctgc aggcagagcg tggttctggc tcttcagatg atgccggtgg agcactttga 2220
ggagtcctca ccccaccgtg ataaccagac attaaaatct tggggctttg catcccagga 2280
tttctctgtg attccttcta gacttgtggc atcatggcag catcactgct gtagatttct 2340
agtcacttgg ttctcaggag ccgtttattt aatggcttca catttaattt cagtgaacaa 2400
ggtagtggca ttgctcttca cagggccgtc ctgttgtcca caggttccag attgactgtt 2460
gccccttatc tatgtgaaca gtcacaactg aggcaggttt ctgttgttta caggacagtt 2520
ctgcagatcg atttctcaac agcttgggaa gatttatgac aggactggac accagaaata 2580
atgtcaaggt aaaccgctgt ctttgttcta gtagcttttt gatgaacaat aatccttatg 2640
tttcctggag tactttcaac tcatggtaaa gttggcaggg gcattcacaa cagaaaagag 2700
caaactatta actttaccag tgaggcagta cggtgtagtg tagtgattca gagaatttgc 2760
tttgccacca gacataccag gtaaccttga ctaagttact taacctatct aaacctcagt 2820
tycctcatct gtgaaatgga gacagtaatc atagctattt ccaaactgtt gtgagaattc 2880
aatgagttaa aggtataagg tcctcaccac agcgcctgcc cacatagtca gtgatcacta 2940
tgtcctgaac actgtaatta cttcgccata ttctctgatc atagtgtttt gccttggtat 3000
gtgactagaa tttctttctg aggtttatgg gcatggttgg tgggtatgca cctgcctgca 3060
ggagcccggt ttgggggcat taccttgtac ctggtatgtt ttctttcagg tgtggttcaa 3120
taacaagggc tggcatgcaa tcagctcttt cctgaatgtc atcaacaatg ccattctccg 3180
ggccaacctg caaaagggag agaaccctag ccattatgga attactgctt tcaatcatcc 3240
cctgaatctc accaagcagc agctctcaga ggtggctctg taagtgtggc tgtgtctgta 3300
tagatggagt ggggcaaggg agagggttat ggagaagggg agaaaaatgt gaatctcatt 3360
gtaggggaac agctgcagag accgttatat tatgataaat ctggattgat ccaggctctg 3420
ggcagaagtg ataagtttac gaattggctg gttgggcttc ttgaactgca gaagagaaaa 3480
tgacactgat atgtaaaaat cgtaacattt agtgaattca tataaagtga gttcaaaaat 3540
tgttaattaa attataattt aattataagt gtttaatcag tttgatttgt ttaaaaacca 3600
ctgttttaaa tttggtggaa tatgttttta ttagcttgta tctttaattc ctaaattaag 3660
ctgtgtgtgt gtgtgtgtgt gtgtgtgtgt gtgtgtgtgt gtgtgtgtgt gaagtttaaa 3720
gccaggatga gctagtttaa agtatgcagc ctttggagtc atacagatct gggtttgaat 3780
ctggtctcta aactttatag atgtatgata ttaaatgagg cagttcatgt aaattgccaa 3840
gcccagcact cagcacagag ttgatatttc acacacatta gatacctttc ctgtatgtgg 3900
agcatggcag ttcctgtttc tgctttactc ctacaggata ctaatatagg acactaggat 3960
ctttatacca agaccccatg taatgggctt atgagaccat tcttcttata aaaatctgac 4020
agaatttttg tatgtgttag atcaataggc tgcatactgt tattttcaag ttgatttaca 4080
gccagaaata ttaatttatt tgagtagtta cagagtaata tttctgctct catttagttt 4140
tcaagcccca ctagtccttt gtgtgtgaaa atttacaact tactgctctt acaaggtcat 4200
gaacagtgga ccaaagtgaa tgccattaac cactctgact tccttcatta gttttattgt 4260
gacagtggac tcttttgacc tcagtaatac cagtttggca tttacattgt catattttta 4320
gacttaaaaa tgatcatctt aaccctgaat aaaatgtgtc tggtgaacag atgtttttcc 4380
ttggctgtgc ctcagatatc tctgtgtgtg tgtacgtgtg tgtttgtctg tgtgtccatg 4440
tcctcactga ttgagcccta actgcatcaa agacccctca gattttcaca cgctttttct 4500
ctccaggatg accacatcag tggatgtcct tgtgtccatc tgtgtcatct ttgcaatgtc 4560
cttcgtccca gccagctttg tcgtattcct gatccaggag cgggtcagca aagcaaaaca 4620
cctgcagttc atcagtggag tgaagcctgt catctactgg ctctctaatt ttgtctggga 4680
tatggtaagg acacaggcct gctgtatctt tctgatgtct gtcagggcca tggattgata 4740
tggataagaa agaaagagct ctggctatca tcaggaaatg ttccagctac tctaaagatg 4800
tatgaaaaag aaatagccag aggcaggtga tcactttcat gacaccaaac acagcattgg 4860
gtaccagagt tcatgtcaca ccagagggaa aattctgtac acaatgatga aaattaatac 4920
cactaccact taagttccta tgtgacaact ttcccaagaa tcagagagat acaagtcaaa 4980
actccaagtc aatgcctcta acttctctga tgggttttaa cctccagagt cagaatgttc 5040
tttgccttac taggaaagcc atctgtcatt tagaaaactc tgtacatttt atcagcagct 5100
tatccatcca ttgcaaatat tgtttttgtg ccasccacaa tatattgctt ctatttggac 5160
caatatgggg gatttgaagg aattctgaag ttctaattat atttcaactc tactttacaa 5220

tatctccctg aaatatatct ccctgtaact tctattaatt ataagctaca cagagcaaat 5280
ctaattcttc tcccaccgaa caagtccctg gatatttaaa aataactctc atactctcat 5340
ttaacctgag tattacccag ataagatgat atatgagaat acaccttgta acctccgaag 5400
cactgtacaa atgtgagcaa tgatggtgga gatgatgatg agatctttgc tgtttatacc 5460
aagcccctta gactgtgtca ctcttctgat ccggttgtcc ttgtatggcc atgctgtata 5520
ttgtgaatgt cccgttttca aaagcaaagc caagaattaa ccttgtgttc aggctgtggt 5580
ctgaatggtt atgggtccag agggagttga tctttagctc acacttctat tactgcagca 5640
caaagatttt gcattttgga aggagcaccg tcttactggc aacttagtgg taaaccaaaa 5700
cctccatttc acacaaatga ttgtgaaatt cgggtctcct tcattctata caaattcatt 5760
tgattttttt gaaactaaac tttatattta tccatattaa attacatggg ttttattttt 5820
gttttatctt gattcagtaa ttactccttt cagtaaacac agactgagtg ctgtgtgtct 5880
gacttatgcc aggcataggt gattcagaga tgaaaggtca agtccctgaa cccatctctt 5940
gtcttcctgg gtattatctg tccctccctg ctttagagct cctgaaattt gctagaagca 6000
tgtcttcatc taagttgttg ataaacacat caagtaggat tggactgagg cagagccctg 6060
tagtctgaag ctgcagttct tctagcggct gacaagcccc actatcactt ccctgctggt 6120
gctttgctct gccagctgtg aattctcata attgtcctat cgtcaagtct ttatttctgc 6180
attttactgc ttgatacact gtcaggacag actttaaaat tattctcagt gcgatgaaac 6240
aattctgaca ttcatgttat gagcagttac ctcataaata gattacatg 6289

<210> 27
<211> 4244
<212> DNA
<213> Homo sapiens

<400> 27
aaattactct gactgggaat ccatcgttca gtaagtttac tgagtgtgac accttggctt 60
gactgttgga aagacagaaa gggcatgtag tttataaaat cagccaaggg gaaaatgctt 120
gtcaaaatgt attgtcgggt attttgatta atagtttatg tggcttcatt aattcagagt 180
tactctccaa tatgtttatc tgccctttct tgtctgataa tggtgaaaac ttgtgtgatg 240
cattgtatat ttgatttagg ggtgaactgg atgtctttgt tttcactttt agtgcaatta 300
cgttgtccct gccacactgg tcattatcat cttcatctgc ttccagcaga agtcctatgt 360
gtcctccacc aatctgcctg tgctagccct tctacttttg ctgtatgggt aagtcacctc 420
tgagtgaggg agctgcacag tggataaggc atttggtgcc cagtgtcaga aggagggcag 480
ggactctcag tagacactta tctttttgtg tctcaacagg tggtcaatca cacctctcat 540
gtacccagcc tcctttgtgt tcaagatccc cagcacagcc tatgtggtgc tcaccagcgt 600
gaacctcttc attggcatta atggcagcgt ggccaccttt gtgctggagc tgttcaccga 660
caatgtgagt catgcagaga gaacactcct gctgggatga gcatctctgg gagccagagg 720
acagtgttta attgtgatct tattccactt gtcagtggta ttgacactgc tgactgcctt 780
gtcctgtctt cagagtctgt cttccctgag aaggcaaagc acctttcttt cttgctgtgc 840
cttacatttt gctggtcaag cctttcagtt tcttttgaca gtttttttta cttctttctt 900
ttttcaatgt tgctcttacc aagagtagct cctctgcctt ccactttaca catgagagct 960
gggcgacgca ttcagtccta aggcttttac catcacctct cttggtgttt ttattgtcat 1020
ctctaagatc aatgccttta gccttgatca taaccttgaa ctctaatctc aaattctcac 1080
ttgcctagtg gattgctcca tttagatagt atatagatac cccaacctgg atatgtccta 1140
gttttctttc cccttggaac ttaatgcttt tcttgccatc cctgtcacac tcagtggcac 1200
taccatccac tcggttgccc aagctggctc ttagagttat cctagatgct tgctttgctg 1260
ttgcagattt cccacattca actggttatg ttgtcagttc ttccaggtat ggacctctaa 1320
aataaggctt cctctccatt ccggttgtca ttgcctttgt ccaaacacag cacacaaggc 1380
cttttacagt tgcacaactc ttcctgtcca tacccaccac accctttccc agctgtaagc 1440
ttcagatgag ttgcctccaa ccaccatgct cctgtaggcc tggcttgaaa tgcccttctt 1500
ctgtcacagg gtctggtagt atatcccttg cccttcaaga tttagctaaa atgtgaagct 1560
ttccttacct gctgggaggt gttctctctt ttctctgtgc tctcagagtc cttagtccat 1620
gcctccagta caacgtacat ccacttacat ggtaatttcc tgtttacata cttttcctac 1680

tcggagtgga gtctgtttct taataatttt gcctctccca tgccctagca cagtgcatcc 1740
agcgtatagc cccttattca gttggtagat atttggccac tgttgccttg tgggatcata 1800
agttctgatg tatttgagaa gaatttctaa aattctgaca aaatcctgaa actcaaatat 1860
tgacccagac atgagcaatt tgcttttcaa atgctaaggg atttttaatg gatttgcttt 1920
aattaaatct agcctgtttc taagctttat tcattatttc tccatactca gagcatttct 1980
ccagattttc taaagaatag aattttattg ctacatatca tcagctatgc ctgctgctat 2040
ttaattggta tctgaattaa aaggtctggt ttgtccctag agaatcaaat tttttcttca 2100
ctcccatatt tcagaacttg atacattttt aggataaacc atgaatgaca cccgtttctt 2160
ctccctcacc ctcccttccc tcccattttt tttttttttt ttttttagaa gctgaataat 2220
atcaatgata tcctgaagtc cgtgttcttg atcttcccac atttttgcct gggacgaggg 2280
ctcatcgaca tggtgaaaaa ccaggcaatg gctgatgccc tggaaaggtt tggtgagtga 2340
agcagtggct gtaggatgct ttaatggaga tggcactctg cataggcctt ggtaccctga 2400
actttgtttt ggaaagaagc aggtgactaa gcacaggatg ttcccccacc cccatgccca 2460
gtgacagggc tcatgccaac acagctggtt gtggcatggg ttttgtgaca caaccatttg 2520
tctgtgtctc tgatagcatt gagaaaagtg aaagggcagt tttgaaggta aggaaaatag 2580
tgttatttgc ttggatccac tggctcatgc cactgtctgg gttggttaga agcactggaa 2640
aagtcaaacc ataactttga gaattaggtg atcagggaat cagaaggaaa gatgcaaact 2700
ttggctcttt taggcgaatc atgtgcctgc agatgaggtc atttattatc ttttacacag 2760
tctataaaat tataatgtat tacatctttt tctaccttta gaatggttaa aaatatttct 2820
ccggtagcca tatgattatt attcatccat tagataatat agtcaaatgg gccatgttat 2880
ttactgttca tagaagaggg gctttttgca acttgggcta caaaggagat atgtaaggaa 2940
tttaaggaat ggttacatgg aactagattt aattgaatct agtggtttaa ttgattcact 3000
aggatatatg ctactgaaag gggaatctgc ttaaagtgct ttctgatatt tattattact 3060
aaaacttaga atttattaaa aatactgact gtgaaaatta cttgggtcgt ttgccttttt 3120
aaaaggattt ttggcatgtc tcattaaaaa aagaaatact agatatcttc agtgaagtta 3180
caaatcgaat acacattggc tctgaaattc tgattgatac tgggtcataa aaagttttcc 3240
caaatcagac ttggaaagtg atcactctct tgttactctt ttttccttgt catgggtgat 3300
agccatttgt gtttattgga agatcggtga attttaagga acataggccc aaatttgagg 3360
aagggccatg gtttttgatc cctccattct gaccggatct ctgcattgtg tctactaggg 3420
gagaatcgct ttgtgtcacc attatcttgg gacttggtgg gacgaaacct cttcgccatg 3480
gccgtggaag gggtggtgtt cttcctcatt actgttctga tccagtacag attcttcatc 3540
aggcccaggt gagctttttc ttagaacccg tggagcacct ggttgagggt cacagaggag 3600
gcgcacaggg aaacactcac caatgggggt tgcattgaac tgaactcaaa atatgtgata 3660
aaactgattt tcctgatgtg ggcatcccgc agccccctcc ctgcccatcc tggagactgt 3720
ggcaagtagg ttttataata ctacgttaga gactgaatct ttgtcctgaa aaatagtttg 3780
aaaggttcat ttttcttgtt ttttccccca agacctgtaa atgcaaagct atctcctctg 3840
aatgatgaag atgaagatgt gaggcgggaa agacagagaa ttcttgatgg tggaggccag 3900
aatgacatct tagaaatcaa ggagttgacg aaggtgagag agtacaggtt acaatagctc 3960
atcttcagtt tttttcagct ttatgtgctg taacccagca gtttgctgac ttgcttaata 4020
aaagggcatg tgttcccaaa atgtacatct ataccaaggt tctgtcaatt ttattttaaa 4080
aacaccatgg agacttctta aagaattctt actgagaatt cttttgtgat atgaattccc 4140
attctcgaat actttggttt tatatgctta catttatgtg ttagttatta aaacatacta 4200
atattgtata tctagtcaaa ctgagtagag agataatggt gatt 4244

<210> 28
<211> 5023
<212> DNA
<213> Homo sapiens

<400> 28
ttttaaaata cctgcaatac atatatatgt tgaatagatg aaaaattatg tagatgataa 60
tgaatgatac ggttctaaaa agacaggtta aaaagtaagt tcacttttat tttgagcttc 120
agaatcattc agaagccagt cgccacaaac gcagaccaag gctcttggca catcaaatat 180
gcctatggct tagggttatt gacaagtctt atgttgcagt gtatgtggtt tatagtcctg 240
ccttccacag ttgcttggga gagctgtgag tcactgaggc ttatgaatgt ttacattttg 300

tttgttgcag atatatagaa ggaagcggaa gcctgctgtt gacaggattt gcgtgggcat 360
tcctcctggt gaggtaaaga cactttgtct atattgcgtt tgtccctatt agttcagact 420
atctctaccc aatcaagcaa cgatgctcgt taagaggtaa aagtggattt taaaggcttc 480
tgtatttatg ccaggatgga gcaattagtc atcgagaaga gagggaccct gtatgtcaag 540
agaatgattt cagagaatcc aatacaattt aagaaaaagc atggggctgg gcgcagtgat 600
tcactcctgt aatcccagca ctttgggagg ccgaggtggg cggactcacg aggtcaggag 660
attgagacca tcctggccaa catggtgaaa ccccatctct actataaata caaaaattag 720
ctgggcatag tagtgcattc ctgtagtccc agctactcgg gaggctgagg caggagaatt 780
gcttgaacct aggaggggga ggttgcccag attgcgctgc tgcactccag cctggtgaca 840
gagtgagact catgtcaaca acaaaaacag aaaaagcacg cacatctaaa acatgctttt 900
gtgatccatt tgggatggtg atgacattca aatagttttt taaaaataga ttttctcctt 960
tctggtttcc gtttgtgttc ttttatgccc ttttgccaga gtaggtggtg caatttggct 1020
agctggcttt cattactgtt tttcacacat taactttggc ctcaacttga caactcaaat 1080
aatatttata aatacagcca cacttaaaat ggtcccatta tgaaatacat atttaaatat 1140
ctatacgatg tgttaaaacc aagaaaatat ttgattcttc tctgatattt aagaattgaa 1200
ggtttgaggt agttacgtgt taggggcatt tatattcatg tttttagagt ttgcttatac 1260
aacttaatct ttccttttca gtgctttggg ctcctgggag ttaatggggc tggaaaatca 1320
tcaactttca agatgttaac aggagatacc actgttacca gaggagatgc tttccttaac 1380
aaaaataggt gagaaaagaa gtggcttgta ttttgctgca aagactttgt ttttaattta 1440
tttaaagaaa taggttgtta tttttgatta cagtggtatt tttagagttc ataaaaatgt 1500
tgaaatatag taaagggtaa agaagcacat aaaatcatcc atgatttcaa tatctagaga 1560
taatcacaat ttacatttcc tttcagtctc attctcttct tttaacagct ttattcaggt 1620
ataatttaca tacaatataa tttgcttgtt ttttaagagt ataatttagt gatttttggt 1680
aaattgagag ttttgcaacc atcaccacaa tccagtttta gaacttttcc atcaccccac 1740
atctgtctta tatacacata taaatgtgcc atacaattga gatcatactg tatgtagaat 1800
ttaaaattag tttttattgt taatgagtgt attatgaata tttcccagtg ggttacattt 1860
cctaagatgt ggaattttac attgctacat aaaatccccc tatgtacatg tacctataat 1920
ttatttaata aattccttat aaatgttgga cacattagtt tccatttttc actatgtaaa 1980
tatgtccctg tatacatctt ttattatttc ctcaggaaca attcctacaa agtaaattgc 2040
cctctctaaa gagcatacaa attgactgag ccaccgttag gccattttct gagactgcac 2100
aggtcacaaa gcaatctgat ctttgggaat acagctacat tttataggct tcttagataa 2160
tgttactcta agtactttaa atatgtgggg cttctctggg cttttttttt tttgagacgg 2220
agtttcactc ttactgccca ggctggagag caatggcgcg accttggctc actgcaacct 2280
ccgcctccca ggttcaagcg attctcctgc ctcagcctcc tgagtagctg agattacagg 2340
tgcccgccac aatgcctgcc taattttttt gtattttcag tagagatggg gtttcaccat 2400
gttggccaga ctggtctcga gctcctgacc tcaggtgatc cacctgcctc agcctcccaa 2460
agttctggga ttacaggcat gagccactgc gcccggcttc tctggactta ttatgtggag 2520
agatagtaca aggcagtggc tttcagagtt ttttgaccat gaccgttgtg ggaaatacat 2580
tttatatctc aacctagtat gtacacacag acatgtagac acatgtataa cctaaagttt 2640
cataaagcag tacctactgt tactaattgt agtgcactct gctatttctt attctacctt 2700
atactgcgtc attaaaaaag tgctggtcat gacccactaa atttatttcc caaaccacta 2760
atgaacaatg actcacaatt tgaacacact ggacaggggg atagccaata aaattgaaaa 2820
gagcaaggaa attaatgtat tcatgatctc ctctcctgtc tcttacattt ttgcagtagc 2880
aatgtaaagg aatcctaaga gaacagacat tctgggaata gcaggcctag cgctgcacaa 2940
ctgctttcct aggcttgctc ctagtaccaa gctcctgacg catatagcag tggcagtaat 3000
aaccagccca tagtaaggtt tgtcacaggg actggttgta agaactgatt tgrttggtat 3060
agctgtgagg gcctggcacg gtgtccacgt gtgcctcaat cctaattctg aaaaaggctg 3120
accctggggg tgctaattag atacacagag aggaatgaat gctgccagaa ggccaagttc 3180
atggcaatgc cgctgtggct gaggtgcagt catcagtctg gaacgtgaac actgaacttc 3240
tctcacatgt gattcttcac ttgactggct tcatagaacc ccaaagccac cccaccacca 3300
cataaattgt gtctctaggt tctgtgttgc tcacactcaa aatttctggg ccttctcatt 3360
tggtgcatgt gaatggtgca tatgagtgaa gtctaggatg gggccttagc gttaaagccc 3420
tggggtagtg tgactgagat tgttggtaaa gaatgtgcag tggttggcat gacctcagaa 3480
attctgaaat gggactgcac ctgcagactg aagtgttcag agagccaggg aggtgcaagg 3540
actggggagg gtagaggcag gaaccctgcc tgccaggaag agctagcatc ctgggggcag 3600

aaaggctgtg ctttcaagta gcagcagatg tattggtatc tttgtaatgg agaagcatac 3660
tttacaggaa cattaggcca gattgtctaa ccagagtatc tctacctgct taaaatctaa 3720
gtagttttct tgtcctttgc agtatcttat caaacatcca tgaagtacat cagaacatgg 3780
gctactgccc tcagtttgat gccatcacag agctgttgac tgggagagaa cacgtggagt 3840
tctttgccct tttgagagga gtcccagaga aagaagttgg caaggtactg tgggcacctg 3900
aaagccagcc tgtctccttt ggcatcctga caatatatac cttatggctt ttccacacgc 3960
attgacttca ggctgttttt cctcatgaat gcagcagcac aaaatgctgg ttctttgtat 4020
ctgctttcag ggtggaaacc tgtaacggtg gtggggcagg gctgggtggg cagagaggga 4080
gtgctgctcc caccacacga gtcccttctc cctgctttgg ctcctcacca gttgtcaggt 4140
tatgattata gaatctagtc ctactcagtg aaagaacttt catacatgta tgtgtaggac 4200
agcatgataa aattcccaag ccagaccaaa gtcaaggtgc tttttatcac tgtaggttgg 4260
tgagtgggcg attcggaaac tgggcctcgt gaagtatgga gaaaaatatg ctggtaacta 4320
tagtggaggc aacaaacgca agctctctac agccatggct ttgatcggcg ggcctcctgt 4380
ggtgtttctg gtgagtataa ctgtggatgg aaaactgttg ttctggcctg agtggaaaac 4440
atgactgttc aaaagtccta tatgtccagg gctgttgtat gattggcttg tcttccccca 4500
gggacagcag agcaaccttg gaaaagcaga gggaagcttc tcccttggca cacactgggg 4560
tggctgtacc atgcctgcag atgctcccaa atagaggcac tccaagcact ttgtttctta 4620
gcgtgattga ggctggatat gtgatttgat ctttctctgg aacattcttt ctaatcatct 4680
ttgtgttcat tccctgaaaa tgaagagtgt ggacacagct ttaaaatccc caaggtagca 4740
actaggtcat agttccttac acacggatag atgaaaaaca gatcagactg ggaagtggcc 4800
cttgaccttt tttcttctgt agataagagc attgatgtta ttacggaaag aagcctttga 4860
ggcttttatg tattccacct cggtctggaa tttgtttctg taaggctaac agttgcaata 4920
tactagggta atctgagtga gctggaatta aaaaaaaaaa ggaatttcac cccaatctta 4980
tactgacttc aatagaggtt tcagacaaaa agttgttttg tat 5023

<210> 29
<211> 5138
<212> DNA
<213> Homo sapiens

<220>
<221> misc_feature
<222> (1)...(5138)
<223> n = a, t, c, or g

<400> 29
ngccnngttn aaaangaaaa tttnnnnnaa attnaanntt annggngnnn tttccccaga 60
aaaaacnaaa angatttccn cccngggggg ncccccnant cnaaaaggcc ccncttnttt 120
gnggngaggg aaagnttttt ttggaatttt taatttttgg tcccccaaaa cctattattg 180
agaatttaat tacataaaaa agtactcaga atatttgagt ttcctgcatc aataagacat 240
ttataataat gaccttgttt acaaatgaat ttgaaagtta ctctaattct ttgattcatc 300
aagaaataac tagaatggca agttaaaatt taagctgttt caaagatgct tctgcattta 360
aaaacaaatt tatctttgat ttttttcccc cccagcaaat aagacttatt ttattctaat 420
tacaggatga acccaccaca ggcatggatc ccaaagcccg gcggttcttg tggaattgtg 480
ccctaagtgt tgtcaaggag gggagatcag tagtgcttac atctcatagg tccgtagtaa 540
agtcttgggt tcctcactgt gggatgtttt aactttccaa gtagaatatg cgatcatttt 600
gtaaaaatta gaaaatacag aaaagcaaag agtaaaacaa ttattacctg aaattatata 660
tgcatattct tacaaaaatg caagcccagt ataaatactg ctctttttca cttaatatat 720
tgtaaacatt attccaagtc agtgcattta ggtgtcattt cttatagctg gatagtattc 780
cattaggata tactcttatt taactattcc cccttttgta gacatttgga ttatttccaa 840
cttgttcaca attgtaaaca ccactacact gaacagcatc atccctatat ccacatgtac 900
ttgtaacaga atacaattcc ctaggaagct ggaatgctgg aagtcatggt gatgttctca 960
tggttacaga gaatctctct aaaactaaaa cctctttctg ttttaccgca gtatggaaga 1020

atgtgaagct ctttgcacta ggatggcaat catggtcaat ggaaggttca ggtgccttgg 1080
cagtgtccag catctaaaaa ataggtaata aagataattt ctttgggata gtgcctagtg 1140
agaaggcttg atatttattc ttttgtgagt atataaatgg tgcctctaaa ataaagggaa 1200
ataaaactga gcaaaacagt atagtggaaa gaatgagggc tttgaagtcc gaactgcatt 1260
caaattctgt ctttaccatt tactggttct gtgactcttg ggcaagttac ttaactactg 1320
taagagttag tttccctgga agatctacct cctagctttg tgctatagat gaaatgaaaa 1380
aaatttacat gtgccagtac tggtgagagc gcaagctttg gagtcaaaca caaatgggtt 1440
tgcatcctgg ccctaccaat tatgagctct gagccatggg caagtgacta actccctggg 1500
cctcagtttc tctgtaacat ctgtcagact tcatgggtcc aggtgaggat taaaggagat 1560
catgtattta cagcacatgg catggtgctt cacataaaat aagtatttag taaatgataa 1620
ctggttcctt ctctcagaaa cttatttctg ggcctgccag gggccgccct ttttcatggc 1680
acaagttggg ttcccagggt tcagtattct tttaaatagt tttctggaga tcctccattt 1740
gggtattttt tcctgctttc aggtttggag atggttatac aatagttgta cgaatagcag 1800
ggtccaaccc ggacctgaag cctgtccagg atttctttgg acttgcattt cctggaagtg 1860
ttcyaaaaga gaaacaccgg aacatgctac aataccagct tccatcttca ttatcttctc 1920
tggccaggat attcagcatc ctctcccaga gcaaaaagcg actccacata gaagactact 1980
ctgtttctca gacaacactt gaccaagtaa gctttgagtg tcaaaacaga tttacttctc 2040
agggtgtgga ttcctgcccc gacactcccg cccataggtc caagagcagt ttgtatcttg 2100
aattggtgct tgaattcctg atctactatt cctagctatg ctttttacta aacctctctg 2160
aacctgaaaa gggagatgat gcctatgtac tctataggat tattgtgaga atttactgta 2220
ataataacca taaaaactac catttagtga gcacctacca tgggccaggc attttacttg 2280
gtgcctaatc ctatttaaat tagataaaaa agtaccaaat aggtcctgac acttaagaag 2340
tactcagtaa atattttctt ccctcttccc tttaatcaag accgtatgtg ccaaagtaaa 2400
tggatgactg agcagttggt gatgtagggg tggggggcga tatagaaagt cagtttttgg 2460
ccgggcgtgg tggctcatgc ctgtaatccc agcactttgg gaggctgagg agcaggcaga 2520
tcatgaggtc aggagatcca gataatcctg gccaacaggg tgaaaccccg tctctactaa 2580
aaatacaaaa attagctggg catggtggtg cgcacttgta gtcccagcta cttgcgaggc 2640
tgaggcagga gaattgctcg aacccaggag gtggaggtta cagtgagcca aggtctcgcc 2700
actgcactcc agcctgggga cagagcaaga ccccatttca aggggggaaa aaaagtctat 2760
ttttaagttg ttattgcttt tttcaagtat tcttccctcc ttcacacaca gttttctagt 2820
taatccattt atgtaattct gtatgctcct acttgaccta atttcaacat ctggaaaaat 2880
agaactagaa taaagaatga gcaagttgag tggtatttat aaaggtccat cttaatcttt 2940
taacaggtat ttgtgaactt tgccaaggac caaagtgatg atgaccactt aaaagacctc 3000
tcattacaca aaaaccagac agtagtggac gttgcagttc tcacatcttt tctacaggat 3060
gagaaagtga aagaaagcta tgtatgaaga atcctgttca tacggggtgg ctgaaagtaa 3120
agaggaacta gactttcctt tgcaccatgt gaagtgttgt ggagaaaaga gccagaagtt 3180
gatgtgggaa gaagtaaact ggatactgta ctgatactat tcaatgcaat gcaattcaat 3240
gcaatgaaaa caaaattcca ttacaggggc agtgcctttg tagcctatgt cttgtatggc 3300
tctcaagtga aagacttgaa tttagttttt tacctatacc tatgtgaaac tctattatgg 3360
aacccaatgg acatatgggt ttgaactcac actttttttt ttttttttgt tcctgtgtat 3420
tctcattggg gttgcaacaa taattcatca agtaatcatg gccagcgatt attgatcaaa 3480
atcaaaaggt aatgcacatc ctcattcact aagccatgcc atgcccagga gactggtttc 3540
ccggtgacac atccattgct ggcaatgagt gtgccagagt tattagtgcc aagtttttca 3600
gaaagtttga agcaccatgg tgtgtcatgc tcacttttgt gaaagctgct ctgctcagag 3660
tctatcaaca ttgaatatca gttgacagaa tggtgccatg cgtggctaac atcctgcttt 3720
gattccctct gataagctgt tctggtggca gtaacatgca acaaaaatgt gggtgtctcc 3780
aggcacggga aacttggttc cattgttata ttgtcctatg cttcgagcca tgggtctaca 3840
gggtcatcct tatgagactc ttaaatatac ttagatcctg gtaagaggca aagaatcaac 3900
agccaaactg ctggggctgc aactgctgaa gccagggcat gggattaaag agattgtgcg 3960
ttcaaaccta gggaagcctg tgcccatttg tcctgactgt ctgctaacat ggtacactgc 4020
atctcaagat gtttatctga cacaagtgta ttatttctgg ctttttgaat taatctagaa 4080
aatgaaaaga tggagttgta ttttgacaaa aatgtttgta ctttttaatg ttatttggaa 4140
ttttaagttc tatcagtgac ttctgaatcc ttagaatggc ctctttgtag aaccctgtgg 4200
tatagaggag tatggccact gcccactatt tttatttcct tatgtaagtt tgcatatcag 4260
tcatgactag tgcctagaaa gcaatgtgat ggtcaggatc tcatgacatt atatttgagt 4320

ttctttcaga tcatttagga tactcttaat ctcacttcat caatcaaata ttttttgagt 4380
gtatgctgta gctgaaagag tatgtacgta cgtataagac tagagagata ttaagtctca 4440
gtacacttcc tgtgccatgt tattcagctc actggtttac aaatataggt tgtcttgtgg 4500
ttgtaggagc ccactgtaac aatactgggc agcctttttt tttttttttt taattgcaac 4560
aatgcaaaag ccaagaaagt ttaagggtca caagtctaaa caatgaattc ttcaacaggg 4620
aaaacagcta gcttgaaaac ttgctgaaaa acacaacttg tgtttatggc atttagtacc 4680
ttcaaataat tggctttgca gatattggat accccattaa atctgacagt ctcaaatttt 4740
tcatctcttc aatcactagt caagaaaaaa tataaaaaca acaaatactt ccatatggag 4800
catttttcag agttttctaa cccagtctta tttttctagt cagtaaacat ttgtaaaaat 4860
actgtttcac taatacttac tgttaactgt cttgagagaa aagaaaaata tgagagaact 4920
attgtttggg gaagttcaag tgatctttca atatcattac taacttcttc cactttttcc 4980
agaatttgaa tattaacgct aaaggtgtaa gacttcagat ttcaaattaa tctttctata 5040
ttttttaaat ttacagaata ttatataacc cactgctgaa aaagaaacaa atgattgttt 5100
tagaagttaa aggtcaatat tgattttaaa atattaag 5138

<210> 30
<211> 20
<212> DNA
<213> Homo sapiens

<400> 30
gtgttcctgc agagggcatg 20

<210> 31
<211> 20
<212> DNA
<213> Homo sapiens

<400> 31
cacttccagt aacagctgac 20

<210> 32
<211> 21
<212> DNA
<213> Homo sapiens

<400> 32
ctttgcgcat gtccttcatg c 21

<210> 33
<211> 21
<212> DNA
<213> Homo sapiens

<400> 33
gacatcagcc ctcagcatct t 21

<210> 34
<211> 19
<212> DNA
<213> Homo sapiens

<400> 34

caacaagcca tgttccctc 19

<210> 35
<211> 18
<212> DNA
<213> Homo sapiens

<400> 35
catgttccct cagccagc 18

<210> 36
<211> 19
<212> DNA
<213> Homo sapiens

<400> 36
cagagctcac agcagggac 19

<210> 37
<211> 21
<212> PRT
<213> Homo sapiens

<400> 37
Cys Ser Val Arg Leu Ser Tyr Pro Pro Tyr Glu Gln His Glu Cys His
1               5                   10                  15
Phe Pro Asn Lys Ala
            20

<210> 38
<211> 14
<212> DNA
<213> Homo sapiens

<400> 38
gcctgtgtgt cccc 14

<210> 39
<211> 14
<212> DNA
<213> Homo sapiens

<220>
<221> misc_feature
<222> (1)...(14)
<223> n = t or c

<400> 39
gcctgtgngt cccc 14

<210> 40
<211> 45
<212> DNA
<213> Homo sapiens

<400> 40
aagaagatgc tgcctgtgtg tcccccaggg gcaggggggc tgcct                    45

<210> 41
<211> 15
<212> PRT
<213> Homo sapiens

<400> 41
Lys Lys Met Leu Pro Val Cys Pro Pro Gly Ala Gly Gly Leu Pro
1               5                   10                  15

<210> 42
<211> 15
<212> PRT
<213> Mus musculus

<400> 42
Lys Lys Met Leu Pro Val Cys Pro Pro Gly Ala Gly Gly Leu Pro
1               5                   10                  15

<210> 43
<211> 15
<212> PRT
<213> Homo sapiens

<400> 43
Lys Lys Met Leu Pro Val Arg Pro Pro Gly Ala Gly Gly Leu Pro
1               5                   10                  15

<210> 44
<211> 5
<212> PRT
<213> Caenorhabditis elegans

<400> 44
Leu Leu Gly Gly Ser
1               5

<210> 45
<211> 45
<212> DNA
<213> Homo sapiens

<400> 45
aagaagatgc tgcctgtgcg tcccccaggg gcaggggggc tgcct                    45

<210> 46
<211> 14
<212> DNA
<213> Homo sapiens

<400> 46
gcctacttgc agga                                                     14

<210> 47
<211> 14
<212> DNA
<213> Homo sapiens

<400> 47
gcctacttgc ggga                                                          14

<210> 48
<211> 45
<212> DNA
<213> Homo sapiens

<400> 48
tgggggggct tcgcctactt gcaggatgtg gtggagcagg caatc                         45

<210> 49
<211> 15
<212> PRT
<213> Homo sapiens

<400> 49
Trp Gly Gly Phe Ala Tyr Leu Gln Asp Val Val Glu Gln Ala Ile
 1               5                   10                  15

<210> 50
<211> 15
<212> PRT
<213> Mus musculus

<400> 50
Trp Gly Gly Phe Ala Tyr Leu Gln Asp Val Val Glu Gln Ala Ile
 1               5                   10                  15

<210> 51
<211> 15
<212> PRT
<213> Homo sapiens

<400> 51
Trp Gly Gly Phe Ala Tyr Leu Arg Asp Val Val Glu Gln Ala Ile
 1               5                   10                  15

<210> 52
<211> 12
<212> PRT
<213> Caenorhabditis elegans

<400> 52
Phe Met Thr Val Gln Arg Ala Val Asp Val Ala Ile
 1               5                   10

<210> 53
<211> 45
<212> DNA

<213> Homo sapiens

<400> 53
tgggggggct tcgcctactt gcgggatgtg gtggagcagg caatc                    45

<210> 54
<211> 25
<212> DNA
<213> Homo sapiens

<220>
<221> misc_feature
<222> (1)...(25)
<223> n is a, t, c, or g.

<400> 54
tcattcctct tgtnngcncn gnncn                                         25

<210> 55
<211> 45
<212> DNA
<213> Homo sapiens

<400> 55
agtagcctca ttcctcttct tgtgagcgct ggcctgctag tggtc                   45

<210> 56
<211> 15
<212> PRT
<213> Homo sapiens

<400> 56
Ser Ser Leu Ile Pro Leu Leu Val Ser Ala Gly Leu Leu Val Val
 1               5                   10                  15

<210> 57
<211> 15
<212> PRT
<213> Mus musculus

<400> 57
Ser Ser Leu Ile Pro Leu Leu Val Ser Ala Gly Leu Leu Val Val
 1               5                   10                  15

<210> 58
<211> 14
<212> PRT
<213> Homo sapiens

<400> 58
Ser Ser Leu Ile Pro Leu Val Ser Ala Gly Leu Leu Val Val
 1               5                   10

<210> 59
<211> 15

<212> PRT
<213> Caenorhabditis elegans

<400> 59
Ile Asn Tyr Ala Lys Leu Thr Phe Ala Val Ile Val Leu Thr Ile
1               5                   10                  15

<210> 60
<211> 42
<212> DNA
<213> Homo sapiens

<400> 60
agtagcctca ttcctcttgt gagcgctggc ctgctagtgg tc                          42

<210> 61
<211> 25
<212> DNA
<213> Homo sapiens

<220>
<221> misc_feature
<222> (1)...(25)
<223> n is a, t, c, or g.

<400> 61
tgatgaagat gananncngn ngcga                                             25

<210> 62
<211> 36
<212> DNA
<213> Homo sapiens

<400> 62
aatgatgaag atgaagatgt gaggcgggaa agacag                                 36

<210> 63
<211> 12
<212> PRT
<213> Homo sapiens

<400> 63
Asn Asp Glu Asp Glu Asp Val Arg Arg Glu Arg Gln
1               5                   10

<210> 64
<211> 12
<212> PRT
<213> Mus musculus

<400> 64
Asn Asp Glu Asp Glu Asp Val Arg Arg Glu Arg Gln
1               5                   10

<210> 65

<211> 10
<212> PRT
<213> Homo sapiens

<400> 65
Asn Asp Glu Asp Val Arg Arg Glu Arg Gln
1               5                   10

<210> 66
<211> 15
<212> PRT
<213> Caenorhabditis elegans

<400> 66
Asp Glu Arg Asp Val Glu Asp Ser Asp Val Ile Ala Glu Lys Ser
1               5                   10                  15

<210> 67
<211> 30
<212> DNA
<213> Homo sapiens

<400> 67
aatgatgaag atgtgaggcg ggaaagacag                                        30

<210> 68
<211> 14
<212> DNA
<213> Homo sapiens

<400> 68
agttgtacga atag                                                         14

<210> 69
<211> 14
<212> DNA
<213> Homo sapiens

<220>
<221> misc_feature
<222> (1)...(14)
<223> n is t or c.

<400> 69
agttgtanga atag                                                         14

<210> 70
<211> 20
<212> DNA
<213> Homo sapiens

<400> 70
ggctggatta gcagtcctca                                                   20

<210> 71

<211> 20
<212> DNA
<213> Homo sapiens

<400> 71
ggatttccca gatcccagtg                                                    20

<210> 72
<211> 20
<212> DNA
<213> Homo sapiens

<400> 72
gacagacttg gcatgaagca                                                    20

<210> 73
<211> 20
<212> DNA
<213> Homo sapiens

<400> 73
gcacttggca gtcacttctg                                                    20

<210> 74
<211> 20
<212> DNA
<213> Homo sapiens

<400> 74
cgtttctcca ctgtcccatt                                                    20

<210> 75
<211> 20
<212> DNA
<213> Homo sapiens

<400> 75
acttcaagga cccagcttcc                                                    20

<210> 76
<211> 24
<212> DNA
<213> Homo sapiens

<400> 76
tcggtttctt gtttgttaaa ctca                                               24

<210> 77
<211> 20
<212> DNA
<213> Homo sapiens

<400> 77
tcccaaggct ttgagatgac                                                    20

<210> 78
<211> 19
<212> DNA
<213> Homo sapiens

<400> 78
ggctccaaag cccttgtaa                                                    19

<210> 79
<211> 20
<212> DNA
<213> Homo sapiens

<400> 79
gctgctgtga tggggtatct                                                   20

<210> 80
<211> 25
<212> DNA
<213> Homo sapiens

<400> 80
tttgtaaatt ttgtagtgct cctca                                             25

<210> 81
<211> 20
<212> DNA
<213> Homo sapiens

<400> 81
tagtcagccc ttgcctccta                                                   20

<210> 82
<211> 20
<212> DNA
<213> Homo sapiens

<400> 82
aaaggggctt ggtaagggta                                                   20

<210> 83
<211> 20
<212> DNA
<213> Homo sapiens

<400> 83
gatgtggtgc tccctctagc                                                   20

<210> 84
<211> 20
<212> DNA
<213> Homo sapiens

<400> 84
caagtgagtg cttgggattg                                                   20

<210> 85
<211> 21
<212> DNA
<213> Homo sapiens

<400> 85
gcaaattcaa atttctccag g                                                21

<210> 86
<211> 20
<212> DNA
<213> Homo sapiens

<400> 86
tcaaggagga aatggacctg                                                  20

<210> 87
<211> 20
<212> DNA
<213> Homo sapiens

<400> 87
ctgaaagttc aagcgcagtg                                                  20

<210> 88
<211> 20
<212> DNA
<213> Homo sapiens

<400> 88
tgcagactga atggagcatc                                                  20

<210> 89
<211> 20
<212> DNA
<213> Homo sapiens

<400> 89
gccaggggac actgtattct                                                  20

<210> 90
<211> 20
<212> DNA
<213> Homo sapiens

<400> 90
aggtcctctg ccttcactca                                                  20

<210> 91
<211> 20
<212> DNA
<213> Homo sapiens

<400> 91
ccagtgctta cccctgctaa                                                  20

<210> 92
<211> 21
<212> DNA
<213> Homo sapiens

<400> 92
cacacaacag agcttcttgg a                                                21

<210> 93
<211> 20
<212> DNA
<213> Homo sapiens

<400> 93
acctggaaca ggtgtggtgt                                                  20

<210> 94
<211> 21
<212> DNA
<213> Homo sapiens

<400> 94
gggctaacat gccactcagt a                                                21

<210> 95
<211> 20
<212> DNA
<213> Homo sapiens

<400> 95
gtttgttgca gatggggaag                                                  20

<210> 96
<211> 20
<212> DNA
<213> Homo sapiens

<400> 96
caccagaaga aggagcatgg                                                  20

<210> 97
<211> 20
<212> DNA
<213> Homo sapiens

<400> 97
ctggactcgt agggatttgc                                                  20

<210> 98
<211> 21
<212> DNA
<213> Homo sapiens

<400> 98
gcctgtcaca gagaaatgct t                                                21

<210> 99
<211> 21
<212> DNA
<213> Homo sapiens

<400> 99
ttacggaatg atcctgtgct c                                                21

<210> 100
<211> 20
<212> DNA
<213> Homo sapiens

<400> 100
agtcaggttt ccggtcacac                                                  20

<210> 101
<211> 22
<212> DNA
<213> Homo sapiens

<400> 101
ccgttcctta tatcctcagg tg                                               22

<210> 102
<211> 21
<212> DNA
<213> Homo sapiens

<400> 102
ccttgtacac actcgcactg a                                                21

<210> 103
<211> 20
<212> DNA
<213> Homo sapiens

<400> 103
tgttgtccac aggttccaga                                                  20

<210> 104
<211> 20
<212> DNA
<213> Homo sapiens

<400> 104
tgaggtttat gggcatggtt                                                  20

<210> 105
<211> 20
<212> DNA
<213> Homo sapiens

<400> 105
atgtttttcc ttggctgtgc                                                  20

<210> 106
<211> 20
<212> DNA
<213> Homo sapiens

<400> 106
atctgccctt tcttgtctga                                                    20

<210> 107
<211> 20
<212> DNA
<213> Homo sapiens

<400> 107
agggagctgc acagtggata                                                    20

<210> 108
<211> 24
<212> DNA
<213> Homo sapiens

<400> 108
tcactcccat atttcagaac ttga                                               24

<210> 109
<211> 22
<212> DNA
<213> Homo sapiens

<400> 109
tgtttattgg aagatcggtg aa                                                 22

<210> 110
<211> 25
<212> DNA
<213> Homo sapiens

<400> 110
cgttagagac tgaatctttg tcctg                                              25

<210> 111
<211> 20
<212> DNA
<213> Homo sapiens

<400> 111
agtcctgcct tccacagttg                                                    20

<210> 112
<211> 21
<212> DNA
<213> Homo sapiens

<400> 112
ggtagttacg tgttaggggc a                                                  21

<210> 113
<211> 21
<212> DNA
<213> Homo sapiens

<400> 113
caggaacatt aggccagatt g                                                21

<210> 114
<211> 23
<212> DNA
<213> Homo sapiens

<400> 114
catgtatgtg taggacagca tga                                              23

<210> 115
<211> 21
<212> DNA
<213> Homo sapiens

<400> 115
ctgtttcaaa gatgcttctg c                                                21

<210> 116
<211> 20
<212> DNA
<213> Homo sapiens

<400> 116
cctaggaagc tggaatgctg                                                  20

<210> 117
<211> 20
<212> DNA
<213> Homo sapiens

<400> 117
gggttcccag ggttcagtat                                                  20

<210> 118
<211> 23
<212> DNA
<213> Homo sapiens

<400> 118
cttgacctaa tttcaacatc tgg                                              23

<210> 119
<211> 20
<212> DNA
<213> Homo sapiens

<400> 119
atccccaact caaaaccaca                                                  20

<210> 120
<211> 21
<212> DNA
<213> Homo sapiens

<400> 120
aagtccaatt tagcccacgt t                                                21

<210> 121
<211> 20
<212> DNA
<213> Homo sapiens

<400> 121
ccagccattc aaaattctcc                                                  20

<210> 122
<211> 20
<212> DNA
<213> Homo sapiens

<400> 122
ggtgcaggtc aatttccaat                                                  20

<210> 123
<211> 20
<212> DNA
<213> Homo sapiens

<400> 123
ccccttcacc accattacaa                                                  20

<210> 124
<211> 20
<212> DNA
<213> Homo sapiens

<400> 124
tgtccaagga aaagcctcac                                                  20

<210> 125
<211> 20
<212> DNA
<213> Homo sapiens

<400> 125
aggacctctt gccagactca                                                  20

<210> 126
<211> 20
<212> DNA
<213> Homo sapiens

<400> 126
aggagatgac acaggccaag                                                  20

<210> 127
<211> 20
<212> DNA
<213> Homo sapiens

<400> 127
cgcacacctc tgaagctacc                                                    20

<210> 128
<211> 20
<212> DNA
<213> Homo sapiens

<400> 128
acctcactca cacctgggaa                                                    20

<210> 129
<211> 20
<212> DNA
<213> Homo sapiens

<400> 129
gcctcctgcc tgaaccttat                                                    20

<210> 130
<211> 23
<212> DNA
<213> Homo sapiens

<400> 130
caaaatcatg acaccaagtt gag                                                23

<210> 131
<211> 20
<212> DNA
<213> Homo sapiens

<400> 131
catgcacatg cacacacata                                                    20

<210> 132
<211> 20
<212> DNA
<213> Homo sapiens

<400> 132
ccttagcccg tgttgagcta                                                    20

<210> 133
<211> 21
<212> DNA
<213> Homo sapiens

<400> 133
tgcttttatt cagggactcc a                                                  21

<210> 134
<211> 20
<212> DNA
<213> Homo sapiens

<400> 134
cccatgcact gcagagattc                                                    20

<210> 135
<211> 19
<212> DNA
<213> Homo sapiens

<400> 135
aaggcaggag acatcgctt                                                     19

<210> 136
<211> 20
<212> DNA
<213> Homo sapiens

<400> 136
gggatcagca tggtttccta                                                    20

<210> 137
<211> 20
<212> DNA
<213> Homo sapiens

<400> 137
gcttaagtcc cactcctccc                                                    20

<210> 138
<211> 20
<212> DNA
<213> Homo sapiens

<400> 138
attttcctcc gcatgtgtgt                                                    20

<210> 139
<211> 20
<212> DNA
<213> Homo sapiens

<400> 139
tcacagaagc ctagccatga                                                    20

<210> 140
<211> 20
<212> DNA
<213> Homo sapiens

<400> 140
aacagagcag ggagatggtg                                                    20

<210> 141
<211> 20
<212> DNA
<213> Homo sapiens

<400> 141
tctgcacctc tcctcctctg                                                    20

<210> 142
<211> 20
<212> DNA
<213> Homo sapiens

<400> 142
actggggcca acattaatca                                                    20

<210> 143
<211> 20
<212> DNA
<213> Homo sapiens

<400> 143
cttccccatc tgcaacaaac                                                    20

<210> 144
<211> 20
<212> DNA
<213> Homo sapiens

<400> 144
gctaaaggcc atccaaagaa                                                    20

<210> 145
<211> 20
<212> DNA
<213> Homo sapiens

<400> 145
tcaagtgcat ctgggcataa                                                    20

<210> 146
<211> 20
<212> DNA
<213> Homo sapiens

<400> 146
tctgaagtcc attcccttgg                                                    20

<210> 147
<211> 20
<212> DNA
<213> Homo sapiens

<400> 147
caatgtggca tgcagttgat                                                    20

<210> 148
<211> 19
<212> DNA
<213> Homo sapiens

<400> 148
gaagctacca gcccatcct                                                    19

<210> 149
<211> 20
<212> DNA
<213> Homo sapiens

<400> 149
catttccccc actgtttcag                                                   20

<210> 150
<211> 20
<212> DNA
<213> Homo sapiens

<400> 150
ccaaggcttt cttcaatcca                                                   20

<210> 151
<211> 20
<212> DNA
<213> Homo sapiens

<400> 151
gatccgttta acctgccaac                                                   20

<210> 152
<211> 19
<212> DNA
<213> Homo sapiens

<400> 152
atgcccctgc caactttac                                                    19

<210> 153
<211> 20
<212> DNA
<213> Homo sapiens

<400> 153
ctctgcagct gttcccctac                                                   20

<210> 154
<211> 20
<212> DNA
<213> Homo sapiens

<400> 154
tatcaatcca tggccctgac                                                   20

<210> 155
<211> 20
<212> DNA
<213> Homo sapiens

<400> 155
agagtccctg ccctccttct                                                    20

<210> 156
<211> 20
<212> DNA
<213> Homo sapiens

<400> 156
aaggcagtca gcagtgtcaa                                                    20

<210> 157
<211> 20
<212> DNA
<213> Homo sapiens

<400> 157
ggggaacatc ctgtgcttag                                                    20

<210> 158
<211> 20
<212> DNA
<213> Homo sapiens

<400> 158
ccattggtga gtgtttccct                                                    20

<210> 159
<211> 20
<212> DNA
<213> Homo sapiens

<400> 159
agtcagcaaa ctgctgggtt                                                    20

<210> 160
<211> 20
<212> DNA
<213> Homo sapiens

<400> 160
attgctccat cctggcataa                                                    20

<210> 161
<211> 23
<212> DNA
<213> Homo sapiens

<400> 161
tcatggatga ttttatgtgc ttc                                                23

<210> 162
<211> 20
<212> DNA
<213> Homo sapiens

<400> 162
gcgtgtggaa aagccataag                                                    20

<210> 163
<211> 20
<212> DNA
<213> Homo sapiens

<400> 163
gccaatcata caacagccct                                                    20

<210> 164
<211> 23
<212> DNA
<213> Homo sapiens

<400> 164
tgatcgcata ttctacttgg aaa                                                23

<210> 165
<211> 22
<212> DNA
<213> Homo sapiens

<400> 165
tccctttatt ttagaggcac ca                                                 22

<210> 166
<211> 21
<212> DNA
<213> Homo sapiens

<400> 166
gatcaggaat tcaagcacca a                                                  21

<210> 167
<211> 24
<212> DNA
<213> Homo sapiens

<400> 167
tgggttccat aatagagttt caca                                               24

<210> 168
<211> 22
<212> DNA
<213> Homo sapiens

<400> 168
tgtcagctgt tactggaagt gg                                                 22

<210> 169
<211> 22
<212> DNA
<213> Homo sapiens

<400> 169
tgtcagctgc tgctggaagt gg                                                22

<210> 170
<211> 21
<212> DNA
<213> Homo sapiens

<400> 170
aggagctggc cgaagccaca a                                                 21

<210> 171
<211> 21
<212> DNA
<213> Homo sapiens

<400> 171
aggagctggc tgaagccaca a                                                 21

<210> 172
<211> 21
<212> DNA
<213> Homo sapiens

<400> 172
aatgatgcca ccaaacaaat g                                                 21

<210> 173
<211> 21
<212> DNA
<213> Homo sapiens

<400> 173
aatgatgcca tcaaacaaat g                                                 21

<210> 174
<211> 21
<212> DNA
<213> Homo sapiens

<400> 174
gaggtggctc cgatgaccac a                                                 21

<210> 175
<211> 21
<212> DNA
<213> Homo sapiens

<400> 175
gaggtggctc tgatgaccac a                                                 21

<210> 176
<211> 21
<212> DNA
<213> Homo sapiens

<400> 176
ttccttaaca gaaatagtat c                                                21

<210> 177
<211> 21
<212> DNA
<213> Homo sapiens

<400> 177
ttccttaaca aaaatagtat c                                                21

<210> 178
<211> 21
<212> DNA
<213> Homo sapiens

<400> 178
ggaagtgttc caaaagagaa a                                                21

<210> 179
<211> 21
<212> DNA
<213> Homo sapiens

<400> 179
ggaagtgttc taaaagagaa a                                                21

<210> 180
<211> 21
<212> DNA
<213> Homo sapiens

<400> 180
agtaaagagg gactagactt t                                                21

<210> 181
<211> 21
<212> DNA
<213> Homo sapiens

<400> 181
agtaaagagg aactagactt t                                                21

<210> 182
<211> 21
<212> DNA
<213> Homo sapiens

<400> 182
gcctacttgc aggatgtggt g                                                21

<210> 183
<211> 21
<212> DNA
<213> Homo sapiens

<400> 183
gcctacttgc gggatgtggt g                                                21

<210> 184
<211> 23
<212> DNA
<213> Homo sapiens

<400> 184
cctcattcct cttcttgtga gcg                                              23

<210> 185
<211> 20
<212> DNA
<213> Homo sapiens

<400> 185
cctcattcct cttgtgagcg                                                  20

<210> 186
<211> 21
<212> DNA
<213> Homo sapiens

<400> 186
gcaggactac gtgggcttca c                                                21

<210> 187
<211> 21
<212> DNA
<213> Homo sapiens

<400> 187
gcaggactac atgggcttca c                                                21

<210> 188
<211> 21
<212> DNA
<213> Homo sapiens

<400> 188
aaaagtctac cgagatggga t                                                21

<210> 189
<211> 21
<212> DNA
<213> Homo sapiens

<400> 189
aaaagtctac tgagatggga t                                                21

<210> 190
<211> 21
<212> DNA
<213> Homo sapiens

<400> 190
ggccagatca cctccttcct g                                                21

<210> 191
<211> 21
<212> DNA
<213> Homo sapiens

<400> 191
ggccagatca tctccttcct g                                                21

<210> 192
<211> 21
<212> DNA
<213> Homo sapiens

<400> 192
acacaccaca tggatgaagc g                                                21

<210> 193
<211> 21
<212> DNA
<213> Homo sapiens

<400> 193
acacaccaca cggatgaagc g                                                21

<210> 194
<211> 21
<212> DNA
<213> Homo sapiens

<400> 194
cctggaagaa gtaagttaag t                                                21

<210> 195
<211> 21
<212> DNA
<213> Homo sapiens

<400> 195
cctggaagaa ctaagttaag t                                                21

<210> 196
<211> 21
<212> DNA
<213> Homo sapiens

<400> 196
gctgcctgtg tgtcccccag g                                                21

<210> 197
<211> 21
<212> DNA
<213> Homo sapiens

<400> 197
gctgcctgtg cgtcccccag g                                                21

<210> 198
<211> 22
<212> DNA
<213> Homo sapiens

<400> 198
tagccattat ggaattactg ct                                               22

<210> 199
<211> 21
<212> DNA
<213> Homo sapiens

<400> 199
tagccattat caattactgc t                                                21

<210> 200
<211> 26
<212> DNA
<213> Homo sapiens

<400> 200
gatgaagatg aagatgtgag gcggga                                           26

<210> 201
<211> 20
<212> DNA
<213> Homo sapiens

<400> 201
gatgaagatg tgaggcggga                                                  20

<210> 202
<211> 21
<212> DNA
<213> Homo sapiens

<400> 202
aatagttgta cgaatagcag g                                                21

<210> 203
<211> 21
<212> DNA
<213> Homo sapiens

<400> 203
aatagttgta tgaatagcag g                                                21

<210> 204
<211> 21
<212> DNA
<213> Homo sapiens

<400> 204
acacgctggg ggtgctggct g                                                21

<210> 205
<211> 21
<212> DNA
<213> Homo sapiens

<400> 205
acacgctggg cgtgctggct g                                                21

<210> 206
<211> 20
<212> DNA
<213> Homo sapiens

<400> 206
gaccagccac ggcgtccctg                                                  20

<210> 207
<211> 21
<212> DNA
<213> Homo sapiens

<400> 207
gaccagccac gggcgtccct g                                                21

<210> 208
<211> 22
<212> DNA
<213> Homo sapiens

<400> 208
cattttctta gaaaagagag gt                                               22

<210> 209
<211> 22
<212> DNA
<213> Homo sapiens

<400> 209
cattttctta gagaagagag gt                                               22

<210> 210
<211> 21
<212> DNA
<213> Homo sapiens

<400> 210
gaaaattagt atgtaaggaa g                                                21

<210> 211
<211> 21
<212> DNA
<213> Homo sapiens

<400> 211
gaaaattagt ctgtaaggaa g                                                21

<210> 212
<211> 25
<212> DNA
<213> Homo sapiens

<400> 212
cctccgcctg ccaggttcag cgatt                                            25

<210> 213
<211> 25
<212> DNA
<213> Homo sapiens

<400> 213
cctccgcctg ccgggttcag cgatt                                            25

<210> 214
<211> 25
<212> DNA
<213> Homo sapiens

<400> 214
tatgtgctga ccatgggagc ttgtt                                            25

<210> 215
<211> 25
<212> DNA
<213> Homo sapiens

<400> 215
tatgtgctga ccgtgggagc ttgtt                                            25

<210> 216
<211> 21
<212> DNA
<213> Homo sapiens

<400> 216
gtgacaccca acggagtagg g                                                21

<210> 217
<211> 21
<212> DNA
<213> Homo sapiens

<400> 217
gtgacaccca gcggagtagg g                                                21

<210> 218
<211> 21
<212> DNA
<213> Homo sapiens

<400> 218
agtatccctt gttcacgaga a                                                21

<210> 219
<211> 25
<212> DNA
<213> Homo sapiens

<400> 219
agtatccctc ccttgttcac gagaa                                            25

<210> 220
<211> 21
<212> DNA
<213> Homo sapiens

<400> 220
ctgggttcct gtatcacaac c                                                21

<210> 221
<211> 21
<212> DNA
<213> Homo sapiens

<400> 221
ctgggttcct atatcacaac c                                                21

<210> 222
<211> 21
<212> DNA
<213> Homo sapiens

<400> 222
ggcctaccaa gggagaaact g                                                21

<210> 223
<211> 21
<212> DNA
<213> Homo sapiens

<400> 223
ggcctaccaa aggagaaact g                                                21

<210> 224
<211> 20
<212> DNA
<213> Homo sapiens

<400> 224
tttaaagggg gtgattagga                                                  20

<210> 225
<211> 20
<212> DNA
<213> Homo sapiens

<400> 225
tttaaagggg ttgattagga                                                    20

<210> 226
<211> 22
<212> DNA
<213> Homo sapiens

<400> 226
gaagaaattt gtttttttga tt                                                 22

<210> 227
<211> 22
<212> DNA
<213> Homo sapiens

<400> 227
   gaagaaattt ttttttttga tt                                                  22

<210> 228
<211> 21
<212> DNA
<213> Homo sapiens

<400> 228
gcgggcatcc cgagggaggg g                                                  21

<210> 229
<211> 21
<212> DNA
<213> Homo sapiens

<400> 229
gcgggcatcc tgagggaggg g                                                  21

<210> 230
<211> 21
<212> DNA
<213> Homo sapiens

<400> 230
agggaggggg gctgaagatc a                                                  21

<210> 231
<211> 21
<212> DNA
<213> Homo sapiens

<400> 231
agggaggggg actgaagatc a                                                  21

<210> 232
<211> 20
<212> DNA
<213> Homo sapiens

<400> 232
aggagccaaa cgctcattgt                                                    20

<210> 233
<211> 21
<212> DNA
<213> Homo sapiens

<400> 233
aggagccaaa gcgctcattg t                                                  21

<210> 234
<211> 21
<212> DNA
<213> Homo sapiens

<400> 234
aagccactgt ttttaaccag t                                                  21

<210> 235
<211> 21
<212> DNA
<213> Homo sapiens

<400> 235
aagccactgt atttaaccag t                                                  21

<210> 236
<211> 21
<212> DNA
<213> Homo sapiens

<400> 236
cgtgggcttc acactcaaga t                                                  21

<210> 237
<211> 21
<212> DNA
<213> Homo sapiens

<400> 237
cgtgggcttc ccactcaaga t                                                  21

<210> 238
<211> 21
<212> DNA
<213> Homo sapiens

<400> 238
tcacactcaa gatcttcgct g                                                  21

<210> 239
<211> 21
<212> DNA
<213> Homo sapiens

<400> 239
tcacactcaa catcttcgct g                                                21

<210> 240
<211> 21
<212> DNA
<213> Homo sapiens

<400> 240
gcagcctcac ccgctcttcc c                                                21

<210> 241
<211> 21
<212> DNA
<213> Homo sapiens

<400> 241
gcagcctcac tcgctcttcc c                                                21

<210> 242
<211> 21
<212> DNA
<213> Homo sapiens

<400> 242
agaagagaat atcagaaatc t                                                21

<210> 243
<211> 21
<212> DNA
<213> Homo sapiens

<400> 243
agaagagaat gtcagaaatc t                                                21

<210> 244
<211> 21
<212> DNA
<213> Homo sapiens

<400> 244
gcgcagtgcc ctgtgtcctt a                                                21

<210> 245
<211> 21
<212> DNA
<213> Homo sapiens

<400> 245
gcgcagtgcg ctgtgtcctt a                                                21

<210> 246
<211> 21
<212> DNA
<213> Homo sapiens

<400> 246
gatctaaggt tgtcattctg g                                                21

<210> 247
<211> 21
<212> DNA
<213> Homo sapiens

<400> 247
gatctaaggt ggtcattctg g                                                21

<210> 248
<211> 23
<212> DNA
<213> Homo sapiens

<400> 248
ctcttctgtt agcacagaag aga                                              23

<210> 249
<211> 23
<212> DNA
<213> Homo sapiens

<400> 249
ctcttctgtt atcacagaag aga                                              23

<210> 250
<211> 21
<212> DNA
<213> Homo sapiens

<400> 250
cattctaggg atcatagcca t                                                21

<210> 251
<211> 21
<212> DNA
<213> Homo sapiens

<400> 251
cattctaggg gtcatagcca t                                                21

<210> 252
<211> 22
<212> DNA
<213> Homo sapiens

<400> 252
aagtacagtg ggaggaacag cg                                               22

<210> 253
<211> 22
<212> DNA
<213> Homo sapiens

<400> 253
aagtacagtg tgaggaacag cg                                                22

<210> 254
<211> 22
<212> DNA
<213> Homo sapiens

<400> 254
attcctaaaa aatagaaatg ca                                                22

<210> 255
<211> 22
<212> DNA
<213> Homo sapiens

<400> 255
attcctaaaa agtagaaatg ca                                                22

<210> 256
<211> 21
<212> DNA
<213> Homo sapiens

<400> 256
ggcccctgcc ttattattac t                                                 21

<210> 257
<211> 21
<212> DNA
<213> Homo sapiens

<400> 257
ggcccctgcc gtattattac t                                                 21

<210> 258
<211> 22
<212> DNA
<213> Homo sapiens

<400> 258
tgagagaatt acttgaaccc gg                                                22

<210> 259
<211> 22
<212> DNA
<213> Homo sapiens

<400> 259
tgagagaatt gcttgaaccc gg                                                22

<210> 260
<211> 21
<212> DNA
<213> Homo sapiens

<400> 260
tttgctgaaa caatcactga c                                                21

<210> 261
<211> 21
<212> DNA
<213> Homo sapiens

<400> 261
tttgctgaaa taatcactga c                                                21

<210> 262
<211> 22
<212> DNA
<213> Homo sapiens

<400> 262
aacctcagtt ccctcatctg tg                                               22

<210> 263
<211> 22
<212> DNA
<213> Homo sapiens

<400> 263
aacctcagtt tcctcatctg tg                                               22

<210> 264
<211> 21
<212> DNA
<213> Homo sapiens

<400> 264
ctggacacca gaaataatgt c                                                21

<210> 265
<211> 21
<212> DNA
<213> Homo sapiens

<400> 265
ctggacacca aaaataatgt c                                                21

<210> 266
<211> 21
<212> DNA
<213> Homo sapiens

<400> 266
tcctatgtgt cctccaccaa t                                                21

<210> 267
<211> 21
<212> DNA
<213> Homo sapiens

<400> 267
tcctatgtgt gctccaccaa t                                                21

<210> 268
<211> 21
<212> DNA
<213> Homo sapiens

<400> 268
aagaagtggc ttgtattttg c                                                21

<210> 269
<211> 21
<212> DNA
<213> Homo sapiens

<400> 269
aagaagtggc ctgtattttg c                                                21

<210> 270
<211> 23
<212> DNA
<213> Homo sapiens

<400> 270
aactgatttg attggtatag ctg                                              23

<210> 271
<211> 23
<212> DNA
<213> Homo sapiens

<400> 271
aactgatttg gttggtatag ctg                                              23

<210> 272
<211> 21
<212> DNA
<213> Homo sapiens

<400> 272
cagggtccaa cccggacctg a                                                21

<210> 273
<211> 21
<212> DNA
<213> Homo sapiens

<400> 273
cagggtccaa tccggacctg a                                                21

<210> 274
<211> 22
<212> DNA
<213> Homo sapiens

<400> 274
ttgggaggct aaggcaggag aa                                                22

<210> 275
<211> 22
<212> DNA
<213> Homo sapiens

<400> 275
ttgggaggct gaggcaggag aa                                                22

<210> 276
<211> 15
<212> DNA
<213> Gallus gallus

<400> 276
accaggggaa tctcc                                                        15

<210> 277
<211> 15
<212> DNA
<213> Gallus gallus

<400> 277
accagggaaa tctcc                                                        15

<210> 278
<211> 45
<212> DNA
<213> Gallus gallus

<400> 278
cgctacccaa caccagggga atctcctggt attgttggaa acttc                       45

<210> 279
<211> 15
<212> PRT
<213> Homo sapiens

<400> 279
Arg Tyr Pro Thr Pro Gly Glu Ala Pro Gly Val Val Gly Asn Phe
 1               5                  10                  15

<210> 280
<211> 15
<212> PRT
<213> Mus musculus

<400> 280

Arg Tyr Pro Thr Pro Gly Glu Ala Pro Gly Val Val Gly Asn Phe
1               5                   10                  15

<210> 281
<211> 15
<212> PRT
<213> Gallus gallus

<400> 281
Arg Tyr Pro Thr Pro Gly Glu Ser Pro Gly Ile Val Gly Asn Phe
1               5                   10                  15

<210> 282
<211> 15
<212> PRT
<213> Gallus gallus

<400> 282
Arg Tyr Pro Thr Pro Gly Lys Ser Pro Gly Ile Val Gly Asn Phe
1               5                   10                  15

<210> 283
<211> 45
<212> DNA
<213> Gallus gallus

<400> 283
cgctacccaa caccagggaa atctcctggt attgttggaa acttc                45

<210> 284
<211> 19
<212> DNA
<213> Homo sapiens

<400> 284
gcgtcaggga tggggacag                                              19

<210> 285
<211> 20
<212> DNA
<213> Homo sapiens

<400> 285
gcgtcaggga ttggggacag                                             20

<210> 286
<211> 17
<212> DNA
<213> Homo sapiens

<400> 286
ccacttcggt ctccatg                                                17

<210> 287
<211> 17

<212> DNA
<213> Homo sapiens

<400> 287
ccacttcgat ctccatg                                                    17

## Description

### Background of the Invention

Low HDL cholesterol (HDL-C), or hypoalphalipoproteinemia, is a blood lipid abnormality which correlates with a high risk of cardiovascular disease (CVD), in particular coronary artery disease (CAD), but also cerebrovascular disease, coronary restenosis, and peripheral vascular disease. HDL, or 'good cholesterol' levels are influenced by both environmental and genetic factors.

Epidemiological studies have consistently demonstrated that plasma HDL-C concentration is inversely related to the incidence of CAD. HDL-C levels are a strong graded and independent cardiovascular risk factor. Protective effects of an elevated HDL-C persist until 80 years of age. A low HDL-C is associated with an increased CAD risk even with normal (<5.2 mmol/l) total plasma cholesterol levels. Coronary disease risk is increased by 2% in men and 3% in women for every 1 mg/dL (0.026 mmol/l) reduction in HDL-C and in the majority of studies this relationship is statistically significant even after adjustment for other lipid and non-lipid risk factors. Decreased HDL-C levels are the most common lipoprotein abnormality seen in patients with premature CAD. Four percent of patients with premature CAD with have an isolated form of decreased HDL-C levels with no other lipoprotein abnormalities while 25% have low HDL levels with accompanying hypertriglyceridemia.

Even in the face of other dyslipidemias or secondary factors, HDL-C levels are important predictors of CAD. In a cohort of diabetics, those with isolated low HDL cholesterol had a 65% increased death rate compared to diabetics with normal HDL cholesterol levels (>0.9 mmol/1). Furthermore, it has been shown that even within high risk populations, such as those with familial hypercholesterolemia, HDL cholesterol level is an important predictor of CAD. Low HDL cholesterol levels thus constitute a major, independent, risk for CAD.

These findings have led to increased attention to HDL cholesterol levels as a focus for treatment, following the recommendations of the National Cholesterol Education Program. These guidelines suggest that HDL cholesterol values below 0.9 mmol/l confer a significant risk for men and women. As such, nearly half of patients with CAD would have low HDL cholesterol. It is therefore crucial that we obtain a better understanding of factors which contribute to this phenotype. In view of the fact that pharmacological intervention of low HDL cholesterol levels has so far proven unsatisfactory, it is also important to understand the factors that regulate these levels in the circulation as this understanding may reveal new therapeutic targets.

Absolute levels of HDL cholesterol may not always predict risk of CAD. In the case of CETP deficiency, individuals display an increased risk of developing CAD, despite increased HDL cholesterol levels. What seems to be important in this case is the functional activity of the reverse cholesterol transport pathway, the process by which intracellular cholesterol is trafficked out of the cell to acceptor proteins such as ApoAl or HDL. Other important genetic determinants of HDL cholesterol levels, and its inverse relation with CAD, may reside in the processes leading to HDL formation and intracellular cholesterol trafficking and efflux. To date, this process is poorly understood, however, and clearly not all of the components of this pathway have been identified. Thus, defects preventing proper HDL-mediated cholesterol efflux may be important predictors of CAD. Therefore it is critical to identify and understand novel genes involved in the intracellular cholesterol trafficking and efflux pathways.

HDL particles are central to the process of reverse cholesterol transport and thus to the maintenance of tissue cholesterol homeostasis. This process has multiple steps which include the binding of HDL to cell surface components, the acquisition of cholesterol by passive absorption, the esterification of this cholesterol by LCAT and the subsequent transfer of esterified cholesterol by CETP, to VLDL and chylomicron remnants for liver uptake. Each of these steps is known to impact the plasma concentration of HDL.

Changes in genes for ApoAI-CIII, lipoprotein lipase, CETP, hepatic lipase, and LCAT all contribute to determination of HDL-C levels in humans. One rare form of genetic HDL deficiency is Tangier disease (TD), diagnosed in approximately 40 patients world-wide, and associated with almost complete absence of HDL cholesterol (HDL-C) levels (listed in OMIM as an autosomal recessive trait (OMIM 205400)). These patients have very low HDL cholesterol and ApoAI levels, which have been ascribed to hypercatabolism of nascent HDL and ApoAI, due to a delayed acquisition of lipid and resulting failure of conversion to mature HDL. TD patients accumulate cholesterol esters in several tissues, resulting in characteristic features, such as enlarged yellow tonsils, hepatosplenomegaly, peripheral neuropathy, and cholesterol ester deposition in the rectal mucosa. Defective removal of cellular cholesterol and phospholipids by ApoAI as well as a marked deficiency in HDL mediated efflux of intracellular cholesterol has been demonstrated in TD fibroblasts. Even though this is a rare disorder, defining its molecular basis could identify pathways relevant for cholesterol regulation in the general population. The decreased availability of free cholesterol for efflux in the surface membranes of cells in Tangier Disease patients appears to be due to a defect in cellular lipid metabolism or trafficking. Approximately 45% of Tangier patients have signs of premature CAD, suggesting a strong link between decreased cholesterol efflux, low HDL cholesterol and CAD. As increased cholesterol is observed in the rectal mucosa of persons with TD, the molecular mechanism responsible for TD may also regulate cholesterol adsorption from the gastrointestinal (GI) tract.

A more common form of genetic HDL deficiency occurs in patients who have low plasma HDL cholesterol usually below the 5th percentile for age and sex (OMIM 10768), but an absence of clinical manifestations specific to Tangier disease (Marcil et al., Arterioscler. Thromb. Vasc. Biol. 19:159-169, 1999; Marcil et al., Arterioscler. Thromb. Vasc. Biol. 15:1015-1024, 1995). These patients have no obvious environmental factors associated with this lipid phenotype, and do not have severe hypertriglyceridemia nor have known causes of severe HDL deficiency (mutations in ApoAI, LCAT, or LPL deficiency) and are not diabetic. The pattern of inheritance of this condition is most consistent with a Mendelian dominant trait (OMIM 10768).

The development of drugs that regulate cholesterol metabolism has so far progressed slowly. Thus, there is a need for a better understanding of the genetic components of the cholesterol efflux pathway. Newly-discovered components can then serve as targets for drug design.

Low HDL levels are likely to be due to multiple genetic factors. The use of pharmacogenomics in the aid of designing treatment tailored to the patient makes it desirable to identify polymorphisms in components of the cholesterol efflux pathway. An understanding of the effect of these polymorphisms on protein function would allow for the design of a therapy that is optimal for the patient.

### Summary of the Invention

In a first aspect, the invention features a substantially pure ABC1 polypeptide having ABC1 biological activity. Preferably, the ABC 1 polypeptide is human ABC 1 (e.g., one that includes amino acids 1 to 60 or amino acids 61 to 2261 of SEQ ID NO: 1). In one preferred embodiment, the ABC1 polypeptide includes amino acids 1 to 2261 of SEQ ID NO: 1.

Specifically excluded from the polypeptides of the invention are the polypeptide having the exact amino acid sequence as GenBank accession number CAA10005.1 and the nucleic acid having the exact sequence as AJ012376.1. Also excluded is protein having the exact amino acid sequence as GenBank accession number X75926.

In a related aspect, the invention features a substantially pure ABC1 polypeptide that includes amino acids 1 to 2261 of SEQ ID NO: 1.

In another aspect, the invention features a substantially pure nucleic acid molecule encoding an ABC1 polypeptide having ABC1 biological activity (e.g., a nucleic acid molecule that includes nucleotides 75 to 254 or nucleotides 255 to 6858 of SEQ ID NO: 2). In one preferred embodiment, the nucleic acid molecule includes nucleotides 75 to 6858 of SEQ ID NO: 2.

In a related aspect, the invention features an expression vector, a cell, or a non-human mammal that includes the nucleic acid molecule of the invention.

In yet another aspect, the invention features a substantially pure nucleic acid molecule that includes nucleotides 75 to 254 of SEQ ID NO: 2, nucleotides 255 to 6858 of SEQ ID NO: 2, or nucleotides 75 to 6858 of SEQ ID NO: 2.

In still another aspect, the invention features a substantially pure nucleic acid molecule that includes at least fifteen nucleotides corresponding to the 5' or 3' untranslated region from a human ABC 1 gene. Preferably, the 3' untranslated region includes nucleotides 7015-7860 of SEQ ID NO: 2.

In a related aspect, the invention features a substantially pure nucleic acid molecule that hybridizes at high stringency to a probe comprising nucleotides 7015-7860 of SEQ ID NO: 2.

In another aspect, the invention features a method of treating a human having low HDL cholesterol or a cardiovascular disease, including administering to the human an ABC1 polypeptide, or cholesterol-regulating fragment thereof, or a nucleic acid molecule encoding an ABC1 polypeptide, or cholesterol-regulating fragment thereof. In a preferred embodiment, the human has a low HDL cholesterol level relative to normal. Preferably, the ABC1 polypeptide is wild-type ABC1, or has a mutation increases its stability or its biological activity. A preferred biological activity is regulation of cholesterol.

In a related aspect, the invention features a method of preventing or treating cardiovascular disease, including introducing into a human an expression vector comprising an *ABC1* nucleic acid molecule operably linked to a promoter and encoding an ABC1 polypeptide having ABC1 biological activity.

In another related aspect, the invention features a method of preventing or ameliorating the effects of a disease-causing mutation in an *ABC1* gene, including introducing into a human an expression vector comprising an *ABC1* nucleic acid molecule operably linked to a promoter and encoding an ABC1 polypeptide having ABC1 biological activity.

In still another aspect, the invention features a method of treating or preventing cardiovascular disease, including administering to an animal (e.g., a human) a compound that mimes the activity of wild-type ABC1 or modulates the biological activity of ABC1.

One preferred cardiovascular disease that can be treated using the methods of the invention is coronary artery disease. Others include cerebrovascular disease and peripheral vascular disease.

The discovery that the ABC1 gene and protein are involved in cholesterol transport that affects serum HDL levels allows the ABC1 protein and gene to be used in a variety of diagnostic tests and assays for identification of HDL-increasing or CVD-inhibiting drugs. In one family of such assays, the ability of domains of the ABC1 protein to bind ATP is utilized; compounds that enhance this binding are potential HDL-increasing drugs. Similarly, the anion transport capabilities and membrane pore-forming functions in cell membranes can be used for drug screening.

ABC1 expression can also serve as a diagnostic tool for low HDL or CVD; determination of the genetic subtyping of the *ABC1* gene sequence can be used to subtype low HDL individuals or families to determine whether the low HDL phenotype is related to ABC1 function. This diagnostic process can lead to the tailoring of drug treatments according to patient genotype (referred to as pharmacogenomics), including prediction of the patient's response (e.g., increased or decreased efficacy or undesired side effects upon administration of a compound or drug.

Antibodies to an ABC1 polypeptide can be used both as therapeutics and diagnostics. Antibodies are produced by immunologically challenging a B-cell-containing biological system, e.g., an animal such as a mouse, with an ABC 1 polypeptide to stimulate production of anti-ABC 1 protein by the B-cells, followed by isolation of the antibody from the biological system. Such antibodies can be used to measure ABC1 polypeptide in a biological sample such as serum, by contacting the sample with the antibody and then measuring immune complexes as a measure of the ABC 1 polypeptide in the sample. Antibodies to ABC 1 can also be used as therapeutics for the modulation of ABC1 biological activity.

Thus, in another aspect, the invention features a purified antibody that specifically binds to ABC1.

In yet another aspect, the invention features a method for determining whether a candidate compound modulates ABC 1 biological activity, comprising: (a) providing an ABC1 polypeptide; (b) contacting the ABC1 polypeptide with the candidate compound; and (c) measuring ABC1 biological activity, wherein altered ABC1 biological activity, relative to an ABC 1 polypeptide not contacted with the compound, indicates that the candidate compound modulates ABC1 biological activity. Preferably, the ABC 1 polypeptide is in a cell or is in a cell-free assay system.

In still another aspect, the invention features a method for determining whether a candidate compound modulates ABC 1 expression. The method includes (a) providing a nucleic acid molecule comprising an ABC1 promoter operably linked to a reporter gene; (b) contacting the nucleic acid molecule with the candidate compound; and (c) measuring reporter gene expression, wherein altered reporter gene expression, relative to a nucleic acid molecule not contacted with the compound, indicates that the candidate compound modulates ABC 1 expression.

In another aspect, the invention features a method for determining whether candidate compound is useful for modulating cholesterol levels, the method including the steps of: (a) providing an ABC1 polypeptide; (b) contacting the polypeptide with the candidate compound; and (c) measuring binding of the ABC1 polypeptide, wherein binding of the ABC1 polypeptide indicates that the candidate compound is useful for modulating cholesterol levels.

In a related aspect, the invention features method for determining whether a candidate compound mimics ABC1 biological activity. The method includes (a) providing a cell that is not expressing an ABC1 polypeptide; (b) contacting the cell with the candidate compound; and (c) measuring ABC1 biological activity of the cell, wherein altered ABC1 biological activity, relative to a cell not contacted with the compound, indicates that the candidate compound modulates ABC 1 biological activity. Preferably, the cell has an *ABC1* null mutation. In one preferred embodiment, the cell is in a mouse or a chicken (e.g., a WHAM chicken) in which its *ABCI* gene has been mutated.

In still another aspect, the invention features a method for determining whether a candidate compound is useful for the treatment of low HDL cholesterol. The method includes (a) providing an ABC transporter (e.g., ABC1); (b) contacting the transporter with the candidate compound; and (c) measuring ABC transporter biological activity, wherein increased ABC transporter biological activity, relative to a transporter not contacted with the compound, indicates that the candidate compound is useful for the treatment of low HDL cholesterol. Preferably the ABC transporter is in a cell or a cell free assay system.

In yet another aspect, the invention features a method for determining whether candidate compound is useful for modulating cholesterol levels. The method includes (a) providing a nucleic acid molecule comprising an ABC transporter promoter operably linked to a reporter gene; (b) contacting the nucleic acid molecule with the candidate compound; and (c) measuring expression of the reporter gene, wherein increased expression of the reporter gene, relative to a nucleic acid molecule not contacted with the compound, indicates that the candidate compound is useful for modulating cholesterol levels.

In still another aspect, the invention features a method for determining whether a candidate compound increases the stability or decreases the regulated catabolism of an ABC transporter polypeptide. The method includes (a) providing an ABC transporter polypeptide; (b) contacting the transporter with the candidate compound; and (c) measuring the half-life of the ABC transporter polypeptide, wherein an increase in the half-life, relative to a transporter not contacted with the compound, indicates that the candidate compound increases the stability or decreases the regulated catabolism of an ABC transporter polypeptide. Preferably the ABC transporter is in a cell or a cell free assay system.

In a preferred embodiment of the screening methods of the present invention, the cell is in an animal. The preferred ABC transporters are ABC1, ABC2, ABCR, and ABC8, and the preferred biological activity is transport of cholesterol (e.g., HDL cholesterol or LDL cholesterol) or interleukin-1, or is binding or hydrolysis of ATP by the ABC1 polypeptide.

Preferably, the ABC1 polypeptide used in the screening methods includes amino acids 1-60 of SEQ ID NO: 1. Alternatively, the ABC1 polypeptide can include a region encoded by a nucleotide sequence that hybridizes under high stringency conditions to nucleotides 75 to 254 of SEQ ID NO: 2.

In another aspect, the invention features a method for determining whether a patient has an increased risk for cardiovascular disease. The method includes determining whether an *ABC1* gene of the patient has a mutation, wherein a mutation indicates that the patient has an increased risk for cardiovascular disease.

In related aspect, the invention features a method for determining whether a patient has an increased risk for cardiovascular disease. The method includes determining whether an *ABC1* gene of the patient has a polymorphism, wherein a polymorphism indicates that the patient has an increased risk for cardiovascular disease.

In another aspect, the invention features a method for determining whether a patient has an increased risk for cardiovascular disease. The method includes measuring ABC1 biological activity in the patient, wherein increased or decreased levels in the ABC1 biological activity, relative to normal levels, indicates that the patient has an increased risk for cardiovascular disease.

In still another aspect, the invention features a method for determining whether a patient has an increased risk for cardiovascular disease. The method includes measuring ABCl expression in the patient, wherein decreased levels in the ABC1 expression relative to normal levels, indicates that the patient has an increased risk for cardiovascular disease. Preferably, the ABC1 expression is determined by measuring levels of ABC1 polypeptide or *ABC1* RNA.

In another aspect, the invention features a non-human mammal having a transgene comprising a nucleic acid molecule encoding a mutated ABC 1 polypeptide. In one embodiment, the mutation is a dominant-negative mutation.

In a related aspect, the invention features a non-human mammal, having a transgene that includes a nucleic acid molecule encoding an ABC1 polypeptide having ABC1 biological activity.

In another related aspect, the invention features a cell from a non-human mammal having a transgene that includes a nucleic acid molecule encoding an ABC 1 polypeptide having ABC1 biological activity.

In still another aspect, the invention features a method for determining whether a candidate compound decreases the inhibition of a dominant-negative ABC1 polypeptide. The method includes (a) providing a cell expressing a dominant-negative ABC polypeptide; (b) contacting the cell with the candidate compound; and (c) measuring ABC 1 biological activity of the cell, wherein an increase in the ABC I biological activity, relative to a cell not contacted with the compound, indicates that the candidate compound decreases the inhibition of a dominant-negative ABC1 polypeptide.

By "polypeptide" is meant any chain of more than two amino acids, regardless of post-translational modification such as glycosylation or phosphorylation.

By "substantially identical" is meant a polypeptide or nucleic acid exhibiting at least 50%, preferably 85%, more preferably 90%, and most preferably 95% identity to a reference amino acid or nucleic acid sequence. For polypeptides, the length of comparison sequences will generally be at least 16 amino acids, preferably at least 20 amino acids, more preferably at least 25 amino acids, and most preferably 35 amino acids. For nucleic acids, the length of comparison sequences will generally be at least 50 nucleotides, preferably at least 60 nucleotides, more preferably at least 75 nucleotides, and most preferably 110 nucleotides.

Sequence identity is typically measured using sequence analysis software with the default parameters specified therein (e.g., Sequence Analysis Software Package of the Genetics Computer Group, University of Wisconsin Biotechnology Center, 1710 University Avenue, Madison, WI 53705). This software program matches similar sequences by assigning degrees of homology to various substitutions, deletions, and other modifications. Conservative substitutions typically include substitutions within the following groups: glycine, alanine, valine, isoleucine, leucine; aspartic acid, glutamic acid, asparagine, glutamine; serine, threonine; lysine, arginine; and phenylalanine, tyrosine.

By "high stringency conditions" is meant hybridization in 2X SSC at 40°C with a DNA probe length of at least 40 nucleotides. For other definitions of high stringency conditions, see F. Ausubel et al., Current Protocols in Molecular Biology, pp. 6.3.1-6.3.6, John Wiley & Sons, New York, NY, 1994, hereby incorporated by reference.

By "substantially pure polypeptide" is meant a polypeptide that has been separated from the components that naturally accompany it. Typically, the polypeptide is substantially pure when it is at least 60%, by weight, free from the proteins and naturally-occurring organic molecules with which it is naturally associated. Preferably, the polypeptide is an ABC1 polypeptide that is at least 75%, more preferably at least 90%, and most preferably at least 99%, by weight, pure. A substantially pure ABC1 polypeptide may be obtained, for example, by extraction from a natural source (e.g., a pancreatic cell), by expression of a recombinant nucleic acid encoding a ABC 1 polypeptide, or by chemically synthesizing the protein. Purity can be measured by any appropriate method, e.g., by column chromatography, polyacrylamide gel electrophoresis, or HPLC analysis.

A polypeptide is substantially free of naturally associated components when it is separated from those contaminants that accompany it in its natural state. Thus, a polypeptide which is chemically synthesized or produced in a cellular system different from the cell from which it naturally originates will be substantially free from its naturally associated components. Accordingly, substantially pure polypeptides include those which naturally occur in eukaryotic organisms but are synthesized in *E. coli* or other prokaryotes.

By "substantially pure nucleic acid" is meant nucleic acid that is free of the genes which, in the naturally-occurring genome of the organism from which the nucleic acid of the invention is derived, flank the nucleic acid. The term therefore includes, for example, a recombinant nucleic acid that is incorporated into a vector; into an autonomously replicating plasmid or virus; into the genomic nucleic acid of a prokaryote or a eukaryote cell; or that exists as a separate molecule (e.g., a cDNA or a genomic or cDNA fragment produced by PCR or restriction endonuclease digestion) independent of other sequences. It also includes a recombinant nucleic acid that is part of a hybrid gene encoding additional polypeptide sequence.

By "modulates" is meant increase or decrease. Preferably, a compound that modulates cholesterol levels (e.g., HDL-cholesterol levels, LDL-cholesterol levels, or total cholesterol levels), or ABC1 biological activity, expression, stability, or degradation does so by at least 10%, more preferably by at least 25%, and most preferably by at least 50%.

By "purified antibody" is meant antibody which is at least 60%, by weight, free from proteins and naturally occurring organic molecules with which it is naturally associated. Preferably, the preparation is at least 75%, more preferably 90%, and most preferably at least 99%, by weight, antibody. A purified antibody may be obtained, for example, by affinity chromatography using recombinantly-produced protein or conserved motif peptides and standard techniques.

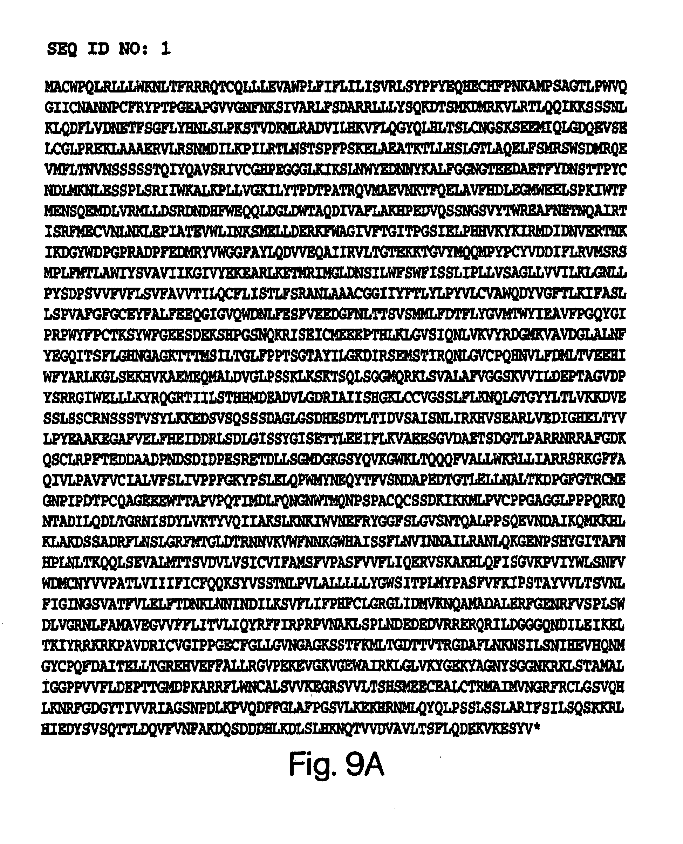

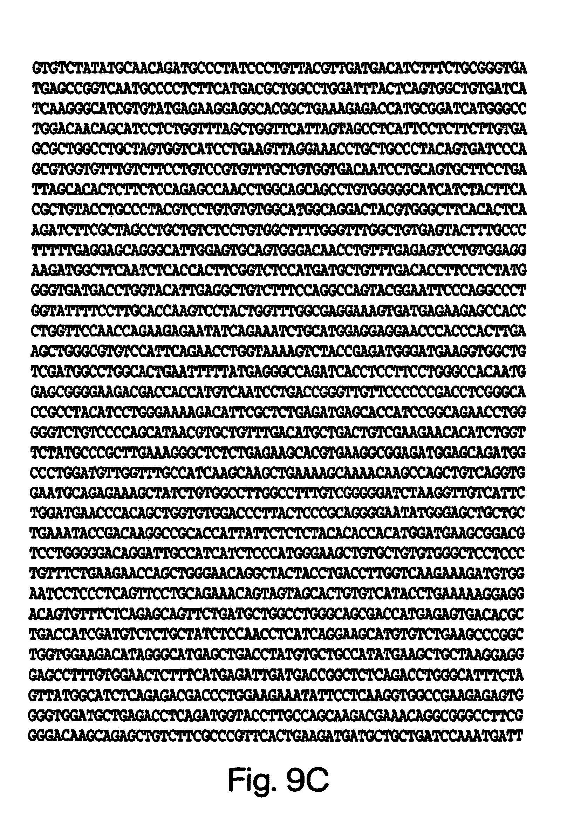

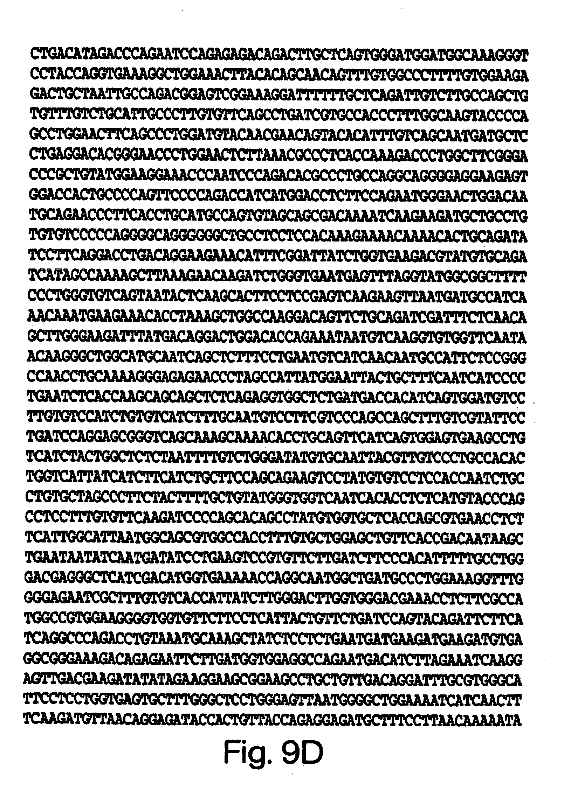

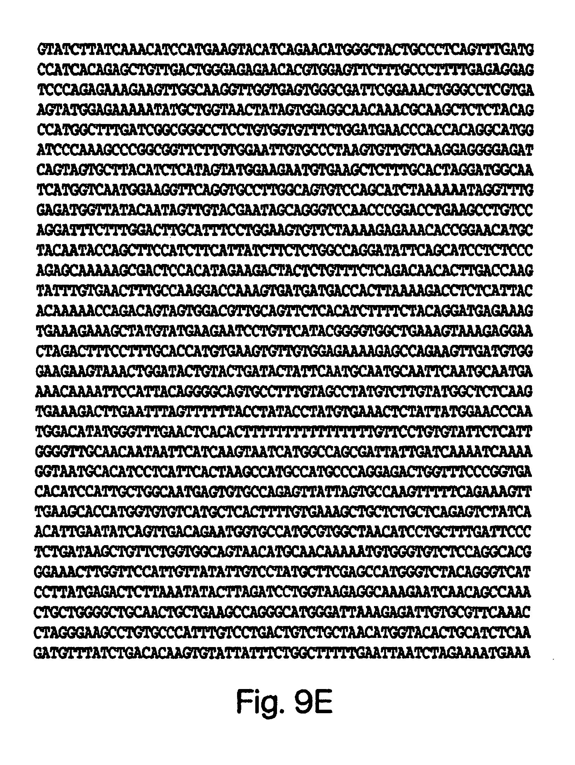

By "specifically binds" is meant an antibody that recognizes and binds to, for example, a human ABC1 polypeptide but does not substantially recognize and bind to other non-ABC1 molecules in a sample, e.g., a biological sample, that naturally includes protein. A preferred antibody binds to the ABC 1 polypeptide sequence of Fig. 9A (SEQ ID NO: 1).

By "polymorphism" is meant that a nucleotide or nucleotide region is characterized as occurring in several different forms. A "mutation" is a form of a polymorphism in which the expression level, stability, function, or biological activity of the encoded protein is substantially altered.

By "ABC transporter" or "ABC polypeptide" is meant any transporter that hydrolyzes ATP and transports a substance across a membrane. Preferably, an ABC transporter polypeptide includes an ATP Binding Cassette and a transmembrane region. Examples of ABC transporters include, but are not limited to, ABC1, ABC2, ABCR, and ABC8.

By "ABC1 polypeptide" is meant a polypeptide having substantial identity to an ABC1 polypeptide having the amino acid sequence of SEQ ID NO: 1.

By "ABC biological activity" or "ABC1 biological activity" is meant hydrolysis or binding of ATP, transport of a compound (e.g., cholesterol, interleukin-1) or ion across a membrane, or regulation of cholesterol or phospholipid levels (e.g., either by increasing or decreasing HDL-cholesterol or LDL-cholesterol levels).

The invention provides screening procedures for identifying therapeutic compounds (cholesterol-modulating or anti-CVD pharmaceuticals) which can be used in human patients. Compounds that modulate ABC biological activity (e.g., ABC1 biological activity) are considered useful in the invention, as are compounds that modulate ABC concentration, protein stability, regulated catabolism, or its ability to bind other proteins or factors. In general, the screening methods of the invention involve screening any number of compounds for therapeutically active agents by employing any number of *in vitro* or *in vivo* experimental systems. Exemplary methods useful for the identification of such compounds are detailed below.

The methods of the invention simplify the evaluation, identification and development of active agents for the treatment and prevention of low HDL and CVD. In general, the screening methods provide a facile means for selecting natural product extracts or compounds of interest from a large population which are further evaluated and condensed to a few active and selective materials. Constitutes of this pool are then purified and evaluated in the methods of the invention to determine their HDL-raising or anti-CVD activities or both.

Other features and advantages of the invention will be apparent from the following description of the preferred embodiments thereof, and from the claims.

### Brief Description of the Drawings

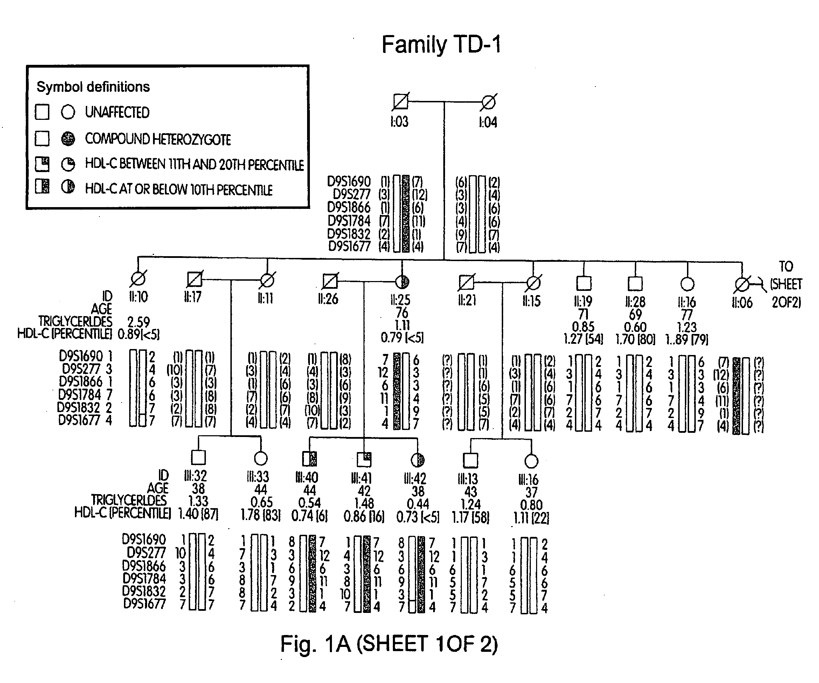

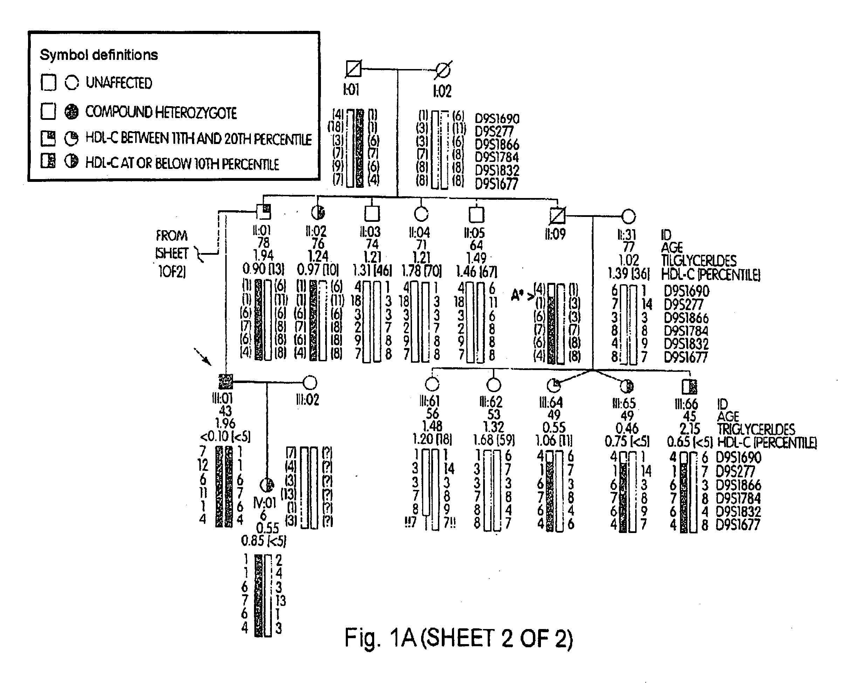

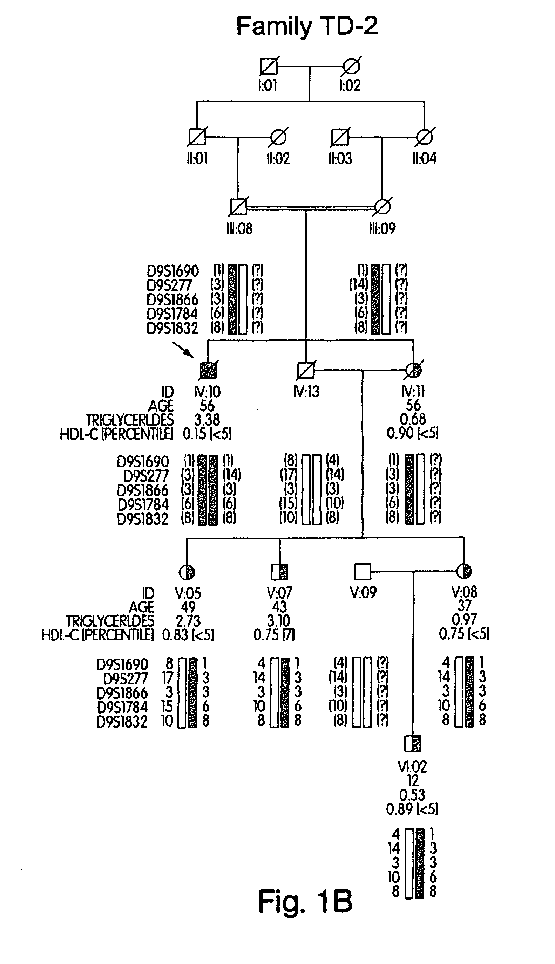

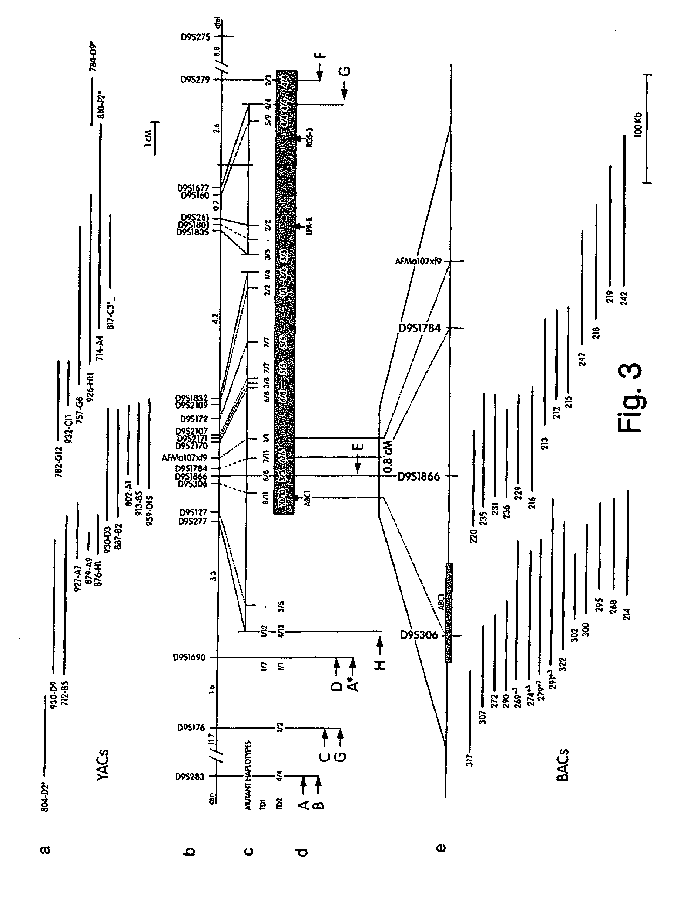

Figs. 1A and 1B are schematic illustrations showing two pedigrees with Tangier Disease, (TD-1 and TD-2). Square and circle symbols represent males and females, respectively. Diagonal lines are placed through the symbols of all deceased individuals. A shaded symbol on both alleles indicates the probands with Tangier Disease. Individuals with half shaded symbols have HDL-C levels at or below the 10th percentile for age and sex, while those with quarter shaded symbols have HDL-C between the 11th and 20th percentiles.
   Each individual's ID number, age at the time of lipid measurement, triglyceride level and HDL cholesterol level followed by their percentile ranking for age and sex are listed below the pedigree symbol. Markers spanning the 9q31.1 region are displayed to the left of the pedigree. The affected allele is represented by the darkened bars which illustrate the mapping of the limits of the shared haplotype region as seen in Fig. 3. Parentheses connote inferred marker data, questions marks indicate unknown genotypes, and large arrows show the probands.

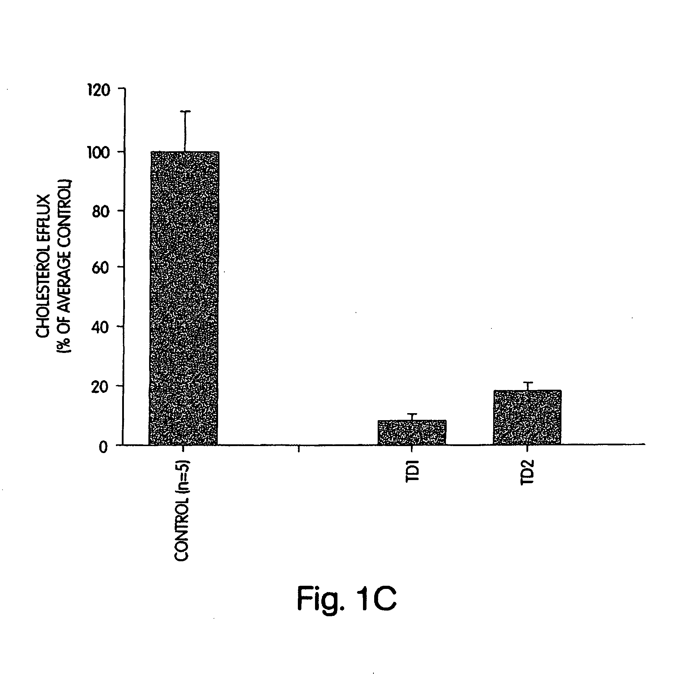

Fig. 1C shows ApoAI (10 µg/mL) -mediated cellular cholesterol efflux in control fibroblasts (n=5, normalized to 100%) and two subjects with Tangier disease (TD). Cells were ³H-cholesterol (0.2 µCi/mL) labeled during growth and cholesterol (20 µg/mL) loaded in growth arrest. Cholesterol efflux is determined as ³H medium/(³H cell + ³H medium)

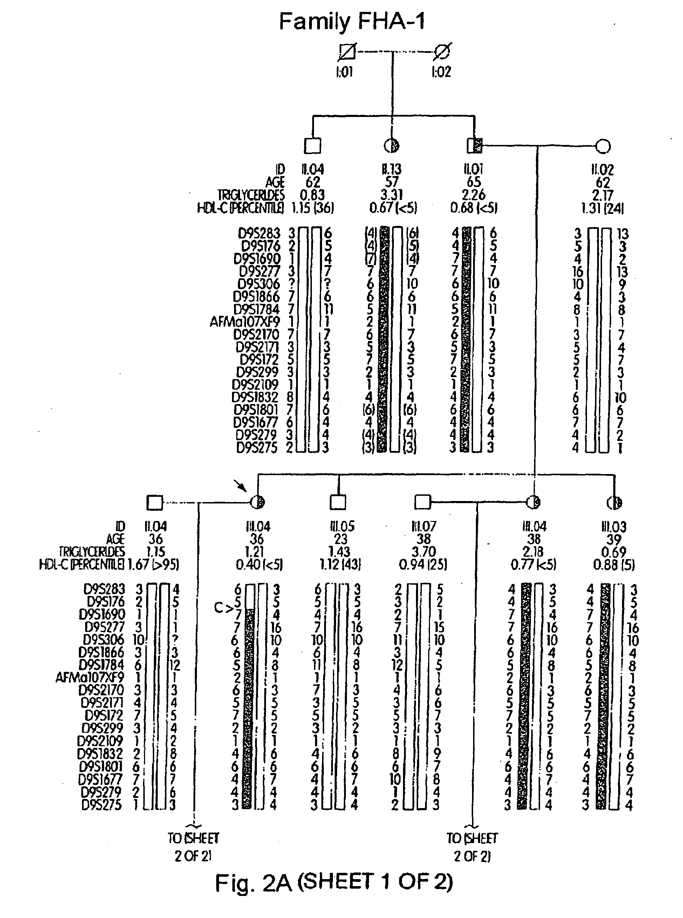

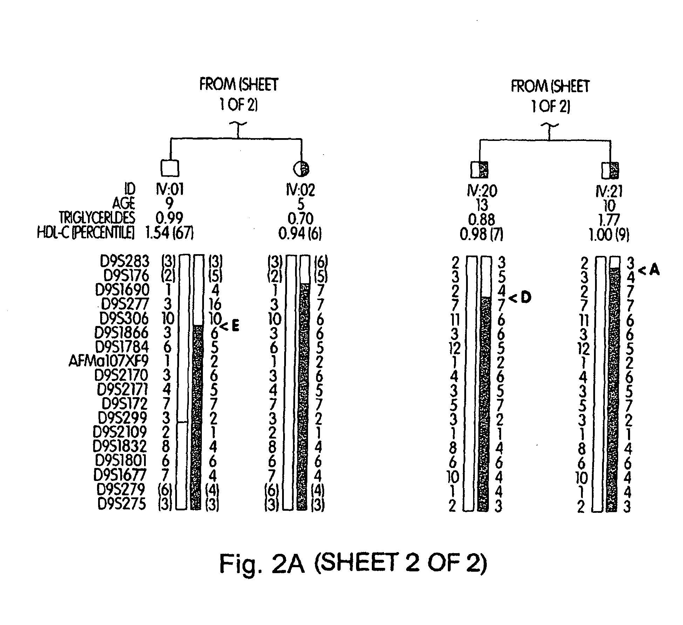

Fig. 2A - 2D are schematic illustrations showing four French Canadian pedigrees with FHA (FHA-1 to -4). The notations are as in Fig. 1. Exclamation points on either side of a genotype (as noted in Families FHA-3 and FHA-4) are used when the marker data appears to be inconsistent due to potential microsatellite repeat expansions. A bar that becomes a single thin line suggests that the haplotype is indeterminate at that marker.
Figs. 3A - 3E are a schematic illustration showing a genetic and physical map of 9q31 spanning 35 cM. Fig. 3A: YACs from the region of 9q22-34 were identified and a YAC contig spanning this region was constructed. Fig. 3B: A total of 22 polymorphic CA microsatellite markers were mapped to the contig and used in haplotype analysis in TD-1 and TD-2. Fig 3C: The mutant haplotypes for probands in TD-1 and -2 indicate a significant region of homozygosity in TD-2, while the proband in TD-1 has 2 different mutant haplotypes. The candidate region can be narrowed to the region of homozygosity for CA markers in proband 2. A critical crossover at D9S1690 in TD-1 (A)* also provides a centromeric boundary for the region containing the gene. Three candidate genes in this region *(ABC1, LPA-R* and *RGS-3)* are shown. Fig. 3D: Meiotic recombinations in the FHA families (A-H) refine the minimal critical region to 1.2 cM between D9S277 and D9S 1866. The heterozygosity of the TD-2 proband at D9S127, which ends a continuous region of homozygosity in TD-2, further refines the region to less than 1 cM. This is the region to which ABC 1 has been mapped. Fig. 3E: Isolated YAC DNA and selected markers from the region were used to probe high-density BAC grid filters, selecting BACs which via STS-content mapping produced an 800 Kb contig. Four BACs containing *ABC1* were sequenced using high-throughput methods.

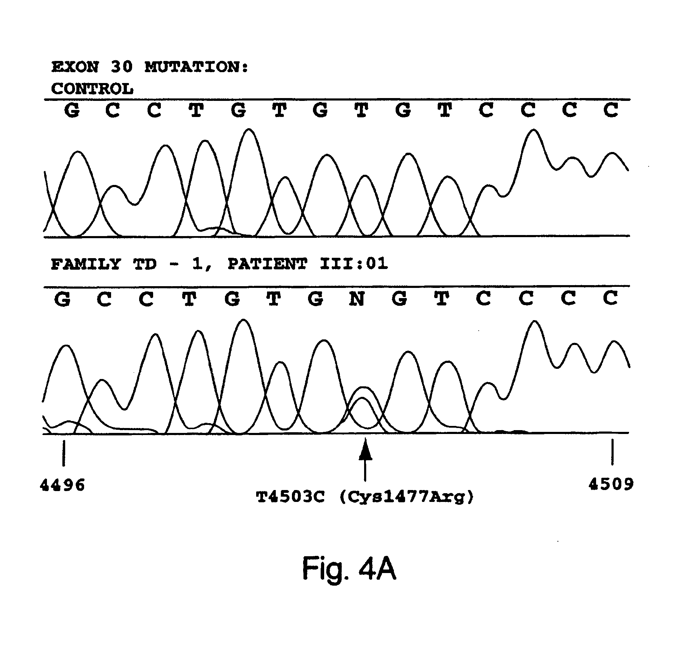

Fig. 4A shows sequence of one mutation in family TD-1. Patient III-01 is heterozygous for a T to C transition at nucleotide 4503 of the cDNA; the control is homozygous for T at this position. This mutation corresponds to a cysteine to arginine substitution in the ABC1 protein (C1477R).

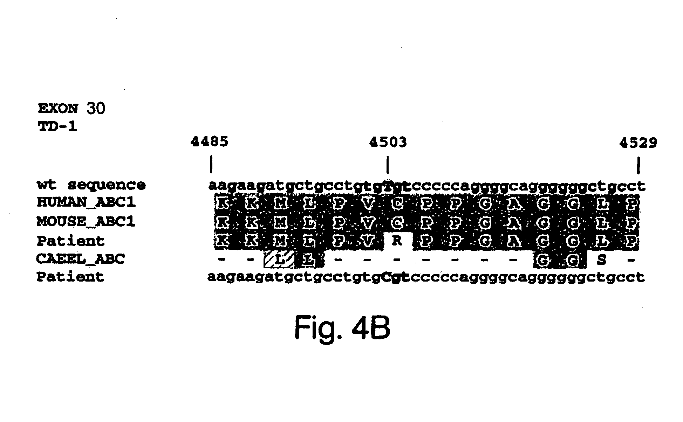

Fig 4B shows the amino acid sequence conservation of residue 1477 in mouse and human, but not a related *C. elegans* gene. A change from cysteine to arginine likely has an important effect on the protein secondary and tertiary structure, as noted by its negative scores in most substitution matrices (Schuler et al., A Practical Guide to the Analysis of Genes and Proteins, eds. Baxevanis, A.D. & Ouellette, B.F.F. 145:171, 1998). The DNA sequences of the normal and mutant genes are shown above and below the amino acid sequences, respectively.

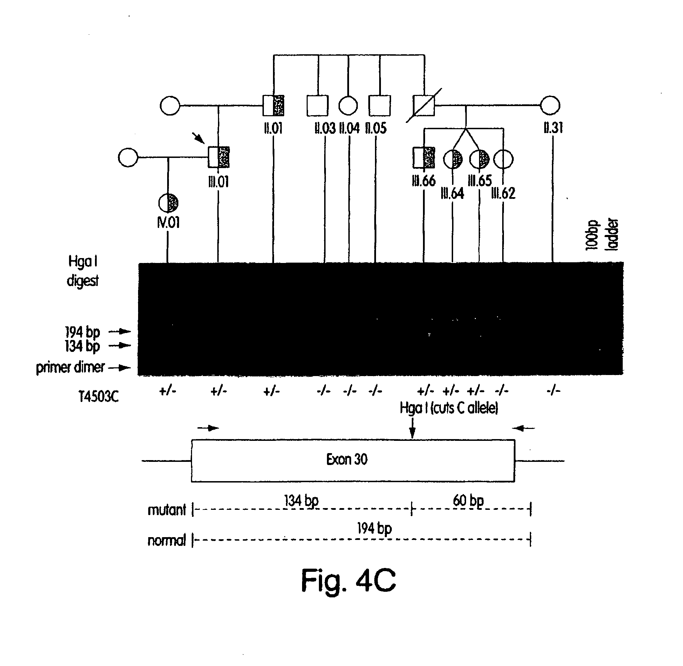

Fig. 4C shows the segregation of the T4503C mutation in TD-1. The presence of the T4503C mutation (+) was assayed by restriction enzyme digestion with *Hga*I*,* which cuts only the mutant (C) allele (↓). Thus, in the absence of the mutation, only the 194 bp PCR product (amplified between → and ←) is observed, while in its presence the PCR product is cleaved into fragments of 134 bp and 60 bp. The proband (individual III.01) was observed to be heterozygous for this mutation (as indicated by both the 194 bp and 134 bp bands), as were his daughter, father, and three paternal cousins. A fourth cousin and three of the father's siblings were not carriers of this mutation.

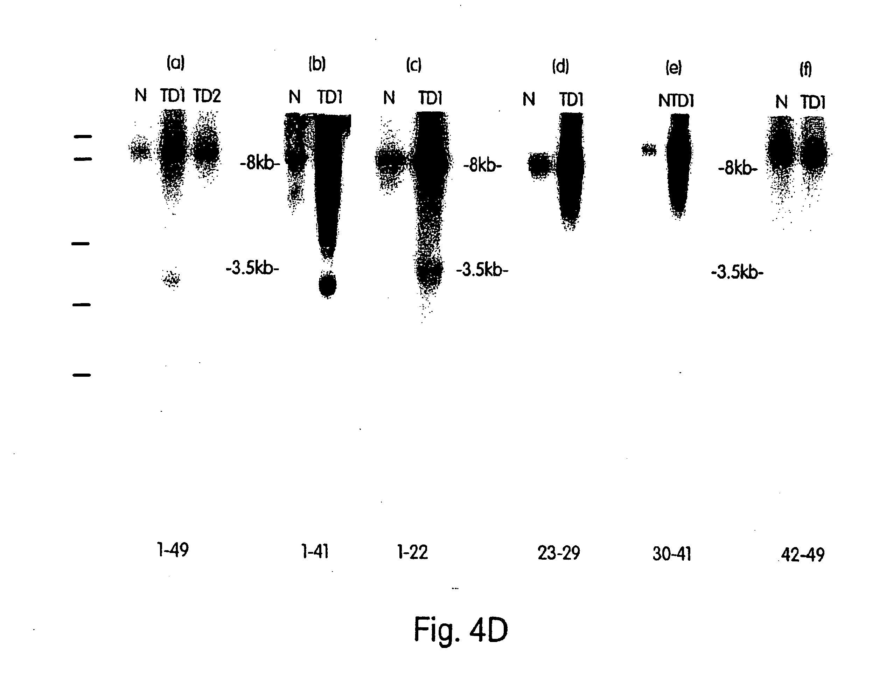

Fig. 4D shows Northern blot analysis with probes spanning the complete ABC1 gene reveal the expected ~8 Kb transcript and, in addition, a ~3.5 kb truncated transcript only seen in the proband TD-1 and not in TD-2 or control. This was detected by probes spanning exons 1-49 (a), 1-41 (b), 1-22 (c), and 23-29 (d), but not with probes spanning exons 30-41 (e) or 42-49 (f).

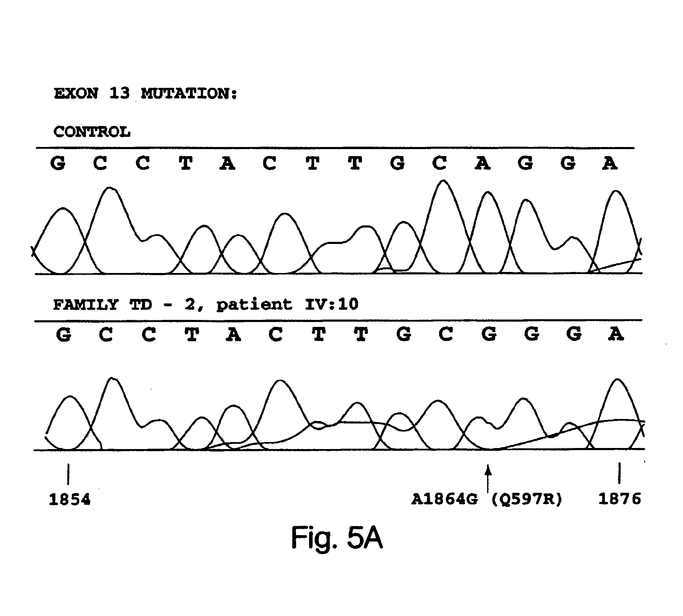

Fig. 5A shows the sequence of the mutation in family TD-2. Patient IV-10 is homozygous for an A to G transition at nucleotide 1864 of the cDNA (SEQ ID NO: 2); the control is homozygous for A at this position. This mutation corresponds to a glutamine to arginine substitution in the ABC1 protein (Q597R).

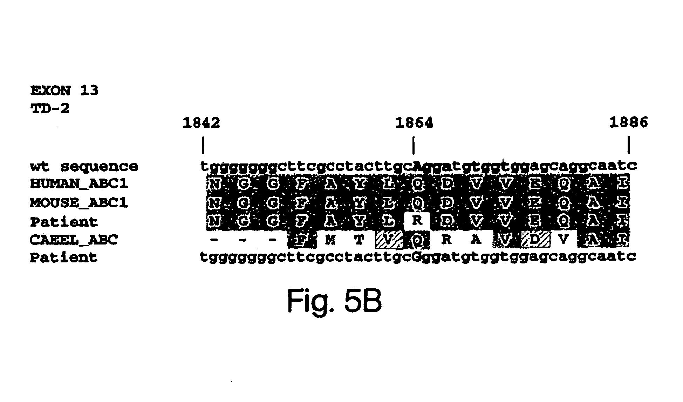

Fig. 5B shows that the glutamine amino acid, which is mutated in the TD-2 proband, is conserved in human and mouse ABC1 as well as in an ABC orthologue from *C. elegans,* revealing the specific importance of this residue in the structure/function of this ABC protein in both worms and mammals. The DNA sequences of the normal and mutant proteins are shown above and below the amino acid sequences, respectively.

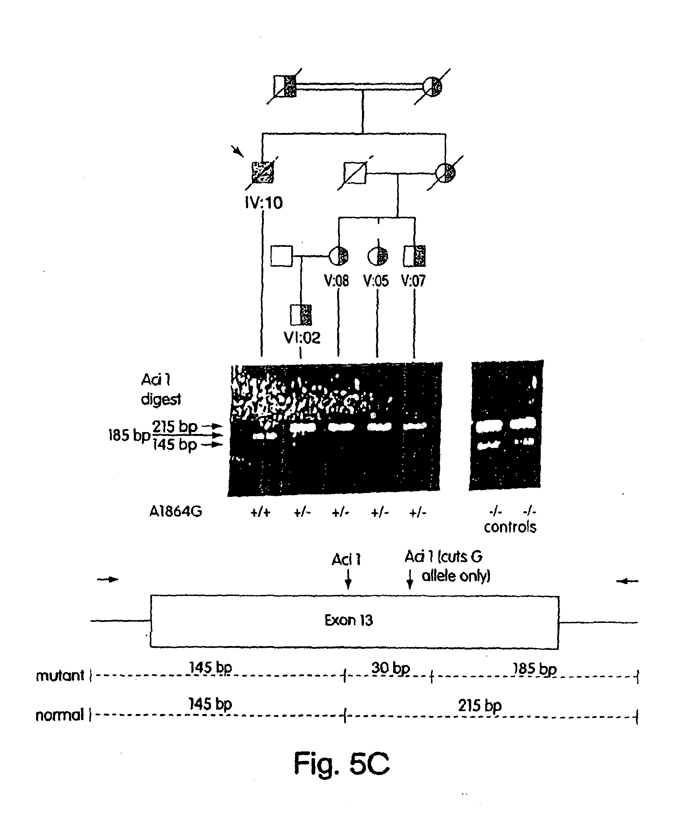

Fig. 5C shows the segregation of the A1864G mutation in TD-2. The presence of the A1864G mutation (indicated by +) was assayed by restriction enzyme digestion with *Aci*I*.* The 360 bp PCR product has one invariant *Aci*I recognition site (↓), and a second one is created by the A1864G mutation. The wild-type allele is thus cleaved to fragments of 215 bp and 145 bp, while the mutant allele (G-allele) is cleaved to fragments of 185 bp, 145 bp and 30 bp. The proband (individual IV-10), the product of a consanguineous mating, was homozygous for the A1864G mutation (+/+), as evidenced by the presence of only the 185 bp and 145 bp bands, while four other family members for whom DNA was tested are heterozygous carriers of this mutation (both the 215 bp and 185 bp fragments were present). Two unaffected individuals (-/-), with only the 215 bp and 145 bp bands are shown for comparison.

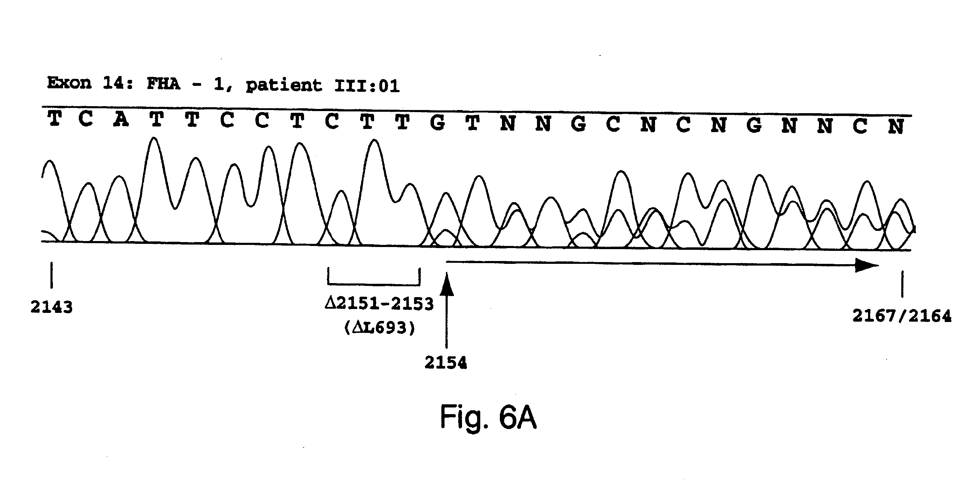

Fig. 6A shows a sequence of the mutation in family FHA-1. Patient III-01 is heterozygous for a deletion of nucleotides 2151-2153 of the cDNA (SEQ ID NO: 2). This deletion was detected as a superimposed sequence starting at the first nucleotide after the deletion. This corresponds to deletion of leucine 693 in the ABC1 protein (SEQ ID NO: 1).

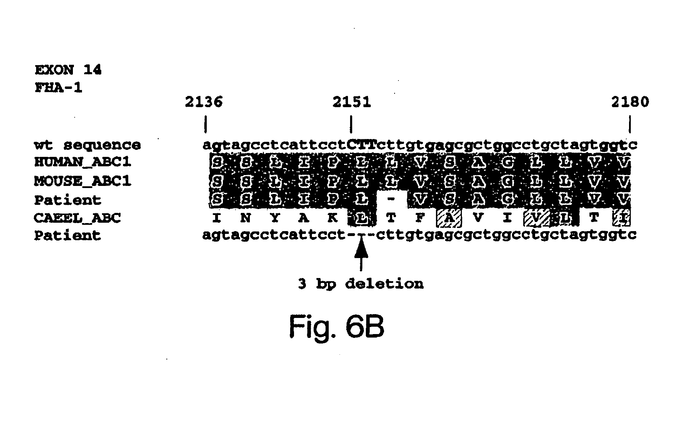

Fig. 6B is an alignment of the human and mouse wild-type amino acid sequences, showing that the human and mouse sequences are identical in the vicinity of ΔL693. L693 is also conserved in *C*. *elegans.* This highly conserved residue lies within a predicted transmembrane domain. The DNA sequences of the normal and mutant proteins are shown above and below the amino acid sequences, respectively.

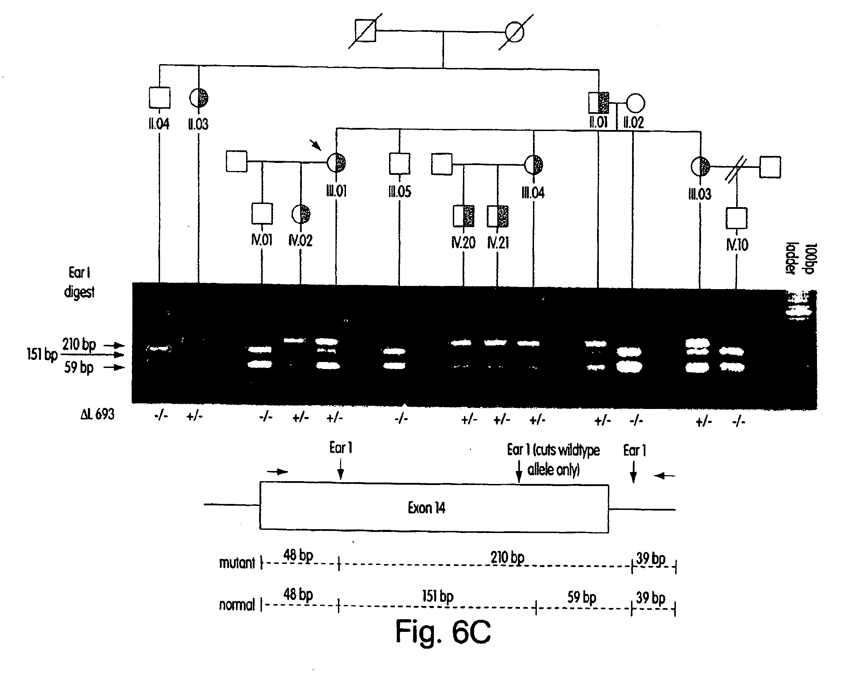

Fig. 6C shows segregation of the ΔL693 mutation in FHA-1, as assayed by *Ear*I restriction digestion. Two invariant *Ear*I restriction sites (indicated by ↓) are present within the 297 bp PCR product located between the horizontal arrows (→ ←) while a third site is present in the wild-type allele only. The presence of the mutant allele is thus distinguished by the presence of a 210 bp fragment (+), while the normal allele produces a 151 bp fragment (-). The proband of this family (III.01) is heterozygous for this mutation, as indicated by the presence of both the 210 and 151 bp bands.

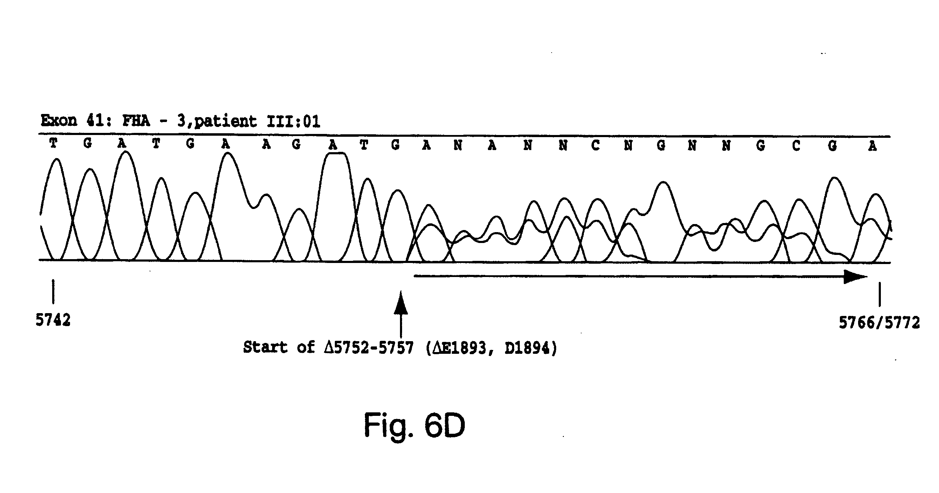

Fig. 6D shows a sequence of the mutation in family FHA-3. Patient III-01 is heterozygous for a deletion of nucleotides 5752-5757 of the cDNA (SEQ ID NO: 2). This deletion was detected as a superimposed sequence starting at the first nucleotide after the deletion. This corresponds to deletion of glutamic acid 1893 and aspartic acid 1894 in the ABC 1 protein (SEQ ID NO: 1).

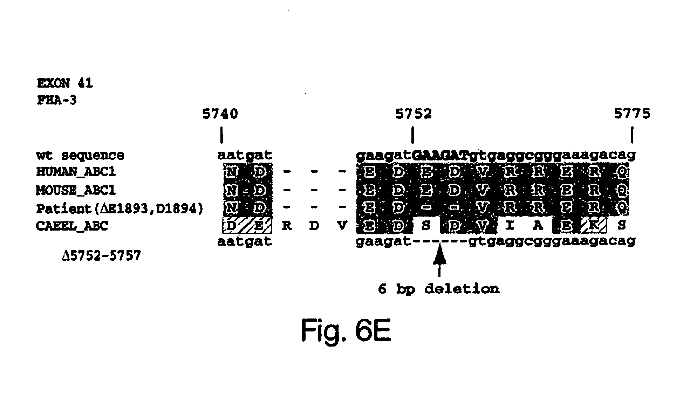

Fig. 6E is an alignment of the human and mouse wild-type amino acid sequences, showing that the human and mouse sequences are identical in the vicinity of Δ5752-5757. This region is highly conserved in *C. elegans.* The DNA sequences of the normal and mutant proteins are shown above and below the amino acid sequences, respectively.

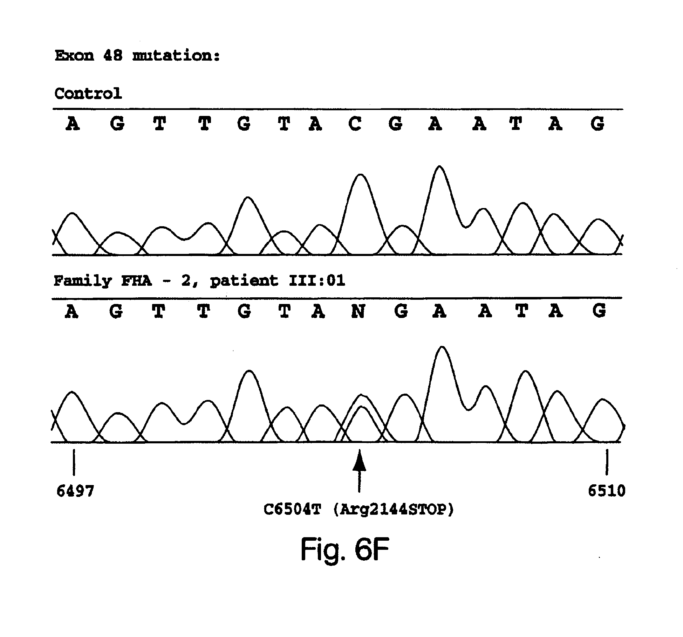

Fig. 6F shows a sequence of the mutation in family FHA-2. Patient III-01 is heterozygous for a for a C to T transition at nucleotide 6504 of the cDNA (SEQ ID NO: 2). This alteration converts an arginine at position 2144 of SEQ ID NO: 1 to a STOP codon, causing truncation of the last 118 amino acids of the ABC1 protein.

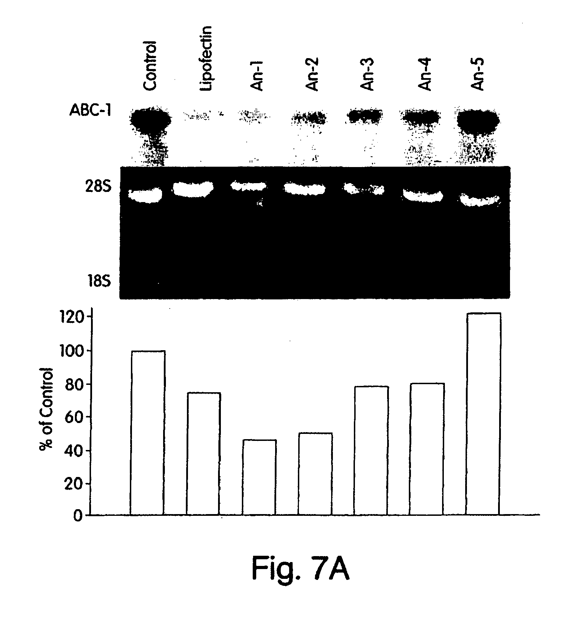

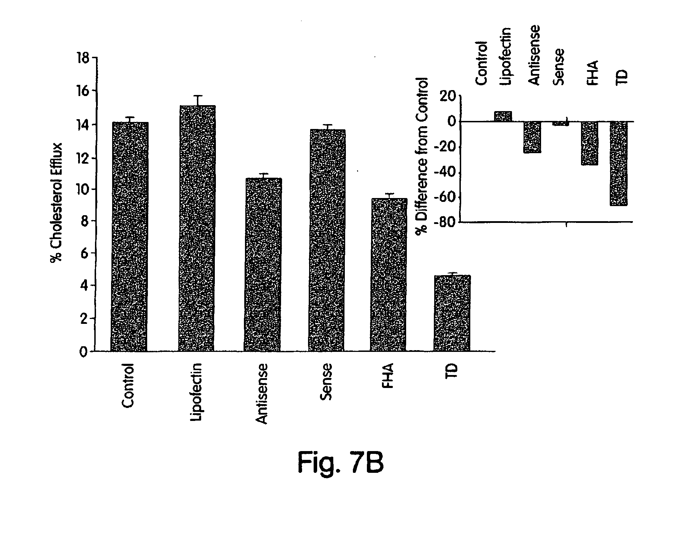

Figs. 7A and 7B show cholesterol efflux from human skin fibroblasts treated with ABC1 antisense oligonucleotides. Fibroblasts from a control subject were labeled with ³H cholesterol (0.2 µCi/mL) during growth for 48 hours and transfected with 500 nM *ABC1* antisense AN-1 (5'-GCA GAG GGC ATG GCT TTA TTT G-3'; SEQ ID NO: 3) with 7.5 µg lipofectin for 4 hours. Following transfection, cells were cholesterol loaded (20 µg/mL) for 12 hours and allowed to equilibrate for 6 hours. Cells were either then harvested for total RNA and 10 µg was used for Northern blot analysis. Cholesterol efflux experiments were carried out as described herein. Fig. 7A: AN-1 was the oligonucleotide that resulted in a predictable decrease in *ABC1* RNA transcript levels. Fig. 7B: A double antisense transfection method was used. In this method, cells were labeled and transfected with AN-1 as above, allowed to recover for 20 hours, cholesterol loaded for 24 hours, and then re-transfected with AN-1. Twenty hours after the second transfection, the cholesterol efflux as measured. A ~50% decrease in *ABC1* transcript levels was associated with a significant decrease in cholesterol efflux intermediate between that seen in wild-type and TD fibroblasts.

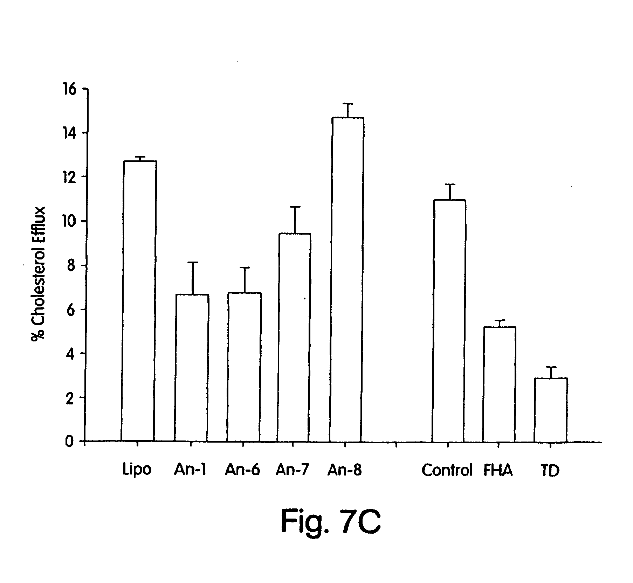

Fig. 7C shows show cholesterol efflux from human skin fibroblasts treated with antisense oligonucleotides directed to the region encoding the amino-terminal 60 amino acids. Note that the antisense oligonucleotide AN-6, which is directed to the previously unrecognized translation start site, produces a substantial decrease in cellular cholesterol efflux.

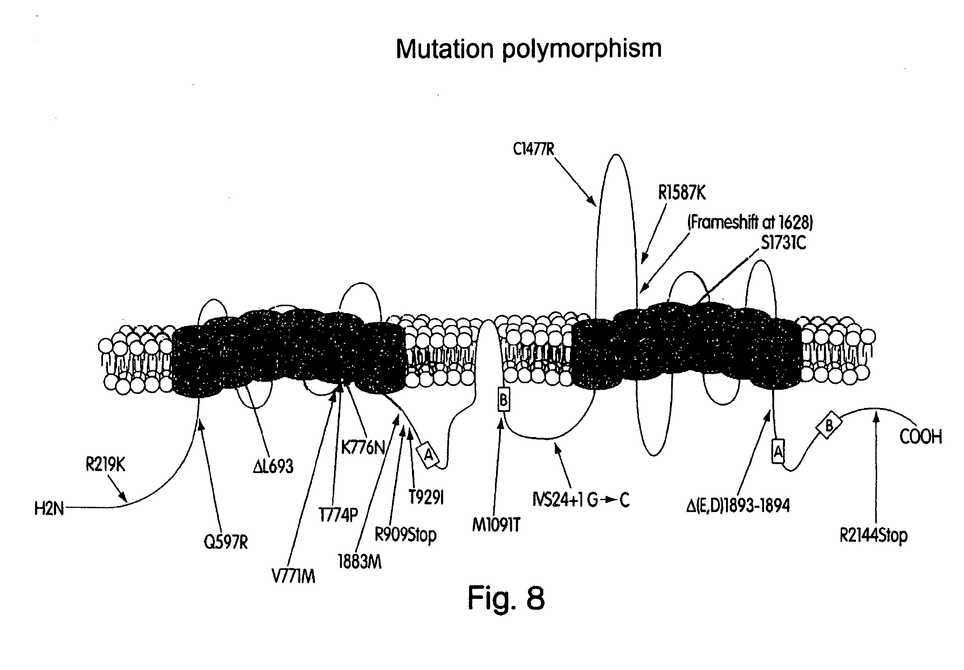

Fig. 8 is a schematic illustration showing predicted topology, mutations, and polymorphisms of ABC1 in Tangier disease and FHA. The two transmembrane and ATP binding domains are indicated. The locations of mutations are indicated by the arrows with the amino acid changes, which are predicted from the human *ABC1* cDNA sequence. These mutations occur in different regions of the ABC 1 protein.
Fig. 9A shows the amino acid sequence of the human ABC 1 protein (SEQ ID NO: 1).

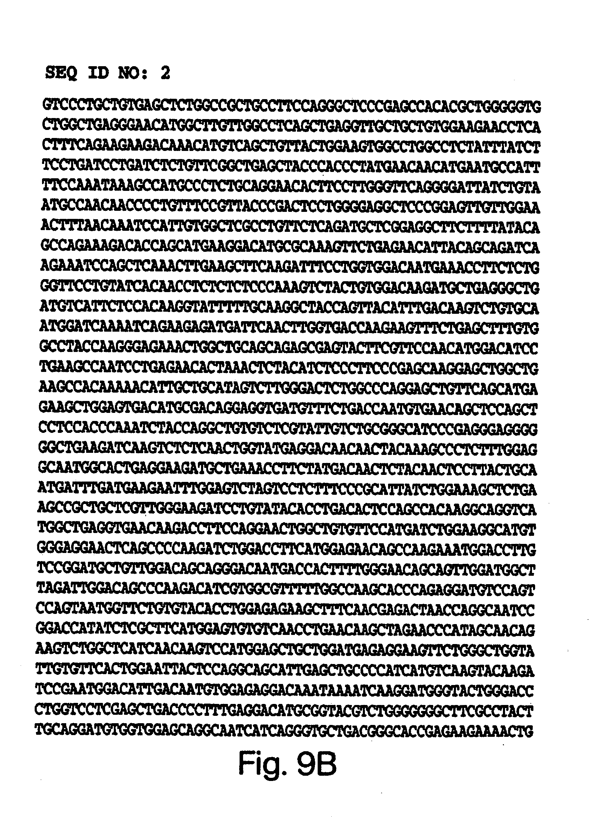

Figs. 9B - 9E show the nucleotide sequence of the human *ABC1* cDNA (SEQ ID NO: 2).

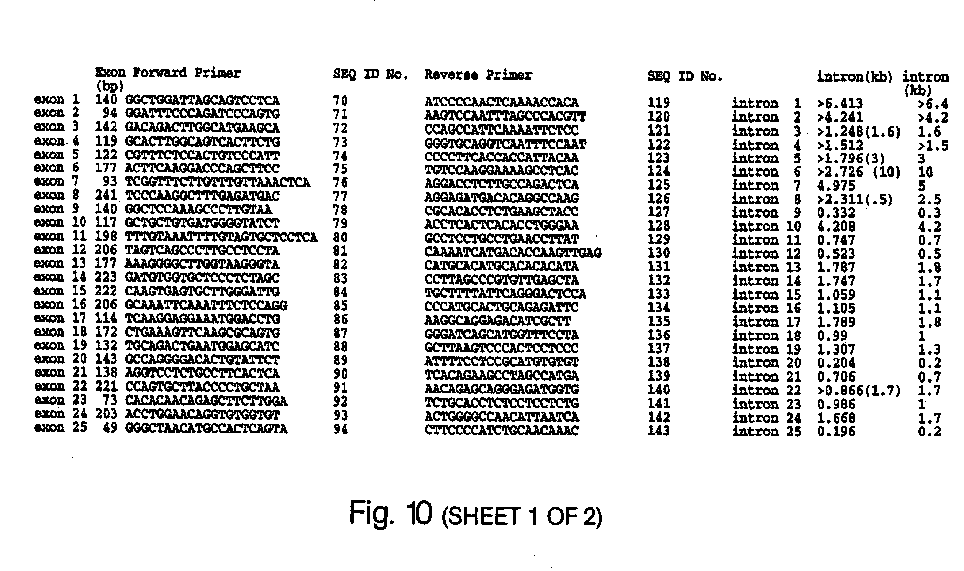

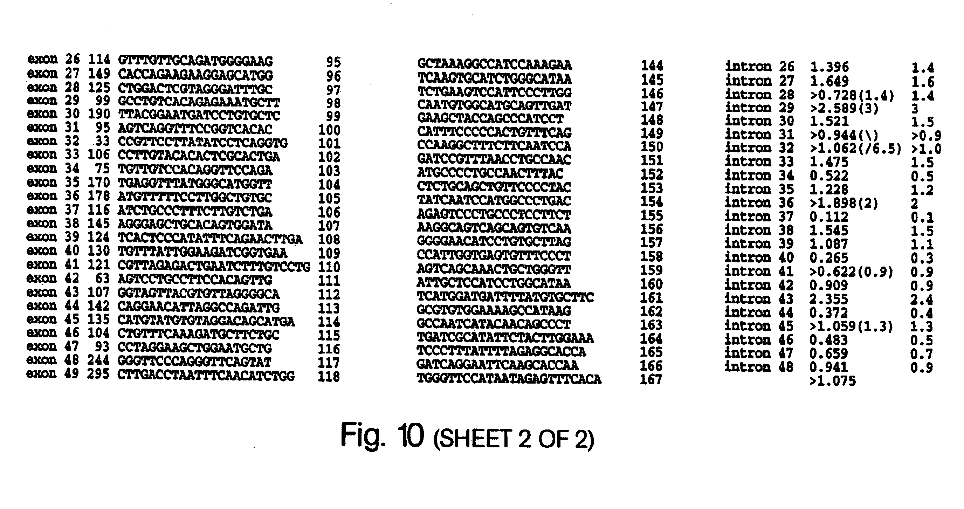

Fig. 10 shows the 5' and 3' nucleotide sequences suitable for use as 5' and 3' PCR primers, respectively, for the amplification of the indicated ABC1 exon.

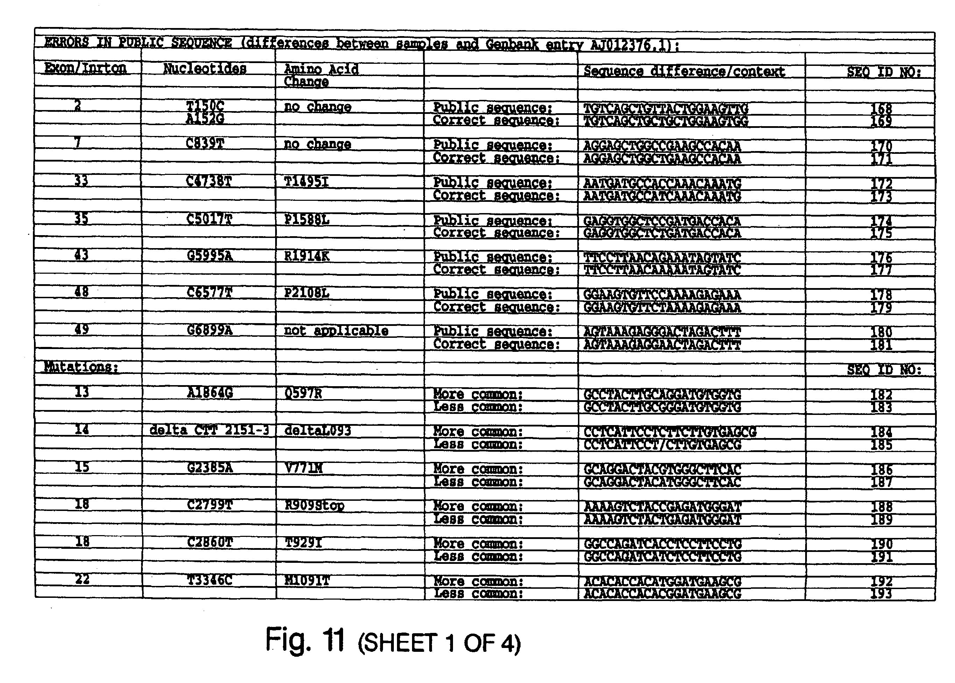

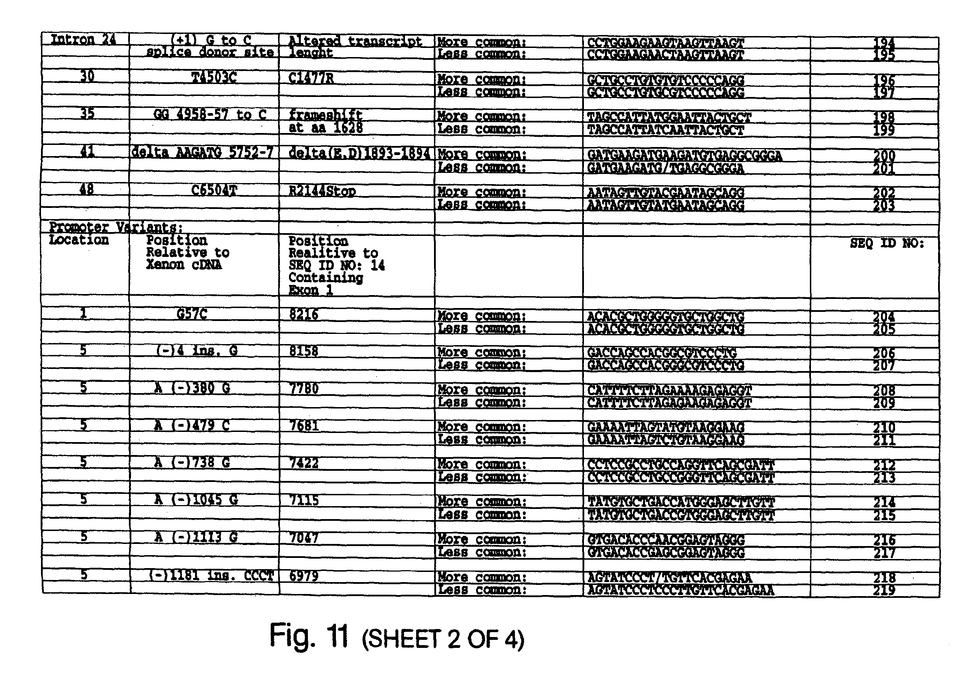

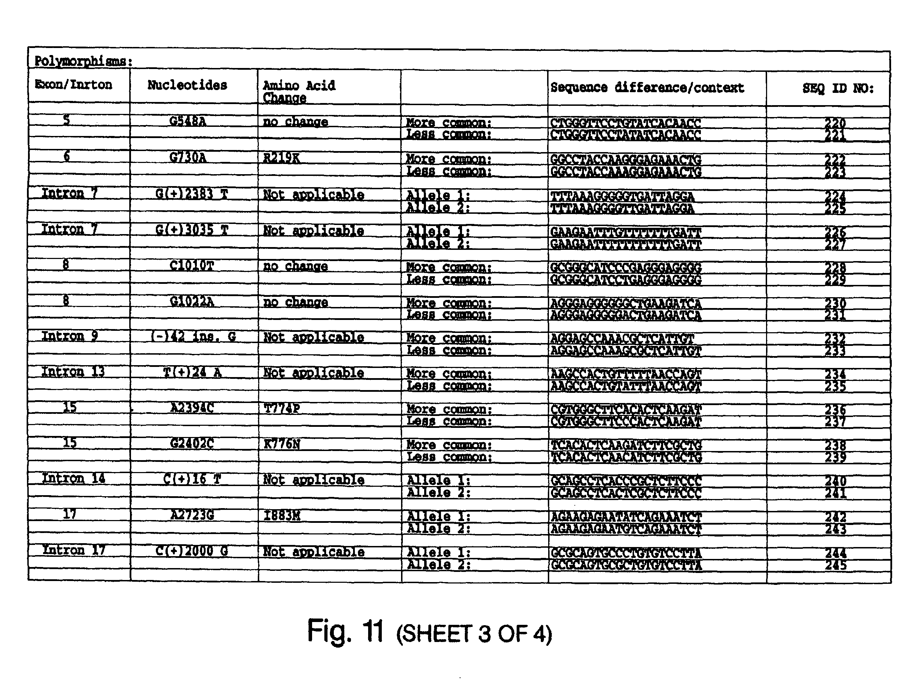

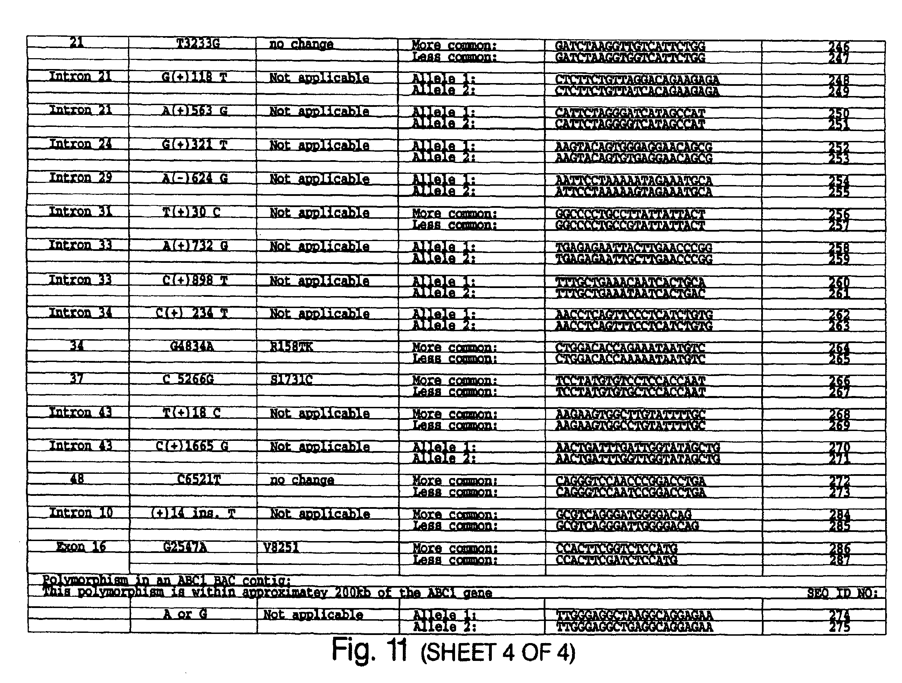

Fig. 11 shows a summary of alterations found in ABC1, including sequencing errors, mutations, and polymorphisms.

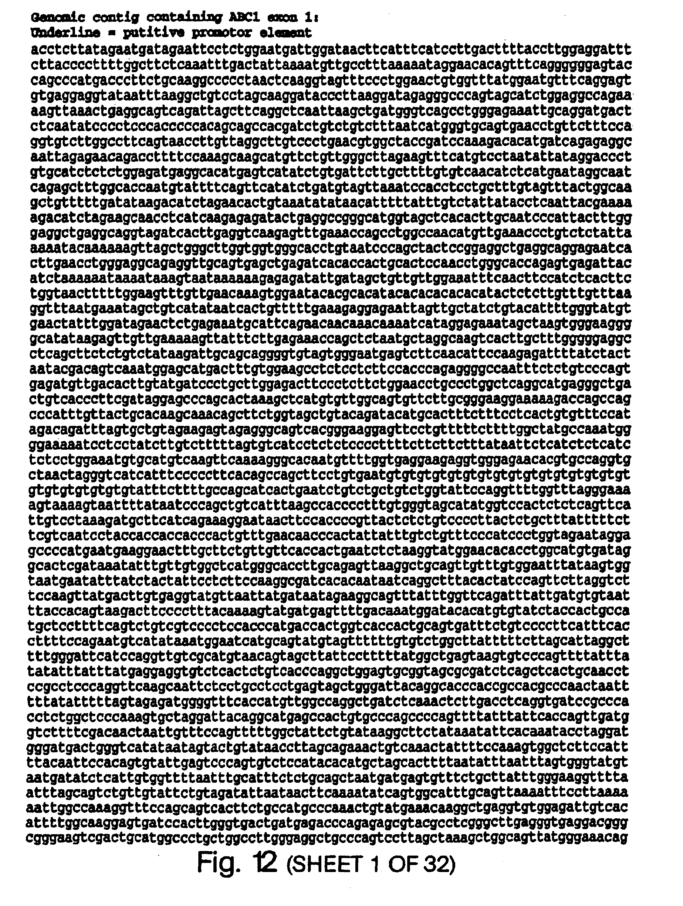

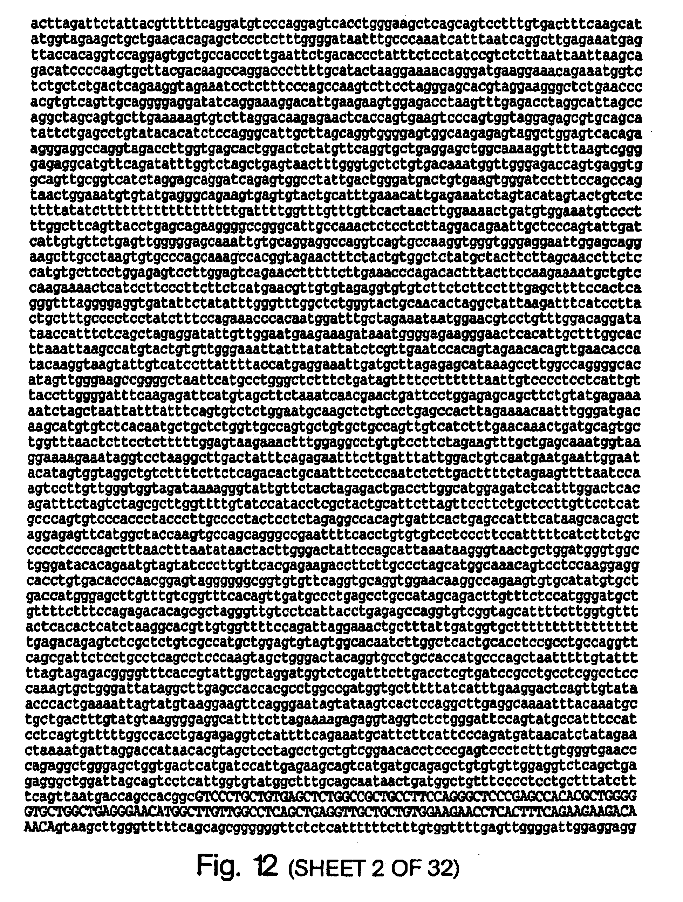

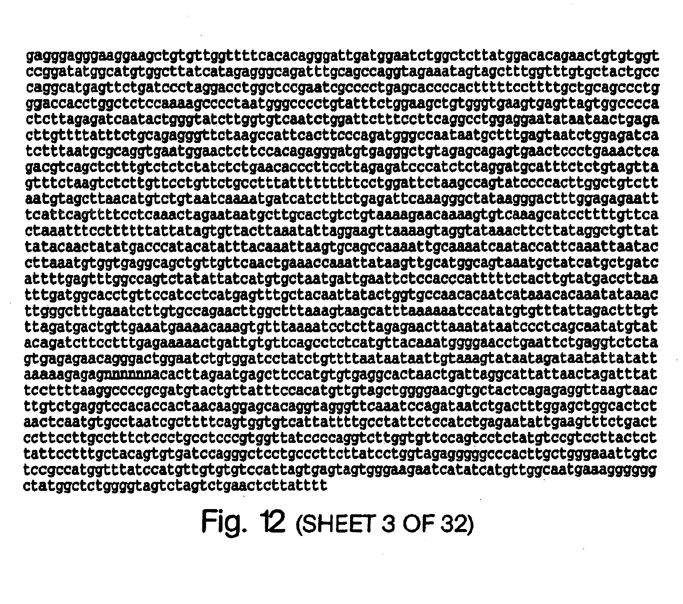

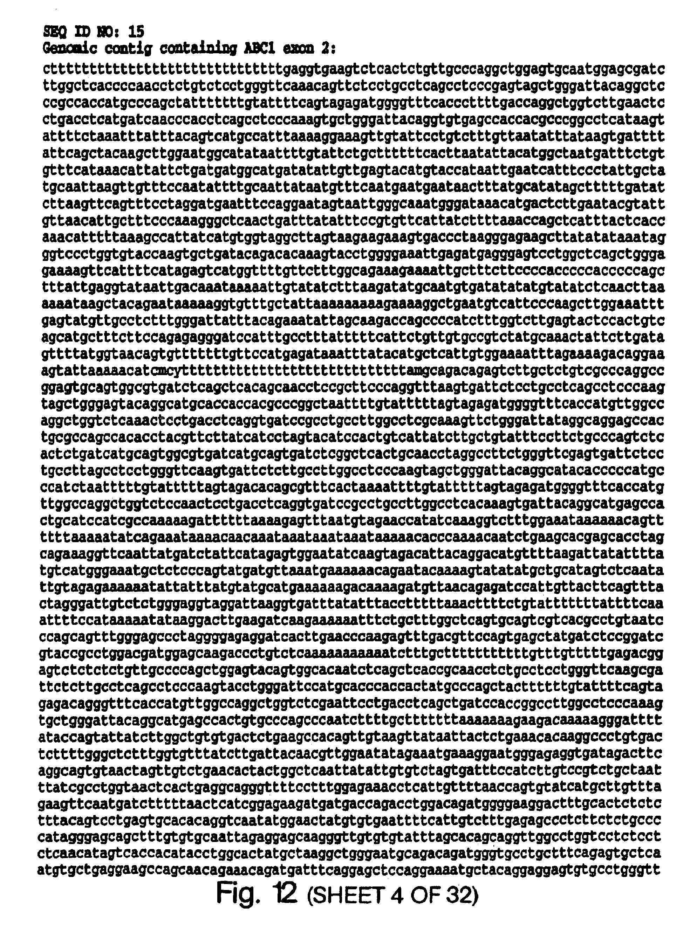

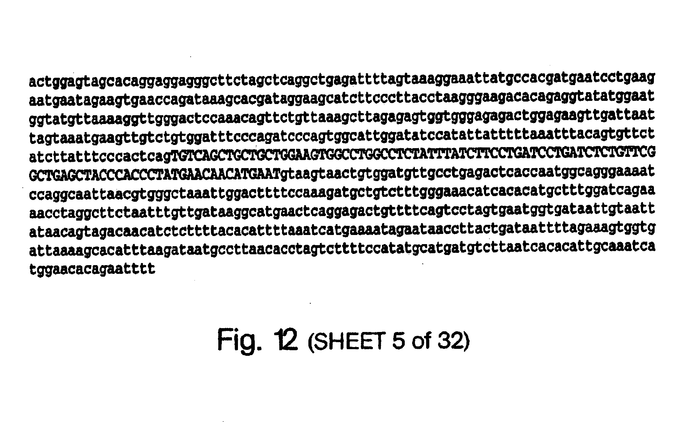

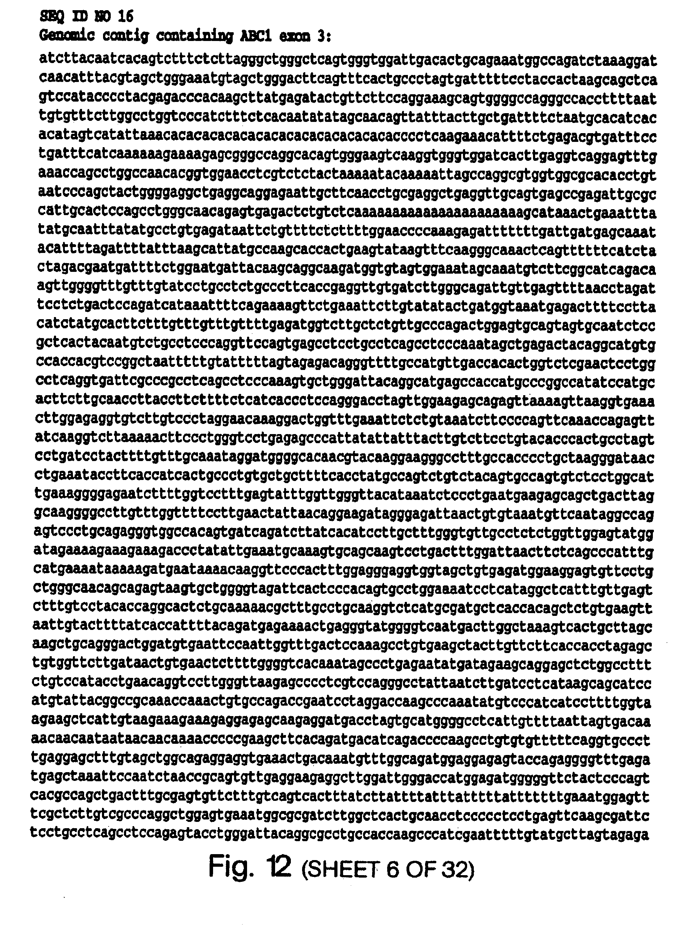

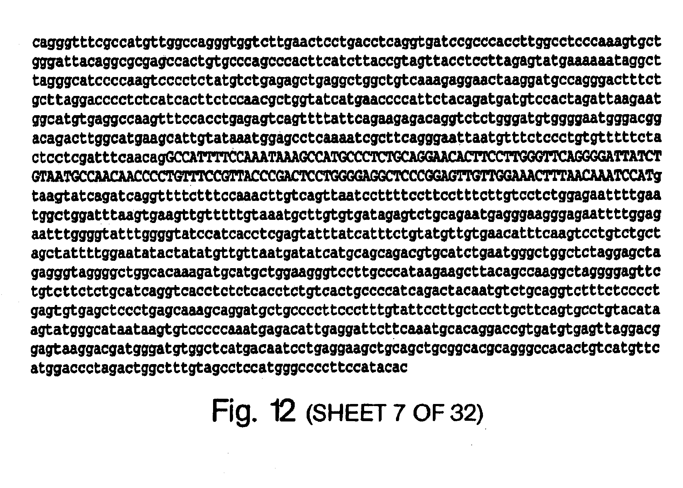

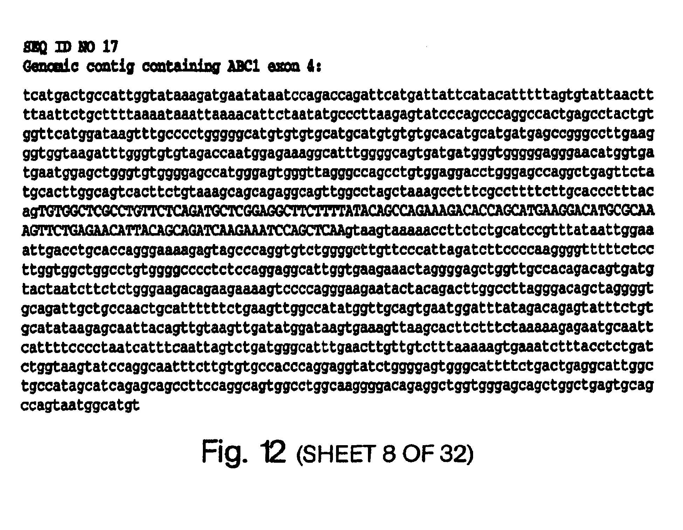

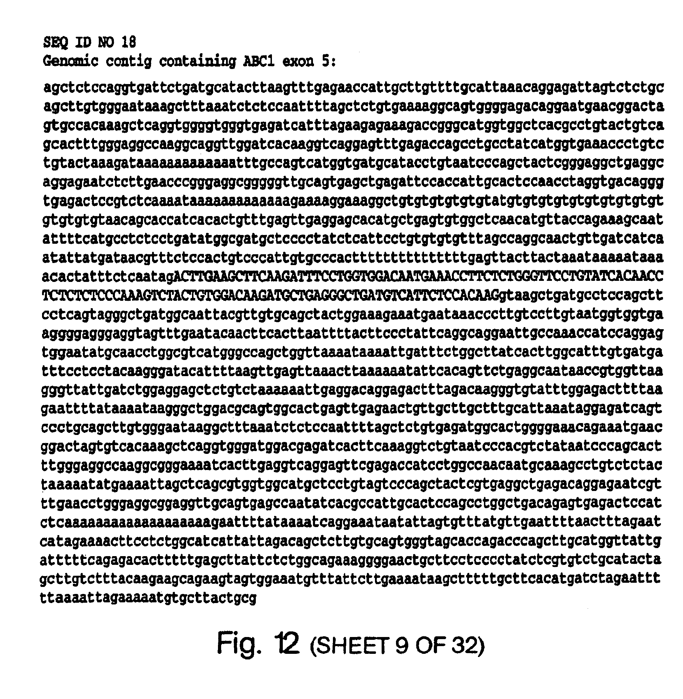

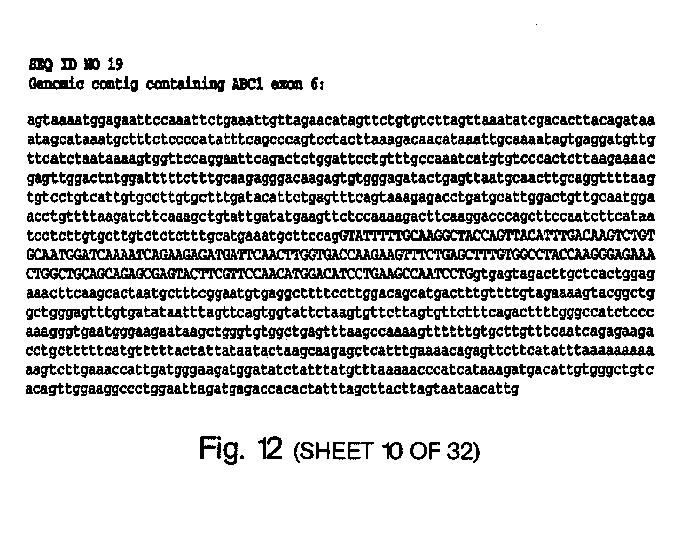

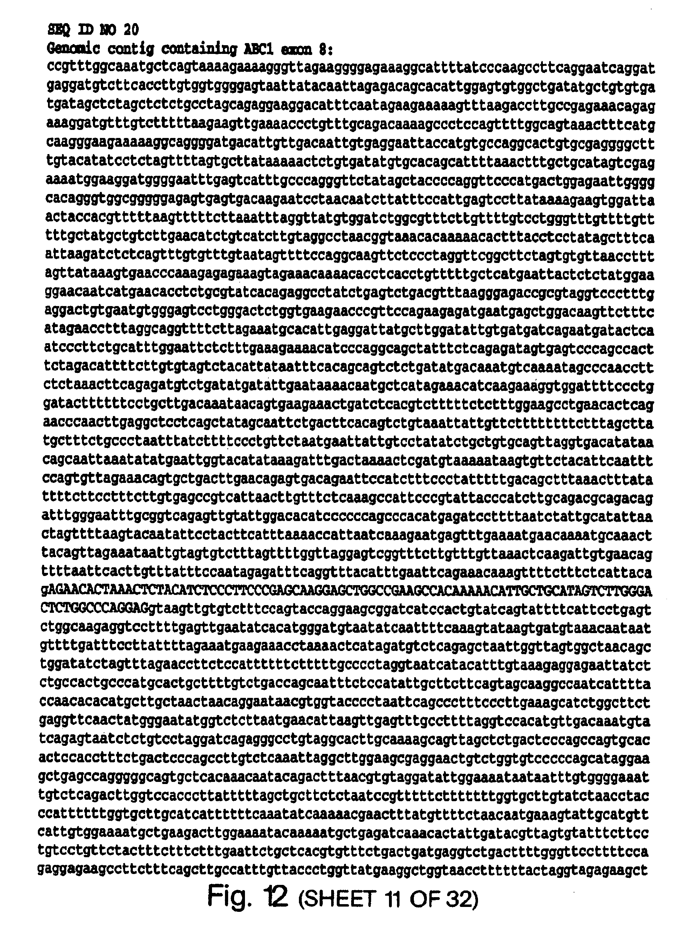

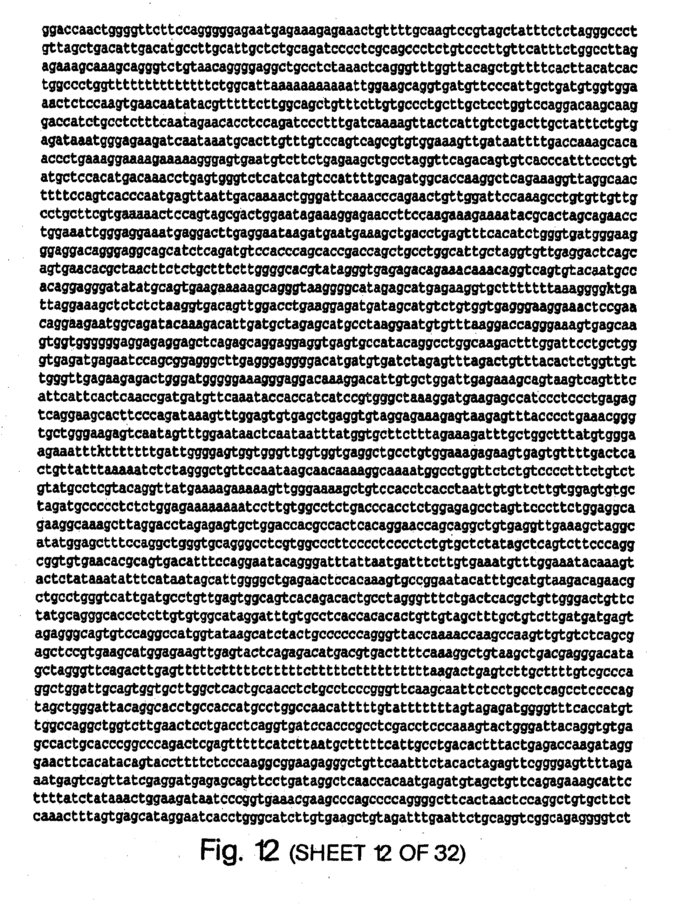

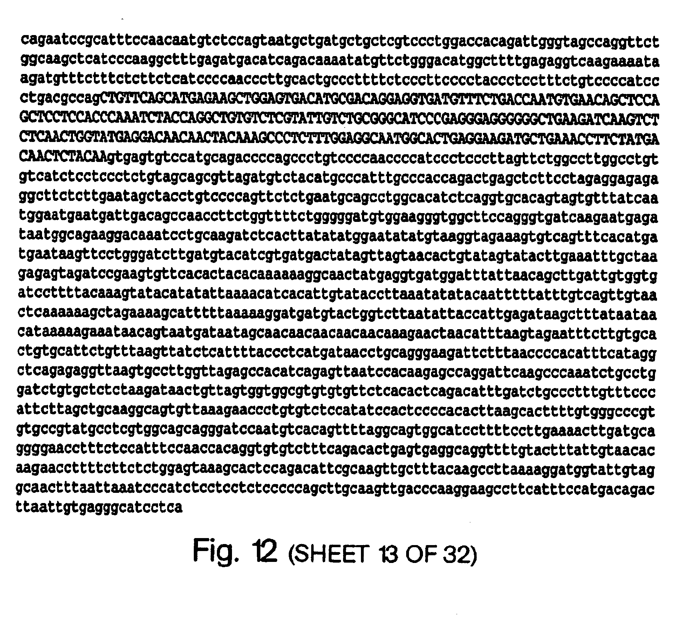

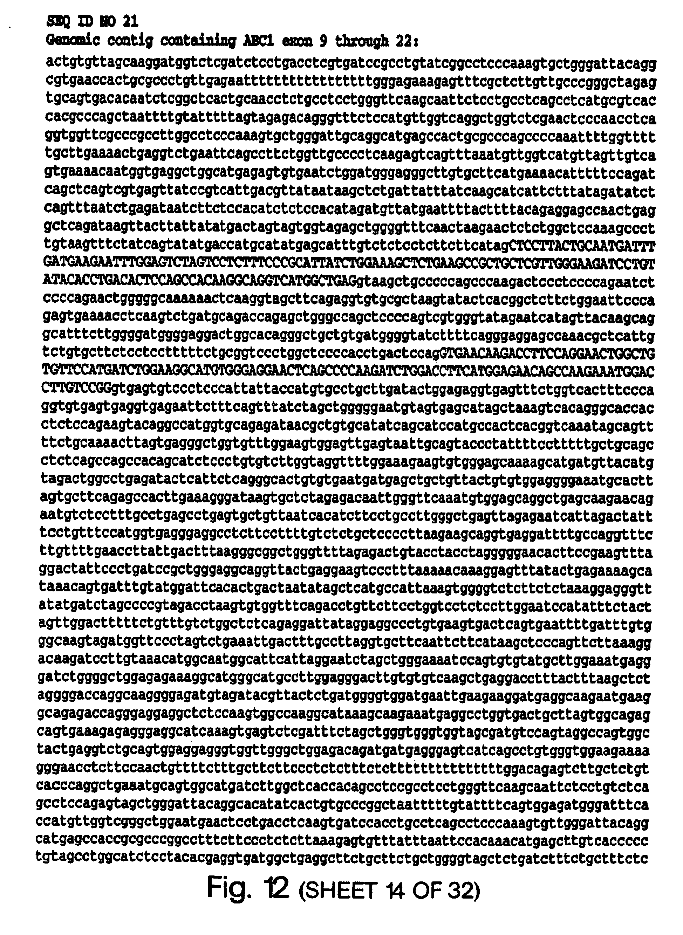

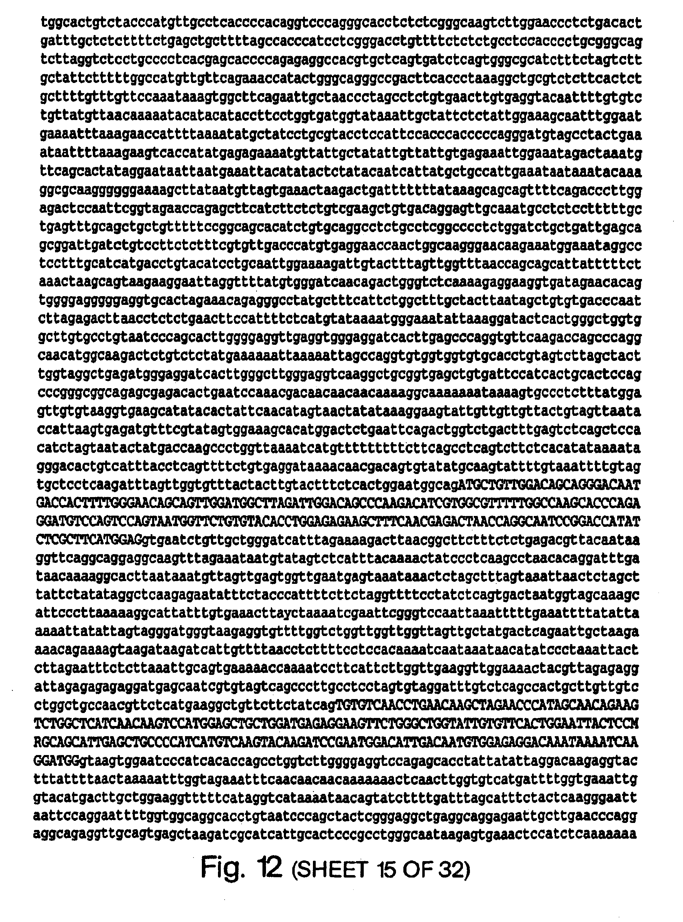

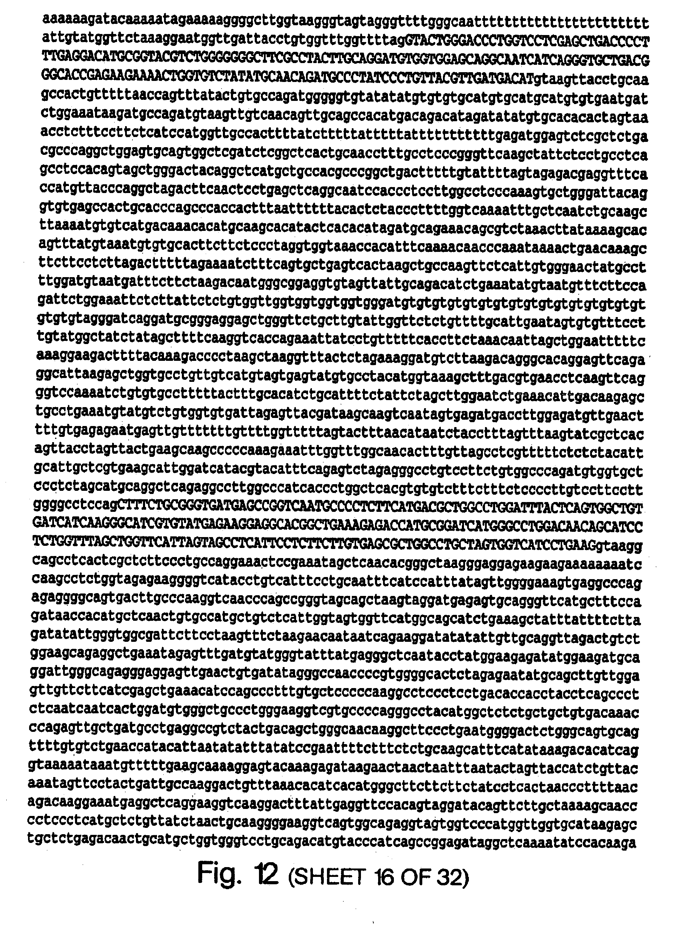

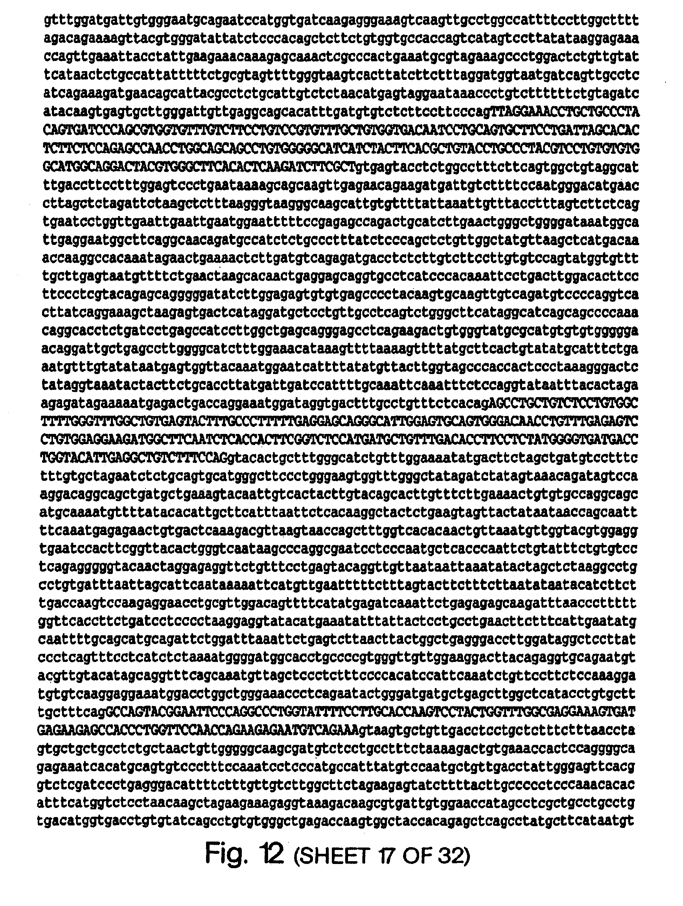

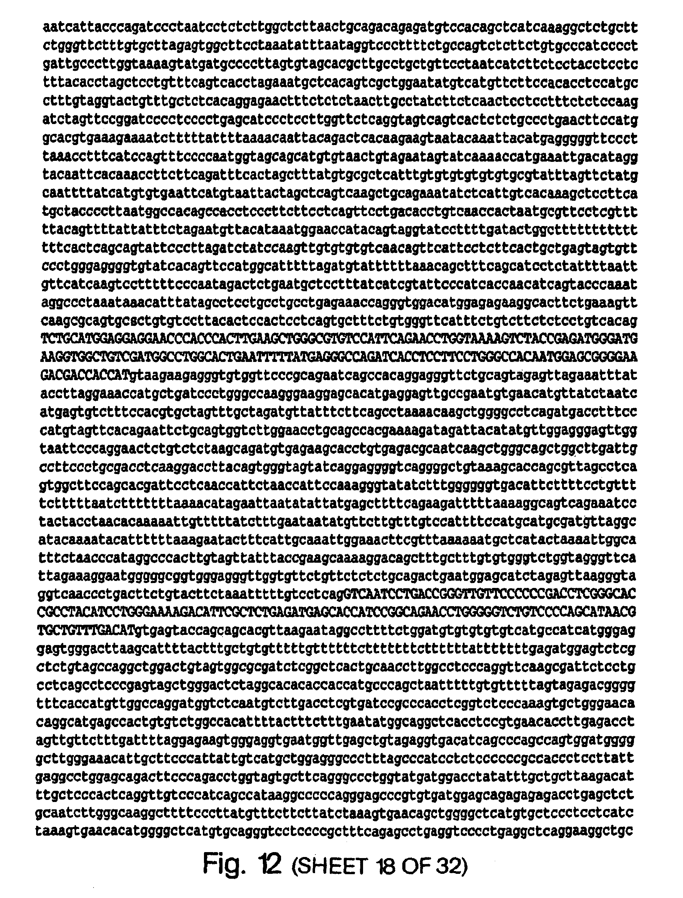

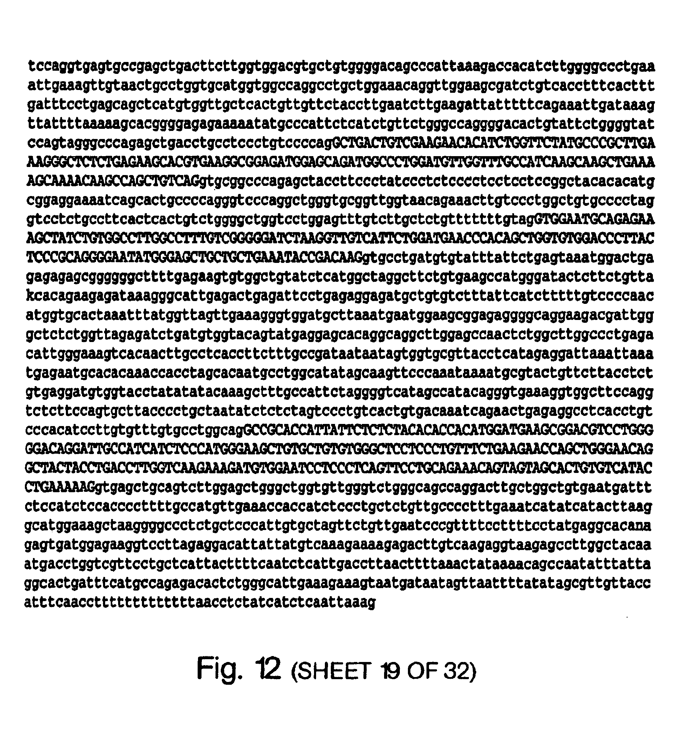

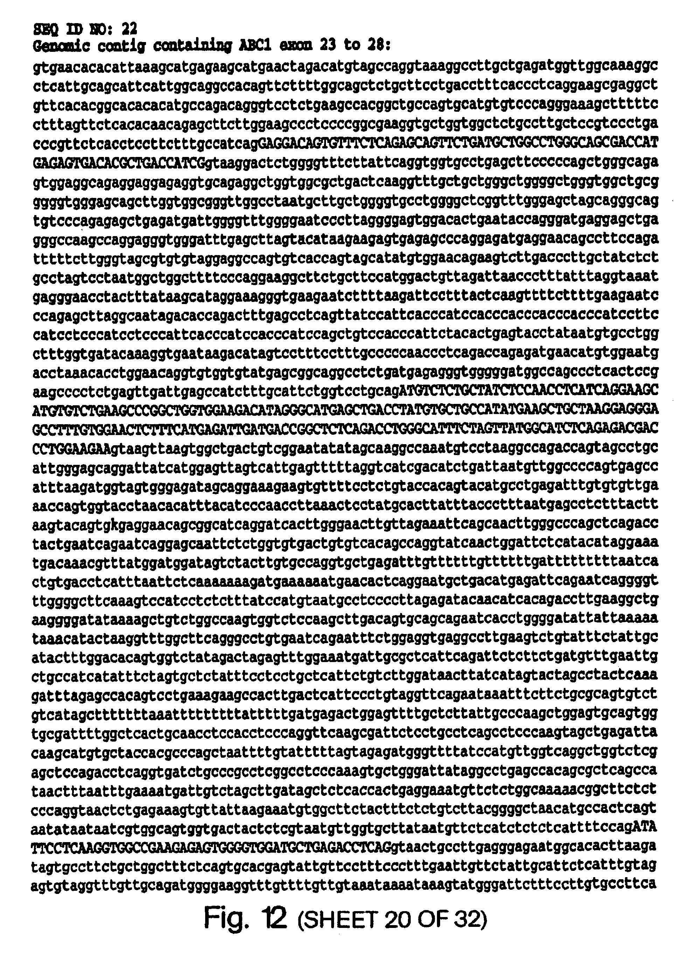

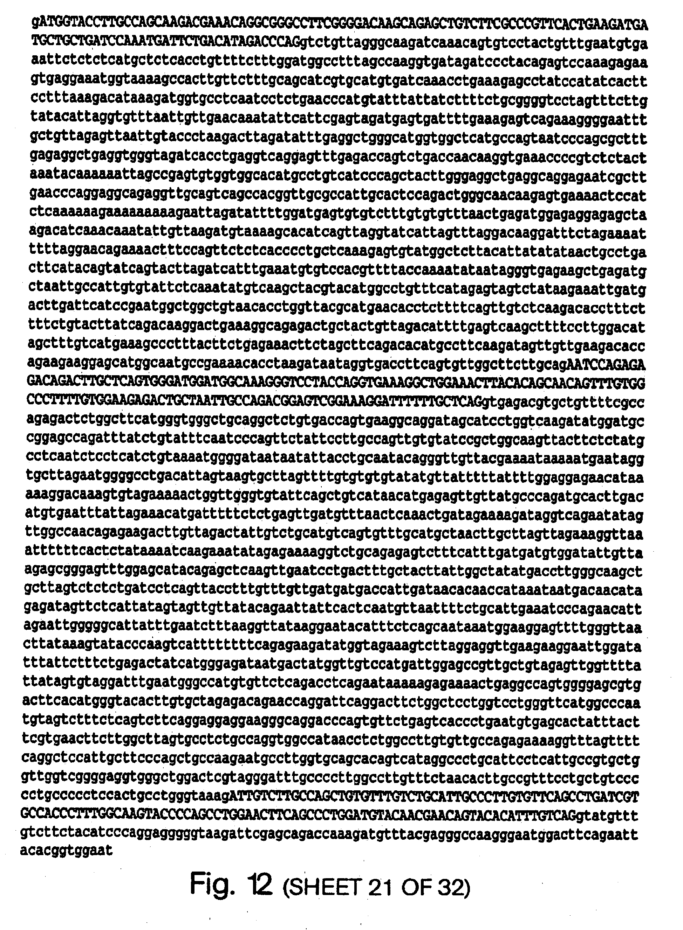

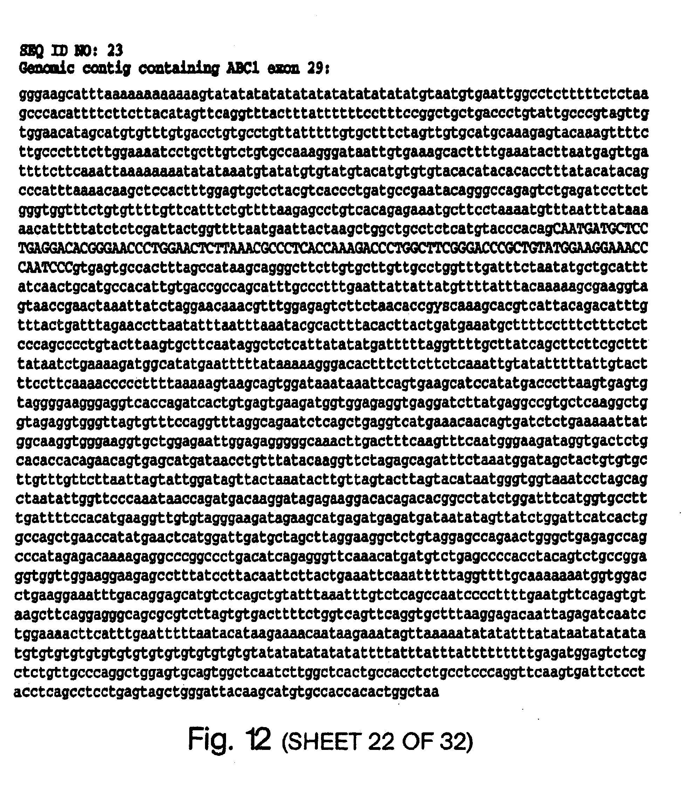

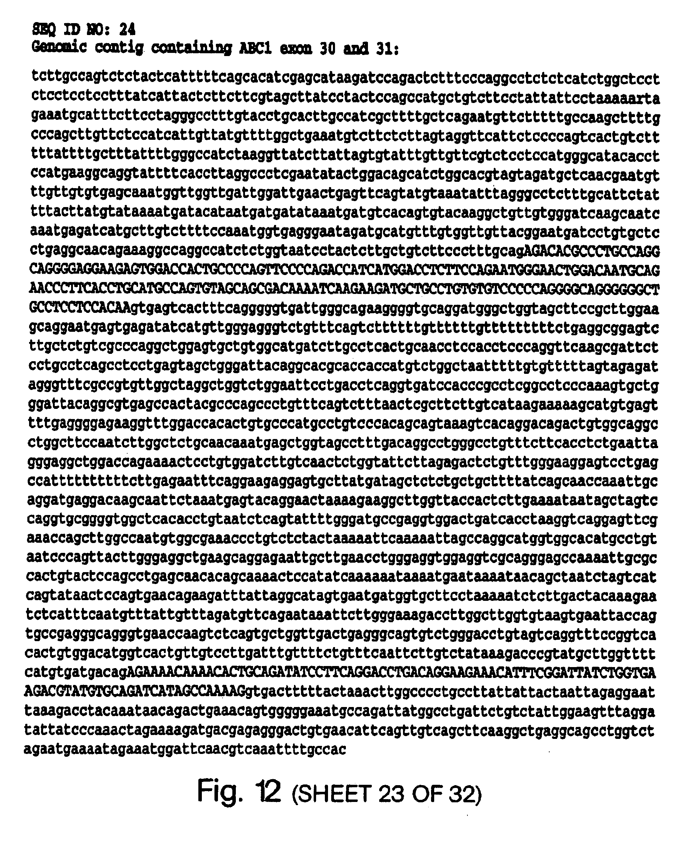

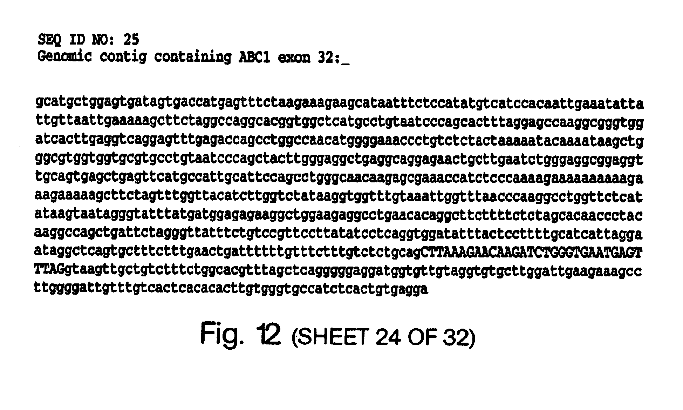

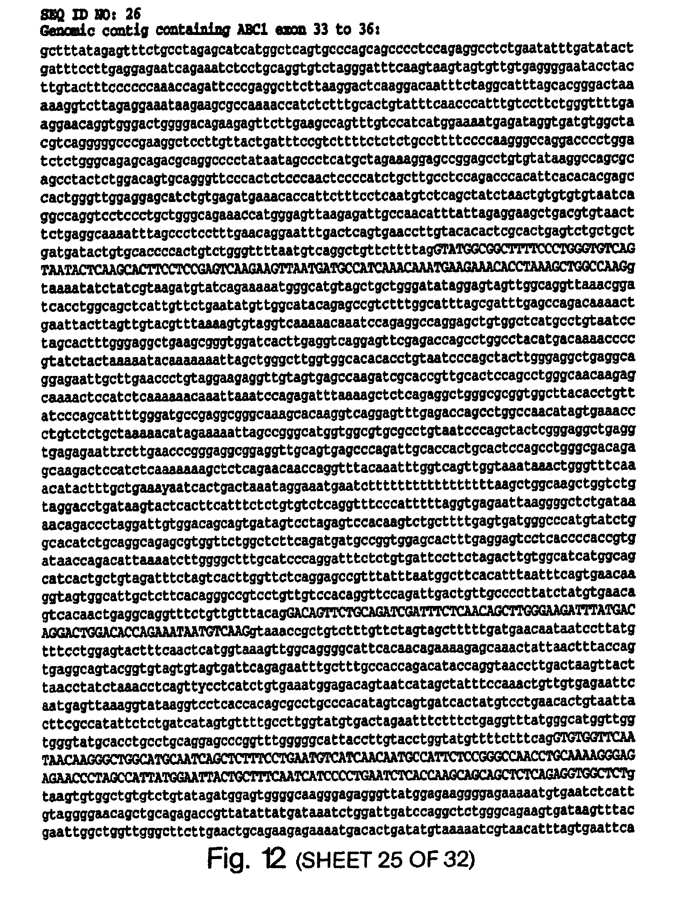

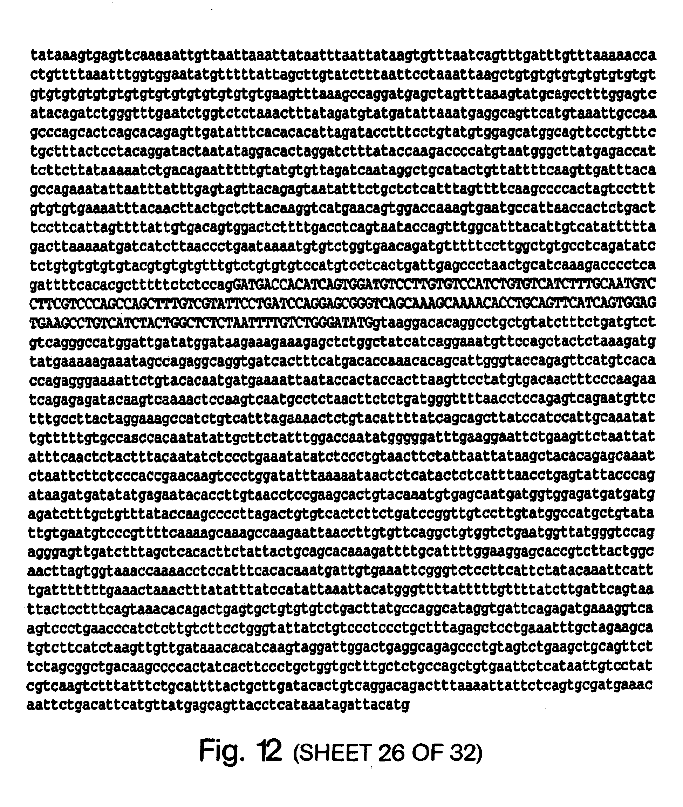

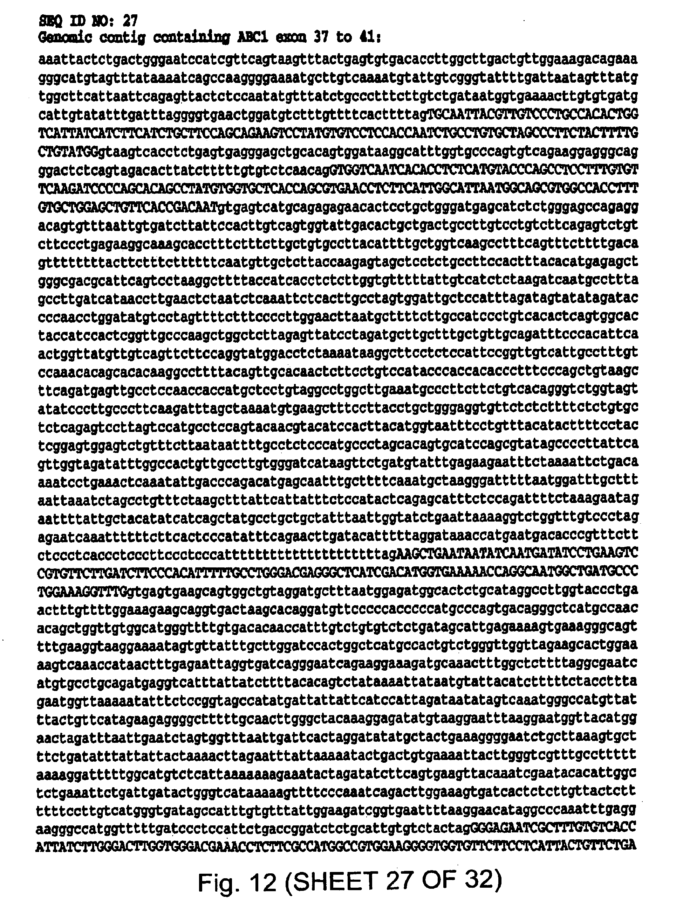

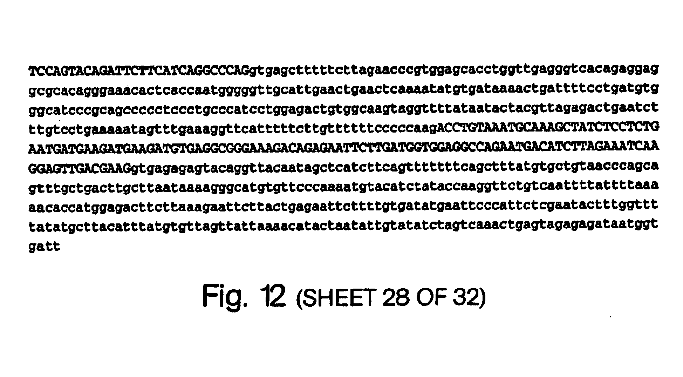

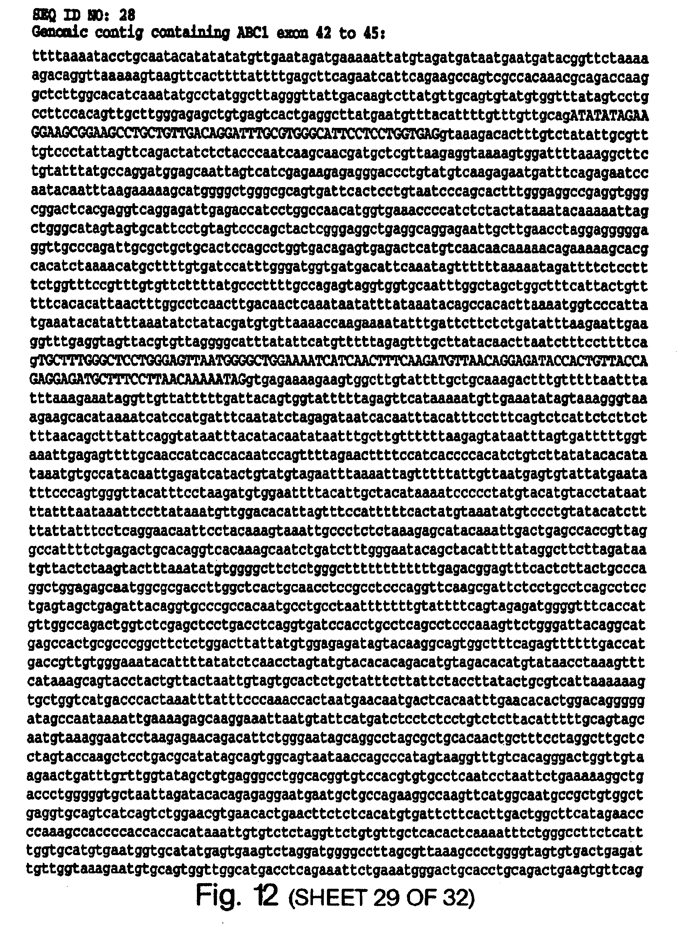

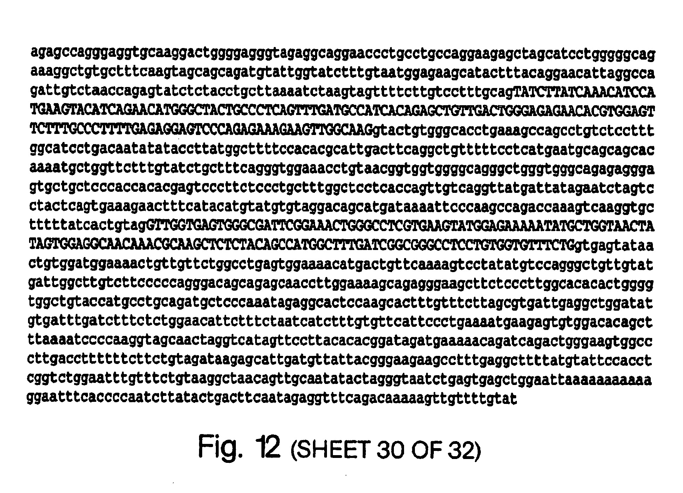

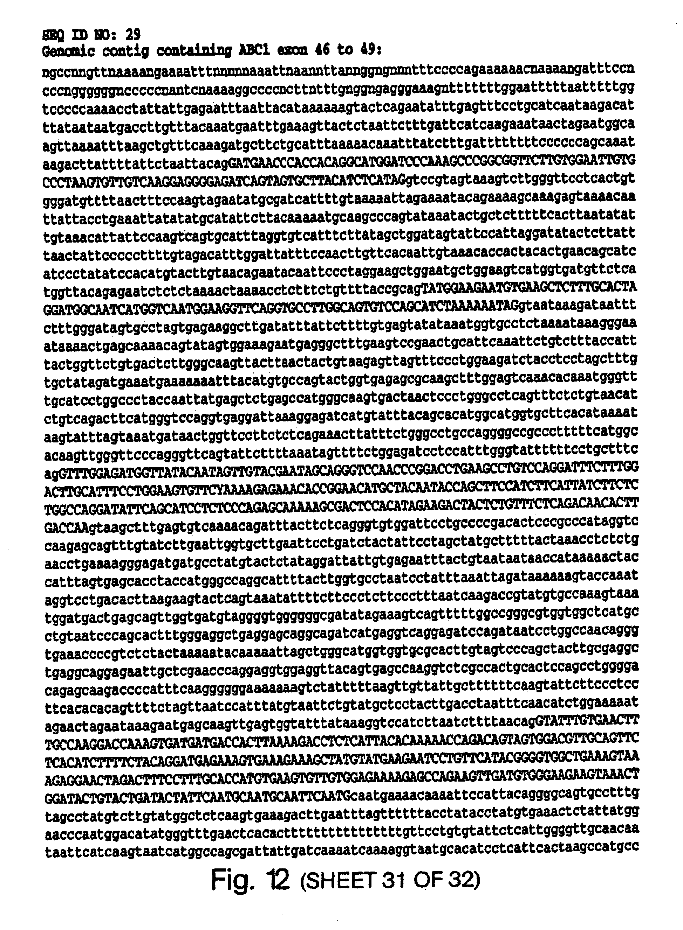

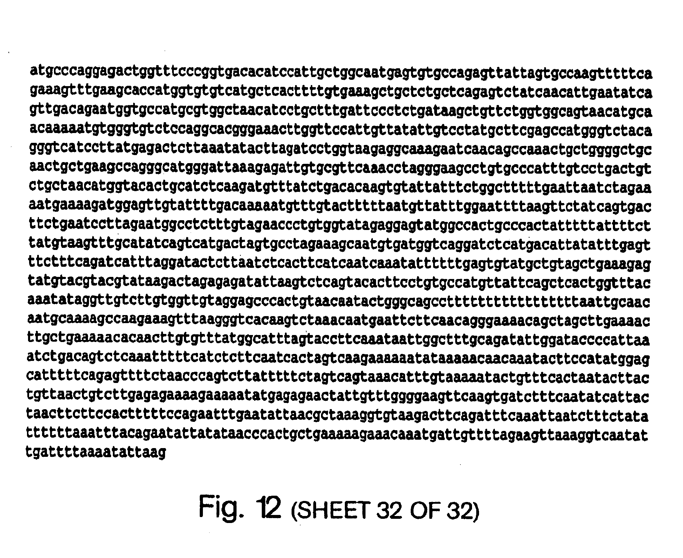

Fig. 12 shows a series of genomic contigs (SEQ ID NOS. 14-29) containing the ABC1 promoter (SEQ ID NO: 14), as well as exons 1-49 (and flanking intronic sequence) of ABC1. The exons (capitalized letters) are found in the contigs as follows: SEQ ID NO: 14--exon 1; SEQ ID NO: 15--exon 2; SEQ ID NO: 16--exon 3; SEQ ID NO: 17--exon 4; SEQ ID NO: 18--exon 5; SEQ ID NO: 19--exon 6; SEQ ID NO: 20--exons 7 and 8; SEQ ID NO: 21--exons 9 through 22; SEQ ID NO: 22--exons 23 through 28; SEQ ID NO: 23--exon 29; SEQ ID NO: 24--exons 30 and 31; SEQ ID NO: 25--exon 32; SEQ ID NO: 26--exons 33 through 36; SEQ ID NO: 27--exons 37 through 41; SEQ ID NO: 28--exons 42-45; SEQ ID NO: 29--exons 46-49.

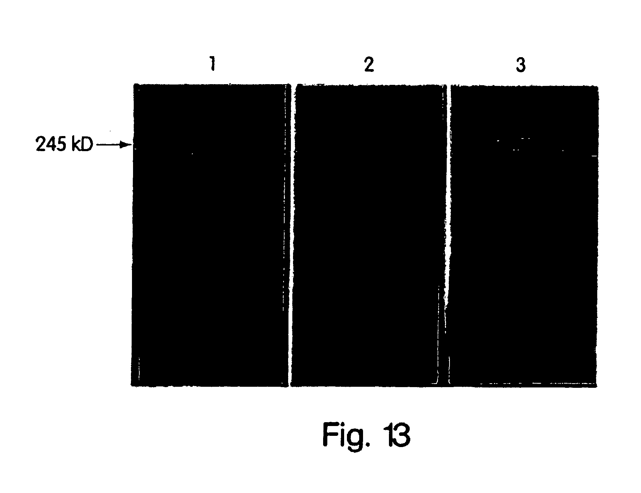

Fig. 13 is a series of illustrations showing that the amino-terminal 60 amino acid region of ABC1 is protein-coding. Lysates of normal human fibroblasts were immunoblotted in parallel with a rabbit polyclonal antibody to amino acids 1-20 of human ABC1 (1); a rabbit polyclonal antibody to amino acids 1430-1449 of human ABC 1 (2); and a mouse monoclonal antibody to amino acids 2236-2259 of human ABC1. The additional bands detected in lane 2 may be due to a lack of specificity of that antibody or the presence of degradation products of ABC 1.

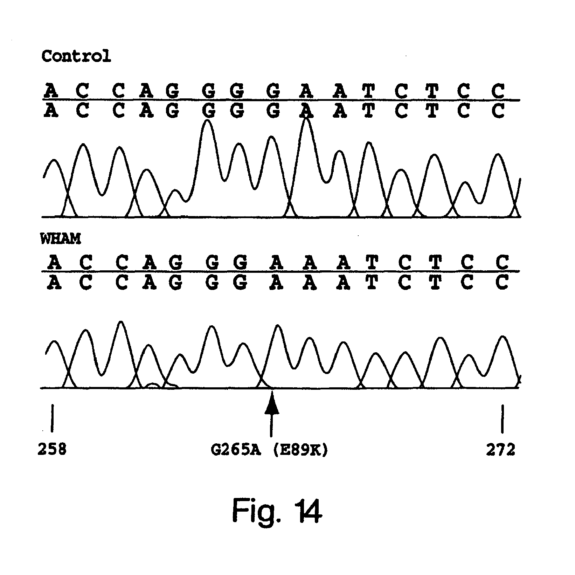

Fig. 14 is a schematic illustration showing that the WHAM chicken contains a non-conservative substitution (G265A) resulting in an amino acid change (E89K).

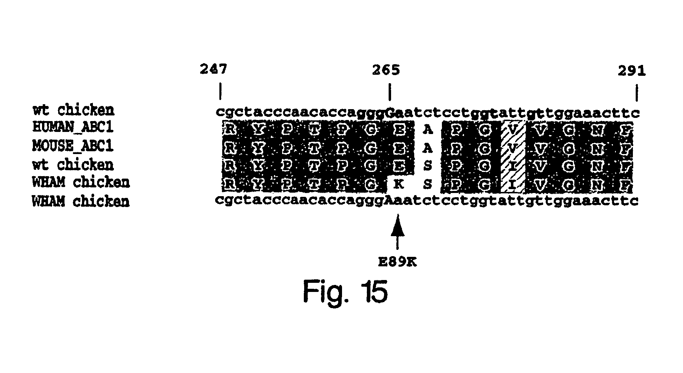

Fig. 15 is a schematic illustration showing that the mutation in the WHAM chicken is at an amino acid that is conserved among human, mouse, and chicken.

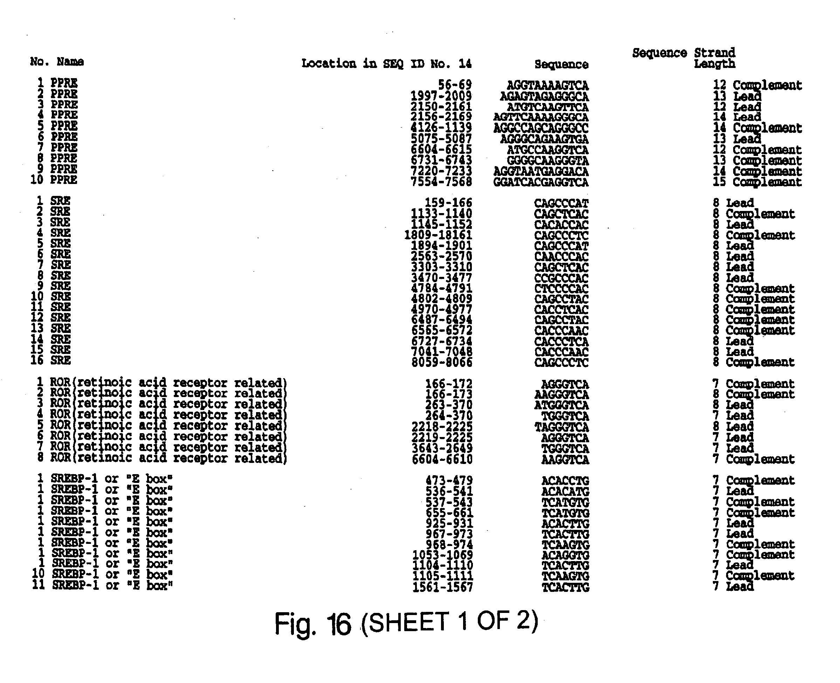

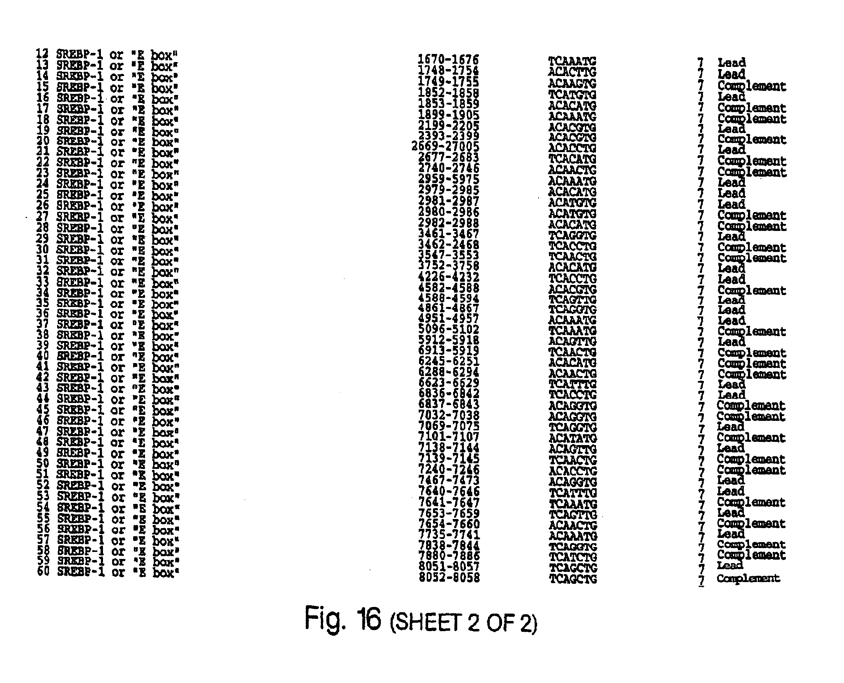

Fig. 16 show a summary of locations of consensus transcription factor binding sites in the human ABC1 promoter (nucleotides 1-8238 of SEQ ID NO: 14). The abbreviations are as follows: PPRE=peroxisome proliferator-activated receptor. SRE=steroid response element-binding protein site. ROR=RAR-related orphan receptor.

### Detailed Description

Genes play a significant role influencing HDL levels. Tangier disease (TD) was the first reported genetic HDL deficiency. The molecular basis for TD is unknown, but has been mapped to 9q31 in three families. We have identified two additional probands and their families, and confirmed linkage and refined the locus to a limited genomic region. Mutations in the *ABC1* gene accounting for all four alleles in these two families were detected. A more frequent cause of low HDL levels is a distinct disorder, familial HDL deficiency (FHA). On the basis of independent linkage, meiotic recombinants and disease associated haplotypes, FHA was localized to a small genomic region encompassing the *ABC1* gene. A mutation in a conserved residue in ABC1 segregated with FHA. Antisense reduction of the *ABC1* transcript in fibroblasts was associated with a significant decrease in cholesterol efflux.

Cholesterol is normally assembled with intracellular lipids and secreted, but in TD the process is diverted and cholesterol is degraded in lysosomes. This disturbance in intracellular trafficking of cholesterol results in an increase in intracellular cholesterol ester accumulation associated with morphological changes of lysosomes and the Golgi apparatus and cholesteryl ester storage in histiocytes, Schwann cells, smooth muscle cells, mast cells and fibroblasts.

The clinical and biochemical heterogeneity in patients with TD has led to the possibility that genetic heterogeneity may also underlie this disorder. Considering this, we initially performed linkage analysis on these two families of different ancestries (TD-1 is Dutch, TD-2 is British; Frohlich et al., Clin. Invest. Med. 10:377-382, 1987) and confirmed that the genetic mutations underlying TD in these families were localized to the same 9q31 region, to which a large family with TD had been assigned (Rust et al., Nature Genetics 20:96-98, 1998). Detailed haplotype analysis, together with the construction of a physical map, refined the localization of this gene. Mutations in the ABC 1 gene were found in TD.

FHA is much more common than TD, although its precise frequency is not known. While TD has been described to date in only 40 families, we have identified more than 40 FHA families in the Netherlands and Quebec alone. After initial suggestions of linkage to 9q31, thirteen polymorphic markers spanning approximately 10 cM in this region were typed and demonstrated the highest LOD score at D9S277. Analysis of the homozygosity of markers in the TD-2 proband, who was expected to be homozygous for markers close to TD due to his parents' consanguinity, placed the TD gene distal to D95127. Combined genetic data from TD and FHA families pointed to the same genomic segment spanning approximately 1,000 kb between D9S 127 and D9S 1866. The *ABC1* transporter gene was contained within the minimal genomic region. RT-PCR analysis in one family demonstrated a deletion of leucine at residue 693 (Δ693) in the first transmembrane domain of ABC1, which segregated with the phenotype of HDL deficiency in this family.

ABC1 is part of the ATP-binding cassette (ABC transporter) superfamily, which is involved in energy-dependent transport of a wide variety of substrates across membranes (Dean et al., Curr. Opin. Gen. Dev. 5:779-785, 1995). These proteins have characteristic motifs conserved throughout evolution which distinguish this class of proteins from other ATP binding proteins. In humans these genes essentially encode two ATP binding segments and two transmembrane domains (Dean et al., Curr. Opin. Gen. Dev. 5:779-785, 1995). We have now shown that the ABC1 transporter is crucial for intracellular cholesterol transport.

We have demonstrated that reduction of the *ABC1* transcript using oligonucleotide antisense approaches results in decreased efflux, clearly demonstrating the link between alterations in this gene and its functional effects. TD and FHA now join the growing list of genetic diseases due to defects in the ABC group of proteins including cystic fibrosis (Zielenski, et al., Annu. Rev. Genet. 29:777-807, 1995), adrenoleukodystrophy (Mosser et al., Nature 361: 726-730, 1993), Zellweger syndrome (Gärtner et al., Nat. Genet. 1:23, 1992), progressive familial intrahepatic cholestatis (Bull et al., Nat. Genet. 18:219-224, 1998), and different eye disorders including Stargardt disease (Allikmets et al., Nat. Genet.15:236-246, 1997), autosomal recessive retinitis pigmentosa (Allikmets et al., Science 277:1805-1807, 1997), and cone-rod dystrophy (Cremers et al., Hum. Mol. Genet. 7:355-362, 1998).

Patients with TD have been distinguished from patients with FHA on the basis that Tangier disease was an autosomal recessive disorder (OMIM 20540) while FHA is inherited as an autosomal dominant trait (OMIM 10768). Furthermore, patients with TD have obvious evidence for intracellular cholesterol accumulation which is not seen in FHA patients. It is now evident that heterozygotes for TD do have reduced HDL levels and that the same mechanisms underlie the HDL deficiency and cholesterol efflux defects seen in heterozygotes for TD as well as FHA. Furthermore, the more severe phenotype in TD represents loss of function from both alleles of the *ABC1* gene.

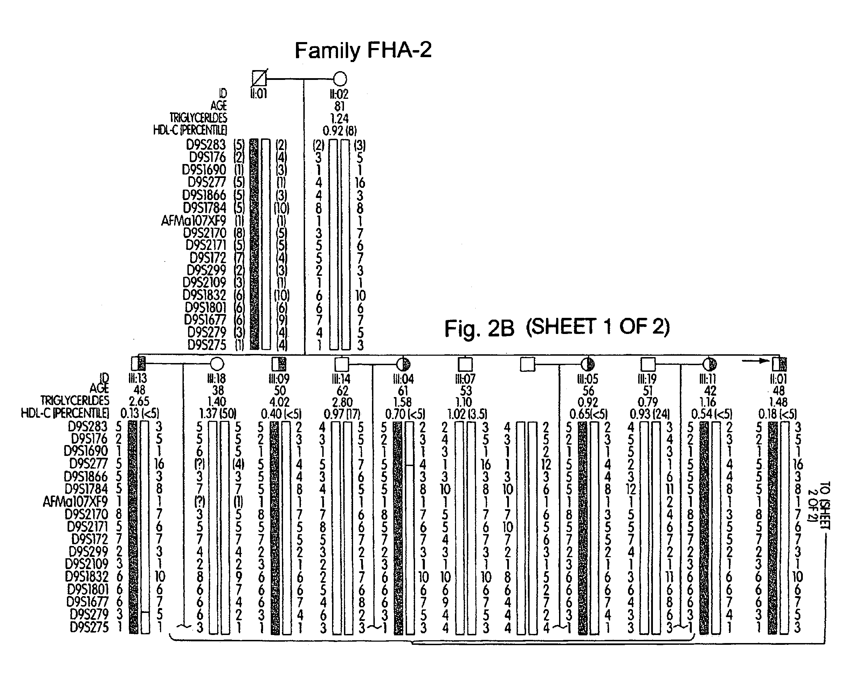

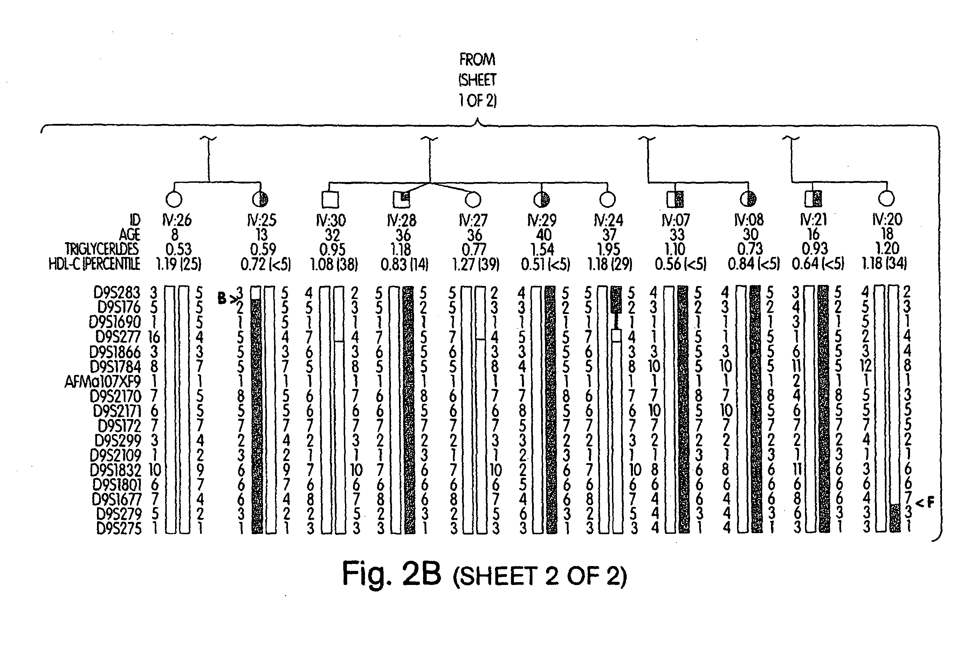

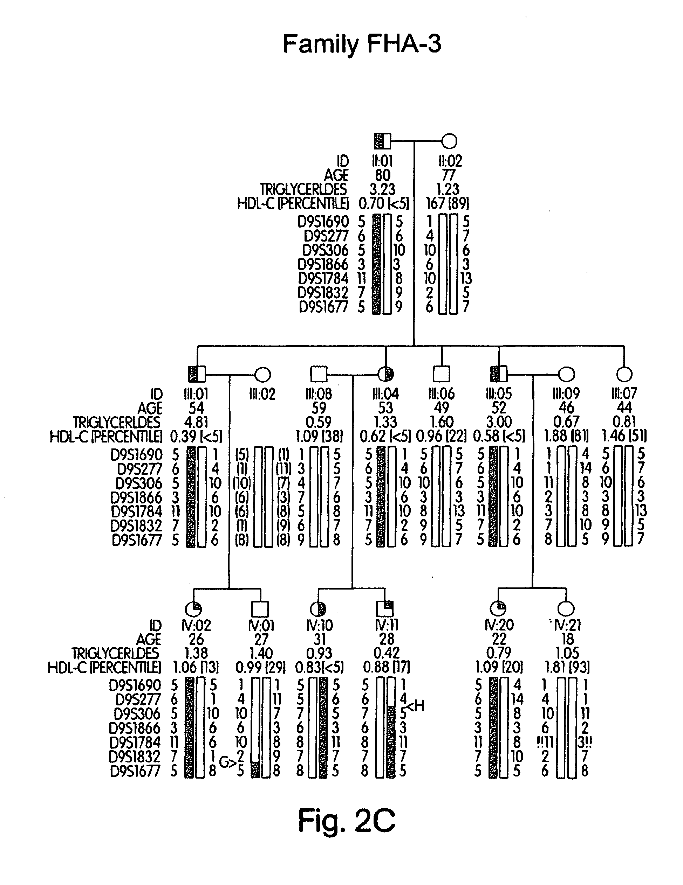

ABC1 is activated by protein kinases, presumably via phosphorylation, which also provides one explanation for the essential role of activation of protein kinase C in promoting cholesterol efflux (Drobnick et al., Arterioscler. Thromb. Vasc. Biol. 15: 1369-1377, 1995). Brefeldin, which inhibits trafficking between the endoplasmic reticulum and the Golgi, significantly inhibits cholesterol efflux, essentially reproducing the effect of mutations in ABC1, presumably through the inhibition of ABC1 biological activity. This finding has significance for the understanding of mechanisms leading to premature atherosclerosis. TD homozygotes develop premature coronary artery disease, as seen in the proband of TD-1 (111-01) who had evidence for coronary artery disease at 38 years. This is particular noteworthy as TD patients, in addition to exhibiting significantly reduced HDL, also have low LDL cholesterol, and yet they develop atherosclerosis despite this. This highlights the importance of HDL intracellular transport as an important mechanism in atherogenesis. There is significant evidence that heterozygotes for TD are also at increased risk for premature vascular disease (Schaefer et al., Ann. Int. Med. 93:261-266, 1980; Serfaty-Lacrosniere et al., Atherosclerosis 107:85-98, 1994). There is also preliminary evidence for premature atherosclerosis in some probands with FHA (Fig. 2B), *e.g.,* the proband in FHA-2 (III-01) had a coronary artery bypass graft at 46 years while the proband in FHA-3 (Fig. 2C) had evidence for GAD around 50 years of age. The TD-1 proband had more severe efflux deficiency than the TD-2 proband (Fig. 1 C). Interestingly, the TD-2 proband had no evidence for CAD by 62 when he died of unrelated causes, providing preliminary evidence for a relationship between the degree of cholesterol efflux (mediated in part by the nature of the mutation) and the likelihood of atherosclerosis.

The *ABC1* gene plays a crucial role in cholesterol transport and, in particular, intracellular cholesterol trafficking in monocytes and fibroblasts. It also appears to play a significant role in other tissues such as the nervous system, GI tract, and the cornea. Completely defective intracellular cholesterol transport results in peripheral neuropathy, corneal opacities, and deposition of cholesterol esters in the rectal mucosa.

HDL deficiency is heterogeneous in nature. The delineation of the genetic basis of TD and FHA underlies the importance of this particular pathway in intracellular cholesterol transport, and its role in the pathogenesis of atherosclerosis. Unraveling of the molecular basis for TD and FHA defines a key step in a poorly defined pathway of cholesterol efflux from cells and could lead to new approaches to treatment of patients with HDL deficiency in the general population.

HDL has been implicated in numerous other biological processes, including but not limited to: prevention of lipoprotein oxidation; absorption of endotoxins; protection against *Trypanosoma brucei* infection; modulation of endothelial cells; and prevention of platelet aggregation (see Genest et al., J. Invest. Med. 47: 31-42, 1999, hereby incorporated by reference). Any compound that modulates HDL levels may be useful in modulating one or more of the foregoing processes. The present discovery that ABC1 functions to regulate HDL levels links, for the first time, ABC1 with the foregoing processes.

The following examples are to illustrate the invention. They are not meant to limit the invention in any way.

### Analysis of TD Families

### Studies of cholesterol efflux

Both probands had evidence of marked deficiency of cholesterol efflux similar to that previously demonstrated in TD patients (Fig. 1C). TD-1 is of Dutch descent while TD-2 is of British descent.

### Linkage analysis and establishment of a physical map

Multiple DNA markers were genotyped in the region of 9q31 to which linkage to TD had been described (Rust et al., Nat. Genet. 20, 96-98, 1998). Two point linkage analysis gave a maximal peak LOD score of 6.49 at D9S 1832 (Table 1) with significant evidence of linkage to all markers in a -10 cM interval. Recombination with the most proximal marker, D9S1690 was seen in II-09 in Family TD-1 (A* in Fig. 3D) providing a centromeric boundary for the disease gene. Multipoint linkage analysis of these data did not increase the precision of the positioning of the disease trait locus.

A physical map spanning approximately 10 cM in this region was established with the development of a YAC contig (Fig. 3A). In addition, 22 other polymorphic multi-allelic markers which spanned this particular region were mapped to the contig (Fig. 3B) and a subset of these were used in construction of a haplotype for further analysis (Figs. 1A and 1B; Table 2).

While the family of Dutch decent did not demonstrate any consanguinity, the proband in TD-2 was the offspring of a first-cousin consanguineous marriage (Fig. 1B). We postulated, therefore, that it was most likely that this proband would be homozygous for the mutation while the proband in the Dutch family was likely to be a compound heterozygote. The Dutch proband shows completely different mutation bearing haplotypes, supporting this hypothesis (Fig. 3C).

The TD-2 proband was homozygous for all markers tested (Fig. 1B) distal to D9S127 but was heterozygous at D9S127 and DNA markers centromeric to it (Fig. 3C). This suggested that the gene for TD was likely located to the genomic region telomeric of D9S127 and encompassed by the markers demonstrating homozygosity (Fig. 3B).

**TABLE 1**

| **Two Point Linkage Analysis of TD-1 and TD-2** | | | | | | | |
|---|---|---|---|---|---|---|---|
| | LOD Score at recombination fraction | | | | | | |
| Marker Locus | 0 | 0.01 | 0.05 | 0.10 | 0.20 | 0.30 | 0.40 |
| *D9S1690* | -infini | 4.25 | 4.52 | 4.26 | 3.39 | 2.30 | 1.07 |
| *D9S277* | 6.22 | 6.11 | 5.67 | 5.10 | 3.90 | 2.60 | 1.17 |
| *D9S1866* | 4.97 | 4.87 | 4.49 | 4.00 | 2.95 | 1.85 | 0.70 |
| *D9S1784* | 5.50 | 5.40 | 5.00 | 4.47 | 3.36 | 2.17 | 0.92 |
| *D9S1832* | 6.49 | 6.37 | 5.91 | 5.31 | 4.05 | 2.69 | 1.21 |
| *D9S1677* | 4.60 | 4.51 | 4.18 | 3.76 | 2.88 | 1.93 | 0.93 |

| | | | | | | | |
|---|---|---|---|---|---|---|---|
| Results of pairwise linkage analysis using MLINK. Values correspond to the LOD score for linkage between the disease locus and a marker locus for specified values of the recombination fraction. | | | | | | | |

**TABLE 2. Microsatellite markers used in this study**

| Genetic Markers | Type | Hetero Zygocity | Number of alleles | Allele frequency^{t} size.bp(proportion) |
|---|---|---|---|---|
| D9S283 | CA | 0.80 | 10 | 179(0.04); 181(0.34); 183(0.19); 185(0.20); 189(0.05); 193(0.04); 197(0.07); 199(0.02); 201(0.04); 203(0.04) |
| D9S176 | CA | 0.82 | 9 | 129(0.03); 131(0.06); 133(0.26); 135(0.12);137(0.25); 139(0.03); 141(0.01); 145(0.05); 147(0.05) |
| D9S1690 | CA | 0.79 | 8 | 225(0.38); 227(0.14); 229(0.04); 231(0.12); 233(0.05); 235(0.16); 237(0.05); 239(0.05) |
| D9S277 | CA | 0.89 | 15 | 167(0.07); 171(0.02); 173(0.15); 175(0.11); 177(0.07); 179(0.04); 181(0.17); 183(0.06); 185(0.02); 187(0.02); 189(0.13); 191(0.13); 193(0.02); 197(0.00); 199(0.00) |
| D9S127 | CA | 0.72 | 6 | 149(0.11); 151(0.07); 153(0.25); 155(0.03); 157(0.45);159(0.06) |
| D9S306 | CA | 0.87 | 13 | 102(0.06); 104(0.01); 110(0.03); 112(0.08); 114(0.16); 116(0.15); 118(0.11); 120(0.23); 122(0.06); 124(0.06); 126(0.03); 134(0.02); 136(0.01) |
| D9S 1866 | CA | 0.62 | 11 | 248(0.06); 252(0.04); 254(0.01 256(58); 258(0.03); 260(0.06); 262(0.02); 264(0.12); 266(0.06); 268(0.03); 270(0.01) |
| D9S 1784 | CA | 0.86 | 15 | 174(0.10); 176(0.02); 178(0.00); 180(0.08);182(0.11); 184(0.22); 136(0.15); 158(0.06); 190(0.04); 192(0.07); 194(0.08): 196(0.07);198(0.01); 200(0.01), 202(0.01) |
| AFMa107xt9 | CA | n.a | n.a. | n.a |
| D9S2170 | CA | n.a | n.a | n.a. |
| D9S2171 | CA | n.a. | n.a. | n.a. |
| D9S2107 | CA | 0.63 | 5 | n.a. |
| D9S 172 | CA | 0.54 | 5 | 291 (0.00); 297(0.05); 299(0.32); 303(0.62); 305(0.02) |
| D9S2109 | CA | 0.51 | 3 | 1(0.42); 2(0.56); 3(0.02) |
| D9S1832 | CA | 0.88 | 12 | 161(0.04); 163(0.02); 167(0.02); 169(0.04); 171(0.10); 173(0.09); 175(0.15); 177(0.28); 179(0.19); 181(0.04)-1183(0.01); 185(0.01) |
| D9S 1835 | CA | 0.48 | 4 | 110(0.02); 112(0.23); 116(0.68); 118(0.07) |
| D9S1801 | CA | 0.7 | 10 | 166(0.10); 172(0.04); 174(0.02); 182(0.02); 184(0.19); 186(0.40); 188(0.15); 190(0.04); 192(0.02); 194(0.02) |
| D9S261 | CA | 0.63 | 7 | 90(0.02); 92(0.52);94(0.02); 98(0.02); 100(0.10); 102(0.04); 104(0.08) |
| D9S160 | CA | 0.62 | 6 | 136(0.25); 138(0.53); 140(0.01); 142(0.12); 144(0.00); 146(0.07) |
| D9S1677 | CA | 0.81 | 10 | 251(0.27); 257(0.27); 259(0.07); 261(0.09); 263(0.27); 265(0.14); 267(0.02); 267(0.02) 271(0.04); 273(0.02) |
| D9S279 | CA | 0.78 | 6 | 244(0.09); 246(0.18); 248(0.29); 250(0.29);252(0.07); 254(0.09) |
| D9S275 | CA | 0.62 | 4 | 190(0.31); 196(0.07); 198(0.52); 200(0.09) |

| | | | | |
|---|---|---|---|---|
| ^{t} In a Caucasian population of French Canadian or French descent (J. Weissenbach, Personal Communication 1993). n.a. = not assessed. | | | | |

These polymorphic microsatellite markers were used for DNA typing in the region of 9q31 seen in Fig. 3. The majority come from the last version of the Généthon human linkage map ⁵³. The ferquency of heterozygosity, the number of alleles as well as the allele frequency of each marker are presented.

### Mutation detection

Based on the defect in intracellular cholesterol transport in patients with TD, we reviewed the EST database for genes in this region which might be relevant to playing a role in this process. One gene that we reviewed as a candidate was the lysophosphatidic acid (LPA) receptor *(EDG2)* which mapped near D9S 1801 (Fig. 3C). This receptor binds LPA and stimulates phospholipase-C (PLC), and is expressed in fibroblasts. It has previously been shown that the coordinate regulation of PLC that is necessary for normal HDL3 mediated cholesterol efflux is impaired in TD (Walter et al., J. Clin. Invest. 98:2315-2323, 1996). Therefore this gene represented an excellent candidate for the TD gene. Detailed assessment of this gene, using Northern blot and RT-PCR and sequencing analysis, revealed no changes segregating with the mutant phenotype in this family, in all likelihood excluding this gene as the cause for TD. Polymorphisms were detected, however, in the RT-PCR product, indicating expression of transcripts from both alleles.

The second candidate gene *(RGS3)* encodes a member of a family regulating G protein signaling which could also be involved in influencing cholesterol efflux (Mendez et al., Trans. Assoc. Amer. Phys. 104:48-53, 1991). This gene mapped 0.7 cM telomeric to the LPA-receptor (Fig. 3C), and is expressed in fibroblasts. It was assessed by exon-specific amplification, as its genomic organization was published (Chatterjee et al., Genomics 45:429-433, 1997). No significant sequence changes were detected.

The *ABC1* transporter gene had previously been mapped to 9q31, but its precise physical location had not been determined (Luciani et al., Genomics 21:150-159, 1994). The *ABC1* gene is a member of the ATP binding cassette transporters which represents a super family of highly conserved proteins involved in membrane transport of diverse substrates including amino acids, peptides, vitamins and steroid hormones (Luciani et al., Genomics 21:150-159, 1994; Dean et al., Curr. Opin. Gen. Dev. 5:779-785, 1995). Primers to the 3' UTR of this gene mapped to YACs spanning D9S306 (887-B2 and 930-D3) compatible with it being a strong candidate for TD. We initiated large scale genomic sequencing ofBACs spanning approximately 800 kb around marker D9S306 (BACs 269, 274, 279 and 291) (Fig 3E). The *ABC1* gene was revealed encompassing 49 exons and a minimum of 75 Kb of genomic sequence. In view of the potential function of a gene in this family as a cholesterol transporter, its expression in fibroblasts and localization to the minimal genomic segment underlying TD, we formally assessed ABC 1 as a candidate.

Patient and control total fibroblast RNA was used in Northern blot analysis and RT-PCR and sequence analyses. RT-PCR and sequence analysis of TD-1 revealed a heterozygous T to C substitution (Fig. 4A) in the TD-1 proband, which would result in a substitution of arginine for cysteine at a conserved residue between mouse and man (Fig. 4B). This mutation, confirmed by sequencing exon 30 of the ABC1 gene, exhibited complete segregation with the phenotype on one side of this family (Fig. 4C). This substitution creates a *Hga*I site, allowing for RFLP analysis of amplified genomic DNA and confirmation of the mutation (Fig. 4C). The point mutation in exon 30 was not seen on over 200 normal chromosomes from unaffected persons of Dutch decent, and 250 chromosomes of Western European decent, indicating it is unlikely to be a polymorphism. Northern blot analysis of fibroblast RNA from this patient, using a cDNA encompassing exons 1 to 49 of the gene, revealed a normal sized ~8 Kb transcript and a truncated mutant transcript which was not visible in control RNA or in RNA from other patients with HDL deficiency (Fig. 4D). Additionally, Northern blot analysis using clones encompassing discrete regions of the cDNA revealed that the mutant transcript was detected with a cDNA compassing exons 1 to 49 (a), 1 to 41 (b), 1 to 22 (c), much more faintly with a probe spanning exon 23 to 29 (d) and not seen with probes encompassing exons 30 to 42 (e), but not seen with cDNA fragment spanning exons 30 to 49 (f). This was repeated on multiple filters with control RNA, RNA from other patients with HDL deficiency and the other TD proband, and only in TD-1 was the truncated transcript observed. Sequence analysis of the coding region did not reveal an alteration in sequence that could account for this finding. Furthermore, DNA analysis by Southern blot did not reveal any major rearrangements. Completion of exon sequencing in genomic DNA showed that this mutation was a G to C transversion at position (+1) of intron 24, (Fig. 11) affecting a splice donor site and causing aberrant splicing.

RT-PCR analysis of fibroblast RNA encoding the *ABC1* gene from the proband in TD-2 (Fig. 1B) revealed a homozygous nucleotide change of A to G at nucleotide 1864 of SEQ ID NO: 2 in exon 13 (Fig. 5A), resulting in a substitution of arginine for glutamine at residue 597 of SEQ ID NO: 1 (Fig. 5B), occurring just proximal to the first predicted transmembrane domain of ABC1 (Fig. 8) at a residue conserved in mouse and as well as a C. *elegans* homolog. This mutation creates a second *AciI* site within exon 13. Segregation analysis of the mutation in this family revealed complete concordance between the mutation and the low HDL phenotype as predicted (Fig 5C). The proband in TD-2 is homozygous for this mutation, consistent with our expectation of a disease causing mutation in this consanguineous family.

### Analysis of FHA families

### Linkage analysis and refinement of the minimal genomic region containing the gene for FHA

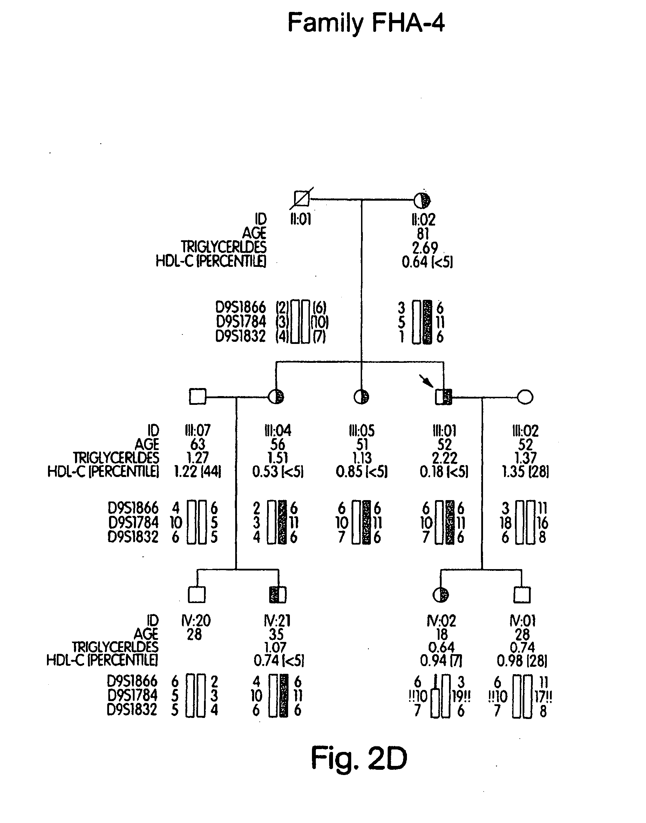

Data from microsatellite typing of individual family members from the four pedigrees of French Canadian origin were analyzed (Fig. 2). A maximum LOD score of 9.67 at a recombination fraction of 0.0 was detected at D9S277 on chromosome 9q31 (Fig. 3; Table 3). Thereafter, 22 markers were typed in a region spanning 10 cM around this locus in these families (Figs. 2 and 3). The frequency for these markers were estimated from a sample of unrelated and unaffected subjects of French ancestry (Table 2).

TD and FHA have thus far been deemed distinct with separate clinical and biochemical characteristics. Even though the genes for these disorders mapped to the same region, it was uncertain whether FHA and TD were due to mutations in the same gene or, alternatively, due to mutations in genes in a similar region. Refinement of the region containing the gene for FHA was possible by examining haplotype sharing and identification of critical recombination events (Fig. 2). Seven separate meiotic recombination events were seen in these families ("A" through "G" in Figs. 2 and 3), clearly indicating that the minimal genomic region containing the potential disease gene was a region of approximately 4.4 cM genomic DNA spanned by marker D9S1690 and D9S 1866 (Figs. 2 and 3). This region is consistent with the results of two point linkage analysis which revealed maximal LOD scores with markers

**TABLE 3**

| Two Point Linkage Analysis of FHA | | | | | | | |
|---|---|---|---|---|---|---|---|
| | LOD Score at recombination fraction | | | | | | |
| Marker Locus | 0 | 0.01 | 0.05 | 0.10 | 0.20 | 0.30 | 0.40 |
| D9S283 | -infini | -2.57 | 0.51 | 1.48 | 1.84 | 1.48 | 0.76 |
| D9S176 | -infini | 1.42 | 3.07 | 3.39 | 3.05 | 2.22 | 1.12 |
| D9S1690 | -infini | 3.11 | 4.04 | 4.04 | 3.33 | 2.24 | 0.96 |
| D9S277 | 9.67 | 9.51 | 8.89 | 8.06 | 6.29 | 4.30 | 2.10 |
| D9S306 | 5.60 | 5.51 | 5.13 | 4.62 | 3.55 | 2.36 | 1.11 |
| D9S1866 | -infini | 7.24 | 7.35 | 6.87 | 5.50 | 3.82 | 1.91 |
| D9S1784 | -infini | 8.85 | 7.76 | 9.03 | 7.09 | 4.78 | 2.25 |
| D9S172 | -infini | 2.63 | 3.00 | 2.87 | 2.26 | 1.50 | 0.67 |
| D9S1832 | -infini | 5.20 | 5.97 | 5.75 | 4.59 | 3.02 | 1.30 |
| D9S1801 | 0.14 | 0.13 | 0.11 | 0.09 | 0.06 | 0.03 | 0,.01 |
| D9S1677 | -infini | 7.83 | 7.90 | 7.38 | 5.90 | 4.08 | 2.01 |
| D9S279 | -infini | 3.43 | 3.80 | 3.66 | 3.01 | 2.12 | 1.05 |
| D9S275 | -infini | 2.57 | 2.98 | 2.91 | 2.41 | 1.69 | 0.81 |

| | | | | | | | |
|---|---|---|---|---|---|---|---|
| Results of pairwise linkage analysis using MLINK. Values correspond to LOD score for linkage between the disease locus and a marker locus for specified values of the recombination fraction. | | | | | | | |

D9S277 and D9S306 and essentially excluded the region centromeric to D9S1690 or telomeric to D9S1866. An 8^{th} meiotic recombination event ("H" in Fig. 3) further refined the FHA region to distal to D9S277.

As described herein, the *ABC1* gene mapped within this interval. The overlapping genetic data strongly suggested that FHA may in fact be allelic to TD. Utilization of sets of genetic data from FHA and TD provided a telomeric boundary at D9S 1866 (meiotic recombinant) (Fig. 3D) and a centromeric marker at D9S127 based on the homozygosity data of TD-2. This refined the locus to approximately 1 Mb between D9S 127 and D9S 1866. The *ABC1* gene mapped within this minimal region (Fig. 3E).

### Mutation detection in FHA

Mutation assessment of the *ABC1* gene was undertaken in FHA-1 (Fig. 2A). Using primers that spanned overlapping segments of the mRNA we performed RT-PCR analysis and subjected these fragments to mutational analysis. A deletion of three nucleotides is evident in the RT-PCR sequence of FHA-1 III.01 (Fig. 6A), resulting in a loss of nucleotides 2151-2153 of SEQ ID NO: 2 and deletion of a leucine (ΔL693) at amino acid position 693 of SEQ ID NO: 1 (Fig. 6A). This leucine is conserved in mouse and C. *elegans* (Fig. 6B). The alteration was detected in the RT-PCR products as well as in genomic sequence from exon 14 specific amplification. This mutation results in a loss of an *Ear*I restriction site. Analysis of genomic DNA from the family indicated that the mutation segregated completely with the phenotype of HDL deficiency. The loss of the *Ear*I site results in a larger fragment being remaining in persons heterozygous for this mutation (Fig. 6C). This mutation maps to the first putative transmembrane domain of ABC1 (Fig. 8) and was not seen in 130 chromosomes from persons of French Canadian descent nor seen in over 400 chromosomes from persons of other Western European ancestry.

A mutation has also been found in patient genomic DNA in pedigree FHA-3 from Quebec. The alteration, a 6 bp deletion of nucleotides 5752-5757 of SEQ ID NO: 2 within exon 41, results in a deletion of amino acids 1893 (Glu) and 1894 (Asp) of SEQ ID NO: 1. The deletion was detected as a double, superimposed, sequence starting from the point of the deletion (Fig. 6D), and was detected in sequence reads in both directions. The deletion can be detected on 3% agarose or 10% polyacrylamide gels, and segregates with disease in FHA-3. It was not seen in 128 normal chromosomes of French-Canadian origin or in 434 other control chromosomes. Amino acids 1893 and 1894 are in a region of the ABC 1 protein that is conserved between human, mouse, and C. *elegans* (Fig. 6E), implying that it is of functional importance.

An additional mutation has been found in patient genomic DNA in pedigree FHA-2 from Quebec (Fig. 6F). The alteration, a C to T transition at position 6504 of SEQ ID NO: 2, converts an arginine at position 2144 of SEQ ID NO: 1 to a STOP codon, causing truncation of the last 118 amino acids of the ABC1 protein. This alteration segregates with disease in family FHA-2.

A summary of all mutations and polymorphisms found in ABC1 is shown in Fig. 11. Each variant indicated as a mutation segregates with low HDL in its family, and was not seen in several hundred control chromosomes.

### Functional relationship between changes in ABC1 transcript levels and cholesterol efflux

Antisense approaches were undertaken to decrease the ABC1 transcript and assess the effect of alteration of the transcript on intracellular cholesterol transport. The use of antisense primers to the 5' end of ABC1 clearly resulted in a decrease to approximately 50% of normal RNA levels (Fig. 7A). This would be expected to mimic in part the loss of function due to mutations on one allele, similar to that seen in heterozygotes for TD and patients with FHA. Importantly, reduction in the mRNA for the ABC 1 gene resulted in a significant reduction in cellular cholesterol efflux (Fig. 7B), further establishing the role of this protein in reverse cholesterol transport and providing evidence that the mutations detected are likely to constitute loss of function mutations. Furthermore, these data support the functional importance of the first 60 amino acids of the protein. Antisense oligonucleotide AN-6 is directed to the novel start codon 5' to the one indicated in AJ012376.1; this antisense oligonucleotide effectively suppresses efflux.

The above-described results were obtained using the following materials and methods.

### Patient selection

The probands in TD families had previously been diagnosed as suffering from TD based on clinical and biochemical data. Study subjects with FHA were selected from the Cardiology Clinic of the Clinical Research Institute of Montréal. The main criterion was an HDL-C level <5th percentile for age and gender, with a plasma concentration of triglycerides <95th percentile in the proband and a first-degree relative with the same lipid abnormality. In addition, the patients did not have diabetes.

### Biochemical studies

Blood was withdrawn in EDTA-containing tubes for plasma lipid, lipoprotein cholesterol, ApoAI, and triglyceride analyses, as well as storage at -80°C. Leukocytes were isolated from the buffy coat for DNA extraction.

Lipoprotein measurement was performed on fresh plasma as described elsewhere (Rogler et al., Arterioscler. Thromb. Vasc. Biol. 15:683-690, 1995). The laboratory participates and meets the criteria of the Lipid Research Program Standardization Program. Lipids, cholesterol and triglyceride levels were determined in total plasma and plasma at density d<1.006 g/mL (obtained after preparative ultracentrifugation) before and after precipitation with dextran manganese. Apolipoprotein measurement was performed by nephelometry for ApoB and ApoAI.

### Linkage analysis

Linkage between the trait locus and microsatellite loci was analyzed using the FASTLINK version (4.0 P). FASTLINK/MLINK was used for two-point linkage analysis assuming an autosomal dominant trait with complete penetrance. In FHA and TD heterozygotes, the phenotype was HDL deficiency <5th percentile for age and sex. The disease allele frequency was estimated to be 0.005. Marker allele frequencies were estimated from the genotypes of the founders in the pedigrees using NEWPREP. Multipoint linkage analysis was carried out using FASTLINK/LINKMAP.

### Genomic clone assembly and physical map construction of the 9q31 region

Using the Whitehead Institute/MIT Center for Genome Research map as a reference, the genetic markers of interest at 9q31 were identified within YAC contigs. Additional markers that mapped to the approximate 9q31 interval from public databases and the literature were then assayed against the YAC clones by PCR and hybridization analysis. The order of markers was based on their presence or absence in the anchored YAC contigs and later in the BAC contig. Based on the haplotype analysis, the region between D9S277 and D9S306 was targeted for higher resolution physical mapping studies using bacterial artificial chromosomes (BACs). BACs within the region of interest were isolated by hybridization of DNA marker probes and whole YACs to high-density filters containing clones from the RPCI-11 human BAC library (Fig. 3).

### Sequence retrieval and alignment

The human *ABC1* mRNA sequence was retrieved from GenBank using the Entrez nucleotide query (Baxevanis et al., A Practical Guide to the Analysis of Genes and Proteins, eds. Baxevanis, A.D. & Ouellette, B.F.F. 98:120, 1998) as GenBank accession number AJ012376.1. The version of the protein sequence we used as wild-type (normal) was CAA10005.1.

We identified an additional 60 amino acids in-frame with the previously-believed start methionine (Fig. 9A). Bioinformatic analysis of the additional amino acids indicates the presence of a short stretch of basic amino acid residues, followed by a hydrophobic stretch, then several polar residues. This may represent a leader sequence, or another transmembrane or membrane-associated region of the ABC 1 protein. In order to differentiate among the foregoing possibilities, antibodies directed to the region of amino acids 1-60 are raised against and used to determine the physical relationship of amino acids 1-60 in relation to the cell membrane. Other standard methods can also be employed, including, for example, expression of fusion proteins and cell fractionation.

We also identified six errors in the previously-reported nucleotide sequence (at positions 839, 4738, 5017, 5995, 6557, and 6899 of SEQ ID NO: 2; Fig. 11). Hence, the sequence of the ABC1 polypeptide of Fig. 9A differs from CAA10005.1 as follows: Thro 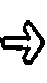 IIe at position 1554; Pro 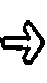 Leu at position 1642; Arg 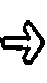 Lys at position 1973; and Pro 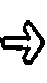 Leu at position 2167. We also identified 5' and 3' UTR sequence (Figs. 9B - 9E).

The mouse *ABC*1 sequence used has accession number X75926. It is very likely that this mouse sequence is incomplete, as it lacks the additional 60 amino acids described herein for human ABC1.

Version 1.7 of ClustalW was used for multiple sequence alignments with BOXSHADE for graphical enhancement (http://www.isrec.isb-sib.ch:8080/software/BOX_form.html) with the default parameter. A *Caenorhabditis elegans* ABC1 orthologue was identified with BLAST (version 2.08) using CAA1005.1 (see above) as a query, with the default parameter except for doing an organism filter for *C. elegans.* The selected protein sequence has accession version number AAC69223.1 with a score of 375, and an E value of 103.

### Genomic DNA sequencing

BAC DNA was extracted from bacterial cultures using NucleoBond Plasmid Maxi Kits (Clontech, Palo Alto, CA). For DNA sequencing, a sublibrary was first constructed from each of the BAC DNAs (Rowen et al., Automated DNA Sequencing and Analysis, eds. Adams, M.D., Fields, C. & Venter, J.C., 1994). In brief, the BAC DNA was isolated and randomly sheared by nebulization. The sheared DNA was then size fractionated by agarose gel electrophoresis and fragments above 2 kb were collected, treated with Mung Bean nuclease followed by T4 DNA polymerase and klenow enzyme to ensure blunt-ends, and cloned into *Sma*I*-*cut M13mp19. Random clones were sequenced with an ABI373 or 377 sequencer and fluorescently labeled primers (Applied BioSystems, Foster City, CA). DNAStar software was used for gel trace analysis and contig assembly. All DNA sequences were examined against available public databases primarily using BLASTn with RepeatMasker (University of Washington).

### Reverse transcription (RT)-PCR amplification and sequence analysis

Total RNA was isolated from the cultured fibroblasts of TD and FHA patients, and reverse transcribed with a CDS primer containing oligo d(T)18 using 250 units of SuperScript II reverse transcriptase (Life Technologies, Inc., Rockville, MD) as described (Zhang et al., J. Biol. Chem. 27:1776-1783, 1996). cDNA was amplified with Taq DNA polymerase using primers derived from the published human *ABC1* cDNA sequence (Luciani et al., Genomics 21:150-159, 1994). Six sets of primer pairs were designed to amplify each cDNA sample, generating six DNA fragments which are sequentially overlapped covering 135 to 7014 bp of the full-length human *ABC1* cDNA. The nucleotides are numbered according to the order of the published human cDNA sequence (AJ012376.1). Primer pairs (1): 135-158 (f) and 1183-1199 (r); (2): 1080-1107 (f) and 2247-2273 (r); (3): 2171-2197 (f) and 3376-3404 (r); (4): 3323-3353 (f) and 4587-4617 (r); (5) 4515-4539 (f) and 5782-5811 (r); (6): 5742-5769 (f) and 6985-7014 (r). RT-PCR products were purified by Qiagen spin columns. Sequencing was carried out in a Model 373A Automated DNA sequencer (Applied Biosystems) using Taq di-deoxy terminator cycle sequencing and Big Dye Kits according to the manufacturer's protocol.

### Northern blot analysis

Northern transfer and hybridizations were performed essentially as described (Zhang et al., J. Biol. Chem. 27:1776-1783, 1996). Briefly, 20 µg of total fibroblast RNA samples were resolved by electrophoresis in a denaturing agarose (1.2%; w/v) gel in the presence of 7% formaldehyde, and transferred to nylon membranes. The filters were probed with ³²P-labeled human ABC1 cDNA as indicated. Pre-hybridization and hybridizations were carried out in an ExpressHyb solution (ClonTech) at 68°C according to the manufacturer's protocol.

### Detection of the mutations in TD

Genotyping for the T4503C and A1864G variants was performed by PCR amplification of exon 30 followed by restriction digestion with *Hga*I and amplification of exon 13 followed by digestion with *Aci*I*,* respectively. PCR was carried out in a total volume of 50 µL with 1.5 mM MgCl₂, 187.5 nM of each dNTP, 2.5U Taq polymerase and 15 pmol of each primer (forward primer in exon 30: 5'-CTG CCA GGC AGG GGA GGA AGA GTG-3' (SEQ ID NO: 4); reverse primer spanning the junction of exon 30 and intron 30: 5'-GAA AGT GAC TCA CTT GTG GAG GA-3' (SEQ ID NO: 5); forward primer in intron 12: 5'-AAA GGG GCT TGG TAA GGG TA-3' (SEQ ID NO: 6); reverse in intron 13: 5'-CAT GCA CAT GCA CAC ACA TA -3' (SEQ ID NO: 7)). Following an initial denaturation of 3 minutes at 95°C, 35 cycles consisting of 95°C 10 seconds, 58°C 30 seconds, 72°C 30 seconds were performed, with a final extension of 10 minutes at 72°C. For detection of the T4503C mutation, 15 µL of exon 30 PCR product was incubated with 4 U *Hga*I in a total volume of 25 µL, for 2 hours at 37°C, and the resulting fragments were separated on a 1.5% agarose gel. The presence of the T4503C mutation creates a restriction site for *Hga*I*,* and thus the 194 bp PCR product will be cut into fragments of 134 and 60 bp in the presence of the T4503C variant, but not in its absence. For detection of the A1864G mutation, 15 µL of exon 13 PCR products were digested with 8 U *Aci*I for three hours at 37°C. Products were separated on 2% agarose gels. The presence of the A1864G mutation creates a second *Aci*I site within the PCR product. Thus, the 360 bp PCR product is cleaved into fragments of 215 bp and 145 bp on the wild-type allele, but 185 bp, 145 bp and 30 bp on the mutant allele.

### Detection of mutation in FHA

Genotyping for the Δ693 variant was performed by PCR amplification of exon 14 followed by restriction enzyme digestion with *Ear*I*.* PCR was carried out in a total volume of 80 µL with 1.5 mM MgCl₂, 187.5 nM of each dNTP, 2.5 U Taq polymerase and 20 pmol of each primer (forward primer in exon 14: 5'- CTT TCT GCG GGT GAT GAG CCG GTC AAT-3' (SEQ ID NO: 8); reverse primer in intron 14: 5'-CCT TAG CCC GTG TTG AGC TA-3' (SEQ ID NO: 9)). Following an initial denaturation of 3 minutes at 95°C, 35 cycles consisting of 95°C 10 seconds, 55°C 30 seconds, 72°C 30 seconds were performed, with a final extension of 10 minutes at 72°C. Twenty microliters of PCR product was incubated with 4 U *Ear*I in a total volume of 25 µL, for two hours at 37°C, and the fragments were separated on a 2 % agarose gel. The presence of the Δ693 mutation destroys a restriction site for *Ear*I*,* and thus the 297 bp PCR product will be cut into fragments of 151 bp, 59 bp, 48 bp and 39 bp in the presence of a wild-type allele, but only fragments of 210 bp, 48 bp and 39 bp in the presence of the deletion.

A 6 bp deletion encompassing nucleotides 5752-5757 (inclusive), was detected in exon 41 in the proband of family FHA-3 by genomic sequencing using primers located within the introns flanking this exon. Genotyping of this mutation in family FHA-3 and controls was carried out by PCR with forward (5'-CCT GTA AAT GCA AAG CTA TCT CCT CT- 3' (SEQ ID NO: 10)) and reverse primers (5'-CGT CAA CTC CTT GAT TTC TAA GAT GT (SEQ ID NO: 11)) located near the 5' and 3' ends of exon 41, respectively. Each PCR was carried out as for the genotyping of the 693 variant, but with annealing temperature of 58°C. Twenty microliters of PCR product was resolved on 3% agarose or 10% acrylamide gels. The wild type allele was detected as a 117 bp band and the mutant allele as a 111 bp band upon staining with ethidium bromide.

A C to T transition was detected at nucleotide 6504 in genomic DNA of the proband of family FHA-2. It was detectable as a double C and T peak in the genomic sequence of exon 48 of this individual, who is heterozygous for the alteration. This mutation, which creates a STOP codon that results in truncation of the last 118 amino acids of the ABC1 protein, also destroys an RsaI restriction site that is present in the wild type sequence. Genotyping of this mutation in family FHA-2 and controls was carried out by PCR with forward (5'-GGG TTC CCA GGG TTC AGT AT-3') (SEQ ID NO: 12)) and reverse (5'-GAT CAG GAA TTC AAG CAC CAA-3') (SEQ ID NO: 13)) primers directed to the intronic sequences flanking exon 48. PCR was done as for the 693 variant. Fifteen microliters of PCR product was digested with 5 Units of RsaI at 37°C for two hours and the digestion products resolved on 1.5% agarose gels. The mutant allele is detected as an uncut 436 bp band. The normal sequence is cut by *Rsa*I to produce 332 and 104 bp bands.

### Cell culture

Skin fibroblast cultures were established from 3.0 mm punch biopsies of the forearm of FHD patients and healthy control subjects as described (Marcil et al., Arterioscler. Thromb. Vasc. Biol. 19:159-169, 1999).

### Cellular cholesterol labeling and loading

The protocol for cellular cholesterol efflux experiments was described in detail elsewhere (Marcil et al., Arterioscler. Thromb. Vasc. Biol. 19:159-169, 1999). The cells were ³H-cholesterol labeled during growth and free cholesterol loaded in growth arrest.

### Cholesterol efflux studies

Efflux studies were carried out from 0 to 24 hours in the presence of purified ApoAI (10 µg protein/mL medium). Efflux was determined as a percent of free cholesterol in the medium after the cells were incubated for specified periods of time. All experiments were performed in triplicate, in the presence of cells from one control subject and the cells from the study subjects to be examined. All results showing an efflux defect were confirmed at least three times.

### Oligonucleotide synthesis

Eight phosphorothioate deoxyoligonucleotides complementary to various regions of the human *ABC1* cDNA sequence were obtained from GIBCO BRL. The oligonucleotides were purified by HPLC. The sequences of the antisense oligonucleotides and their location are listed. One skilled in the art will recognize that other *ABC1* antisense sequences can also be produced and tested for their ability to decrease ABC1-mediated cholesterol regulation.

| Name | Sequence (5'- 3') | mRNA target | % control |
|---|---|---|---|
| AN-1 | GCAGAGGGCATGGCTTTATTTG (SEQ ID NO: 3) | AUG codon | 46 |
| AN-2 | GTGTTCCTGCAGAGGGCATG (SEQ ID NO: 30) | AUG codon | 50 |
| AN-3 | CACTTCCAGTAACAGCTGAC (SEQ ID NO: 31) | 5' -Untranslated | 79 |
| AN-4 | CTTTGCGCATGTCCTTCATGC (SEQ ID NO: 32) | Coding | 80 |
| AN-5 | GACATCAGCCCTCAGCATCTT (SEQ ID NO: 33) | Coding | 120 |
| AN-6: | CAACAAGCCATGTTCCCTC (SEQ ID NO: 34) | Coding | |
| AN-7: | CATGTTCCCTCAGCCAGC (SEQ ID NO: 35) | Coding | |
| AN-8: | CAGAGCTCACAGCAGGGA C (SEQ ID NO: 36) | Coding | |

### Cell transfection with antisense oligonucleotides

Cells were grown in 35 mm culture dishes until 80 % confluent, then washed once with DMEM medium (serum and antibiotics free). One milliliter of DMEM (serum and antibiotics free) containing 500 nM antisense oligonucleotides and 5 µg/ml or 7.5 µg/ml of lipofectin (GIBCO BRL) were added to each well according to the manufacturer's protocol. The cells were incubated at 37°C for 4 hours, and then the medium was replaced by DMEM containing 10% FCS. Twenty-four hours after the transfection, the total cell RNA was isolated. Ten micrograms of total RNA was resolved on a 1 % of agarose-formaldehyde gel and transferred to nylon membrane. The blot was hybridized with α-³²P dCTP labeled human *ABC1* cDNA overnight at 68°C. The membrane was subsequently exposed to x-ray film. The hybridizing bands were scanned by optical densitometry and standard to 28S ribosome RNA.

### Cholesterol efflux with anti-ABCI oligonucleotides

Human skin fibroblasts were plated in 6-well plates. The cells were labeled with ³H-cholesterol (0.2 µCi /ml) in DMEM with 10% FBS for two days when the cell reached 50% confluence. The cells were then transfected with the antisense *ABC1* oligonucleotides at 500nM in DMEM (serum and antibiotic free) with 7.5 µg/ml Lipofectin (GIBCO BRL) according to the manufacturer's protocol. Following the transfection, and the cells were loaded with nonlipoprotein (20 µg/ml) for 12 hours in DMEM containing 2 mg/ml BSA without serum. The cellular cholesterol pools were then allowed to equilibrate for 6 hours in DMEM-BSA. The cholesterol efflux mediated by ApoAl (10pg /ml, in DMEM-BSA) were then carried out which is 48 hours after transfection.

Radiolabeled cholesterol released into the medium is expressed as a percentage of total ³H-cholesterol per well (medium + cell). Results are the mean +/-SD of triplicate dishes.

### Determination of genomic structure of the ABC1 gene

Most splice junction sequences were determined from genomic sequence generated from BAC clones spanning the ABC1 gene. More than 160 kb of genomic sequence were generated. Genomic sequences were aligned with cDNA sequences to identify intron/exon boundaries. In some cases, long distance PCR between adjacent exons was used to amplify intron/exon boundary sequences using amplification primers designed according to the cDNA sequence.

### Functionality of the newly-discovered 60 amino acids at the N-terminus

### Antisense experiments

Phosphorothioate antisentisense oligonucleotides were designed to be complementary to the regions of the cDNA near newly discovered translation start site. AN-6 and AN-7 both overlap the initiator methionine codon; this site is in the middle of oligonucleotide AN-6. AN-8 is complementary to the very 5' end of the ABC1 cDNA. Antisense oligonucleotide AN-1 is complementary to the region of the ABC 1 cDNA corresponding to the site identified as the ABC1 initiator methionine in AJ012376. Fig. 7C shows that antisense oligonucleotide AN-6 interferes with cellular cholesterol efflux in normal fibroblasts to the same extent as does antisense oligonucleotide AN-1. Transfection with either of these antisense oligonucleotides results in a decrease in cellular cholesterol efflux almost as severe as that seen in FHA cells. In general, antisense oligonucleotides complementary to coding sequences, especially near the 5' end of a gene's coding sequence, are expected to be more effective in decreasing the effective amount of transcript than are oligonucleotides directed to more 3' sequences or to non-coding sequences. The observation that AN-6 depresses cellular cholesterol efflux as effectively as AN-1 implies that both of these oligonucleotides are complementary to ABC 1 coding sequences, and that the amino terminal 60 amino acids are likely to be contained in ABC 1 protein. In contrast, the ineffectiveness of AN-8 shows that it is likely to be outside the protein coding region of the transcript, as predicted by presence of an in-frame stop codon between the initiator methionine and the region targeted by AN-8.

### Antibody experiments

Polyclonal and monoclonal antibodies have been generated using peptides corresponding to discrete portions of the ABC1 amino acid sequence. One of these, 20-amino acid peptide #2 (Pep2: CSVRLSYPPYEQHECHFPNKA (SEQ ID NO: 37), in which the N-terminal cysteine was added to facilitate conjugation of the peptide) corresponds to a protein sequence within the 60 amino-terminal amino acids of the newly-discovered ABC1 protein sequence. The peptide was coupled to the KLH carrier protein and 300 µg injected at three intervals into two Balb/c mice over a four week period. The spleen was harvested from the mouse with the highest ELISA-determined immune response to free peptide, and the cells fused to NS-1 myeloma cells by standard monoclonal antibody generation methods. Positive hybridomas were selected first by ELISA and then further characterized by western blotting using cultured primary human fibroblasts. Monoclonal cell lines producing a high antibody titre and specifically recognizing the 245 kD human ABC1 protein were saved. The same size ABC1 protein product was detected by antibodies directed to four other discrete regions of the same protein. The 245 kD band could be eliminated in competition experiments with appropriate free peptide, indicating that it represents ABC1 protein (Fig. 13).

The foregoing experiments indicate that ABC1 protein is detected not only by antibodies corresponding to amino acid sequences within the previously-described ABC1 amino acid sequence, but also by the Pep2 monoclonal antibody that recognizes an epitope within the newly-discovered N-terminal 60 amino acids. The N-terminal 60 amino acid region is therefore coding, and is part of the ABC1 protein.

The epitope recognized by the Pep2 monoclonal antibody is also conserved among human, mouse, and chicken. Liver tissues from these three species employed in a Western blot produced an ABC 1 band of 245 kD when probed with the Pep2 monoclonal antibody. This indicates that the 60 amino acid N-terminal sequence is part of the ABC1 coding sequence in humans, mice, and chickens. Presence of this region is therefore evolutionarily conserved and likely to be of important functional significance for the ABC1 protein.

### Bioinformatic analyses of ABC1 protein sequences

Transmembrane prediction programs indicate 13 transmembrane (TM) regions, the first one being between amino acids 26 and 42 (http://psort.nibb.ac.jp:8800/psort/helpwww2.html#ealom). The tentative number of TM regions for the threshold 0.5 is 13. (INTEGRAL Likelihood = -7.75 Transmembrane 26-42): The other 12 TM range in value between -0.64 and -12 (full results below). It is therefore very likely that the newly-discovered 60 amino acids contain a TM domain, and that the amino end of ABC 1 may be on the opposite side of the membrane than originally thought.

### ALOM: TM region allocation

Init position for calculation: 1
Tentative number of TMs for the threshold 0.5: 13
INTEGRAL Likelihood = -7.75 Transmembrane 26 - 42
INTEGRAL Likelihood = -3.98 Transmembrane 640 - 656
INTEGRAL Likelihood = -8.70 Transmembrane 690 - 706
INTEGRAL Likelihood = -9.61 Transmembrane 717 - 733
INTEGRAL Likelihood =-1.44 Transmembrane 749 - 765
INTEGRAL Likelihood = -0.64 Transmembrane 771 - 787
INTEGRAL Likelihood = -1.28 Transmembrane 1041 -1057
INTEGRAL Likelihood =-12.79 Transmembrane 1351 -1367
INTEGRAL Likelihood = -8.60 Transmembrane 1661 -1677
INTEGRAL Likelihood = -6.79 Transmembrane 1708 - 1724
INTEGRAL Likelihood = -3.40 Transmembrane 1737 -1753
INTEGRAL Likelihood = -1.49 Transmembrane 1775-1791
INTEGRAL Likelihood =-8.39 Transmembrane 1854 -1870
PERIPHERAL Likelihood = 0.69 (at 1643)
ALOM score: -12.79 (number of TMSs: 13)

There does not appear to be an obvious cleaved peptide, so this first 60 amino acid residues are not likely to be cleaved, and are therefore not specifically a signal/targeting sequence. No other signals (e.g., for targeting to specific organelles) are apparent.

### Agonists and Antagonists

Useful therapeutic compounds include those which modulate the expression, activity, or stability of ABC1. To isolate such compounds, ABC1 expression, biological activity, or regulated catabolism is measured following the addition of candidate compounds to a culture medium of ABC1-expressing cells. Alternatively, the candidate compounds may be directly administered to animals (for example mice, pigs, or chickens) and used to screen for their effects on ABC1 expression.

In addition its role in the regulation of cholesterol, ABC1 also participates in other biological processes for which the development of ABC 1 modulators would be useful. In one example, ABC1 transports interleukin-1β (IL- 1β) across the cell membrane and out of cells. IL-1β is a precursor of the inflammatory response and, as such, inhibitors or antagonists of ABC1 expression or biological activity may be useful in the treatment of any inflammatory disorders, including but not limited to rheumatoid arthritis, systemic lupus erythematosis (SLE), hypo- or hyper- thyroidism, inflammatory bowel disease, and diabetes mellitus. In another example, ABC1 expressed in macrophages has been shown to be engaged in the engulfment and clearance of dead cells. The ability of macrophages to ingest these apoptotic bodies is impaired after antibody-mediated blockade of ABC1. Accordingly, compounds that modulate ABC1 expression, stability, or biological activity would be useful for the treatment of these disorders.

ABC1 expression is measured, for example, by standard Northern blot analysis using an *ABC1* nucleic acid sequence (or fragment thereof) as a hybridization probe, or by Western blot using an anti-ABC 1 antibody and standard techniques. The level of ABC1 expression in the presence of the candidate molecule is compared to the level measured for the same cells, in the same culture medium, or in a parallel set of test animals, but in the absence of the candidate molecule. ABC1 activity can also be measured using the cholesterol efflux assay.

### Transcriptional Regulation of ABC Expression

ABC1 mRNA is increased approximately 8-fold upon cholesterol loading. This increase is likely controlled at the transcriptional level. Using the promoter sequence described herein, one can identify transcription factors that bind to the promoter by performing, for example, gel shift assays, DNAse protection assays, or *in vitro* or *in vivo* reporter gene-based assays. The identified transcription factors are themselves drug targets. In the case of ABC1, drug compounds that act through modulation of transcription of ABC1 could be used for HDL modulation, atherosclerosis prevention, and the treatment of cardiovascular disease. For example, using a compound to inhibit a transcription factor that represses ABC1 would be expected to result in up-regulation of ABC1 and, therefore, HDL levels. In another example, a compound that increases transcription factor expression or activity would also increase ABC1 expression and HDL levels.

Transcription factors known to regulate other genes in the regulation of apolipoprotein genes or other cholesterol- or lipid-regulating genes are of particular relevance. Such factors include, but are not limited to, the steroid response element binding proteins (SREBP-1 and SREBP-2), the PPAR (peroxisomal proliferation-activated receptor) transcription factors. Several consensus sites for certain elements are present in the sequenced region 5' to the ABC1 gene (Fig. 16) and are likely to modulate ABC 1 expression. For example, PPARs may alter transcription of ABC1 by mechanisms including heterodimerization with retinoid X receptors (RXRs) and then binding to specific proliferator response elements (PPREs). Examples of such PPARs include PPARα, β,γ and δ. These distinct PPARs have been shown to have transcriptional regulatory effects on different genes. PPARα is expressed mainly in liver, whereas PPARγ is expressed in predominantly in adipocytes. Both PPARα and PPARγ are found in coronary and carotid artery atherosclerotic plaques and in endothelial cells, smooth muscle cells, monocytes and monocyte-derived macrophages. Activation of PPARα results in altered lipoprotein metabolism through PPARα's effect on genes such as lipoprotein lipase (LPL), apolipoprotein CIII (apo CIII) and apolipoprotein AI (apo AI) and AII (apo AII). PPARα activation results in overexpression of LPL and apoA-1 and apoA-II, but inhibits the expression of apo CIII. PPARα activation also inhibits inflammation, stimulates lipid oxidation and increases the hepatic uptake and esterification of free fatty acids (FFA's). PPARα and PPARγ activation may inhibit nitric oxide (NO) synthase in macrophages and prevent interleukin-1 (IL-1) induced expression of IL-6 and cyclo-oxygenase-2 (COX-2) and thrombin induced endothelin-1 expression secondary to negative transcriptional regulation of NF-KB and activation of protein-1 signaling pathway. It has also been shown that PPARα induces apoptosis in monocyte-derived macrophages through the inhibition of NF-KB activity.

Activation of PPARα can be achieved by compounds such as fibrates, β-estradiol, arachidonic acid derivatives, WY-14,643 and LTB4 or 8(s)HETE. PPARγ activation can be achieved through compounds such as thiozolidinedione antidiabetic drugs, 9-HODE and 13-HODE. Additional compounds such as nicotinic acid or HMG CoA reductase inhibitors may also alter the activity of PPARs.

Compounds which alter activity of any of the PPARs (e.g., PPARα or PPARγ) may have an effect on ABC1 expression and thereby could affect HDL levels, atherosclerosis and risk of CAD. PPARs are also regulated by fatty acids (including modified fatty acids such as 3 thia fatty acids), leukotrienes such as leukotriene B4 and prostaglandin J2, which is a natural activator/ligand for PPARγ. Drugs that modulate PPARs may therefore have an important effect on modulating lipid levels (including HDL and triglyceride levels) and altering CAD risk. This effect could be achieved through the modulation of ABC1 gene expression. Drugs may also effect ABC1 gene expression and thereby HDL levels, by an indirect effect on PPARs via other transcriptional factors such as adipocyte differentiation and determination factor-1 (ADD-1) and sterol regulatory element binding protein-1 and 2 (SREBP-1 and 2). Drugs with combined PPARα and PPARγ agonist activity or PPARα and PPARγ agonists given in combination for example, may increase HDL levels even more.

A PPAR binding site (PPRE element) is found 5' to the ABC1 gene (nucleotides 2150 to 2169 of SEQ ID NO: 14). Like the PPRE elements found in the C-ACS, HD, CYP4A6 and ApoA-I genes, this PPRE site is a trimer related to the PPRE consensus sequence. Partly because of its similarity in the number and arrangement of repeats in this PPAR binding site, this element in particular is very likely to be of physiological relevance to the regulation of the ABC1 gene.

Additional transcription factors which may also have an effect in modulating ABC1 gene expression and thereby HDL levels, atherosclerosis and CAD risk include; REV-ERBα, SREBP-1 & 2, ADD-1, EBPα, CREB binding protein, P300, HNF 4, RAR, LXR, and RORα. Additional degenerate binding sites for these factors can be found through examination of the sequence in SEQ ID NO: 14.

### Additional utility of ABC 1 polypeptides, nucleic acids, and modulators

ABC1 may act as a transporter of toxic proteins or protein fragments (e.g., APP) out of cells. Thus, ABC 1 agonists/upregulators may be useful in the treatment of other disease areas, including Alzheimer's disease, Niemann-Pick disease, and Huntington's disease.

ABC transporters have been shown to increase the uptake of long chain fatty acids from the cytosol to peroxisomes and, moreover, to play a role in β-oxidation of very long chain fatty acids. Importantly, in x-linked adrenoleukodystrophy (ALD), fatty acid metabolism is abnormal, due to defects in the peroxisomal ABC transporter. Any agent that upregulates ABC transporter expression or biological activity may therefor be useful for the treatment of ALD or any other lipid disorder.

ABC 1 is expressed in macrophages and is required for engulfment of cells undergoing programmed cell death. The apoptotic process itself, and its regulation, have important implications for disorders such as cancer, one mechanism of which is failure of cells to undergo cell death appropriately. ABC1 may facilitate apoptosis, and as such may represent an intervention point for cancer treatment. Increasing ABC1 expression or activity or otherwise up-regulating ABC1 by any method may constitute a treatment for cancer by increasing apoptosis and thus potentially decreasing the aberrant cellular proliferation characterized by this disease. Conversely, down-regulation of ABC1 by any method may provide opportunity for decreasing apoptosis and allowing increased proliferation of cells in conditions where cell growth is limited. Such disorders include but are not limited to neurodeficiencies and neurodegeneration, and growth disorders. ABC1 could, therefore, potentially be used as a method for identification of compounds for use in the treatment of cancer, or in the treatment of degenerative disorders.

Agents that have been shown to inhibit ABC1 include, for example, the antidiabetic agents glibenclamide and glyburide, flufenamic acid, diphenylamine-2-carbonic acid, sulfobromophthalein, and DIDS.

Agents that upregulate ABC 1 expression or biological activity include but are not limited to protein kinase A, protein kinase C, vanadate, okadaic acid, and IBMX1.

Those in the art will recognize that other compounds can also modulate ABC1 biological activity, and these compounds are also in the spirit of the invention.

### Drug screens based on the ABC I gene or protein

The ABC 1 protein and gene can be used in screening assays for identification of compounds which modulate its activity and may be potential drugs to regulate cholesterol levels. Useful ABC 1 proteins include wild-type and mutant ABC 1 proteins or protein fragments, in a recombinant form or endogenously expressed. Drug screens to identify compounds acting on the ABC1 expression product may employ any functional feature of the protein. In one example, the phosphorylation state or other post-translational modification is monitored as a measure of ABC1 biological activity. ABC1 has ATP binding sites, and thus assays may wholly or in part test the ability of ABC1 to bind ATP or to exhibit ATPase activity. ABC1, by analogy to similar proteins, is thought to be able to form a channel-like structure; drug screening assays could be based upon assaying for the ability of the protein to form a channel, or upon the ability to transport cholesterol or another molecule, or based upon the ability of other proteins bound by or regulated by ABC1 to form a channel. Alternatively, phospholipid or lipid transport can also be used as measures of ABC1 biological activity.

There is evidence that, in addition to its role as a regulator of cholesterol levels, ABC 1 also transports anions. Functional assays could be based upon this property, and could employ drug screening technology such as (but not limited to) the ability of various dyes to change color in response to changes in specific ion concentrations in such assays can be performed in vesicles such as liposomes, or adapted to use whole cells.

Drug screening assays can also be based upon the ability of ABC1 or other ABC transporters to interact with other proteins. Such interacting proteins can be identified by a variety of methods known in the art, including, for example, radioimmunoprecipitation, co-immunoprecipitation, co-purification, and yeast two-hybrid screening. Such interactions can be further assayed by means including but not limited to fluorescence polarization or scintillation proximity methods. Drug screens can also be based upon functions of the ABC1 protein deduced upon X-ray crystallography of the protein and comparison of its 3-D structure to that of proteins with known functions. Such a crystal structure has been determined for the prokaryotic ABC family member HisP, histidine permease. Drug screens can be based upon a function or feature apparent upon creation of a transgenic or knockout mouse, or upon overexpression of the protein or protein fragment in mammalian cells *in vitro.* Moreover, expression of mammalian (e.g., human) ABC1 in yeast or C. *elegans* allows for screening of candidate compounds in wild-type and mutant backgrounds, as well as screens for mutations that enhance or suppress an ABC1-dependent phenotype. Modifier screens can also be performed in ABC 1 transgenic or knock-out mice.

Additionally, drug screening assays can also be based upon ABC1 functions deduced upon antisense interference with the gene function. Intracellular localization of ABC 1, or effects which occur upon a change in intracellular localization of the protein, can also be used as an assay for drug screening. Immunocytochemical methods will be used to determine the exact location of the ABC1 protein.

Human and rodent ABC1 protein can be used as an antigen to raise antibodies, including monoclonal antibodies. Such antibodies will be useful for a wide variety of purposes, including but not limited to functional studies and the development of drug screening assays and diagnostics. Monitoring the influence of agents (e.g., drugs, compounds) on the expression or biological activity of ABC 1 can be applied not only in basic drug screening, but also in clinical trials. For example, the effectiveness of an agent determined by a screening assay as described herein to increase ABC1 gene expression, protein levels, or biological activity can be monitored in clinical trails of subjects exhibiting altered ABC1 gene expression, protein levels, or biological activity. Alternatively, the effectiveness of an agent determined by a screening assay to modulate ABC 1 gene expression, protein levels, or biological activity can be monitored in clinical trails of subjects exhibiting decreased altered gene expression, protein levels, or biological activity. In such clinical trials, the expression or activity of ABC 1 and, preferably, other genes that have been implicated in, for example, cardiovascular disease can be used to ascertain the effectiveness of a particular drug.

For example, and not by way of limitation, genes, including ABC1, that are modulated in cells by treatment with an agent (e.g., compound, drug or small molecule) that modulates ABC1 biological activity (e.g., identified in a screening assay as described herein) can be identified. Thus, to study the effect of agents on cholesterol levels or cardiovascular disease, for example, in a clinical trial, cells can be isolated and RNA prepared and analyzed for the levels of expression of ABC1 and other genes implicated in the disorder. The levels of gene expression can be quantified by Northern blot analysis or RT-PCR, or, alternatively, by measuring the amount of protein produced, by one of a number of methods known in the art, or by measuring the levels of biological activity of ABC 1 or other genes. In this way, the gene expression can serve as a marker, indicative of the physiological response of the cells to the agent. Accordingly, this response state may be determined before, and at various points during, treatment of the individual with the agent.

In a preferred embodiment, the present invention provides a method for monitoring the effectiveness of treatment of a subject with an agent (e.g., an agonist, antagonist, peptidomimetic, protein, peptide, nucleic acid, small molecule, or other drug candidate identified by the screening assays described herein) including the steps of (i) obtaining a pre-administration sample from a subject prior to administration of the agent; (ii) detecting the level of expression of an ABC1 protein, mRNA, or genomic DNA in the preadministration sample; (iii) obtaining one or more post-administration samples from the subject; (iv) detecting the level of expression or activity of the ABC1 protein, mRNA, or genomic DNA in the post-administration samples; (v) comparing the level of expression or activity of the ABC1 protein, mRNA, or genomic DNA in the pre-administration sample with the ABC1 protein, mRNA, or genomic DNA in the post administration sample or samples; and (vi) altering the administration of the agent to the subject accordingly. For example, increased administration of the agent may be desirable to increase the expression or activity of ABC1 to higher levels than detected, i.e., to increase the effectiveness of the agent. Alternatively, decreased administration of the agent may be desirable to decrease expression or activity of ABC1 to lower levels than detected.

The *ABC1* gene or a fragment thereof can be used as a tool to express the protein in an appropriate cell *in vitro* or *in vivo* (gene therapy), or can be cloned into expression vectors which can be used to produce large enough amounts of ABC1 protein to use in *in vitro* assays for drug screening. Expression systems which may be employed include baculovirus, herpes virus, adenovirus, adeno-associated virus, bacterial systems, and eucaryotic systems such as CHO cells. Naked DNA and DNA-liposome complexes can also be used.

Assays of ABC 1 activity includes binding to intracellular interacting proteins; interaction with a protein that up-regulates ABC 1 activity; interaction with HDL particles or constituents; interaction with other proteins which facilitate interaction with HDL or its constituents; and measurement of cholesterol efflux. Furthermore, assays may be based upon the molecular dynamics of macromolecules, metabolites and ions by means of fluorescent-protein biosensors. Alternatively, the effect of candidate modulators on expression or activity may be measured at the level of ABC I protein production using the same general approach in combination with standard immunological detection techniques, such as Western blotting or immunoprecipitation with an ABC1-specific antibody. Again, useful cholesterol-regulating or anti-CVD therapeutic modulators are identified as those which produce an change in ABC I polypeptide production. Agonists may also affect ABC1 activity without any effect on expression level.

Candidate modulators may be purified (or substantially purified) molecules or may be one component of a mixture of compounds (e.g., an extract or supernatant obtained from cells). In a mixed compound assay, ABC1 expression is tested against progressively smaller subsets of the candidate compound pool (e.g., produced by standard purification techniques, e.g., HPLC or FPLC; Ausubel et al.) until a single compound or minimal compound mixture is demonstrated to modulate ABC1 expression.

Agonists, antagonists, or mimetics found to be effective at modulating the level of cellular ABC1 expression or activity may be confirmed as useful in animal models (for example, mice, pigs, rabbits, or chickens). For example, the compound may ameliorate the low HDL levels of mouse or chicken hypoalphalipoproteinemias.

A compound that promotes an increase in ABC1 expression or activity is considered particularly useful in the invention; such a molecule may be used, for example, as a therapeutic to increase the level or activity of native, cellular ABC1 and thereby treat a low HDL condition in an animal (for example, a human).

One method for increasing ABC biological activity is to increase the stabilization of the ABC protein or to prevent its degradation. Thus, it would be useful to identify mutations in an ABC polypeptide (e.g., ABC1) that lead to increased protein stability. These mutations can be incorporated into any protein therapy or gene therapy undertaken for the treatment of low HDL-C or any other condition resulting from loss of ABC1 biological activity. Similarly, compounds that increase the stability of a wild-type ABC polypeptide or decrease its catabolism may also be useful for the treatment of low HDL-C or any other condition resulting from loss of ABC1 biological activity. Such mutations and compounds can be identified using the methods described herein.

In one example, cells expressing an ABC polypeptide having a mutation are transiently metabolically labeled during translation and the half-life of the ABC polypeptide is determined using standard techniques. Mutations that increase the half-life of an ABC polypeptide are ones that increase ABC protein stability. These mutations can then be assessed for ABC biological activity. They can also be used to identify proteins that affect the stability of ABC1 mRNA or protein. One can then assay for compounds that act on these factors or on the ability of these factors to bind ABC1.

In another example, cells expressing wild-type ABC polypeptide are transiently metabolically labeled during translation, contacted with a candidate compounds, and the half-life of the ABC polypeptide is determined using standard techniques. Compounds that increase the half-life of an ABC polypeptide are useful compounds in the present invention.

If desired, treatment with an agonist of the invention may be combined with any other HDL-raising or anti-CVD therapies.

It is understood that, while ABC1 is the preferred ABC transporter for the drug screens described herein, other ABC transporters can also be used. The replacement of ABC1 with another ABC transporter is possible because it is likely that ABC transporter family members, such as ABC2, ABCR, or ABC8 will have a similar mechanism of regulation.

Exemplary assays are described in greater detail below.

### Protein-based assays

ABC 1 polypeptide (purified or unpurified) can be used in an assay to determine its ability to bind another protein (including, but not limited to, proteins found to specifically interact with ABC1). The effect of a compound on that binding is then determined.

### Protein Interaction Assays

ABC1 protein (or a polypeptide fragment thereof or an epitope-tagged form or fragment thereof) is harvested from a suitable source (e.g., from a prokaryotic expression system, eukaryotic cells, a cell-free system, or by immunoprecipitation from ABC1-expressing cells). The ABC1 polypeptide is then bound to a suitable support (e.g., nitrocellulose or an antibody or a metal agarose column in the case of, for example, a his-tagged form of ABC1). Binding to the support is preferably done under conditions that allow proteins associated with ABC1 polypeptide to remain associated with it. Such conditions may include use of buffers that minimize interference with protein-protein interactions. The binding step can be done in the presence and absence of compounds being tested for their ability to interfere with interactions between ABC1 and other molecules. If desired, other proteins (e.g., a cell lysate) are added, and allowed time to associate with the ABC polypeptide. The immobilized ABC1 polypeptide is then washed to remove proteins or other cell constituents that may be non-specifically associated with it the polypeptide or the support. The immobilized ABC1 polypeptide is then dissociated from its support, and so that proteins bound to it are released (for example, by heating), or, alternatively, associated proteins are released from ABC1 without releasing the ABC1 polypeptide from the support. The released proteins and other cell constituents can be analyzed, for example, by SDS-PAGE gel electrophoresis, Western blotting and detection with specific antibodies, phosphoamino acid analysis, protease digestion, protein sequencing, or isoelectric focusing. Normal and mutant forms of ABC can be employed in these assays to gain additional information about which part of ABC 1 a given factor is binding to. In addition, when incompletely purified polypeptide is employed, comparison of the normal and muatant forms of the protein can be used to help distinguish true binding proteins.

The foregoing assay can be performed using a purified or semipurified protein or other molecule that is known to interact with ABC1. This assay may include the following steps.
1. Harvest ABC1 protein and couple a suitable fluorescent label to it;
2. Label an interacting protein (or other molecule) with a second, different fluorescent label. Use dyes that will produce different quenching patterns when they are in close proximity to each other vs. when they are physically separate (i.e., dyes that quench each other when they are close together but fluoresce when they are not in close proximity);
3. Expose the interacting molecule to the immobilized ABC1 in the presence or absence of a compound being tested for its ability to interfere with an interaction between the two; and
4. Collect fluorescent readout data.

Another assay is includes Fluorescent Resonance Energy Transfer (FRET) assay. This assay can be performed as follows.
1. Provide ABC 1 protein or a suitable polypeptide fragment thereof and couple a suitable FRET donor (e.g.,. nitro-benzoxadiazole (NBD)) to it;
2. Label an interacting protein (or other molecule) with a FRET acceptor (e.g., rhodamine);
3. Expose the acceptor-labeled interacting molecule to the donor-labeled ABC1 in the presence or absence of a compound being tested for its ability to interfere with an interaction between the two; and
4. Measure fluorescence resonance energy transfer.

Quenching and FRET assays are related. Either one can be applied in a given case, depending on which pair of fluorophores is used in the assay.

### Membrane permeability assay

The ABC1 protein can also be tested for its effects on membrane permeability. For example, beyond its putative ability to translocate lipids, ABC1 might affect the permeability of membranes to ions. Other related membrane proteins, most notably the cystic fibrosis transmembrane conductance regulator and the sulfonylurea receptor, are associated with and regulate ion channels.

ABC1 or a fragment of ABC1 is incorporated into a synthetic vesicle, or, alternatively, is expressed in a cell and vesicles or other cell sub-structures containing ABC are isolated. The ABC1-containing vesicles or cells are loaded with a reporter molecule (such as a fluorescent ion indicator whose fluorescent properties change when it binds a particular ion) that can detect ions (to observe outward movement), or alternatively, the external medium is loaded with such a molecule (to observe inward movement). A molecule which exhibits differential properties when it is inside the vesicle compared to when it is outside the vesicle is preferred. For example, a molecule that has quenching properties when it is at high concentration but not when it is at another low concentration would be suitable. The movement of the charged molecule (either its ability to move or the kinetics of its movement) in the presence or absence of a compound being tested for its ability to affect this process can be determined.

In another assay, membrane permeability is determined electro-physiologically by measuring ionic influx or efflux mediated by or modulated by ABC1 by standard electrophysiological techniques. A suitable control (e.g., TD cells or a cell line with very low endogenous ABC1 expression) can be used as a control in the assay to determine if the effect observed is specific to cells expressing ABC1.

In still another assay, uptake of radioactive isotopes into or out of a vesicle can be measured. The vesicles are separated from the extravesicular medium and the radioactivity in the vesicles and in the medium is quantitated and compared.

### Nucleic acid-based assays

ABC1 nucleic acid may be used in an assay based on the binding of factors necessary for ABC 1 gene transcription. The association between the *ABC1* DNA and the binding factor may be assessed by means of any system that discriminates between protein-bound and non-protein-bound DNA (e.g., a gel retardation assay). The effect of a compound on the binding of a factor to *ABC1* DNA is assessed by means of such an assay. In addition to *in vitro* binding assays, *in vivo* assays in which the regulatory regions of the *ABC1* gene are linked to reporter genes can also be performed.

### \Assays measuring ARC1 stability

A cell-based or cell-free system can be used to screen for compounds based on their effect on the half-life of *ABC1* mRNA or ABC1 protein. The assay may employ labeled mRNA or protein. Alternatively, *ABC1* mRNA may be detected by means of specifically hybridizing probes or a quantitative PCR assay. Protein can be quantitated, for example, by fluorescent antibody-based methods.

### In vitro mRNA stability assay

1. Isolate or produce, by *in vitro* transcription, a suitable quantity of ABC1 mRNA;
2. Label the ABC1 mRNA;
3. Expose aliquots of the mRNA to a cell lysate in the presence or absence of a compound being tested for its ability to modulate *ABC1* mRNA stability;
4. Assess intactness of the remaining mRNA at suitable time points.

### In vitro protein stability assay

1. Express a suitable amount of ABC1 protein;
2. Label the protein;
3. Expose aliquots of the labeled protein to a cell lysate in the presence or absence of a compound being tested for its ability to modulate ABC1 protein stability;
4. Assess intactness of the remaining protein at suitable time points

### In vivo mRNA or protein stability assay

1. Incubate cells expressing ABC1 mRNA or protein with a tracer (radiolabeled ribonucleotide or radiolabeled amino acid, respectively) for a very brief time period (e.g., five minutes) in the presence or absence of a compound being tested for its effect on mRNA or protein stability;
2. Incubate with unlabeled ribonucleotide or amino acid; and
3. Quantitate the ABC 1 mRNA or protein radioactivity at time intervals beginning with the start of step 2 and extending to the time when the radioactivity in ABC 1 mRNA or protein has declined by approximately 80%. It is preferable to separate the intact or mostly intact mRNA or protein from its radioactive breakdown products by a means such as gel electrophoresis in order to quantitate the mRNA or protein.

### Assays measuring inhibition of dominant negative activity

Mutant ABC1 polypeptides are likely to have dominant negative activity (i.e., activity that interferes with wild-type ABC1 function). An assay for a compound that can interfere with such a mutant may be based on any method of quantitating normal ABC1 activity in the presence of the mutant. For example, normal ABC1 facilitates cholesterol efflux, and a dominant negative mutant would interfere with this effect. The ability of a compound to counteract the effect of a dominant negative mutant may be based on cellular cholesterol efflux, or on any other normal activity of the wild-type ABC 1 that was inhibitable by the mutant.

### Assays measuring phosphorylation

The effect of a compound on ABC 1 phosphorylation can be assayed by methods that quantitate phosphates on proteins or that assess the phosphorylation state of a specific residue of a ABC 1. Such methods include but are not limited to ³²p labelling and immunoprecipitation, detection with antiphosphoamino acid antibodies (e.g., antiphosphoserine antibodies), phosphoamino acid analysis on 2-dimensional TLC plates, and protease digestion fingerprinting of proteins followed by detection of ³²P-labeled fragments.

### Assays measuring other post-translational modifications

The effect of a compound on the post-translational modification of ABC1 is based on any method capable of quantitating that particular modification. For example, effects of compounds on glycosylation may be assayed by treating ABC 1 with glycosylase and quantitating the amount and nature of carbohydrate released.

### Assays measuring ATP binding

The ability of ABC1 to bind ATP provides another assay to screen for compounds that affect ABC1. ATP binding can be quantitated as follows.
1. Provide ABC1 protein at an appropriate level of purity and reconsititute it in a lipid vesicle;
2. Expose the vesicle to a labeled but non-hydrolyzable ATP analog (such as gamma ³⁵S-ATP) in the presence or absence of compounds being tested for their effect on ATP binding. Note that azido-ATP analogs can be used to allow covalent attachment of the azido-ATP to protein (by means of U.V. light), and permit easier quantitation of the amount of ATP bound to the protein.
3. Quantitate the amount of ATP analog associated with ABC1

### Assays measuring ATPase activity

Quantitation of the ATPase activity of ABC1 can also be assayed for the effect of compounds on ABC 1. This is preferably performed in a cell-free assay so as to separate ABC 1 from the many other ATPases in the cell. An ATPase assay may be performed in the presence or absence of membranes, and with or without integration of ABC1 protein into a membrane. If performed in a vesicle-based assay, the ATP hydrolysis products produced or the ATP hydrolyzed may be measured within or outside of the vesicles, or both. Such an assay may be based on disappearance of ATP or appearance of ATP hydrolysis products.

For high-throughput screening, a coupled ATPase assay is preferable. For example, a reaction mixture containing pyruvate kinase and lactate dehydrogenase can be used. The mixture includes phosphoenolpyruvate (PEP), nicotinamide adenine dinucleotide (NAD+), and ATP. The ATPase activity of ABC1 generates ADP from ATP. The ADP is then converted back to ATP as part of the pyruvate kinase reaction. The product, pyruvate, is then converted to lactate. The latter reaction generates a colored quinone (NADH) from a colorless substrate (NAD+), and the entire reaction can be monitored by detection of the color change upon formation of NADH. Since ADP is limiting for the pyruvate kinase reaction, this coupled system precisely monitors the ATPase activity of ABC1.

### Assays measuring cholesterol efflux

A transport-based assay can be performed *in vivo* or *in vitro.* For example, the assay may be based on any part of the reverse cholesterol transport process that is readily re-created in culture, such as cholesterol or phospholipid efflux. Alternatively, the assay may be based on net cholesterol transport in a whole organism, as assessed by means of a labeled substance (such as cholesterol).

For high throughput, fluorescent lipids can be used to measure ABC1-catalyzed lipid efflux. For phospholipids, a fluorescent precursor, C6-NBD-phosphatidic acid, can be used. This lipid is taken up by cells and dephosphorylated by phosphatidic acid phosphohydrolase. The product, NBD-diglyceride, is then a precursor for synthesis of glycerophospholipids like phosphatidylcholine. The efflux of NBD-phosphatidylcholine can be monitored by detecting fluorescence resonance energy transfer (FRET) of the NBD to a suitable acceptor in the cell culture medium. This acceptor can be rhodamine-labeled phosphatidylethanolamine, a phospholipid that is not readily taken up by cells. The use of short-chain precursors obviates the requirement for the phospholipid transfer protein in the media. For cholesterol, NBD-cholesterol ester can be reconstituted into LDL. The LDL can efficiently deliver this lipid to cells via the LDL receptor pathway. The NBD-cholesterol esters are hydrolyzed in the lysosomes, resulting in NBD-cholesterol that can now be transported back to the plasma membrane and efflux from the cell. The efflux can be monitored by the aforementioned FRET assay in which NBD transfers its fluorescence resonance energy to the rhodamine-phosphatidylethanoline acceptor.

### Animal Model Systems

Compounds identified as having activity in any of the above-described assays are subsequently screened in any available animal model system, including, but not limited to, pigs, rabbits, and WHAM chickens. Test compounds are administered to these animals according to standard methods. Test compounds may also be tested in mice bearing mutations in the *ABC1* gene. Additionally, compounds may be screened for their ability to enhance an interaction between ABC1 and any HDL particle constituent such as ApoAI, ApoAII, or ApoE.

### The cholesterol efflux assay as a drug screen

The cholesterol efflux assay measures the ability of cells to transfer cholesterol to an extracellular acceptor molecule and is dependent on ABC1 function. In this procedure, cells are loaded with radiolabeled cholesterol by any of several biochemical pathways (Marcil et al., Arterioscler. Thromb. Vasc. Biol. 19:159-169, 1999). Cholesterol efflux is then measured after incubation for various times (typically 0 to 24 hours) in the presence of HDL3 or purified ApoAI. Cholesterol efflux is determined as the percentage of total cholesterol in the culture medium after various times of incubation. ABC 1 expression levels and/or biological activity are associated with increased efflux while decreased levels of ABC1 are associated with decreased cholesterol efflux.

This assay can be readily adapted to the format used for drug screening, which may consist of a multi-well (e.g., 96-well) format. Modification of the assay to optimize it for drug screening would include scaling down and streamlining the procedure, modifying the labeling method, using a different cholesterol acceptor, altering the incubation time, and changing the method of calculating cholesterol efflux. In all these cases, the cholesterol efflux assay remains conceptually the same, though experimental modifications may be made. A transgenic mouse overexpressing ABC1 would be expected to have higher than normal HDL levels.

### Knock-out mouse model

An animal, such as a mouse, that has had one or both ABC 1 alleles inactivated (e.g., by homologous recombination) is likely to have low HDL-C levels, and thus is a preferred animal model for screening for compounds that raise HDL-C levels. Such an animal can be produced using standard techniques. In addition to the initial screening of test compounds, the animals having mutant ABC1 genes are useful for further testing of efficacy and safety of drugs or agents first identified using one of the other screening methods described herein. Cells taken from the animal and placed in culture can also be exposed to test compounds. HDL-C levels can be measured using standard techniques, such as those described herein.

### WHAM chickens: an animal model for low HDL cholesterol

Wisconsin Hypo-Alpha Mutant (WHAM) chickens arose by spontaneous mutation in a closed flock. Mutant chickens came to attention through their a Z-linked white shank and white beak phenotype referred to as 'recessive white skin' (McGibbon, 1981) and were subsequently found to have a profound deficiency of HDL (Poernama *et al.,* 1990).

This chicken low HDL locus (Y) is Z-linked, or sex-linked. (In birds, females are ZW and males are ZZ). Genetic mapping placed the Y locus on the long arm of the Z chromosome (Bitgood, 1985), proximal to the ID locus (Bitgood, 1988). Examination of current public mapping data for the chicken genome mapping project, ChickMap (maintained by the Roslin Institute; http://www.ri.bbsrc.ac.ukl chickmap/ChickMapHomePage.html) showed that a region of synteny with human chromosome 9 lies on the long arm of the chicken Z chromosome (Zq) proximal to the ID locus. Evidence for this region of synteny is the location of the chicken aldolase B locus (ALDOB) within this region. The human ALDOB locus maps to chromosome 9q22.3 (The Genome Database, http://gdbwww.gdb.org/), not far from the location of human ABC1. This comparison of maps showed that the chicken Zq region near chicken ALDOB and the human 9q region near human ALDOB represent a region of synteny between human and chicken.

Since a low HDL locus maps to the 9q location in humans and to the Zq region in chickens, these low HDL loci are most probably located within the syntenic region. Thus we predicted that ABC1 is mutated in WHAM chickens. In support of this, we have identified an E 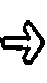 K mutation at a position that corresponds to amino acid 89 of human ABC 1 (Figs. 14 and 15). This non-conservative substitution is at a position that is conserved among human, mouse, and chicken, indicating that it is in a region of the protein likely to be of functional importance.

Discovery of the WHAM mutation in the amino-terminal portion of the ABC 1 protein also establishes the importance of the amino-terminal region. This region may be critical because of association with other proteins required to carry out cholesterol efflux or related tasks. It may be an important regulatory region (there is a phosphorylation site for casein kinase near the mutated residue), or it may help to dictate a precise topological relationship with cellular membranes (the N-terminal 60 amino acid region contains a putative membrane-spanning or membrane-associated segment).

The amino-terminal region of the protein (up to the first 6-TM region at approximately amino acid 639) is an ideal tool for screening factors that affect ABC1 activity. It can be expressed as a truncated protein in ABC1 wild type cells in order to test for interference of the normal ABC1 function by the truncated protein. If the fragment acts in a dominant negative way, it could be used in immunoprecipitations to identify proteins that it may be competing away from the normal endogenous protein.

The C-terminus also lends itself to such experiments, as do the intracellular portions of the molecule, expressed as fragments or tagged or fusion proteins, in the absence of transmembrane regions.

Since it is possible that there are several genes in the human genome which affect cholesterol efflux, it is important to establish that any animal model to be used for a human genetic disease represents the homologous locus in that animal, and not a different locus with a similar function. The evidence above establishes that the chicken Y locus and the human chromosome 9 low HDL locus are homologous. WHAM chickens are therefore an important animal model for the identification of drugs that modulate cholesterol efflux.

The WHAM chickens' HDL deficiency syndrome is not, however, associated with an increased susceptibility to atherosclerosis in chickens. This probably reflects the shorter lifespan of the chicken rather than an inherent difference in the function of the chicken ABC1 gene compared to the human gene. We propose the WHAM chicken as a model for human low HDL for the development and testing of drugs to raise HDL in humans. Such a model could be employed in several forms, through the use of cells or other derivatives of these chickens, or by the use of the chickens themselves in tests of drug effectiveness, toxicity, and other drug development purposes.

### Therapy

Compounds of the invention, including but not limited to, ABC1 polypeptides, *ABC1* nucleic acids, other ABC transporters, and any therapeutic agent that modulates biological activity or expression of ABC1 identified using any of the methods disclosed herein, may be administered with a pharmaceutically-acceptable diluent, carrier, or excipient, in unit dosage form. Conventional pharmaceutical practice may be employed to provide suitable formulations or compositions to administer such compositions to patients. Although intravenous administration is preferred, any appropriate route of administration may be employed, for example, perenteral, subcutaneous, intramuscular, intracranial, intraorbital, ophthalmic, intraventricular, intracapsular, intraspinal, intracisternal, intraperitoneal, intranasal, aerosol, or oral administration. Therapeutic formulations may be in the form of liquid solutions or suspension; for oral administration, formulations may be in the form of tablets or capsules; and for intranasal formulations, in the form of powders, nasal drops, or aerosols.

Methods well known in the art for making formulations are found in, for example, Remington: The Science and Practice of Pharmacy (19th ed.) ed. A.R. Gennaro AR., 1995, Mack Publishing Company, Easton, PA. Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems for agonists of the invention include ethylenevinyl acetate copolymer particles, osmotic pumps, implantable infusion systems, and liposomes. Formulations for inhalation may contain excipients, or example, lactose, or may be aqueous solutions containing, for example, polyoxyethylene-9-lauryl ether, glycocholate and deoxycholate, or may be oily solutions for administration in the form of nasal drops, or as a gel.

### Compounds

In general, novel drugs for the treatment of aberrant cholesterol levels and/or CVD are identified from large libraries of both natural product or synthetic (or semisynthetic) extracts or chemical libraries according to methods known in the art. Those skilled in the field or drug discovery and development will understand that the precise source of test extracts or compounds is not critical to the screening procedure(s) of the invention. Accordingly, virtually any number of chemical extracts or compounds can be screened using the exemplary methods described herein. Examples of such extracts or compounds include, but are not limited to, plant-, fungal-, prokaryotic- or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-, lipid-, peptide-, and nucleic acid-based compounds. Synthetic compound libraries are commercially available from Brandon Associates (Merrimack, NH) and Aldrich Chemical (Milwaukee, WI). Alternatively, libraries of natural compounds in the form of bacterial, fungal, plant, and animal extracts are commercially available from a number of sources, including Biotics (Sussex, UK), Xenova (Slough, UK), Harbor Branch Oceangraphics Institute (Ft. Pierce, FL), and PharmaMar, U.S.A. (Cambridge, MA). In addition, natural and synthetically produced libraries are produced, if desired, according to methods known in the art, e.g., by standard extraction and fractionation methods. Furthermore, if desired, any library or compound is readily modified using standard chemical, physical, or biochemical methods.

In addition, those skilled in the art of drug discovery and development readily understand that methods for dereplication (e.g., taxonomic dereplication, biological dereplication, and chemical dereplication, or any combination thereof) or the elimination of replicates or repeats of materials already known for their HDL-raising and anti-CVD activities should be employed whenever possible.

When a crude extract is found to have cholesterol-modulating or anti-CVD activities or both, further fractionation of the positive lead extract is necessary to isolate chemical constituent responsible for the observed effect. Thus, the goal of the extraction, fractionation, and purification process is the careful characterization and identification of a chemical entity within the crude extract having cholesterol-modulating or anti-CVD activities. The same *in vivo* and *in vitro* assays described herein for the detection of activities in mixtures of compounds can be used to purify the active component and to test derivatives thereof. Methods of fractionation and purification of such heterogeneous extracts are known in the art. If desired, compounds shown to be useful agents for the treatment of pathogenicity are chemically modified according to methods known in the art. Compounds identified as being of therapeutic value are subsequently analyzed using any standard animal model of diabetes or obesity known in the art.

It is understood that compounds that modulate activity of proteins that modulate or are modulated by ABC1 are useful compounds for modulating cholesterol levels. Exemplary compounds are provided herein; others are known in the art.

Compounds that are structurally related to cholesterol, or that mimic ApoAI or a related apolipoprotein, and increase ABC1 biological activity are particularly useful compounds in the invention. Other compounds, known to act on the MDR protein, can also be used or derivatized and assayed for their ability to increase ABC1 biological activity. Exemplary MDR modulators are PSC833, bromocriptine, and cyclosporin A.

### Screening patients having low HDL-C

ABC1 expression, biological activity, and mutational analysis can each serve as a diagnostic tool for low HDL; thus determination of the genetic subtyping of the *ABC1* gene sequence can be used to subtype low HDL individuals or families to determine whether the low HDL phenotype is related to ABC 1 function. This diagnostic process can lead to the tailoring of drug treatments according to patient genotype, including prediction of side effects upon administration of HDL increasing drugs (referred to herein as pharmacogenomics). Pharmacogenomics allows for the selection of agents (e.g., drugs) for therapeutic or prophylactic treatment of an individual based on the genotype of the individual (e.g., the genotype of the individual is examined to determine the ability of the individual to respond to a particular agent).

Agents, or modulators which have a stimulatory or inhibitory effect on ABC1 biological activity or gene expression can be administered to individuals to treat disorders (e.g., cardiovascular disease or low HDL cholesterol) associated with aberrant ABC1 activity. In conjunction with such treatment, the pharmacogenomics (i.e., the study of the relationship between an individual's genotype and that individual's response to a foreign compound or drug) of the individual may be considered. Differences in efficacy of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of ABC1 protein, expression of ABC1 nucleic acid, or mutation content of ABC1 genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons (Eichelbaum, M., Clin. Exp. Pharmacol. Physiol., 23:983-985, 1996; Linder, M. W., Clin. Chem., 43:254-266, 1997). In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). Altered drug action may occur in a patient having a polymorphism (e.g., an single nucleotide polymorphism or SNP) in promoter, intronic, or exonic sequences of ABC1. Thus by determining the presence and prevalence of polymorphisms allow for prediction of a patient's response to a particular therapeutic agent. In particular, polymorphisms in the promoter region may be critical in determining the risk of HDL deficiency and CVD.

In addition to the mutations in the *ABC1* gene described herein, we have detected polymorphisms in the human *ABC1* gene (Fig. 11). These polymorphisms are located in promoter, intronic, and exonic sequence of ABC1. Using standard methods, such as direct sequencing, PCR, SSCP, or any other polymorphism-detection system, one could easily ascertain whether these polymorphisms are present in a patient prior to the establishment of a drug treatment regimen for a patient having low HDL, cardiovascular disease, or any other ABC1-mediated condition. It is possible that some these polymorphisms are, in fact, weak mutations. Individuals harboring such mutations may have an increased risk for cardiovascular disease; thus, these polymorphisms may also be useful in diagnostic assays.

### Association Studies of ABC1 Gene Variants and HDL Levels or Cardiovascular Disease

The following polymorphisms have been examined for their effect on cholesterol regulation and the predisposition for the development of cardiovascular disease.

*Substitution of G for A at nucleotide -1045 [G(-1045)A].* This variant is in complete linkage disequilibrium with the variant at -738 in the individuals we have sequenced, and thus any potential phenotypic effects currently attributed to the variant at -738 may at least in part be due to changes at this site.

*Substitution of G for A at nucleotide* -738 *[G(-738)A].* This variant has been found at very high frequencies in populations selected for low HDL cholesterol or premature coronary artery disease.

*Insertion of a G nucleotide at position -4 [G ins (-4)].* This variant has been associated with less coronary artery disease in its carriers than in non-carriers.

*Substitution of a C for G at nucleotide -57 [G(-57)C].* This variant is in complete linkage disequilibrium with the variant at -4 in the individuals we have sequenced, and thus the phenotypic effects currently attributed to the variant at -4 may at least in part be due to changes at this site.

*Substitution of A for G at nucleotide 730 (R219K).* We have found carriers to have significantly less cardiovascular disease.

*Substitution of C for T at nucleotide 1270 (V399A).* Within the French Canadian population, this variant has only been found in individuals from the low HDL population. It has also been seen in individuals with low HDL or premature coronary artery disease in individuals of Dutch ancestry.

*Substitution of A for G at nucleotide 2385 (V771M).* This variant has been found at an increased frequency in a Dutch population selected for low HDL and at an increased frequency in a population selected for premature coronary artery disease compared to a control Dutch population, indicating carriers of this variant may have reduced HDL and an increased susceptibility to coronary artery disease.

*Substitution of C for A at nucleotide 2394 (T774P).* This variant has been seen at lower frequencies in populations with coronary artery disease or low HDL than in individuals without.

*Substitution of C for G at nucleotide 2402 (K776N).* This variant has been found at a significantly lower frequency (0.56% vs. 2.91%, p=0.02) in a coronary artery disease population vs. a control population of similar Dutch background.

*Substitution of C for G at nucleotide 3590 (E1172D).* This variant is seen at lower frequencies in individuals with low HDL and in some populations with premature coronary artery disease.

*Substitution of A for G at nucleotide 4384 (R1587K).* This variant has been found at decreased frequencies in the 1/3 of individuals with the highest HDL levels in our large Dutch coronary artery disease population (p=0.036), at increased frequencies in those with HDL cholesterol <0.9 mmol/L (p<0.0001) and at decreased frequencies in the cohorts with HDL cholesterol >1.4 mmol/L in both this population (p=0.02) and the Dutch control population (p=0.003).

*Substitution of G for C at nucleotide 5266 (S1731C).* Two FHA individuals who have this variant on the other allele have much lower HDL cholesterol (0.155±0.025) than the FHA individuals in the family who do not have this variant on the other allele (0.64±0.14, p=0.0009). This variant has also been found in one general population French Canadian control with HDL at the 8th percentile (0.92) and one French Canadian individual from a population selected for low HDL and coronary disease (0.72).

*Substitution of G for A at nucleotide* -1113 *[A(-1113)Gj.* This variant has been seen at varying frequencies in populations distinguished by their HDL levels.

Additional polymorphisms that may be associated with altered risk for cardiovascular disease or altered cholesterol levels are as follows:
*Substitution of G for A at nucleotide* 2723 *(1883M).* This variant has been seen at a much higher frequency in individuals of Dutch ancestry with premature coronary artery disease.
*Insertion of 4 nucleotides (CCCT) at position -1181.*
*Substitution of C for A at nucleotide* -479 *(linkage disequilibrium with -518).*
*Substitution of G for A at nucleotide -380.*

### Preferred Embodiments

In a first embodiment the present invention relates to a substantially pure ABC 1 polypeptide having ABC 1 biological activity.

In a preferred embodiment of the substantially pure ABC1 polypeptide of the present invention said ABC 1 polypeptide is human ABC 1.

In a preferred embodiment of the substantially pure ABC 1 polypeptide of the present invention said polypeptide comprises amino acids 1 to 60 of SEQ ID NO: 1.

In a preferred embodiment of the substantially pure ABC 1 polypeptide of the present invention said polypeptide comprises amino acids 61 to 2261 of SEQ ID NO: 1.

In a preferred embodiment of the substantially pure ABC 1 polypeptide of the present invention said polypeptide comprises amino acids 1 to 2261 of SEQ ID NO: 1.

In a second embodiment the present invention relates to a substantially pure ABC1 polypeptide comprising amino acids 1 to 60 of SEQ ID NO: 1.

In a third embodiment the present invention relates to a substantially pure ABC 1 polypeptide comprising amino acids 61 to 2261 of SEQ ID NO: 1.

In a fourth embodiment the present invention relates to a substantially pure ABC1 polypeptide comprising amino acids 1 to 2261 of SEQ ID NO: 1.

In a fifth embodiment the present invention relates to a substantially pure nucleic acid molecule that hybridizes at high stringency conditions to nucleotides 75 to 254 of SEQ ID NO: 2 and encodes a polypeptide having ABC1 biological activity.

In a sixth embodiment the present invention relates to a substantially pure nucleic acid molecule encoding an ABC 1 polypeptide having ABC 1 biological activity.

In a preferred embodiment of the substantially pure nucleic acid molecule of the fifth and sixth embodiment said nucleic acid molecule comprises nucleotides 75 to 254 of SEQ ID NO: 2.

In a preferred embodiment of the substantially pure nucleic acid molecule of the fifth and sixth embodiment said nucleic acid molecule comprises nucleotides 255 to 6857 of SEQ ID NO: 2.

In a preferred embodiment of the substantially pure nucleic acid molecule of the fifth and sixth embodiment said nucleic acid molecule comprises nucleotides 75 to 6857 of SEQ ID NO: 2.

In a seventh embodiment the present invention relates to an expression vector comprising the nucleic acid molecule of the fifth embodiment of the present invention.

In an eighth embodiment the present invention relates to a cell expressing the nucleic acid molecule of the fifth embodiment of the present invention.

In a ninth embodiment the present invention relates to a non-human mammal expressing the nucleic acid molecule of the fifth embodiment of the present invention.

In a tenth embodiment the present invention relates to a substantially pure nucleic acid molecule comprising nucleotides 75 to 254 of SEQ ID NO: 2.

In an eleventh embodiment the present invention relates to a substantially pure nucleic acid molecule comprising nucleotides 255 to 6857 of SEQ ID NO: 2.

In a twelfth embodiment the present invention relates to a substantially pure nucleic acid molecule comprising nucleotides 75 to 6857 of SEQ ID NO: 2.

In a thirteenth embodiment the present invention relates to a substantially pure nucleic acid molecule comprising at least thirty consecutive nucleotides corresponding to nucleotides 7015-7860 of SEQ ID NO: 2.

In a fourteenth embodiment the present invention relates to a substantially pure nucleic acid molecule of claim 20, wherein said nucleic acid molecule comprises nucleotides 7015-7860 of SEQ ID NO: 2.

In a fifteenth embodiment the present invention relates to a substantially pure nucleic acid molecule that hybridizes at high stringency to a probe comprising nucleotides 7015-7860 of SEQ ID NO: 2.

In a sixteenth embodiment the present invention relates to a method of treating a human having low HDL cholesterol or cardiovascular disease, said method comprising administering to said human an ABC 1 polypeptide, or cholesterol-regulating fragment thereof.

In a preferred embodiment of the method of the sixteenth embodiment of the present invention said ABC1 polypeptide has the sequence of SEQ ID NO: 1.

In a preferred embodiment of the method of the sixteenth embodiment of the present invention said ABC1 polypeptide comprises a mutation that increases its stability.

In a preferred embodiment of the method of the sixteenth embodiment of the present invention said ABC1 polypeptide comprises a mutation that increases its biological activity.

In a seventeenth embodiment the present invention relates to a method of treating a human having low HDL cholesterol or cardiovascular disease, said method comprising administering to said human a nucleic acid molecule encoding an ABC 1 polypeptide or a cholesterol-regulating fragment thereof.

In a preferred embodiment of the method of the seventeenth embodiment of the present invention said ABC 1 polypeptide has the amino acid sequence of SEQ ID NO: 1.

In a preferred embodiment of the method of the seventeenth embodiment of the present invention said ABC1 polypeptide comprises a mutation that increases its stability.

In a preferred embodiment of the method of the seventeenth embodiment of the present invention said ABC1 polypeptide comprises a mutation that increases its biological activity.

In a preferred embodiment of the method of the seventeenth embodiment of the present invention said biological activity is regulation of cholesterol.

In a preferred embodiment of the method of the seventeenth embodiment of the present invention said human has low HDL cholesterol levels relative to normal.

In an eighteenth embodiment the present invention relates to a method of increasing ABC1 biological activity in a human, said method comprising administering to said human a nucleic acid molecule that hybridizes at high stringency conditions to nucleotides 75 to 254 of SEQ ID NO: 2 and encodes a polypeptide having ABC 1 biological activity.

In a preferred embodiment of the method of the eighteenth embodiment of the present invention said human has a disease selected from the group consisting of Alzheimer's disease, Niemann-Pick disease, Huntington's disease, x-linked adrenoleukodystrophy, and cancer.

In a nineteenth embodiment the present invention relates to a method of increasing ABC 1 biological activity in a human, said method comprising administering to said human a compound that increases ABC 1 biological activity.

In a preferred embodiment of the method of the nineteenth embodiment of the present invention said human has a disease selected from the group consisting of Alzheimer's disease, Niemann-Pick disease, Huntington's disease, x-linked adrenoleukodystrophy, and cancer.

In a twentieth embodiment the present invention relates to a method of preventing cardiovascular disease in a human, said method comprising administering to said human an expression vector comprising an *ABC1* nucleic acid molecule operably linked to a promoter, said *ABC1* nucleic acid molecule encoding an ABC 1 polypeptide having ABC 1 biological activity.

In a twenty-first embodiment the present invention relates to a method of preventing or ameliorating the effects of a disease-causing mutation in an *ABC1* gene in a human, said method comprising introducing into said human an expression vector comprising a promoter operably linked to an *ABC1* nucleic acid molecule encoding an ABC 1 polypeptide having ABC 1 biological activity.

In a twenty-second embodiment the present invention relates to a method of treating or preventing cardiovascular disease in an animal, said method comprising administering to said animal a compound that mimics the activity of wild-type ABC 1.

In a preferred embodiment of the method of the twenty-second embodiment of the present invention said animal is a human.

In a twenty-third embodiment the present invention relates to a method of treating or preventing cardiovascular disease in an animal, said method comprising administering to said animal a compound that modulates the biological activity of ABC 1.

In a preferred embodiment of the method of the twenty-third embodiment of the present invention said animal is a human.

In a preferred embodiment of the method of the twenty-third embodiment of the present invention said compound is selected from a group consisting of protein kinase A, protein kinase C, vanadate, okadaic acid, IBMX1, fibrates, β-estradiol, arachidonic acid derivatives, WY-14,643, LTB4, 8(s)HETE, thiozolidinedione antidiabetic drugs, 9-HODE, 13-HODE, nicotinic acid, HMG CoA reductase inhibitors, and compounds that increase PPAR-mediated ABC1 expression.

In a preferred embodiment of the method of the sixteenth, seventeenth, twenty-second or twenty-third embodiment of the present invention said cardiovascular disease is coronary artery disease, cerebrovascular disease, coronary restenosis, or peripheral vascular disease.

In a twenty-fourth embodiment the present invention relates to a method for determining whether a candidate compound is useful for modulating cholesterol levels, said method comprising the steps of:
(a) providing a chicken comprising a mutation in an *ABC1* gene;
(b) administering said candidate compound to said chicken; and
(c) measuring ABC 1 biological activity in said chicken,
wherein altered ABC 1 biological activity, relative to a WHAM chicken not contacted with said compound, indicates that said candidate compound modulates cholesterol levels.

In a preferred embodiment of the method of the twenty-fourth embodiment of the present invention said ABC1 biological activity is transport of cholesterol.

In a twenty-fifth embodiment the present invention relates to a method for determining whether a candidate compound modulates ABC biological activity, said method comprising the steps of:
(a) providing a cell expressing an ABC polypeptide comprising amino acids 1 to 60 of SEQ ID NO: 1;
(b) contacting said cell with said candidate compound; and
(c) measuring ABC 1 biological activity of said cell,
wherein altered ABC 1 biological activity, relative to a cell not contacted with said compound, indicates that said candidate compound modulates ABC1 biological activity.

In a twenty-sixth embodiment the present invention relates to a method for determining whether a candidate compound modulates ABC 1 expression, said method comprising the steps of:
(a) providing a cell expressing an *ABC1* gene comprising nucleotides 75 to 254 of SEQ ID NO: 2;
(b) contacting said cell with said candidate compound; and
(c) measuring ABC 1 expression of said cell,
wherein altered ABC1 expression, relative to a cell not contacted with said compound, indicates that said candidate compound modulates ABC1 expression.

In a twenty-seventh embodiment the present invention relates to a method for determining whether a candidate compound modulates ABC 1 expression, said method comprising the steps of:
(a) providing a nucleic acid molecule comprising an ABC 1 promoter operably linked to a reporter gene;
(b) contacting said nucleic acid molecule with said candidate compound; and
(c) measuring expression of said reporter gene,
wherein altered reporter gene expression, relative to a control not contacted with said compound, indicates that said candidate compound modulates ABC 1 expression.

In a preferred embodiment of the method of the twenty-seventh embodiment of the present invention said promoter comprises 50 consecutive nucleotides selected from nucleotides 1 to 8238 of SEQ ID NO: 14.

In a preferred embodiment of the method of the twenty-seventh embodiment of the present invention said promoter comprises a binding site for a transcription factor selected from a group consisting of steroid response element binding proteins, peroxisomal proliferation-activated receptors, retinoid X receptors, and RAR-related orphan receptors.

In a twenty-eighth embodiment the present invention relates to a method for determining whether a candidate compound modulates ABC1 biological activity, said method comprising the steps of:
(a) providing an ABC 1 polypeptide comprising amino acids 1 to 60 of SEQ ID NO: 1;
(b) contacting said polypeptide with said candidate compound; and
(c) measuring ABC 1 biological activity,
wherein a change in ABC1 biological activity, relative to a control not contacted with said compound, indicates that said candidate compound modulates ABC1 biological activity.

In a twenty-ninth embodiment the present invention relates to a method for determining whether a candidate compound modulates ABC1 expression, said method comprising the steps of:
(a) providing an ABC1 polypeptide comprising amino acids 1 to 60 of SEQ ID NO: 1;
(b) contacting said polypeptide with said candidate compound; and
(c) measuring expression of said ABC1 polypeptide,
wherein a change in expression of said ABC1 polypeptide, relative to a control not contacted with said compound, indicates that said candidate compound modulates ABC 1 expression.

In a thirtieth embodiment the present invention relates to a method for determining whether candidate compound modulates ABC1 biological activity, said method comprising the steps of:
(a) providing an ABC1 polypeptide comprising amino acids 1 to 60 of SEQ ID NO:1;
(b) contacting said polypeptide with said candidate compound; and
(c) measuring binding of said ABC1 polypeptide to said candidate compound,
wherein binding of said ABC1 polypeptide to said compound indicates that said candidate compound modulates ABC 1 biological activity.

In a thirty-first embodiment the present invention relates to a method for determining whether candidate compound modulates ABC 1 biological activity, said method comprising the steps of:
(a) providing (i) an ABC 1 polypeptide comprising amino acids 1 to 60 of SEQ ID NO: 1, and (ii) a second polypeptide that interacts with said ABC1 polypeptide ;
(b) contacting said polypeptides with said candidate compound; and
(c) measuring interaction of said ABC1 polypeptide with said second polypeptide,
wherein an alteration in the interaction of said ABC1 polypeptide with said second polypeptide indicates that said candidate compound modulates ABC1 biological activity.

In a thirty-second embodiment the present invention relates to a method for determining whether a candidate compound increases the stability or decreases the regulated catabolism of an ABC1 polypeptide, said method comprising the steps of:
(a) providing a cell comprising an ABC 1 polypeptide comprising amino acids 1 to 60 of SEQ ID NO: 1;
(b) contacting said cell with said candidate compound; and
(c) measuring the half-life of said ABC 1 polypeptide,
wherein an increase in said half-life, relative to a control not contacted with said compound, indicates that said candidate compound increases the stability or decreases the regulated catabolism of an ABC 1 polypeptide.

In a thirty-third embodiment the present invention relates to a method for determining whether a candidate compound modulates ABC1 biological activity, said method comprising the steps of:
(a) providing an ABC 1 polypeptide in a lipid membrane;
(b) contacting said polypeptide with said candidate compound; and
(c) measuring ABC1-mediated lipid transport across said lipid membrane,
wherein a change in lipid transport, relative to a control not contacted with said compound, indicates that said candidate compound modulates ABC 1 biological activity.

In a preferred embodiment of the method of the twenty-seventh, twenty-eighth, twenty-ninth, thirtieth, thirty-first or thirty-third embodiment of the present invention said ABC 1 polypeptide is in a cell-free system.

In a preferred embodiment of the method of the twenty-seventh, twenty-eighth, twenty-ninth, thirtieth, thirty-first or thirty-third embodiment of the present invention said ABC 1 polypeptide is in a cell.

In a preferred embodiment of the method of the twenty-seventh, twenty-eighth, twenty-ninth, thirtieth, thirty-first or thirty-third embodiment of the present invention said cell is from a WHAM chicken.

In a preferred embodiment of the method of the twenty-seventh, twenty-eighth, twenty-ninth, thirtieth, thirty-first or thirty-third embodiment of the present invention said cell is in a human or in a non-human mammal.

In a preferred embodiment of the method of the twenty-seventh, twenty-eighth, twenty-ninth, thirtieth, thirty-first or thirty-third embodiment of the present invention said animal is a WHAM chicken.

In a preferred embodiment of the method of the twenty-eighth embodiment of the present invention said biological activity is transport of lipid or interleukin-1.

In a preferred embodiment of the method of the twenty-seventh, twenty-eighth, twenty-ninth, thirtieth, thirty-first or thirty-third embodiment of the present invention said lipid is cholesterol.

In a preferred embodiment of the method of the twenty-seventh, twenty-eighth, twenty-ninth, thirtieth, thirty-first or thirty-third embodiment of the present invention said cholesterol is HDL-cholesterol.

In a preferred embodiment of the method of the twenty-eighth embodiment of the present invention said biological activity is binding or hydrolysis of ATP by the ABC 1 polypeptide.

In a thirty-fourth embodiment the present invention relates to a method for determining whether a patient has an increased risk for cardiovascular disease, said method comprising determining whether an *ABC1* gene of said patient has a mutation, wherein a mutation indicates that said patient has an increased risk for cardiovascular disease.

In a thirty-fifth embodiment the present invention relates to a method for determining whether a patient has an increased risk for cardiovascular disease, said method comprising measuring ABC 1 biological activity in said patient or in a cell from said patient, wherein increased or decreased levels in said ABC 1 biological activity, relative to normal levels, indicates that said patient has an increased risk for cardiovascular disease.

In a thirty-sixth embodiment the present invention relates to a method for determining whether a patient has an increased risk for cardiovascular disease, said method comprising measuring ABC1 expression in said patient or in a cell from said patient, wherein decreased levels in said ABC1 expression relative to normal levels, indicates that said patient has an increased risk for cardiovascular disease.

In a preferred embodiment of the method of the thirty-sixth embodiment of the present invention said ABC 1 expression is determined by measuring levels of ABC 1 polypeptide.

In a preferred embodiment of the method of the thirty-sixth embodiment of the present invention said ABC1 expression is determined by measuring levels of *ABC1* RNA.

In a thirty-seventh embodiment the present invention relates to a non-human mammal comprising a transgene comprising a nucleic acid molecule encoding a dominant-negative ABC 1 polypeptide.

In a thirty-eighth embodiment the present invention relates to a cell isolated from a non-human mammal comprising a transgene comprising a nucleic acid molecule encoding an ABC 1 polypeptide having biological activity.

In a thirty-ninth embodiment the present invention relates to a method for determining whether a candidate compound decreases the inhibition of a dominant-negative ABC 1 polypeptide, said method comprising the steps of:
(a) providing a cell expressing a dominant-negative ABC1 polypeptide;
(b) contacting said cell with said candidate compound; and
(c) measuring ABC 1 biological activity of said cell,
wherein an increase in said ABC1 biological activity, relative to a cell not contacted with said compound, indicates that said candidate compound decreases the inhibition of a dominant-negative ABC 1 polypeptide.

In a fortieth embodiment the present invention relates to a method for determining whether a person has an altered risk for developing cardiovascular disease, comprising examining the person's ABC 1 gene for polymorphisms,
wherein the presence of a polymorphism associated with cardiovascular disease indicates the person has an altered risk for developing cardiovascular disease.

In a forty-first embodiment the present invention relates to a method for predicting a person's response to a drug, comprising determining whether the person has a polymorphism in an ABC1 gene that alters the person's response to said drug.

In a forty-second embodiment the present invention relates to a method for predicting a person's response to a drug, comprising determining whether the person has a polymorphism in an ABC1 promoter that alters the person's response to said drug.

In a forty-third embodiment the present invention relates to a method for altering ABC1 expression in a cell, said method comprising contacting said cell with a compound selected from a group consisting of fibrates, β-estradiol, arachidonic acid derivatives, WY-14,643, LTB4, 8(s)HETE, thiozolidinedione antidiabetic drugs, 9-HODE, 13-HODE, nicotinic acid, HMG CoA reductase inhibitors, and compounds that increase PPAR-mediated ABC 1 expression.

In a forty-fourth embodiment the present invention relates to a pharmaceutical composition comprising (i) a nucleic acid molecule that hybridizes under high stringency conditions to nucleotides 75 to 254 of SEQ ID NO: 2 and encodes a polypeptide having ABC1 biological activity; and (ii) a pharmaceutically acceptable carrier.

In a forty-fifth embodiment the present invention relates to a nucleic acid that hybridizes under high stringency conditions to nucleotides 1 to 8236 of SEQ ID NO: 14.

In a forty-sixth embodiment the present invention relates to a nucleic acid comprising a region that is 80% identical to at least thirty contiguous nucleotides of nucleotides 1 to 8236 of SEQ ID NO: 14.

In a forty-seventh embodiment the present invention relates to a method for determining whether candidate compound modulates ABC 1 biological activity, said method comprising the steps of:
(a) providing an ABC 1 polypeptide;
(b) contacting said polypeptide with cholesterol and said candidate compound; and
(c) measuring binding of said cholesterol to said ABC1 polypeptide, wherein binding of said cholesterol to said ABC 1 polypeptide indicates that said candidate compound modulates ABC 1 biological activity.

In a preferred embodiment of the method of the forty-seventh embodiment of the present invention said cholesterol is HDL cholesterol.

In a preferred embodiment of the method of the forty-seventh embodiment of the present invention said method is performed in a cell free assay.

In a preferred embodiment of the method of the forty-seventh embodiment of the present invention said ABC 1 polypeptide comprises amino acids 1 to 60 of SEQ ID NO: 1.

In a preferred embodiment of the method of the forty-seventh embodiment of the present invention said cholesterol or said ABC 1 polypeptide is detectably labeled.

### Other Embodiments

All publications mentioned in this specification are herein incorporated by reference to the same extent as if each independent publication was specifically and individually indicated to be incorporated by reference.

While the invention has been described in connection with specific embodiments thereof, it will be understood that it is capable of further modifications. This application is intended to cover any variations, uses, or adaptations following, in general, the principles of the invention and including such departures from the present disclosure within known or customary practice within the art to which the invention pertains and may be applied to the essential features hereinbefore set forth.

## Claims

1. A substantially pure ABC 1 polypeptide having ABC 1 biological activity.

2. The substantially pure ABC1 polypeptide of claim 1, wherein said ABC 1 polypeptide is human ABC 1.

3. The substantially pure ABC1 polypeptide of claim 1, wherein said polypeptide comprises amino acids 1 to 60 of SEQ ID NO: 1.

4. The substantially pure ABC1 polypeptide of claim 1, wherein said polypeptide comprises amino acids 61 to 2261 of SEQ ID NO: 1.

5. The substantially pure ABC1 polypeptide of claim 1, wherein said polypeptide comprises amino acids 1 to 2261 of SEQ ID NO: 1.

6. A substantially pure ABC 1 polypeptide comprising amino acids 1 to 60 of SEQ ID NO: 1.

7. A substantially pure ABC 1 polypeptide comprising amino acids 61 to 2261 of SEQ ID NO: 1.

8. A substantially pure ABC 1 polypeptide comprising amino acids 1 to 2261 of SEQ ID NO: 1.

9. A substantially pure nucleic acid molecule that hybridizes at high stringency conditions to nucleotides 75 to 254 of SEQ ID NO: 2 and encodes a polypeptide having ABC1 biological activity.

10. A substantially pure nucleic acid molecule encoding an ABC1 polypeptide having ABC 1 biological activity.

11. The substantially pure nucleic acid molecule of claim 9 or 10, wherein said nucleic acid molecule comprises nucleotides 75 to 254 of SEQ ID NO: 2.

12. The substantially pure nucleic acid molecule of claim 9 or 10, wherein said nucleic acid molecule comprises nucleotides 255 to 6857 of SEQ ID NO: 2.

13. The substantially pure nucleic acid molecule of claim 9 or 10, wherein said nucleic acid molecule comprises nucleotides 75 to 6857 of SEQ ID NO: 2.

14. An expression vector comprising the nucleic acid molecule of claim 9.

15. A cell expressing the nucleic acid molecule of claim 9.

16. A non-human mammal expressing the nucleic acid molecule of claim 9.

17. A substantially pure nucleic acid molecule comprising nucleotides 75 to 254 of SEQ ID NO: 2.

18. A substantially pure nucleic acid molecule comprising nucleotides 255 to 6857 of SEQ ID NO: 2.

19. A substantially pure nucleic acid molecule comprising nucleotides 75 to 6857 of SEQ ID NO: 2.

20. A substantially pure nucleic acid molecule comprising at least thirty consecutive nucleotides corresponding to nucleotides 7015-7860 of SEQ ID NO: 2.

21. The substantially pure nucleic acid molecule of claim 20, wherein said nucleic acid molecule comprises nucleotides 7015-7860 of SEQ ID NO: 2.

22. A substantially pure nucleic acid molecule that hybridizes at high stringency to a probe comprising nucleotides 7015-7860 of SEQ ID NO: 2.

23. A nucleic acid that hybridizes under high stringency conditions to nucleotides 1 to 8236 of SEQ ID NO: 14.

24. A nucleic acid comprising a region that is 80% identical to at least thirty contiguous nucleotides of nucleotides 1 to 8236 of SEQ ID NO: 14.

25. A method of treating a human having low HDL cholesterol or cardiovascular disease, said method comprising administering to said human an ABC 1 polypeptide, or cholesterol-regulating fragment thereof.

26. A method of treating a human having low HDL cholesterol or cardiovascular disease, said method comprising administering to said human a nucleic acid molecule encoding an ABC1 polypeptide or a cholesterol-regulating fragment thereof.

27. A method of increasing ABC 1 biological activity in a human, said method comprising administering to said human a nucleic acid molecule that hybridizes at high stringency conditions to nucleotides 75 to 254 of SEQ ID NO: 2 and encodes a polypeptide having ABC 1 biological activity.

28. A method of increasing ABC 1 biological activity in a human, said method comprising administering to said human a compound that increases ABC 1 biological activity.

29. A method of preventing cardiovascular disease in a human, said method comprising administering to said human an expression vector comprising an *ABC1* nucleic acid molecule operably linked to a promoter, said *ABC1* nucleic acid molecule encoding an ABC1 polypeptide having ABC1 biological activity.

30. A method of preventing or ameliorating the effects of a disease-causing mutation in an *ABC1* gene in a human, said method comprising introducing into said human an expression vector comprising a promoter operably linked to an *ABC1* nucleic acid molecule encoding an ABC1 polypeptide having ABC 1 biological activity.

31. A method of treating or preventing cardiovascular disease in an animal, said method comprising administering to said animal a compound that mimics the activity of wild-type ABC 1.

32. A method of treating or preventing cardiovascular disease in an animal, said method comprising administering to said animal a compound that modulates the biological activity of ABC 1.

33. A method for determining whether a candidate compound is useful for modulating cholesterol levels, said method comprising the steps of:
(a) providing a chicken comprising a mutation in an *ABC1* gene;
(b) administering said candidate compound to said chicken; and
(c) measuring ABC 1 biological activity in said chicken,
wherein altered ABC biological activity, relative to a WHAM chicken not contacted with said compound, indicates that said candidate compound modulates cholesterol levels.

34. A method for determining whether a candidate compound modulates ABC1 biological activity, said method comprising the steps of:
(a) providing a cell expressing an ABC1 polypeptide comprising amino acids 1 to 60 of SEQ ID NO: 1;
(b) contacting said cell with said candidate compound; and
(c) measuring ABC 1 biological activity of said cell,
wherein altered ABC1 biological activity, relative to a cell not contacted with said compound, indicates that said candidate compound modulates ABC1 biological activity.

35. A method for determining whether a candidate compound modulates ABC 1 expression, said method comprising the steps of:
(a) providing a cell expressing an *ABC1* gene comprising nucleotides 75 to 254 of SEQ ID NO: 2;
(b) contacting said cell with said candidate compound; and
(c) measuring ABC 1 expression of said cell,
wherein altered ABC1 expression, relative to a cell not contacted with said compound, indicates that said candidate compound modulates ABC1 expression.

36. A method for determining whether a candidate compound modulates ABC 1 expression, said method comprising the steps of:
(a) providing a nucleic acid molecule comprising an ABC1 promoter operably linked to a reporter gene;
(b) contacting said nucleic acid molecule with said candidate compound; and
(c) measuring expression of said reporter gene,
wherein altered reporter gene expression, relative to a control not contacted with said compound, indicates that said candidate compound modulates ABC 1 expression.

37. A method for determining whether a candidate compound modulates ABC 1 biological activity, said method comprising the steps of:
(a) providing an ABC 1 polypeptide comprising amino acids 1 to 60 of SEQ ID NO: 1;
(b) contacting said polypeptide with said candidate compound; and
(c) measuring ABC1 biological activity,
wherein a change in ABC1 biological activity, relative to a control not contacted with said compound, indicates that said candidate compound modulates ABC 1 biological activity.

38. A method for determining whether a candidate compound modulates ABC 1 expression, said method comprising the steps of:
(a) providing an ABC1 polypeptide comprising amino acids 1 to 60 of SEQ ID NO: 1;
(b) contacting said polypeptide with said candidate compound; and
(c) measuring expression of said ABC1 polypeptide,
wherein a change in expression of said ABC1 polypeptide, relative to a control not contacted with said compound, indicates that said candidate compound modulates ABC1 expression.

39. A method for determining whether candidate compound modulates ABC1 biological activity, said method comprising the steps of:
(a) providing an ABC 1 polypeptide comprising amino acids 1 to 60 of SEQ ID NO: 1;
(b) contacting said polypeptide with said candidate compound; and
(c) measuring binding of said ABC 1 polypeptide to said candidate compound,
wherein binding of said ABC 1 polypeptide to said compound indicates that said candidate compound modulates ABC 1 biological activity.

40. A method for determining whether candidate compound modulates ABC1 biological activity, said method comprising the steps of:
(a) providing (i) an ABC 1 polypeptide comprising amino acids 1 to 60 of SEQ ID NO: 1, and (ii) a second polypeptide that interacts with said ABC1 polypeptide;
(b) contacting said polypeptides with said candidate compound; and
(c) measuring interaction of said ABC1 polypeptide with said second polypeptide, wherein an alteration in the interaction of said ABC1 polypeptide with said second polypeptide indicates that said candidate compound modulates ABC 1 biological activity.

41. A method for determining whether a candidate compound increases the stability or decreases the regulated catabolism of an ABC1 polypeptide, said method comprising the steps of:
(a) providing a cell comprising an ABC1 polypeptide comprising amino acids 1 to 60 of SEQ ID NO: 1;
(b) contacting said cell with said candidate compound; and
(c) measuring the half-life of said ABC 1 polypeptide,
wherein an increase in said half-life, relative to a control not contacted with said compound, indicates that said candidate compound increases the stability or decreases the regulated catabolism of an ABC 1 polypeptide.

42. A method for determining whether a candidate compound modulates ABC 1 biological activity, said method comprising the steps of:
(a) providing an ABC 1 polypeptide in a lipid membrane;
(b) contacting said polypeptide with said candidate compound; and
(c) measuring ABC1-mediated lipid transport across said lipid membrane,
wherein a change in lipid transport, relative to a control not contacted with said compound, indicates that said candidate compound modulates ABC1 biological activity.

43. A method for determining whether a patient has an increased risk for cardiovascular disease, said method comprising determining whether an *ABC1* gene of said patient has a mutation, wherein a mutation indicates that said patient has an increased risk for cardiovascular disease.

44. A method for determining whether a patient has an increased risk for cardiovascular disease, said method comprising measuring ABC1 biological activity in said patient or in a cell from said patient, wherein increased or decreased levels in said ABC1 biological activity, relative to normal levels, indicates that said patient has an increased risk for cardiovascular disease.

45. A method for determining whether a patient has an increased risk for cardiovascular disease, said method comprising measuring ABC 1 expression in said patient or in a cell from said patient, wherein decreased levels in said ABC 1 expression relative to normal levels, indicates that said patient has an increased risk for cardiovascular disease.

46. A non-human mammal comprising a transgene comprising a nucleic acid molecule encoding a dominant-negative ABC 1 polypeptide.

47. A cell isolated from a non-human mammal comprising a transgene comprising a nucleic acid molecule encoding an ABC 1 polypeptide having biological activity.

48. A method for determining whether a candidate compound decreases the inhibition of a dominant-negative ABC 1 polypeptide, said method comprising the steps of:
(a) providing a cell expressing a dominant-negative ABC 1 polypeptide;
(b) contacting said cell with said candidate compound; and
(c) measuring ABC 1 biological activity of said cell,
wherein an increase in said ABC 1 biological activity, relative to a cell not contacted with said compound, indicates that said candidate compound decreases the inhibition of a dominant-negative ABC1 polypeptide.

49. A method for determining whether a person has an altered risk for developing cardiovascular disease, comprising examining the person's ABC 1 gene for polymorphisms, wherein the presence of a polymorphism associated with cardiovascular disease indicates the person has an altered risk for developing cardiovascular disease.

50. A method for predicting a person's response to a drug, comprising determining whether the person has a polymorphism in an ABC1 gene that alters the person's response to said drug.

51. A method for predicting a person's response to a drug, comprising determining whether the person has a polymorphism in an ABC 1 promoter that alters the person's response to said drug.

52. A method for altering ABC 1 expression in a cell, said method comprising contacting said cell with a compound selected from a group consisting of fibrates, β-estradiol arachidonic acid derivatives, WY-14,643, LTB4, 8(s)HETE, thiozolidinedione antidiabetic drugs, 9-HODE, 13-HODE, nicotinic acid, HMG CoA reductase inhibitors, and compounds that increase PPAR-mediated ABC 1 expression.

53. A pharmaceutical composition comprising (i) a nucleic acid molecule that hybridizes under high stringency conditions to nucleotides 75 to 254 of SEQ ID NO: 2 and encodes a polypeptide having ABC 1 biological activity; and (ii) a pharmaceutically acceptable carrier.

54. A method for determining whether candidate compound modulates ABC 1 biological activity, said method comprising the steps of:
(a) providing an ABC 1 polypeptide;
(b) contacting said polypeptide with cholesterol and said candidate compound; and
(c) measuring binding of said cholesterol to said ABC1 polypeptide,
wherein binding of said cholesterol to said ABC 1 polypeptide indicates that said candidate compound modulates ABC 1 biological activity.
